# EUROPEAN PATENT APPLICATION

(11) **EP 4 286 376 A1**
(43) Date of publication of application: **06.12.2023**
(21) Application number: 22781692.3
(22) Date of filing: 01.04.2022
(51) Int. Cl.: C07D 401/12, A61K 31/506, A61K 31/5377, A61P 35/00, C07D 401/14, C07D 405/14

(54) **PYRIMIDINE DERIVATIVE HAVING PROTEIN KINASE INHIBITORY ACTIVITY, AND THERAPEUTIC PHARMACEUTICAL COMPOSITION COMPRISING SAME**

(30) Priority: 01.04.2021 KR 20210042821; 30.03.2022 KR 20220039968
(71) Applicant: Therapex Co., Ltd., Seoul 05835 (KR); Industry-Academic Cooperation Foundation, Yonsei University, Seoul 03722 (KR)
(72) Inventor: SIM, Tae Bo, Seoul 06635 (KR); CHO, Byoung Chul, Seoul 04425 (KR); CHO, Han Na, Seoul 02583 (KR); SENGUPTA, Sandip, Seoul 02793 (KR); MALLA REDDY, Gannarapu, Seoul 03727 (KR); KIM, Young Hoon, Seoul 03724 (KR)
(74) Representative: Prüfer & Partner mbB Patentanwälte · Rechtsanwälte
(86) International application number: PCT/KR2022/004702
(87) International publication number: WO 2022/211573

(57) **Abstract**

The present invention relates to a pharmaceutical composition for preventing and treating a cancer disease, including lung cancer, caused by abnormal cell growth containing a novel trisubstituted pyrimidine derivative with inhibitory activity against protein kinases, including EGFR, and a pharmaceutically acceptable salt thereof as active ingredients. The novel compound of the present invention has strong inhibitory activity against protein kinases. Particularly, the novel compound of the present invention has potent inhibitory effects against various drug-resistant EGFR mutants (including exon 19 deletion-T790M-C797S-EGFR, L858R-T790M-C797S-EGFR, exon 19 deletion-T790M-EGFR, and L858R-T790M-EGFR) and is useful as a prophylactic and therapeutic agent for cancer diseases caused by these EGFR mutants.

## Description

### Technical Field

The present invention relates to a compound selected from a novel trisubstituted pyrimidine derivative with inhibitory activity against protein kinases, a pharmaceutically acceptable salt thereof, a hydrate thereof, and a stereoisomer thereof, a method for preparing the compound, and a pharmaceutical composition for preventing, ameliorating or treating a cancer disease caused by abnormal cell growth including the compound as an active ingredient.

### Background Art

Protein kinases are enzymes that catalyze phosphorylation reactions, where the gamma-phosphate groups of ATP are transferred to the hydroxyl groups of tyrosine, serine, and threonine in proteins. Protein kinases are responsible for cell metabolism, gene expression, cell growth, differentiation, and cell division and play an important role in signal transduction. Protein kinases account for nearly 2% of the eukaryotic genome. The human genome contains approximately 518 protein kinases. Protein kinases are classified into tyrosine-phosphorylating tyrosine protein kinases and serine- and threonine-phosphorylating serine/threonine kinases. At least about 90 protein kinases are tyrosine kinases, which are divided into receptor tyrosine kinases (RTKs) and non-receptor tyrosine kinases (NRTKs). Receptor tyrosine kinases are membrane proteins that have domains capable of accommodating growth factors on the cell surface and active sites where tyrosine residues in the cytoplasm are phosphorylated. Protein kinases are molecular switches and the transition between their active and inactive states in cells should be regulated smoothly. Abnormal regulation of the transition leads to excessive activation of intracellular signal transduction to induce uncontrolled cell division and proliferation. Further, abnormal activation of protein kinases by genetic variation, amplification, and overexpression plays a crucial role in the growth and metastasis of cancer cells due to its association with the development and progression of various tumors.

Lung cancer is a malignant tumor in the lung as a respiratory organ. Lung cancer is divided into non-small cell lung cancer and small cell lung cancer depending on the size and shape of the cancer cells. Non-small cell lung cancer accounts for 80% of all lung cancers. Non-small cell lung cancer can be entirely cured by surgical treatment when detected early but 20 to 50% of patients with non-small cell lung cancer who had undergone surgery showed recurrence. Non-small cell lung cancer is more likely to metastasize to the brain, bone, liver, and the opposite lung than other cancers.

The most common causes of non-small cell lung cancer are mutations in the epidermal growth factor receptor (EGFR) tyrosine kinase gene. 30-40% of Asian non-small cell lung cancer patients, 10-15% of U.S. and European non-small cell lung cancer patients, and 40-50% of Asian female non-smoker non-small cell lung cancer patients have EGFR mutations. Mutations in the EGFR gene cause lung cancer regardless of smoking or non-smoking. 90% of all EGFR mutations are activating mutations (exon 19 deletion mutations (43%) and L858R point mutations (41%)) where EGFR can be activated even without epidermal growth factor.

EGFR is a transmembrane tyrosine kinase receptor that forms a dimer with a ligand such as epidermal growth factor and is activated upon phosphorylation of tyrosine residues. Activated EGFR creates conditions for easy binding to downstream molecules and phosphorylates them while activating downstream pathways (for example, RAS-mitogen-activated protein kinase (MAPK), phosphotidylinositol 3-kinase (PI3-K)-Akt, phospholipase-Cγ (PLCy)/protein kinase-C (PKC), and signal transducer and activator of transcription (STAT)) to inhibit apoptosis, promote cell growth and proliferation, and regulate gene expression. The occurrence of activating mutations such as exon 19 deletion mutations or L858R point mutations causes infinite cell proliferation and forms cancer because the activated structures of the mutations are always maintained even without ligands.

Gefitinib (Iressa^{®}, AstraZeneca) and erlotinib (Tarceva^{®}, Roche) are first-generation EGFR tyrosine kinase inhibitors that can be administered orally. These first-generation inhibitors extend the survival of patients by 10 months compared to existing cytotoxic anticancer drugs as standard therapy, demonstrating their superiority. However, T790M mutation occurs in 60% of patients due to resistance to the first-generation inhibitors. Afatinib (Gilotrif^{®}, Boehringer Ingelheim) has emerged as a second-generation EGFR tyrosine kinase inhibitor that can inhibit T790M mutation. Although Afatinib has proved to be clinically effective, it failed to ensure a therapeutic window. Second-generation inhibitors with relatively narrow indications have been approved only as first-line therapeutic agents for locally advanced or metastatic non-small cell lung cancer with EGFR mutations. Osimertinib (Tagrisso^{®}, AstraZeneca) is a third-generation EGFR tyrosine kinase inhibitor in which a Michael acceptor capable of covalently binding to cysteine is introduced. Osimertinib was first FDA approved and launched as a third-generation EGFR-targeted anticancer drug. However, osimertinib induced acquired resistance in patients with C797S mutation who had received osimertinib for about 10 months. The C797S mutation refers to a mutation in which the cysteine residue at the binding site of Tagrisso is replaced by serine. The C797S mutation neutralizes the covalent bonds in Tagrisso. Since Tagrisso can be used for T790M mutation occurring after treatment with first- and second-generation EGFR inhibitors, patients who develop resistance to Tagrisso have three mutations, including EGFR activating (exon 19 deletion or L858R), T790M, and C797S mutations. Therefore, fourth-generation EGFR inhibitors should have moderate efficacies against double mutations (exon 19 deletion/T790M or L858R/T790M) and activating mutations (exon 19 deletion or L858R) as well as triple mutations (exon 19 deletion/T790M/C797S or L858R/T790M/C797S).

Third-generation inhibitors bearing Michael acceptors, such as Tagrisso, were discontinued from clinical trials due to their serious side effects (including Stevens-Johnson Syndrome, skin rash, and heart diseases). There is thus a need to develop fourth-generation EGFR inhibitors that have reduced side effects while overcoming resistance to third-generation inhibitors. Such fourth-generation inhibitors should be able to distinguish between EGFR wild type and mutants in order to reduce side effect. Despite their high efficacy, drugs developed hitherto have a narrow window between EGFR wild type and mutants, showing high toxicity.

Lung cancer is a highly fatal cancer, with more than 20,000 new cases diagnosed in South Korea each year. Lung cancer is the first leading cause of death in South Korea. Particularly, lung cancer has a 5-year survival rate as low as 8.9% when it metastasizes to other organs. The number of lung cancer patients in South Korea in 2021 was approximately 30,000, of which 30-40% are EGFR mutation positive. EGFR mutations are also found in several carcinomas such as glioblastoma (50%), triple negative breast cancer (13%-30%), and colorectal cancer (25%-77%) as well as in lung cancer. EGFR inhibitors are being clinically tested for a wide range of carcinomas, including lung cancer and head and neck cancer.

Under these circumstances, the present inventors have conducted research to develop new substances capable of overcoming the limitations of third-generation EGFR inhibitors and finally arrived at the present invention.

### [Prior Art Documents]

### [Patent Documents]

(Patent Document 1) PCT Patent Publication No. WO/2018/230934/A1
(Patent Document 2) U.S. Patent Publication No. 20200207768A1
(Patent Document 3) Korean Patent Publication No. 10-2019-0114910
(Patent Document 4) PCT Patent Publication No. WO/2019/112344/A1
(Patent Document 5) PCT Patent Publication No. WO/2020/147702/A1

### [Non-Patent Documents]

(Non-Patent Document 1) J. Med. Chem. 2018, 61, 4290-4300
(Non-Patent Document 2) J. Med. Chem. 2019, 62, 10272-10293

### Detailed Description of the Invention

### Technical Problems

Accordingly, one object of the present invention is to provide a novel pyrimidine derivative with inhibitory activity against protein kinases.

A further object of the present invention is to provide a pharmaceutical composition useful for the treatment, prevention, and amelioration of a cancer disease, including, as an active ingredient, a novel pyrimidine derivative, a pharmaceutically acceptable salt thereof, a hydrate thereof, a solvate thereof or a stereoisomer thereof.

Another object of the present invention is to provide a therapeutic agent for a cancer disease caused by an EGFR mutation, including, as an active ingredient, a novel pyrimidine derivative, a pharmaceutically acceptable salt thereof, a hydrate thereof, a solvate thereof or a stereoisomer thereof.

One aspect of the present invention is intended to provide a method for preparing the novel compound.

One aspect of the present invention is intended to provide a novel intermediate synthesized during preparation of the novel compound.

### Means for Solving the Problems

One aspect of the present invention provides a compound selected from a pyrimidine derivative represented by Formula 10, 11 or 12: 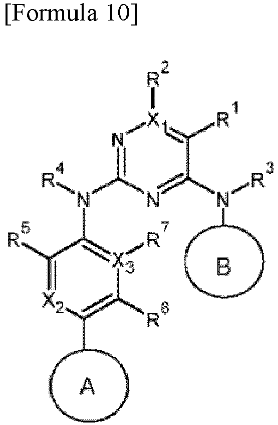
wherein R₁ is hydrogen, hydroxyl, halo, C₁-C₁₃ alkyl or C₁-C₆ alkoxy,
R₂ is hydrogen, halo, C₁-C₁₃ alkyl, C₃-C₁₀ cyclyl, amino (-NR₈R₉) or nitro (-N(O)₂),
R₃ and R₄ are each independently hydrogen, C₁-C₁₃ alkyl, C₃-C₁₀ cyclyl, sulfido (-SR₈), sulfonyl (-S(O)₂R₈) or phosphoryl (-P(O)R₈R₉),
R₅, R₆, and R₇ are each independently hydrogen, hydroxyl, halo, C₁-C₁₃ alkyl, C₁-C₆ alkoxy, C₃-C₁₀ cyclyl, C₃-C₁₀ heterocyclyl, -C(O)-(C₁-C₁₃ alkyl), amino (-NR₈R₉), nitro (-N(O)₂), amido (-(C=O)NR₈R₉), carboxyl (-C(O)OH), nitrile (-CN), ureido (-NR₈(C=O)NR₉-), sulfonamido (-NHS(O)₂R₈), sulfido (-SR₈), sulfonyl (-S(O)₂R₈) or phosphoryl (-P(O)R₈R₉),
X₁, X₂, and X₃ are each independently carbon or nitrogen,
A is C₁-C₁₃ alkyl, amino (-NR₈R₉), C₆-C₁₀ aryl, C₃-C₁₀ cyclyl, C₃-C₁₀ heteroaryl or C₃-C₁₀ heterocyclyl or together with the carbon atom to which R₆ is attached forms a 3- to 7-membered saturated ring optionally containing at least one of N, O, S, NH, C=N, C=O, -NHC(O)-, -NHC(O)NH-, -NHS(O)z-, and SO₂ and optionally substituted with at least one of hydrogen, C₁-C₁₃ alkyl, C₆-C₁₀ aryl, C₃-C₁₀ heteroaryl, hydroxyl, halo, and cyano,
B is C₆-C₁₀ aryl, C₃-C₁₀ cyclyl, C₃-C₁₀ heteroaryl or C₃-C₁₀ heterocyclyl, provided that
when X₁ and X₃ are each independently nitrogen, R₂ or R₇ is nothing,
the C₁-C₆ alkoxy, C₁-C₁₃ alkyl or C₃-C₁₀ cyclyl includes one or more substituents selected from the group consisting of hydrogen, hydroxyl, halo, C₁-C₁₃ alkyl, C₁-C₆ alkoxy, amino (-NR₈R₉), nitro (-N(O)₂), amido (-(C=O)NR₈R₉), carboxyl (-C(O)OH), nitrile (-CN), ureido (-NR₈(C=O)NR₉-), sulfonamido (-NHS(O)z-), sulfido (-S-), sulfonyl (-S(O)₂-), phosphoryl (-P(O)R₈R₉),
C₆-C₁₀ aryl, C₃-C₁₀ heteroaryl, and C₃-C₁₀ heterocyclyl, the C₆-C₁₀ aryl, C₃-C₁₀ heteroaryl or C₃-C₁₀ heterocyclyl includes one or more substituents selected from the group consisting of hydrogen, hydroxyl, halo, carbonyl (-(C=O)R₈R₉), C₁-C₃ alkyl unsubstituted or substituted with halo or C₃-C₁₀ heterocyclyl, C₁-C₃ alkoxy unsubstituted or substituted with halo or C₃-C₁₀ heterocyclyl, C₆-C₁₀ phenoxy, amino (-NR₈R₉), nitro (-N(O)z), amido (-(C=O)NR₈R₉), carboxyl (-C(O)OH), nitrile (-CN), ureido (-NR₈(C=O)NR₉-), sulfonamido (-NHS(O)z-), sulfido (-S-), sulfonyl (-S(O)₂-), phosphoryl (-P(O)R₈R₉), C₆-C₁₀ aryl, C₃-C₁₀ heteroaryl, and C₃-C₁₀ heterocyclyl,
R₈ and R₉ are each independently hydrogen, C₁-C₆ alkyl, C₁-C₆ alkenyl, C₁-C₆ alkynyl, C₆-C₁₀ aryl, C₃-C₁₀ heteroaryl or C₃-C₁₀ heterocyclyl, with the proviso that R₈ together with the nitrogen or carbon atom to which R₉ is attached optionally forms a 3-to 7-membered saturated ring optionally containing at least one of N, O, S, NH, C=N, C=O, -NHC(O)-, -NHC(O)NH-, -NHS(O)z-, and SO₂ and optionally substituted with at least one of hydrogen, C₁-C₁₃ alkyl, C₆-C₁₀ aryl, C₃-C₁₀ heteroaryl, hydroxyl, halo, and cyano, and the C₃-C₁₀ heteroaryl and
C₃-C₁₀ heterocyclyl include at least one heteroatom selected from the group consisting of N, O, and S, 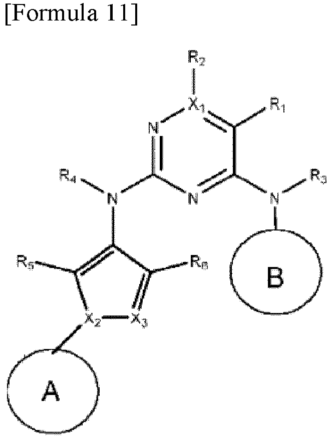
wherein R₁ to R₆, X₁ to X₃, A, and B are as defined in Formula 10, 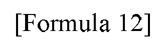 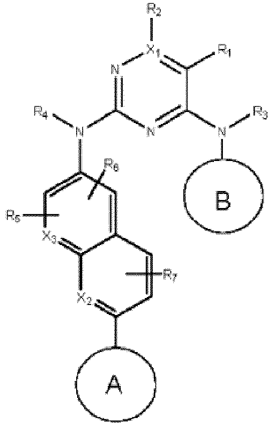
wherein R₁ to R₇, X₁ to X₃, A, and B are as defined in Formula 10; a pharmaceutically acceptable salt thereof; a hydrate thereof; and a stereoisomer thereof.

A further aspect of the present invention provides a pharmaceutical composition for preventing, ameliorating or treating a disease caused by the EGFR protein kinase including the compound as an active ingredient.

In another aspect of the present invention, the disease caused by the EGFR protein kinase may be a metabolic disease caused by the EGFR protein kinase or a neoplastic disease resulting from abnormal cell growth caused by the EGFR protein kinase.

In another aspect of the present invention, the disease may be selected from the group consisting of lung cancer, liver cancer, esophageal cancer, stomach cancer, colorectal cancer, small intestine cancer, pancreatic cancer, melanoma, breast cancer, oral cancer, brain tumor, thyroid cancer, parathyroid cancer, kidney cancer, cervical cancer, sarcoma, prostate cancer, urethral cancer, bladder cancer, testicular cancer, blood cancer, lymphoma, skin cancer, psoriasis, and fibroadenoma.

In another aspect of the present invention, the active ingredient may inhibit the activity of the EGFR kinase or EGFR mutation to prevent, ameliorate or treat the disease.

In another aspect of the present invention, the EGFR mutation may be the activity of del19, L858R, T790M or C797S or a combination thereof.

In another aspect of the present invention, the active ingredient may be co-administered with a cytotoxic therapeutic agent.

In another aspect of the present invention, 100 parts by weight of the active ingredient may be co-administered with 10 to 1000 parts by weight of the cytotoxic therapeutic agent.

### Effects of the Invention

The compound of the present invention can be used as an active ingredient of a pharmaceutical composition for preventing and treating a disease caused by abnormal cell growth and metabolism due to its outstanding ability to inhibit the activity of the EGFR protein kinase.

Therefore, the compound of the present invention can be used as a prophylactic and therapeutic agent for diseases caused by abnormal cell growth and metabolism, for example, metabolic diseases such as diabetes and obesity and a variety of tumor diseases selected from endometrial cancer, bladder cancer, stomach cancer, lung cancer, liver cancer, colorectal cancer, small intestine cancer, pancreatic cancer, brain cancer, bone cancer, melanoma, breast cancer, sclerosing adenosis, head and neck cancer, esophageal cancer, thyroid cancer, parathyroid cancer, kidney cancer, sarcoma, prostate cancer, urethral cancer, blood cancers such as leukemia, multiple myeloma, and myelodysplastic syndrome, lymphomas such as Hodgkin's disease and non-Hodgkin's lymphoma, and fibroadenoma.

### Best Mode for Carrying out the Invention

### Definitions

Unless otherwise specified, all numbers, values, and/or representations expressing components, reaction conditions, and contents of components used herein are taken to mean that these numbers are approximations reflecting various uncertainties of the measurements that essentially occur in obtaining these values among others, and thus should be understood to be modified by the term "about" in all cases. Where a numerical range is disclosed herein, then such a range is continuous, inclusive of both the minimum and maximum values of the range, as well as every value between such minimum and maximum values, unless otherwise indicated. Still further, where a range refers to integers, every integer between the minimum and maximum values of such range is included, unless otherwise indicated.

In the present specification, when a range is described for a variable, it will be understood that the variable includes all values including the end points described within the stated range. For example, the range of "5 to 10" will be understood to include any subranges such as 6 to 10, 7 to 10, 6 to 9, and 7 to 9, as well as individual values of 5, 6, 7, 8, 9 and 10, and will also be understood to include any value between the valid integers within the stated ranges, such as 5.5, 6.5, 7.5, 5.5 to 8.5, and 6.5 to 9. Also, for example, the range of "10% to 30%" will be understood to include any subranges such as 10% to 15%, 12% to 18%, and 20% to 30%, as well as all integers including values of 10%, 11%, 12%, and 13% and up to 30%, and will also be understood to include any value between the valid integers within the stated ranges, such as 10.5%, 15.5%, and 25.5%.

As used herein, the terms "individual(s)", "subject(s)", and "patient(s)" mean any mammal. In some embodiments, the mammal is a human. In some embodiments, the mammal is a non-human mammal. None of the terms require or are limited to situations characterized by the supervision (e.g., constant or intermittent) of a health care worker (e.g., a doctor, a registered nurse, a nurse practitioner, a physician's assistant, an orderly or a hospice worker).

"Treatment" is an intervention performed with the intention of preventing the development or altering the pathology or symptoms of a disorder. Accordingly, "treatment" refers to both therapeutic treatment and prophylactic or preventative measures. Those in need of treatment include those already with the disorder as well as those in which the disorder is to be prevented. In tumor (e.g., cancer) treatment, a therapeutic agent may directly decrease the pathology of tumor cells or render the tumor cells more susceptible to treatment by other therapeutic agents, e.g., radiotherapy and/or chemotherapy and/or immunotherapy. As used herein, the term "amelioration" or "treatment" refers to a symptom which approaches a normalized value (for example, a value obtained in a healthy patient or individual), e.g., is less than 50% different from a normalized value, preferably is less than 25% different from a normalized value, more preferably, is less than 10% different from a normalized value, still more preferably, is not significantly different from a normalized value as determined using routine statistical tests.

"Treatment of cancer" refers to one or more of the following effects: (1) inhibition of tumor growth, including, (i) slowing down and (ii) complete growth arrest; (2) reduction in the number of tumor cells; (3) maintaining tumor size; (4) reduction in tumor size; (5) inhibition, including (i) reduction, (ii) slowing down or (iii) complete prevention, of tumor cell infiltration into peripheral organs; (6) inhibition, including (i) reduction, (ii) slowing down or (iii) complete prevention, of metastasis; and (7) enhancement of anti-tumor immune response, which may result in (i) maintaining tumor size, (ii) reducing tumor size, (iii) slowing the growth of a tumor, (iv) reducing, slowing or preventing invasion.

As used herein, the term "effective amount" or "therapeutically effective amount" refers to a sufficient amount of a compound disclosed herein to relieve to some extent one or more symptoms of a disease or condition being treated. In some embodiments, the result is 1) a reduction and/or alleviation of the signs, symptoms, or causes of a disease, or 2) any other desired alteration of a biological system in a clinical environment. In some embodiments, an appropriate "effective" amount in any individual case is determined using any suitable technique, such as a dose escalation study.

In some embodiments, the "effective amount" is an amount of a compound disclosed that, when administered to an individual in one or more doses, in monotherapy or in combination therapy, is effective to inhibit EGFR by about 20% (20% inhibition), at least about 30% (30% inhibition), at least about 40% (40% inhibition), at least about 50% (50% inhibition), about 60% or more (60% inhibition), about 70% or more (70% inhibition), about 80% or more (80% inhibition) or about 90% or more (90% inhibition), compared to the EGFR activity in the individual in the absence of treatment with the compound or compared to the EGFR activity in the individual before or after treatment with the compound.

In some embodiments, the "therapeutically effective amount" is an amount of a compound disclosed that, when administered to a subject in one or more doses, in monotherapy or in combination therapy, is effective to decrease tumor burden in the subject by about 20%, about 30% or more, about 40% or more, about 50% or more, about 60% or more, about 70% or more, about 80% or more or about 90% or more, compared to the tumor burden in the subject in the absence of treatment with the compound or compared to the tumor burden in the subject before or after treatment with the compound. As used herein, the term "tumor burden" refers to the total mass of tumor tissue carried by a subject with cancer.

In some embodiments, the "therapeutically effective amount" is an amount of a compound disclosed that, when administered to a subject in one or more doses, in monotherapy or in combination therapy, is effective to reduce the dose of radiotherapy required to observe tumor shrinkage in the subject by about 20%, about 30% or more, about 40% or more, about 50% or more, about 60% or more, about 70% or more, about 80% or more or about 90% or more, compared to the dose of radiotherapy required to observe tumor shrinkage in the subject in the absence of treatment with the compound.

### Pharmaceutical Composition

Pharmaceutically acceptable excipients such as vehicles, adjuvants, carriers, and diluents are readily available to those skilled in the art. Pharmaceutically acceptable auxiliary substances such as pH adjusting agents, buffering agents, tonicity adjusting agents, stabilizers, and wetting agents are readily available to those skilled in the art.

In some embodiments, the compound of the present invention may be formulated in an aqueous buffer. Suitable aqueous buffers include, but are not limited to, acetate, succinate, citrate, and phosphate buffers varying in strengths from 5 mM to 1000 mM. In some embodiments, the aqueous buffer includes reagents that provide for an isotonic solution. Such reagents include, but are not limited to, sodium chloride and sugars, e.g., mannitol, dextrose, sucrose, etc. In some embodiments, the aqueous buffer further includes a non-ionic surfactant such as polysorbate 20 or 80. The formulations may optionally further include a preservative. Suitable preservatives include, but are not limited to, benzyl alcohol, phenol, chlorobutanol, benzalkonium chloride etc. In other embodiments, the formulations may be stored at about 4 °C. The formulations may also be freeze-dried, in which case they generally include cryoprotectants such as sucrose, trehalose, lactose, maltose and mannitol. When freeze-dried, the formulations can be stored over extended periods of time, even at ambient temperature.

The present invention provides a pharmaceutical composition including or consisting essentially of a compound disclosed herein, a pharmaceutically acceptable salt, an isomer thereof, a tautomer thereof, and further including or consisting essentially of one or more additional active agents of interest. Any convenient active agents can be utilized in the method of the present invention in conjunction with the compound of the present invention. In one embodiment, the compound of the present invention and an immune checkpoint inhibitor, as well as additional therapeutic agents as described herein for combination therapies, can be administered orally, subcutaneously, intramuscularly, intranasally, parenterally, or another route. In other embodiments, the compound of the present invention and a chemotherapeutic agent (particularly, one that can induce the production of cGAMP in the body), as well as additional therapeutic agents as described herein for combination therapies, can be administered orally, subcutaneously, intramuscularly, intranasally, parenterally, or another route. The compound of the present invention and a second active agent (if present) may be administered by the same route of administration or by different routes of administration. The therapeutic agents can be administered by any suitable means including, but not limited to, for example, oral, rectal, nasal, topical, vaginal, parenteral, intravenous, intranasal or intratumoral injection into an affected organ.

The compound of the present invention may be administered in a unit dosage form and may be prepared by any methods well known in the art. Such methods include combining the compound of the present invention with a pharmaceutically acceptable carrier or diluent which constitutes one or more accessory ingredients. The pharmaceutically acceptable carrier is selected on the basis of the chosen route of administration and standard pharmaceutical practice. Each carrier must be "pharmaceutically acceptable" in the sense of being compatible with the other ingredients of the formulation and not injurious to the subject or patient. This carrier can be a solid or liquid and the type is generally chosen based on the type of administration being used.

Examples of suitable solid carriers include lactose, sucrose, gelatin, agar, and bulk powders. Examples of suitable liquid carriers include water, pharmaceutically acceptable fats and oils, alcohols, or other organic solvents, including esters, emulsions, syrups or elixirs, suspensions, and solutions and/or suspensions reconstituted from non-effervescent granules. Such liquid carriers may contain, for example, suitable solvents, preservatives, emulsifying agents, suspending agents, diluents, sweeteners, thickeners, and melting agents.

The present invention will now be described in detail.

One aspect of the present invention provides a compound selected from a pyrimidine derivative represented by Formula 10, 11 or 12: 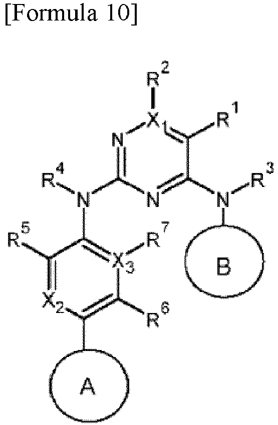
wherein R₁ is hydrogen, hydroxyl, halo, C₁-C₁₃ alkyl or C₁-C₆ alkoxy,
R₂ is hydrogen, halo, C₁-C₁₃ alkyl, C₃-C₁₀ cyclyl, amino (-NR₈R₉) or nitro (-N(O)₂),
R₃ and R₄ are each independently hydrogen, C₁-C₁₃ alkyl, C₃-C₁₀ cyclyl, sulfido (-SR₈), sulfonyl (-S(O)₂R₈) or phosphoryl (-P(O)R₈R₉),
R₅, R₆, and R₇ are each independently hydrogen, hydroxyl, halo, C₁-C₁₃ alkyl, C₁-C₆ alkoxy, C₃-C₁₀ cyclyl, C₃-C₁₀ heterocyclyl, -C(O)-(C₁-C₁₃ alkyl), amino (-NR₈R₉), nitro (-N(O)₂), amido (-(C=O)NR₈R₉), carboxyl (-C(O)OH), nitrile (-CN), ureido (-NR₈(C=O)NR₉-), sulfonamido (-NHS(O)₂R₈), sulfido (-SR₈), sulfonyl (-S(O)₂R₈) or phosphoryl (-P(O)R₈R₉),
X₁, X₂, and X₃ are each independently carbon or nitrogen,
A is C₁-C₁₃ alkyl, amino (-NR₈R₉), C₆-C₁₀ aryl, C₃-C₁₀ cyclyl, C₃-C₁₀ heteroaryl or C₃-C₁₀ heterocyclyl or together with the carbon atom to which R₆ is attached forms a 3- to 7-membered saturated ring optionally containing at least one of N, O, S, NH, C=N, C=O, -NHC(O)-, -NHC(O)NH-, -NHS(O)z-, and SO₂ and optionally substituted with at least one of hydrogen, C₁-C₁₃ alkyl, C₆-C₁₀ aryl, C₃-C₁₀ heteroaryl, hydroxyl, halo, and cyano,
B is C₆-C₁₀ aryl, C₃-C₁₀ cyclyl, C₃-C₁₀ heteroaryl or C₃-C₁₀ heterocyclyl, provided that
when X₁ and X₃ are each independently nitrogen, R₂ or R₇ is nothing,
the C₁-C₆ alkoxy, C₁-C₁₃ alkyl or C₃-C₁₀ cyclyl includes one or more substituents selected from the group consisting of hydrogen, hydroxyl, halo, C₁-C₁₃ alkyl, C₁-C₆ alkoxy, amino (-NR₈R₉), nitro (-N(O)₂), amido (-(C=O)NR₈R₉), carboxyl (-C(O)OH), nitrile (-CN), ureido (-NR₈(C=O)NR₉-), sulfonamido (-NHS(O)z-), sulfido (-S-), sulfonyl (-S(O)₂-), phosphoryl (-P(O)R₈R₉),
C₆-C₁₀ aryl, C₃-C₁₀ heteroaryl, and C₃-C₁₀ heterocyclyl, the C₆-C₁₀ aryl, C₃-C₁₀ heteroaryl or C₃-C₁₀ heterocyclyl includes one or more substituents selected from the group consisting of hydrogen, hydroxyl, halo, carbonyl (-(C=O)R₈R₉), C₁-C₃ alkyl unsubstituted or substituted with halo or C₃-C₁₀ heterocyclyl, C₁-C₃ alkoxy unsubstituted or substituted with halo or C₃-C₁₀ heterocyclyl, C₆-C₁₀ phenoxy, amino (-NR₈R₉), nitro (-N(O)z), amido (-(C=O)NR₈R₉), carboxyl (-C(O)OH), nitrile (-CN), ureido (-NR₈(C=O)NR₉-), sulfonamido (-NHS(O)z-), sulfido (-S-), sulfonyl (-S(O)₂-), phosphoryl (-P(O)R₈R₉), C₆-C₁₀ aryl, C₃-C₁₀ heteroaryl, and C₃-C₁₀ heterocyclyl,
R₈ and R₉ are each independently hydrogen, C₁-C₆ alkyl, C₁-C₆ alkenyl, C₁-C₆ alkynyl, C₆-C₁₀ aryl, C₃-C₁₀ heteroaryl or C₃-C₁₀ heterocyclyl, with the proviso that R₈ together with the nitrogen or carbon atom to which R₉ is attached optionally forms a 3-to 7-membered saturated ring optionally containing at least one of N, O, S, NH, C=N, C=O, -NHC(O)-, -NHC(O)NH-, -NHS(O)z-, and SO₂ and optionally substituted with at least one of hydrogen, C₁-C₁₃ alkyl, C₆-C₁₀ aryl, C₃-C₁₀ heteroaryl, hydroxyl, halo, and cyano, and
the C₃-C₁₀ heteroaryl and C₃-C₁₀ heterocyclyl include at least one heteroatom selected from the group consisting of N, O, and S, 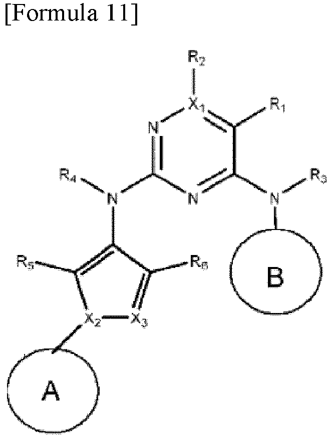
wherein R₁ to R₆, X₁ to X₃, A, and B are as defined in Formula 10, 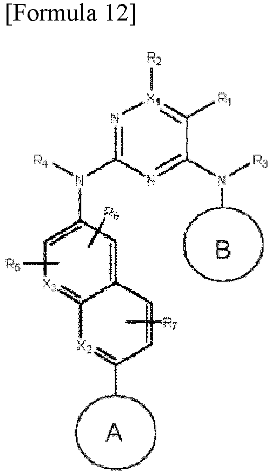
wherein R₁ to R₇, X₁ to X₃, A, and B are as defined in Formula 10, a pharmaceutically acceptable salt thereof, a hydrate thereof, and a stereoisomer thereof.

A pharmaceutically acceptable salt of the pyrimidine compound represented by Formula 10, 11 or 12 according to the present invention can be prepared by a general method known in the art. The pharmaceutically acceptable salt should have low toxicity to humans and do not adversely affect the biological activity and physicochemical properties of its parent compound. Free acids usable to prepare the pharmaceutically acceptable salt can be divided into inorganic acids and organic acids. The inorganic acids may be, for example, hydrochloric acid, sulfuric acid, nitric acid, phosphoric acid, perchloric acid, and hydrobromic acid. The organic acids may be, for example, acetic acid, methanesulfonic acid, ethanesulfonic acid, p-toluenesulfonic acid, fumaric acid, maleic acid, malonic acid, phthalic acid, succinic acid, lactic acid, citric acid, gluconic acid, tartaric acid, salicylic acid, malic acid, oxalic acid, benzoic acid, embonic acid, aspartic acid, glutamic acid, etc. Examples of organic bases usable to prepare organic base addition salts include tris(hydroxymethyl)methylamine dicyclohexylamine, etc. Examples of amino acids usable to prepare amino acid addition bases include natural amino acids such as alanine and glycine.

The pyrimidine compound represented by Formula 10, 11 or 12 according to the present invention is intended to include all hydrates and solvates thereof in addition to the pharmaceutically acceptable salts. A hydrate and a solvate of the compound represented by Formula 10, 11 or 12 can be prepared by dissolving the compound represented by Formula 10, 11 or 12 in a water miscible solvent such as methanol, ethanol, acetone or 1,4-dioxane, adding a free acid or base to the solution, and crystallizing or recrystallizing the mixture. Accordingly, the compound of the present invention is intended to include stoichiometric solvates, including hydrates, in addition to compounds containing various amounts of water that can be prepared by suitable methods such as lyophilization freeze-drying.

A more detailed description will be given of the substituents used to define the compound of the present invention.

In the definition of the substituents in the present invention, the term "alkyl" refers to an aliphatic hydrocarbon radical. The alkyl may be "saturated alkyl" containing no alkenyl or alkynyl moiety or "unsaturated alkyl" containing at least one alkenyl or alkynyl moiety. The term "alkenyl" refers to a group containing at least one carbon-carbon double bond and the term "alkynyl" refers to a group containing at least one carbon-carbon triple bond. The alkyl may be cyclic, branched or straight chained when used alone or in combination with another radical.

The term "aryl" as used herein, either alone or in combination with another radical, means either a carbocyclic aromatic monocyclic group containing 6 carbon atoms which may be further fused to a second 5- or 6-membered carbocyclic group which may be aromatic, saturated or unsaturated. Such aryl groups include, but are not limited to, phenyl, indanyl, 1-naphthyl, 2-naphthyl, and tetrahydronaphthyl. The aryl may be connected to one or more other groups at appropriate positions on the aromatic ring.

The term "alkoxy" as used herein refers to an alkyl group connected to another group through an oxygen atom (*i.e.* -O-alkyl). The alkoxy group may be unsubstituted or substituted with one or more appropriate substituents. Examples of alkoxy groups include, but are not limited to, (C₁-C₆) alkoxy groups such as -O-methyl, -O-ethyl, -O-propyl, -O-isopropyl, -O-2-methyl-1-propyl, -O-2-methyl-2-propyl, -O-2-methyl-1-butyl, -O-3-methyl-1-butyl, -O-2-methyl-3-butyl, -O-2,2-dimethyl-1-propyl, -O-2-methyl-1-pentyl, 3-O-methyl-1-pentyl, -O-4-methyl-1-pentyl, -O-2-methyl-2-pentyl, -O-3-methyl-2-pentyl, -O-4-methyl-2-pentyl, -O-2,2-dimethyl-1-butyl, -O-3,3-dimethylbutyl, -O-2-ethyl-1-butyl, -O-butyl, -O-isobutyl, -O-t-butyl, -O-pentyl, -O-isopentyl, -O-neopentyl, and -O-hexyl.

The term "phenoxy" refers to a phenyl group connected to another group through an oxygen atom (*i.e.* -O-aryl). The phenoxy group may be unsubstituted or substituted with one or more substituents selected from, but not limited to, halo, alkyl, aryl, and hetero aryl groups.

The term "amino" refers to an alkyl group connected to another group through a nitrogen atom (*i.e.* -NH- or -N-alkyl). The amino group may be unsubstituted or substituted with one or more appropriate substituents. Examples of amino groups include, but are not limited to, (C₁-C₆) amino groups such as -NH-methyl, -NH-ethyl, -NH-propyl, -NH-isopropyl, -NH-2-methyl-1-propyl, -NH-2-methyl-2-propyl, -NH-2-methyl-1-butyl, -NH-3-methyl-1-butyl, -NH-2-methyl-3-butyl, -NH-2,2-dimethyl-1-propyl, -NH-2-methyl-1-pentyl, 3-NH-methyl-1-pentyl, -NH-4-methyl-1-pentyl, -NH-2-methyl-2-pentyl, -NH-3-methyl-2-pentyl, -NH-4-methyl-2-pentyl, -NH-2,2-dimethyl-1-butyl, - NH-3,3-dimethyl-butyl, -NH-2-ethyl-1-butyl, -NH-butyl, -NH-isobutyl, -NH-t-butyl, - NH-pentyl, -NH-isopentyl, -NH-neopentyl, -NH-hexyl, -N,N-dimethyl, -N-methyl-N-ethyl, -N-methyl-N-propyl, -N-methyl-isopropyl, -N-methyl-N-butyl, -N-methyl-N-isobutyl, -N-methyl-N-pentyl, -N-methyl-N-isopentyl, N-methyl-N-hexyl, N-methyl-N-isohexyl, -N,N-diethyl, -N-ethyl-N-propyl, -N-ethyl-N-isopropyl, -N-ethyl-N-butyl, -N-ethyl-N-isobutyl, -N-ethyl-N-pentyl, -N-ethyl-N-isopentyl, -N-ethyl-N-hexyl, -N-ethyl-N-isohexyl, -N,N-dipropyl, -N-propyl-N-isopropyl, -N-propyl-N-butyl, -N-propyl-N-isobutyl, -N-propyl-N-pentyl, -N-propyl-N-isopentyl, -N-propyl-N-hexyl, -N-propyl-N-isohexyl, -N,N-dibutyl, -N-butyl-N-isobutyl, -N-butyl-N-pentyl, -N-butyl-N-isopentyl, - N-butyl-N-hexyl, -N-butyl-N-isohexyl, -N,N-dipentyl, -N-pentyl-N-hexyl, -N-pentyl-N-isohexyl, and -N,N-dihexyl.

The term "halo" refers to fluoro, chloro, bromo or iodo.

The term "heterocyclyl" refers to a heteroaromatic group containing at least one heteroatom selected from the group consisting of N, O, and S, unless otherwise mentioned. Examples of preferred heterocyclyl groups include, but are not limited to, pyrrolidine, furan, morpholine, piperazine, and piperidine groups. Examples of more preferred heterocyclyl groups include, but are not limited to, pyrrolidine, piperidine, piperazine, and morpholine groups.

The term "heteroaryl" refers to a heteroaromatic group containing at least one heteroatom selected from the group consisting of N, O, and S, unless otherwise mentioned. Examples of preferred heteroaryl groups include, but are not limited to, pyridine, pyrazine, pyrimidine, pyridazine, pyrazole, imidazole, triazole, indole, oxadiazole, thiadiazole, quinoline, isoquinoline, isoxazole, oxazole, thiazole, and pyrrole groups.

In one aspect of the present invention, R₁ is hydrogen, halo or C₁-C₃ alkyl, R₂ is hydrogen, C₁-C₃ alkyl or amino (-NR₈R₉), R₃ is hydrogen, C₁-C₃ alkyl, sulfido (-SR₈) or sulfonyl (-S(O)₂R₈), R₄ is hydrogen or C₁-C₃ alkyl, R₅ is hydrogen, hydroxyl, halo, C₁-C₃ alkyl or C₁-C₆ alkoxy, R₆ is hydrogen, halo or C₁-C₃ alkyl, and R₇ is hydrogen or C₁-C₃ alkyl.

In one aspect of the present invention, A is amino (-NR₈R₉), C₃-C₁₀ heteroaryl or C₃-C₁₀ heterocyclyl or together with the carbon atom to which R₆ is attached forms a 3-to 7-membered saturated ring optionally containing at least one of N, O, S, NH, C=N, C=O, -NHC(O)-, -NHC(O)NH-, -NHS(O)₂-, and SO₂ and optionally substituted with at least one of hydrogen, C₁-C₁₃ alkyl, C₆-C₁₀ aryl, C₃-C₁₀ heteroaryl, hydroxyl, halo, and cyano and B is C₆-C₁₀ aryl or C₃-C₁₀ heteroaryl.

In one aspect of the present invention, R₁ is halo, R₂ is hydrogen or amino (-NR₈R₉), R₃ is hydrogen, C₁-C₃ alkyl or sulfonyl (-S(O)₂R₈), R₄ is hydrogen, R₅ is hydrogen, halo or C₁-C₆ alkoxy, R₆ is hydrogen, halo or C₁-C₃ alkyl, R₇ is hydrogen, A is selected from the group consisting of piperazine, piperidine, morpholine, pyrrolidine, and imidazole, and B is selected from the group consisting of benzene, thiazole, thiophene, pyrazole, benzothiophene, pyridazine, pyrazine, imidazole, oxadiazole, triazole, furan, pyrimidine, oxazole, pyrrole, pyridine, oxadiazole, triazine, thiadiazole, isoxazole, and tetrazole.

In one aspect of the invention, R₁ is Cl or Br, R₂ is hydrogen or -NH₂, R₃ is hydrogen, -CH₃ or -S(O)₂CH₃, R₄ is hydrogen, R₅ is hydrogen, Cl, Br, -OCH₃, -OCH₂CH₃ or -OCH₂CF₃, R₆ is hydrogen, Cl, Br, F, -CH₃ or -CF₃, R₇ is hydrogen, A is selected from the group consisting of piperazine, piperidine, morpholine, and pyrrolidine, and B is selected from the group consisting of benzene, benzothiazole, quinoline, and quinoxaline.

In one aspect of the present invention, A is selected from the group consisting of

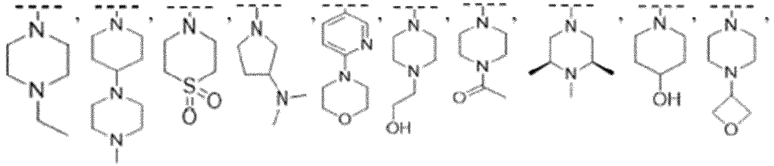

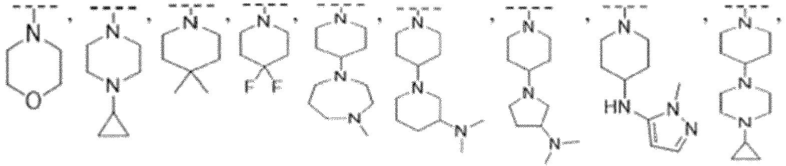

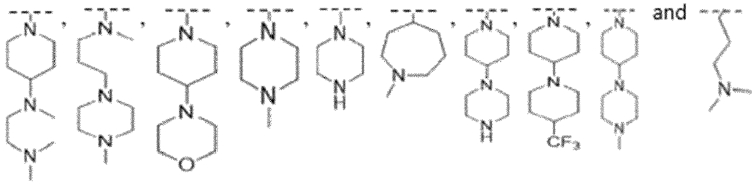

In one aspect of the present invention, B is selected from the group consisting of

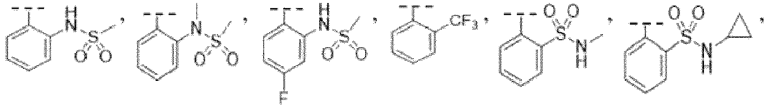

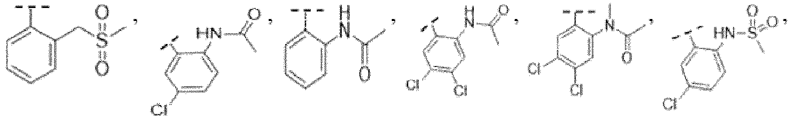

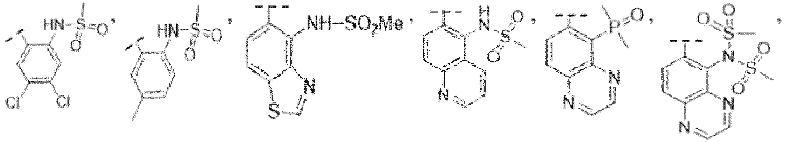

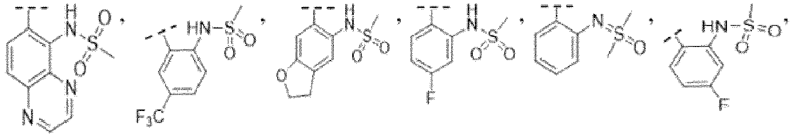

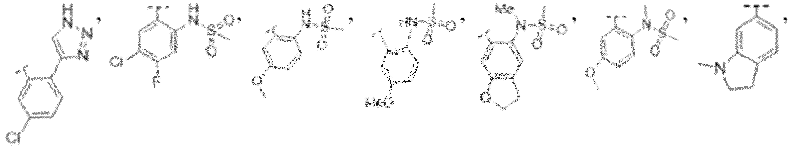

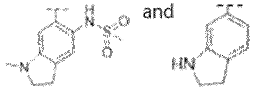

In one aspect of the present invention, R₁ is Cl or Br, R₂ is hydrogen or -NH₂, R₃ is hydrogen, -CH₃ or -S(O)₂CH₃, R₄ is hydrogen, R₅ is hydrogen, Cl, Br, -OCH₃,-OCH₂CH₃ or -OCH₂CF₃, R₆ is hydrogen, Cl, Br, F -CH₃ or -CF₃, R₇ is hydrogen, A is selected from the group consisting of 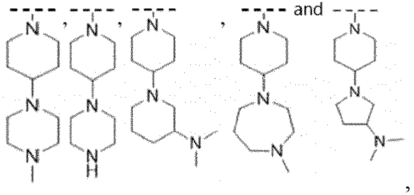 and B is selected from the group consisting of

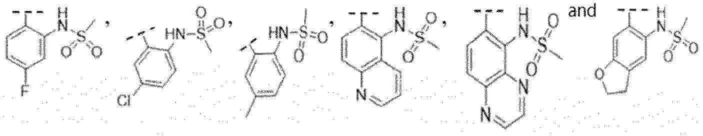

In one aspect of the present invention, the compound is selected from the group consisting of the following compounds Nos. 1 to 283:
Compound No. 1: *N*-(2-((5-chloro-2-((6-(4-ethylpiperazin-1-yl)-2-methoxypyridin-3-yl)amino)-4-yl)amino)phenyl)methanesulfonamide;
Compound No. 2: *N*-(2-((5-chloro-2-((2-methoxy-5-methyl-4-(4-(4-methylpiperazin-1-yl)piperidin-1-yl)phenyl)amino)pyrimidin-4-yl)amino)phenyl)methanesulfonamide;
Compound No. 3: *N*-(2-((5-chloro-2-((6-(4-(4-methylpiperazin-1-yl)piperidin-1-yl)pyridin-3-yl)amino)pyrimidin-4-yl)amino)phenyl)methanesulfonamide;
Compound No. 4: *N*-(2-((5-chloro-2-((6-(4-(4-methylpiperazin-1-yl)piperidin-1-yl)-2-(2,2,2-trifluoroethoxy)pyridin-3-yl)amino)pyrimidin-4-yl)amino)phenyl)methanesulfonamide;
Compound No. 5: *N*-(2-((5-chloro-2-((3-fluoro-4-(4-(4-methylpiperazin-1-yl)piperidin-1-yl)phenyl)amino)pyrimidin-4-yl)amino)phenyl)methanesulfonamide;
Compound No. 6: *N*-(2-((5-chloro-2-((6-(1,1-dioxythiomorpholino)-2-methoxypyridin-3-yl)amino)pyrimidin-4-yl)amino)phenyl)methanesulfonamide;
Compound No. 7: *N*-(2-((5-chloro-2-((5-chloro-2-methoxy-4-(4-(4-methylpiperazin-1-yl)piperidin-1-yl)phenyl)amino)pyrimidin-4-yl)amino)phenyl)methanesulfonamide;
Compound No. 8: *N*-(5-chloro-2-((6-(3-(dimethylamino)pyrrolidin-1-yl)-2-methoxypyridin-3-yl)amino)pyrimidin-4-yl)-*N*-(2-(methylsulfonamido)phenyl)methanesulfonamide;
Compound No. 9: *N*-(5-chloro-2-((2-methoxy-5-methyl-4-(4-(4-methylpiperazin-1-yl)piperidin-1-yl)phenyl)amino)pyrimidin-4-yl)-*N*-(2-(methylsulfonamido)phenyl)methanesulfonamide;
Compound No. 10: *N*-(5-chloro-2-((2-methoxy-6-(4-(4-methylpiperazin-1-yl)piperidin-1-yl)pyridin-3-yl)amino)pyrimidin-4-yl)-N (2-(methylsulfonamido)phenyl)methanesulfonamide;
Compound No. 11: *N*-(2-((5-chloro-2-((2-methoxy-5-methyl-4-(4-(4-methylpiperazin-1-yl)piperidin-1-yl)phenyl)amino)pyrimidin-4-yl)(methyl)amino)phenyl)-*N*-methylsulfonamide;
Compound No. 12: *N*-(2-((5-chloro-2-((2-methoxy-5-methyl-4-(4-(4-methylpiperazin-1-yl)piperidin-1-yl)phenyl)amino)pyrimidin-4-yl)amido)-5-fluorophenyl)methanesulfonamide;
Compound No. 13: *N*-(2-((5-chloro-2-((6-morpholinopyridin-3-yl)amino)pyrimidin-4-yl)amido)-5-fluorophenyl)methanesulfonamide;
Compound No. 14: *N-*(2-((5-chloro-2-((6-(4-(4-methylpiperazin-1-yl)piperidin-1-yl)pyridin-3-yl)amido)pyrimidin-4-yl)amino)-5-fluorophenyl)methanesulfonamide;
Compound No. 15: *N*-(2-((5-chloro-2-((2-methoxy-5-methyl-6-(4-(4-methylpiperazin-1-yl)piperidin-1-yl)pyridin-3-yl)amino)pyrimidin-4-yl)amino)-5-fluorophenyl)methanesulfonamide;
Compound No. 16: *N*-(2-((5-chloro-2-((5-fluoro-6-(4-(4-methylpiperazin-1-yl)piperidin-1-yl)pyridin-3-yl)amino)pyrimidin-4-yl)amino)-5-fluorophenyl)methanesulfonamide;
Compound No. 17: *N*-(2-((5-chloro-2-((2-methoxy-6-(4-(4-methylpiperazin-1-yl)piperidin-1-yl)pyridin-3-yl)amino)pyrimidin-4-yl)amino)-5-fluorophenyl)methanesulfonamide;
Compound No. 18: *N*-(2-((5-chloro-2-((6-(4-(2-hydroxymethyl)piperazin-1-yl)-2-methoxypyridin-3-yl)amino)pyrimidin-4-yl)amino)-5-fluorophenyl)methanesulfonamide;
Compound No. 19: *N*-(2-((2-((6-(4-acetylpiperazin-1-yl)-2-methoxypyridin-3-yl)amino)-5-chloropyrimidin-4-yl)amino)-5-fluorophenyl)methanesulfonamide;
Compound No. 20: *N*-(2-((5-chloro-2-((2-methoxy-6-((3S,5R)-3,4,5-trimethylpiperazin-1-yl)pyridin-3-yl)amino)pyrimidin-4-yl)amino)-5-fluorophenyl)methanesulfonamide;
Compound No. 21: *N*-(2-((5-chloro-((6-(4-hydroxypiperidin-1-yl)-2-methoxypyridin-3-yl)amino)pyrimidin-4-yl)amino)-5-fluorophenyl)methanesulfonamide;
Compound No. 22: *N*-(2-((5-chloro-2-((2-methoxy-6-(4-(oxetan-3-yl)piperazin-1-yl)pyridin-3-yl)amino)pyrimidin-4-yl)amino)-5-fluorophenyl)methanesulfonamide;
Compound No. 23: *N*-(2-((5-chloro-2-((6-(3-(dimethylamino)pyrrolidin-1-yl)-2-methoxypyridin-3-yl)amino)pyrimidin-4-yl)amino)-5-fluorophenyl)methanesulfonamide;
Compound No. 24: *N*-(2-((5-bromo-2-((2-methoxy-5-methyl-4-(4-(4-methylpiperazin-1-yl)piperidin-1-yl)phenyl)amino)pyrimidin-4-yl)amido)-5-fluorophenyl)methanesulfonamide;
Compound No. 25: *N*-(2-((5-bromo-2-((5-fluoro-6-(4-(4-methylpiperazin-1-yl)piperidin-1-yl)pyridin-3-yl)amino)pyrimidin-4-yl)amino)-5-fluorophenyl)methanesulfonamide;
Compound No. 26: *N*-(2-((5-bromo-2-((5-chloro-2-methoxy-4-(4-(4-methylpiperazin-1-yl)piperidin-1-yl)phenyl)amino)pyrimidin-4-yl)amino)-5-fluorophenyl)methanesulfonamide;
Compound No. 27: *N*-(2-((5-bromo-2-((2-chloro-4-(4-(4-methylpiperazin-1-yl)piperidin-1-yl)phenyl)amino)pyrimidin-4-yl)amino)-5-fluorophenyl)methanesulfonamide;
Compound No. 28: *N*-(2-((5-bromo-2-((2-methoxy-6-(4-(4-methylpiperazin-1-yl)piperidin-1-yl)pyridin-3-yl)amino)pyrimidin-4-yl)amino)-5-fluorophenyl)methanesulfonamide;
Compound No. 29: *N*-(2-((5-bromo-2-((2-methoxy-6-morpholinopyridin-3-yl)amino)pyrimidin-4-yl)amino)-5-fluorophenyl)methanesulfonamide;
Compound No. 30: *N*-(2-((5-bromo-2-((6-(4-cyclopropylpiperazin-1-yl)-2-methoxypyridin-3-yl)amino)pyrimidin-4-yl)amino)-5-fluorophenyl)methanesulfonamide;
Compound No. 31: *N*-(2-((5-bromo-2-((2-methoxy-6-(4-morpholinopiperidin-1-yl)pyridin-3-yl)amino)pyrimidin-4-yl)amino)-5-fluorophenyl)methanesulfonamide;
Compound No. 32: *N*-(2-((5-bromo-2-((6-(4,4-dimethylpiperidin-1-yl)-2-methoxypyridin-3-yl)amino)pyrimidin-4-yl)amino)-5-fluorophenyl)methanesulfonamide;
Compound No. 33: *N*-(2-((5-bromo-2-((6-(4,4-difluoropiperidin-1-yl)-2-methoxypyridin-3-yl)amino)pyrimidin-4-yl)amino)-5-fluorophenyl)methanesulfonamide;
Compound No. 34: *N*-(2-((5-bromo-2-((2-chloro-6-(4-(4-methylpiperazin-1-yl)piperidin-1-yl)-5-(trifluoromethyl)pyridin-3-yl)amino)pyrimidin-4-yl)amino)-5-fluorophenyl)methanesulfonamide;
Compound No. 35: *N*-(2-((5-bromo-2-((2-(4-(4-methylpiperazin-1-yl)piperidin-1-yl)quinolin-6-yl)amino)pyrimidin-4-yl)amino)-5-fluorophenyl)methanesulfonamide;
Compound No. 36: *N*-(2-((5-bromo-2-((2-methoxy-6-(4-(1-methylpiperidin-4-yl)piperazin-1-yl)pyridin-3-yl)amino)pyrimidin-4-yl)amino)-5-fluorophenyl)methanesulfonamide;
Compound No. 37: *N*-(2-((5-bromo-2-((2-methoxy-6-(4-(1-methyl-4-yl)piperazin-1-yl)-5-(trifluoromethyl)pyridin-3-yl)amino)pyrimidin-4-yl)amino)-5-fluorophenyl)methanesulfonamide;
Compound No. 38: *N*-(2-((5-bromo-2-((2-methoxy-5-methyl-4-(4-(4-methyl-1,4-diazepan-1-yl)piperidin-1-yl)phenyl)amino)pyrimidin-4-yl)amino)-5-fluorophenyl)methanesulfonamide;
Compound No. 39: *N*-(2-((5-bromo-2-((4-(3-(dimethylamino)-[1,4'-bipiperidin]-1'-yl)-2-methoxy-5-methylphenyl)amino)pyrimidin-4-yl)amino)-5-fluorophenyl)methanesulfonamide;
Compound No. 40: *N*-(2-((5-bromo-2-((4-(4-(3-(dimethylamino)pyrrolidin-1-yl)piperidin-1-yl)-2-methoxy-5-methylphenyl)amino)pyrimidin-4-yl)amino)-5-fluorophenyl)methanesulfonamide;
Compound No. 41: *N*-(2-((5-bromo-2-((2-methoxy-5-methyl-4-(4-((1-methyl-1H-pyrazol-5-yl)amino)piperidin-1-yl)phenyl)amino)pyrimidin-4-yl)amino)-5-fluorophenyl)methanesulfonamide;
Compound No. 42: *N*-(2-((5-bromo-2-((4-(4-(4-cyclopropylpiperazin-1-yl)piperidin-1-yl)-2-methoxy-5-methylphenyl)amino)pyrimidin-4-yl)amino)-5-fluorophenyl)methanesulfonamide;
Compound No. 43: *N*-(2-((5-bromo-2-((2-chloro-5-methyl-4-(4-(4-methylpiperidin-1-yl)piperidin-1-yl)phenyl)amino)pyrimidin-4-yl)amino)-5-fluorophenyl)methanesulfonamide;
Compound No. 44: *N*-(2-((5-bromo-2-((2-chloro-5-methyl-4-(4-(4-methyl-1,4-diazepan-1-yl)piperidin-1-yl)phenyl)amino)pyrimidin-4-yl)amino)-5-fluorophenyl)methanesulfonamide;
Compound No. 45: *N*-(2-((5-bromo-2-((2-chloro-4-(3-(dimethylamino)-[1,4'-bipiperidin]-1'-yl)-5-methylphenyl)amino)pyrimidin-4-yl)amino)fluorophenyl)methanesulfonamide;
Compound No. 46: 5-chloro-*N*²-(2-methoxy-5-methyl-4-(4-(4-methylpiperazin-1-yl)piperidin-1-yl)phenyl)-*N*⁴-(2-(trifluoromethyl)phenyl)pyrimidine-2,4-diamine;
Compound No. 47: 5-chloro-*N*²-(2-methoxy-4-(4-(4-methylpiperazin-1-yl)piperidin-1-yl)phenyl)-*N*⁴-(2-(trifluoromethyl)phenyl)pyrimidine-2,4-diamine;
Compound No. 48: 5-chloro-*N*²-(4-(4-ethylpiperazin-1-yl)phenyl)-*N*⁴-(2-(trifluoromethyl)phenyl)pyrimidine-2,4-diamine;
Compound No. 49: 5-chloro-*N*²-(6-morpholinopyridin-3-yl)-*N*⁴-(2-(trifluoromethyl)phenyl)pyrimidine-2,4-diamine;
Compound No. 50: 2-((5-chloro-2-((2-methoxy-5-methyl-4-(4-(4-methylpiperazin-1-yl)piperidin-1-yl)phenyl)amino)pyrimidin-4-yl)amino)-*N-*methylbenzenesulfonamide;
Compound No. 51: 2-((5-chloro-2-((2-methoxy-4-(4-(4-methylpiperazin-1-yl)piperidin-1-yl)phenyl)amino)pyrimidin-4-yl)amino)-*N-*methylbenzenesulfonamide;
Compound No. 52: 2-((5-chloro-2-((6-(4-(4-methylpiperazin-1-yl)piperidin-1-yl)pyridin-3-yl)amino)pyrimidin-4-yl)amino)-*N-*methylbenzenesulfonamide;
Compound No. 53: 2-((5-chloro-2-((6-morpholinopyridin-3-yl)amino)pyrimidin-4-yl)amino)-*N*-methylbenzenesulfonamide;
Compound No. 54: 2-((5-chloro-2-((6-(4-ethylpiperazin-1-yl)-2-methoxypyridin-3-yl)amino)pyrimidin-4-yl)amino)-*N*-methylbenzenesulfonamide;
Compound No. 55: 2-((5-chloro-2-((2-methoxy-6-(4-(4-methylpiperazin-1-yl)piperidin-1-yl)pyridin-3-yl)amino)pyrimidin-4-yl)amino)-*N-*methylbenzenesulfonamide;
Compound No. 56: 2-((5-chloro-2-((6-(4-(2-hydroxyethyl)piperazin-1-yl)-2-methoxypyridin-3-yl)amino)pyrimidin-4-yl)amino)-*N*-methylbenzenesulfonamide;
Compound No. 57: 2-((5-chloro-2-((6-(3-(dimethylamino)pyrrolidin-1-yl)-2-methoxypyridin-3-yl)amino)pyrimidin-4-yl)amino)-*N*-methylbenzenesulfonamide;
Compound No. 58: 2-((5-chloro-2-((2-methoxy-5-methyl-6-(4-(4-methylpiperazin-1-yl)piperidin-1-yl)pyridin-3-yl)amino)pyrimidin-4-yl)amino)-*N-*methylbenzenesulfonamide;
Compound No. 59: 2-((5-chloro-2-((2-methoxy-5-methyl-4-(4-(4-methylpiperazin-1-yl)piperidin-1-yl)phenyl)amino)pyrimidin-4-yl)amino)-*N-*cyclopropylbenzenesulfonamide;
Compound No. 60: 2-((5-chloro-2-((3-fluoro-4-(4-(4-methylpiperazin-1-yl)piperidin-1-yl)phenyl)amino)pyrimidin-4-yl)amino)-*N-*cyclopropylbenzenesulfonamide;
Compound No. 61: 2-((5-chloro-2-((2-methoxy-6-(4-(4-methylpiperazin-1-yl)piperadin-1-yl)pyridin-3-yl)amino)pyrimidin-4-yl)amino)-*N-*cyclopropylbenzenesulfonamide;
Compound No. 62: 5-chloro-*N*²-(2-methoxy-5-methyl-4-(4-(4-methylpiperazin-1-yl)piperidin-1-yl)phenyl)-*N*⁴-(2-((methylsulfonyl)methyl)phenyl)pyrimidine-2,4-diamine;
Compound No. 63: 5-chloro-*N*²-(3-fluoro-4-(4-(4-methylpiperazin-1-yl)piperidin-1-yl)phenyl)-*N*⁴-(2-((methylsulfonyl)methyl)phenyl)pyrimidine-2,4-diamine;
Compound No. 64: 5-chloro-*N*²-(2-methoxy-6-(4-(4-methylpiperazin-1-yl)piperidin-1-yl)pyridin-3-yl)-*N*⁴-(2-((methylsulfonyl)methyl)phenyl)pyrimidine-2,4-diamine;
Compound No. 65: *N*-(4-chloro-2-((5-chloro-2-((2-methoxy-5-methyl-4-(4-(4-methylpiperazin-1-yl)piperidin-1-yl)phenyl)amino)pyrimidin-4-yl)amino)phenyl)acetamide;
Compound No. 66: *N*-(4-chloro-2-((5-chloro-2-((2-methoxy-5-methyl-6-(4-(4-methylpiperazin-1-yl)piperidin-1-yl)pyridin-3-yl)amino)pyrimidin-4-yl)amino)phenyl)acetamide;
Compound No. 67: *N*-(4-chloro-2-((5-chloro-2-((3-fluoro-4-(4-(4-methylpiperazin-1-yl)piperidin-1-yl)phenyl)amino)pyrimidin-4-yl)amino)phenyl)acetamide;
Compound No. 68: *N*-2-((5-chloro-2-((2-methoxy-5-methyl-4-(4-(4-methylpiperazin-1-yl)piperidin-1-yl)phenyl)amino)pyrimidin-4-yl)amino)phenyl)acetamide;
Compound No. 69: *N*-(2-((5-chloro-2-((3-fluoro-4-(4-(4-methylpiperazin-1-yl)piperidin-1-yl)phenyl)amino)pyrimidin-4-yl)amino)phenyl)acetamide;
Compound No. 70: *N*-(2-((5-chloro-2-((5-fluoro-6-(4-(4-methylpiperazin-1-yl)piperidin-1-yl)pyridin-3-yl)-amino)pyrimidin-4-yl)amino)phenyl)acetamide;
Compound No. 71: *N*-(4,5-dichloro-2-((5-chloro-2-((2-methoxy-5-methyl-4-(4-(4-methylpiperazin-1-yl)piperidin-1-yl)phenyl)amino)pyrimidin-4-yl)amino)phenyl)acetamide;
Compound No. 72: *N*-(4,5-dichloro-2-((5-chloro-2-((2-methoxy-6-(4-(4-methylpiperazin-1-yl)piperidin-1-yl)pyridin-3-yl)amino)pyrimidin-4-yl)amino)phenyl)acetamide;
Compound No. 73: *N*-(4,5-dichloro-2-((5-chloro-2-((5-fluoro-6-(4-(4-methylpiperazin-1-yl)piperidin-1-yl)pyridin-3-yl)amino)pyrimidin-4-yl)amino)phenyl)acetamide;
Compound No. 74: *N*-(4,5-dichloro-2-((5-chloro-2-((6-morpholinopyridin-3-yl)amino)pyrimidin-4-yl)amino)phenyl)acetamide;
Compound No. 75: *N*-(4,5-dichloro-2-((5-chloro-2-((2-methoxy-4-(4-(4-methylpiperazin-1-yl)piperidin-1-yl)phenyl)amino)pyrimidin-4-yl)amino)phenyl)acetamide;
Compound No. 76: *N*-(4,5-dichloro-2-((5-chloro-2-((3-fluoro-4-(4-(4-methylpiperazin-1-yl)piperidin-1-yl)phenyl)amino)pyrimidin-4-yl)amino)phenyl)acetamide;
Compound No. 77: *N*-(4,5-dichloro-2-((5-chloro-2-((2-chloro-4-(4-(4-methylpiperazin-1-yl)piperidin-1-yl)phenyl)amino)pyrimidin-4-yl)amino)phenyl)acetamide;
Compound No. 78: *N*-(4,5-dichloro-2-((5-chloro-2-((2-methoxy-5-methyl-4-(4-(4-methylpiperazin-1-yl)piperidin-1-yl)phenyl)amino)pyrimidin-4-yl)(methyl)amino)phenyl)-*N*-methylacetamide;
Compound No. 79: *N*-(4,5-dichloro-2-((5-chloro-2-((2-methoxy-6-(4-(4-methylpiperazin-1-yl)piperidin-1-yl)pyridin-3-yl)amino)pyrimidin-4-yl)(methyl)amino)phenyl)-*N*-methylacetamide;
Compound No. 80: *N*-(4-chloro-2-((5-chloro-2-((2-methoxy-5-methyl-4-(4-(4-methylpiperazin-1-yl)piperidin-1-yl)phenyl)amino)pyrimidin-4-yl)amino)phenyl)methanesulfonamide;
Compound No. 81: *N*-(4-chloro-2-((5-chloro-2-((2-methoxy-6-(4-(4-methylpiperazin-1-yl)piperidin-1-yl)pyridin-3-yl)amino)pyrimidin-4-yl)aminophenyl)methanesulfonamide;
Compound No. 82: *N*-(4-chloro-2-((5-chloro-2-((2-methoxy-4-(4-(4-methylpiperazin-1-yl)piperidin-1-yl)phenyl)amino)pyrimidin-4-yl)amino)phenyl)methanesulfonamide;
Compound No. 83: *N*-(4-chloro-2-((5-chloro-2-((2-methoxy-5-methyl-6-(4-(4-methylpiperazin-1-yl)piperidin-1-yl)pyridin-3-yl)amino)pyrimidin-4-yl)amino)phenyl)methanesulfonamide;
Compound No. 84: *N*-(4-chloro-2-((5-chloro-2-((3-fluoro-4-(4-(4-methylpiperazin-1-yl)piperidin-1-yl)phenyl)amino)pyrimidin-4-yl)amino)phenyl)methanesulfonamide;
Compound No. 85: *N*-(2-((5-bromo-2-((2-methoxy-5-methyl-4-(4-(4-methylpiperazin-1-yl)piperidin-1-yl)phenyl)amino)pyrimidin-4-yl)amino-4-chlorophenyl)methanesulfonamide;
Compound No. 86: *N*-(2-((5-bromo-2-((2-methoxy-5-methyl-6-(4-(4-methylpiperazin-1-yl)piperidin-1-yl)pyridin-3-yl)amino)pyrimidin-4-yl)amino)-4-chlorophenyl)methanesulfonamide;
Compound No. 87: *N*-(2-((5-bromo-2-((3-fluoro-4-(4-(4-methylpiperazin-1-yl)piperidin-1-yl)phenyl)amino)pyrimidin-4-yl)amino)-4-chlorophenyl)methanesulfonamide;
Compound No. 88: *N*-(2-((5-bromo-2-((5-chloro-2-methoxy-4-(4-(4-methylpiperazin-1-yl)piperidin-1-yl)phenyl)amino)pyrimidin-4-yl)amino)-4-chlorophenyl)methanesulfonamide;
Compound No. 89: *N*-(2-((5-bromo-2-((2-methoxy-5-methyl-4-(4-(4-methyl-1,4-diazepan-1-yl)piperidin-1-yl)phenyl)amino)pyrimidin-4-yl)amino)-4-chlorophenyl)methanesulfonylamide;
Compound No. 90: *N*-(2-((5-bromo-2-((4-(3-(dimethylamino)-[1,4'-bipiperidin]-1'-yl)-2-methoxy-5-methylphenyl)amino)pyrimidin-4-yl)amino)-4-chlorophenyl)methanesulfonamide;
Compound No. 91: *N*-(2-((5-bromo-2-((4-(4-(3-(dimethylamino)pyrrolidin-1-yl)piperidin-1-yl)-2-methoxy-5-methylphenyl)amino)pyrimidin-4-yl)amino)-4-chlorophenyl)methanesulfonamide;
Compound No. 92: *N*-(2-((5-bromo-2-((5-fluoro-2-methoxy-4-(4-(4-methylpiperazin-1-yl)piperidin-1-yl)phenyl)amino)pyrimidin-4-yl)amino)-4-chlorophenyl)methanesulfonamide;
Compound No. 93: *N*-(2-((5-bromo-2-((2-chloro-5-methyl-4-(4-(4-methylpiperazin-1-yl)piperidin-1-yl)phenyl)amino)pyrimidin-4-yl)amino)-4-chlorophenyl)methanesulfonamide;
Compound No. 94: *N*-(2-((5-bromo-2-((2-methoxy-5-methyl-4-(4-(piperazin-1-yl)piperidin-1-yl)phenyl)amino)pyrimidin-4-yl)amino)-4-chlorophenyl)methanesulfonamide;
Compound No. 95: *N*-(2-((5-bromo-2-((5-chloro-2-methoxy-4-(4-(4-methyl-1,4-diazepan-1-yl)piperidin-1-yl)phenyl)amino)pyrimidin-4-yl)amino)-4-chlorophenyl)methanesulfonamide;
Compound No. 96: *N*-(2-((5-bromo-2-((2-chloro-4-(4-(3-(dimethylamino)pyrrolidin-1-yl)piperidin-1-yl)-5-methylphenyl)amino)pyrimidin-4-yl)amino)-4-chlorophenyl)methanesulfonamide;
Compound No. 97: *N*-(2-((5-bromo-2-((2-chloro-5-methyl-4-(4-(4-methyl-1,4-diazepan-1-yl)piperidin-1-yl)phenyl)amino)pyrimidin-4-yl)amino)-4-chlorophenyl)methanesulfonamide;
Compound No. 98: *N*-(2-((5-bromo-2-((2-chloro-4-(3-(dimethylamino)-[1,4'-bipiperidin]-1'-yl)-5-methylphenyl)amino)pyrimidin-4-yl)amino)-4-chlorophenyl)methanesulfonamide;
Compound No. 99: *N*-(4,5-dichloro-2-((5-chloro-2-((2-methoxy-5-methyl-4-(4-(4-methylpiperazin-1-yl)piperidin-1-yl)phenyl)amino)pyrimidin-4-yl)amino)phenyl)methanesulfonamide;
Compound No. 100: *N*-(4,5-dichloro-2-((5-chloro-2-((2-methoxy-5-methyl-6-(4-(4-methylpiperazin-1-yl)piperidin-1-yl)pyridin-3-yl)amino)pyrimidin-4-yl)amino)phenylmethanesulfonamide;
Compound No. 101: *N*-(4,5-dichloro-2-((5-chloro-2-((3-fluoro-4-(4-(4-methylpiperazin-1-yl)piperidin-1-yl)phenyl)amino)pyrimidin-4-yl)amino)phenyl)methanesulfonamide;
Compound No. 102: *N*-(4,5-dichloro-2-((5-chloro-2-((2-(4-(4-methylpiperazin-1-yl)piperidin-1-yl)quinolin-6-yl)amino)pyrimidin-4-yl)amino)phenyl)methanesulfonamide;
Compound No. 103: *N*-(4,5-dichloro-2-((5-chloro-2-((5-chloro-2-methoxy-4-(4-(4-methylpiperazin-1-yl)piperidin-1-yl)phenyl)amino)pyrimidin-4-yl)aminophenyl)methanesulfonamide;
Compound No. 104: *N*-(4,5-dichloro-2-((5-chloro-2-((4-(4-((2-(dimethylamino)ethyl)(methyl)amino)piperidin-1-yl)-2-methoxy-5-methylphenyl)amino)pyrimidin-4-yl)amino)phenyl)methanesulfonamide;
Compound No. 105: *N*-(4,5-dichloro-2-((5-chloro-2-((2-methoxy-5-methyl-4-(methyl(3-(4-methylpiperazin-1-yl)propyl)amino)phenyl)amino)pyrimidin-4-yl)amino)phenyl)methanesulfonamide;
Compound No. 106: *N*-(2-((5-bromo-2-((2-methoxy-5-methyl-4-(4-(4-methylpiperazin-1-yl)piperidin-1-yl)phenyl)amino)pyrimidin-4-yl)amino)-4-methylphenyl)methanesulfonamide;
Compound No. 107: *N*-(2-((5-bromo-2-((5-chloro-2-methoxy-4-(4-(4-methylpiperazin-1-yl)piperidin-1-yl)phenyl)amino)pyrimidin-4-yl)amino)-4-methylphenyl)methanesulfonamide;
Compound No. 108: *N*-(2-((5-bromo-2-((5-fluoro-2-methoxy-4-(4-(4-methoxy-1-yl)piperidin-1-yl)phenyl)amino)pyrimidin-4-yl)amino)-4-methylphenyl)methanesulfonamide;
Compound No. 109: *N*-2-((5-bromo-2-((2-chloro-5-methyl-4-(4-(4-methylpiperazin-1-yl)piperidin-1-yl)phenyl)amino)pyrimidin-4-yl)amino)-4-methylphenyl)methanesulfonamide;
Compound No. 110: *N*-(5-chloro-2-((2-methoxy-5-methyl-4-(4-(4-methylpiperazin-1-yl)piperidin-1-yl)phenyl)amino)pyrimidin-4-yl)-N (4-(methylsulfonamido)benzo[d]thiazol-5-yl)methanesulfonamide;
Compound No. 111: *N*-(5-chloro-2-((2-methoxy-6-(4-(4-methylpiperazin-1-yl)piperidin-1-yl)pyridin-3-yl)amino)pyrimidin-4-yl)-*N*-(4-(methylsulfonamido)benzo[d]thiazol-5-yl)methanesulfonamide;
Compound No. 112: *N*-(5-chloro-2-((3-fluoro-4-(4-(4-methylpiperazin-1-yl)piperidin-1-yl)phenyl)amino)pyrimidin-4-yl)-*N*-(4-(methylsulfonamido)benzo[d]thiazol-5-yl)methanesulfonamide;
Compound No. 113: *N*-(5-chloro-2-((5-fluoro-6-(4-(4-methylpiperazin-1-yl)piperidin-1-yl)pyridin-3-yl)amino)pyrimidin-4-yl)-*N*-(4-(methylsulfonamido)benzo[d]thiazol-5-yl)methanesulfonamide;
Compound No. 114: *N*-(5-chloro-2-((5-chloro-2-methoxy-4-(4-(4-methylpiperazin-1-yl)piperidin-1-yl)phenyl)amino)pyrimidin-4-yl)-N (4-(methylsulfonamido)benzo[d]thiazol-5-yl)methanesulfonamide;
Compound No. 115: *N*-(6-((5-chloro-2-((2-methoxy-5-methyl-4-(4-(4-methylpiperazin-1-yl)piperidin-1-yl)phenyl)amino)pyrimidin-4-yl)amino)quinolin-5-yl)methanesulfonamide;
Compound No. 116: *N*-(6-((5-chloro-2-((2-methoxy-5-methyl-6-(4-(4-methylpiperazin-1-yl)piperidin-1-yl)pyridin-3-yl)amino)pyrimidin-4-yl)amino)quinolin-5-yl)methanesulfonamide;
Compound No. 117: *N*-(6-((5-chloro-2-((2-methoxy-6-(4-morpholinopiperidin-1-yl)pyridin-3-yl)amino)pyrimidin-4-yl)amino)quinolin-5-yl)methanesulfonamide;
Compound No. 118: *N*-(6-((5-chloro-2-((5-chloro-2-methoxy-4-(4-(4-methoxypiperazin-1-yl)piperidin-1-yl)phenyl)amino)pyrimidin-4-yl)amino)quinolin-5-yl)methanesulfonamide;
Compound No. 119: *N*-(6-((5-chloro-2-((2-methoxy-4-(4-methylpiperazin-1-yl)phenyl)amino)pyrimidin-4-yl)amino)quinolin-5-yl)methanesulfonamide;
Compound No. 120: *N*-(2-((5-chloro-2-((2-methoxy-5-methyl-4-(4-(4-methylpiperazin-1-yl)piperidin-1-yl)phenyl)amino)pyrimidin-4-yl)(methyl)amino)phenyl)methanesulfonamide;
Compound No. 121: *N*-(2-((5-chloro-2-((2-methoxy-5-methyl-6-(4-(4-methylpiperazin-1-yl)piperidin-1-yl)pyridin-3-yl)amino)pyrimidin-4-yl)(methyl)amino)phenyl)methanesulfonamide;
Compound No. 122: (6-((5-chloro-2-((2-methoxy-5-methyl-6-(4-(4-methylpiperazin-1-yl)piperidin-1-yl)pyridin-3-yl)amino)pyrimidin-4-yl)amino)quinoxalin-5-yl)dimethylphosphine oxide;
Compound No. 123: (6-((5-chloro-2-((2-methoxy-6-(4-(4-methylpiperazin-1-yl)piperidin-1-yl)pyridin-3-yl)amino)pyrimidin-4-yl)amino)quinoxalin-5-yl)dimethylphosphine oxide;
Compound No. 124: (6-((5-chloro-2-((5-fluoro-6-(4-(4-methylpiperazin-1-yl)piperidin-1-yl)pyridin-3-yl)amino)pyrimidin-4-yl)amino)quinoxalin-5-yl)dimethylphosphine oxide;
Compound No. 125: (6-((5-chloro-2-((3-fluoro-4-(4-(4-methylpiperazin-1-yl)piperidin-1-yl)phenyl)amino)pyrimidin-4-yl)amino)quinoxalin-5-yl)dimethylphosphine oxide;
Compound No. 126: (6-((5-chloro-2-((2-methoxy-6-((3S,5R)-3,4,5-trimethylpiperazin-1-yl)pyridin-3-yl)amino)pyrimidin-4-yl)amino)quinoxalin-5-yl)dimethylphosphine oxide;
Compound No. 127: (6-((5-chloro-2-((6-(3-(dimethylamino)pyrrolidin-1-yl)-2-methoxypyridin-3-yl)amino)pyrimidin-4-yl)amino)quinoxalin-5-yl)dimethylphosphine oxide;
Compound No. 128: (6-((5-chloro-2-((2-methoxy-6-(piperazin-1-yl)pyridin-3-yl)amino)pyrimidin-4-yl)amino)quinoxalin-5-yl)dimethylphosphine oxide;
Compound No. 129: 4-(5-((5-chloro-4-((5-(dimethylphosphoryl)quinoxalin-6-yl)amino)pyrimidin-2-yl)amino)-6-methoxypyridin-2-yl)thiomorpholine 1,1-dioxide;
Compound No. 130: (6-((5-bromo-2-((2-methoxy-5-methyl-4-(4-(4-methylpiperazin-1-yl)piperidin-1-yl)phenyl)amino)pyrimidin-4-yl)amino)quinoxalin-5-yl)dimethylphosphine oxide;
Compound No. 131: *N*-(6-((5-chloro-2-((2-methoxy-5-methyl-4-(4-(4-methylpiperazin-1-yl)piperidin-1-yl)phenyl)amino)pyrimidin-4-yl)amino)quinoxalin-5-yl)methanesulfonamide;
Compound No. 132: *N*-(6-((5-chloro-2-((2-methoxy-6-(4-(4-methylpiperazin-1-yl)piperidin-1-yl)pyridin-3-yl)amino)pyrimidin-4-yl)amino)quinoxalin-5-yl)methanesulfonamide;
Compound No. 133: *N*-(6-((5-chloro-2-((2-methoxy-4-(4-(4-methylpiperazin-1-yl)piperidin-1-yl)phenyl)amino)pyrimidin-4-yl)amino)quinoxalin-5-yl)methanesulfonamide;
Compound No. 134: *N*-(6-((5-chloro-2-((2-methoxy-5-methyl-6-(4-(4-methylpiperazin-1-yl)piperidin-1-yl)pyridin-3-yl)amino)pyrimidin-4-yl)amino)quinoxalin-5-yl)methanesulfonamide;
Compound No. 135: *N*-(6-((5-chloro-2-((2-methoxy-6-((3S,5R)-3,4,5-trimethylpiperazin-1-yl)pyridin-3-yl)amino)pyrimidin-4-yl)amino)quinoxalin-5-yl)methanesulfonamide;
Compound No. 136: *N*-(6-((5-chloro-2-((5-chloro-2-methoxy-4-(4-(4-methylpiperazin-1-yl)piperidin-1-yl)phenyl)amino)pyrimidin-4-yl)amino)quinoxalin-5-yl)methanesulfonamide;
Compound No. 137: *N*-(2-((5-chloro-2-((5-chloro-2-methoxy-4-(4-(4-methylpiperazin-1-yl)piperidin-1-yl)phenyl)amino)pyrimidin-4-yl)amino)-4-(trifluoromethyl)phenyl)methanesulfonamide;
Compound No. 138: *N*-(2-((5-chloro-2-((2-methoxy-5-methyl-4-(4-(4-methylpiperazin-1-yl)piperidin-1-yl)phenyl)amino)pyrimidin-4-yl)amino)-4-(trifluoromethyl)phenyl)methanesulfonamide;
Compound No. 139: *N*-(2-((5-chloro-2-((4-(4-(4-cyclopropylpiperazin-1-yl)piperidin-1-yl)-2-methoxy-5-methylphenyl)amino)pyrimidin-4-yl)amino)-4-(trifluoromethyl)phenyl)methanesulfonamide;
Compound No. 140: *N*-6-((5-chloro-2-((2-methoxy-5-methyl-4-(4-(4-methylpiperazin-1-yl)piperidin-1-yl)phenyl)amino)pyrimidin-4-yl)amino)-2,3-dihydrobenzofuran-5-yl)methanesulfonamide;
Compound No. 141: *N*-(2-((6-amino-5-chloro-2-((2-methoxy-5-methyl-4-(4-(4-methylpiperazin-1-yl)piperidin-1-yl)phenyl)amino)pyrimidin-4-yl)amino)-5-fluorophenyl)methanesulfonamide;
Compound No. 142: ((2-((5-bromo-2-((2-methoxy-5-methyl-4-(4-(4-methylpiperazin-1-yl)piperidin-1-yl)phenyl)amino)pyrimidin-4-yl)amino)phenyl)imino)dimethyl-*λ*⁶-sulfanone;
Compound No. 143: ((2-((5-bromo-2-((5-chloro-2-methoxy-4-(4-(4-methylpiperazin-1-yl)piperidin-1-yl)phenyl)amino)pyrimidin-4-yl)amino)phenyl)imino)dimethyl-*λ*⁶-sulfanone;
Compound No. 144: ((2-((5-bromo-2-((2-chloro-5-methyl-4-(4-(4-methylpiperazin-1-yl)piperidin-1-yl)phenyl)amino)pyrimidin-4-yl)amino)phenyl)imino)dimethyl-*λ*⁶-sulfanone;
Compound No. 145: *N*-(2-((5-bromo-2-((2-methoxy-6-(1-methylazepan-4-yl)pyridin-3-yl)amino)pyrimidin-4-yl)amino)-5-fluorophenyl)methanesulfonamide;
Compound No. 146: *N*-(2-((5-bromo-2-((5-fluoro-2-methoxy-4-(4-(4-methylpiperazin-1-yl)piperidin-1-yl)phenyl)amino)pyrimidin-4-yl)amino)-5-fluorophenyl)methanesulfonamide;
Compound No. 147: *N*-(2-((5-bromo-2-((2-methoxy-6-(4-(4-methylpiperazin-1-yl)piperidin-1-yl)pyridin-3-yl)amino)pyrimidin-4-yl)amino)-5-fluorophenyl)methanesulfonamide;
Compound No. 148: *N*-(2-((5-bromo-2-((2-methoxy-5-methyl-4-(4-(trifluoromethyl)-[1,4'-bipiperidin]-1'-yl)phenyl)amino)pyrimidin-4-yl)amino)-5-fluorophenyl)methanesulfonamide;
Compound No. 149: *N*-(2-((5-bromo-2-((5-chloro-4-(3-(dimethylamino)-[1,4'-bipiperidin]-1'-yl)-2-methoxyphenyl)amino)pyrimidin-4-yl)amino)-5-fluorophenyl)methanesulfonamide;
Compound No. 150: *N*-(2-((5-bromo-2-((2-methoxy-5-methyl-4-(4-(trifluoromethyl)-[1,4'-bipiperidin]-1'-yl)phenyl)amino)pyrimidin-4-yl)amino)-4-chlorophenyl)methanesulfonamide;
Compound No. 151: *N*-(4-chloro-2-((5-chloro-2-((5-chloro-2-methoxy-4-(4-(4-methylpiperazin-1-yl)piperidin-1-yl)phenyl)amino)pyrimidin-4-yl)amino)phenyl)methanesulfonamide;
Compound No. 152: *N*-(4-chloro-2-((6-chloro-3-((-2-methoxy-5-methyl-4-(4-(4-methylpiperazin-1-yl)piperidin-1-yl)phenyl)amino)-1,2,4-triazin-5-yl)amino)phenyl)methanesulfonamide;
Compound No. 153: (4-chloro-2-((5-chloro-2-((2-methoxy-5-methyl-4-(4-(4-methylpiperazin-1-yl)piperidin-1-yl)phenyl)amino)pyrimidin-4-yl)amino)phenyl)dimethylphosphine oxide;
Compound No. 154: (4-chloro-2-((5-chloro-2-((2-chloro-5-methyl-4-(4-(4-methylpiperazin-1-yl)piperidin-1-yl)phenyl)amino)pyrimidin-4-yl)amino)phenyl)dimethylphosphine oxide;
Compound No. 155: *N*-(2-((5-bromo-2-((2-methoxy-5-methyl-4-(4-(4-methylpiperazin-1-yl)piperidin-1-yl)phenyl)amino)pyrimidin-4-yl)amino)-4-chlorophenyl)-*N*-methylmethanesulfonamide;
Compound No. 156: *N*-(2-((5-bromo-2-((5-chloro-2-methoxy-4-(4-(4-methylpiperazin-1-yl)piperidin-1-yl)phenyl)amino)pyrimidin-4-yl)amino)-4-chlorophenyl)-*N*-methylmethanesulfonamide;
Compound No. 157: *N*-(4-chloro-2-((5-chloro-2-((2-chloro-4-(4-(3-(dimethylamino)pyrrolidin-1-yl)piperidin-1-yl)-5-methylphenyl)amino)pyrimidin-4-yl)amino)phenyl)methanesulfonamide;
Compound No. 158: *N*-(4-chloro-2-((5-chloro-2-((2-chloro-5-methyl-4-(4-(4-methylpiperazin-1-yl)piperidin-1-yl)phenyl)amino)pyrimidin-4-yl)amino)phenyl)methanesulfonamide;
Compound No. 159: *N*-(4-chloro-2-((5-chloro-2-((5-chloro-2-methoxy-4-(4-(4-methylpiperazin-1-yl)piperidin-1-yl)phenyl)amino)pyrimidin-4-yl)amino)phenyl)methanesulfonamide;
Compound No. 160: *N*-(2-((5-chloro-2-((2-methoxy-5-methyl-4-(4-(4-methylpiperazin-1-yl)piperidin-1-yl)phenyl)amino)pyrimidin-4-yl)amino)-4-isopropylphenyl)methanesulfonamide;
Compound No. 161: *N*-(2-((5-chloro-2-((5-chloro-2-methoxy-4-(4-(4-methylpiperazin-1-yl)piperidin-1-yl)phenyl)amino)pyrimidin-4-yl)amino)-4-isopropylphenyl)methanesulfonamide;
Compound No. 162: *N*-(4-chloro-2-((5-chloro-2-((2-chloro-5-methyl-4-(4-(4-methyl-1,4-diazepan-1-yl)piperidin-1-yl)phenyl)amino)pyrimidin-4-yl)amino)phenyl)methanesulfonamide;
Compound No. 163: *N*-(4-chloro-2-((5-chloro-2-((5-chloro-2-methoxy-4-(4-(4-methyl-1,4-diazepan-1 -yl)piperidin-1 -yl)phenyl)amino)pyrimidin-4-yl)amino)phenyl)methanesulfonamide;
Compound No. 164: *N*-(2-((6-chloro-3-((2-methoxy-5-methyl-4-(4-(4-methylpiperazin-1-yl)piperidin-1-yl)phenyl)amino)-1,2,4-triazin-5-yl)amino)-5-fluorophenyl)methanesulfonamide;
Compound No. 165: *N*-(6-((5-chloro-2-((2-methoxy-5-methyl-4-(4-(4-methyl-1,4-diazepan-1-yl)piperidin-1-yl)phenyl)amino)pyrimidin-4-yl)amino)-2,3-dihydrobenzofuran-5-yl)methanesulfonamide;
Compound No. 166: 5-chloro-*N*⁴-(5-chloro-2-(1*H*-1,2,3-triazol-4-yl)phenyl)-*N*²-(2-methoxy-5-methyl-4-(4-(4-methylpiperazin-1-yl)piperidin-1-yl)phenyl)pyrimidine-2,4-diamine;
Compound No. 167: *N*-(4-chloro-2-((5-chloro-2-((1-(3-(dimethylamino)propyl)-1*H*-pyrazol-4-yl)amino)pyrimidin-4-yl)amino)phenyl)methanesulfonamide;
Compound No. 168: *N*-(6-((5-chloro-2-((5-chloro-2-methoxy-4-(4-(4-methylpiperazin-1-yl)piperidin-1-yl)phenyl)amino)pyrimidin-4-yl)amino)-2,3-dihydrobenzofuran-5-yl)methanesulfonamide;
Compound No. 169: *N*-(6-((5-bromo-2-((2-methoxy-5-methyl-4-(4-(4-methylpiperazin-1-yl)piperidin-1-yl)phenyl)amino)pyrimidin-4-yl)amino)-2,3-dihydrobenzofuran-5-yl)methanesulfonamide;
Compound No. 170: *N*-(4-chloro-2-((5-chloro-2-((5-chloro-2-methoxy-4-(4-(4-methylpiperazin-1-yl)piperidin-1-yl)phenyl)amino)pyrimidin-4-yl)amino)phenyl)-*N-*methylmethanesulfonamide;
Compound No. 171: *N*-(4-chloro-2-((5-chloro-2-((2-chloro-5-methyl-4-(4-(4-methylpiperazin-1-yl)piperidin-1-yl)phenyl)amino)pyrimidin-4-yl)amino)phenyl)-*N-*methylmethanesulfonamide;
Compound No. 172: *N*-(6-((5-chloro-2-((2-chloro-5-methyl-4-(4-(4-methyl-1,4-diazepan-1-yl)piperidin-1-yl)phenyl)amino)pyrimidin-4-yl)amino)-2,3-dihydrobenzofuran-5-yl)methanesulfonamide;
Compound No. 173: *N*-(6-((5-chloro-2-((2-chloro-5-methyl-4-(4-(4-methylpiperazin-1-yl)piperidin-1-yl)phenyl)amino)pyrimidin-4-yl)amino)-2,3-dihydrobenzofuran-5-yl)methanesulfonamide;
Compound No. 174: *N*-(6-((5-chloro-2-((5-chloro-2-methoxy-4-(4-(4-methyl-1,4-diazepan-1-yl)piperidin-1-yl)phenyl)amino)pyrimidin-4-yl)amino)-2,3-dihydrobenzofuran-5-yl)methanesulfonamide;
Compound No. 175: *N*-(6-((5-bromo-2-((2-chloro-5-methyl-4-(4-(4-methylpiperazin-1-yl)piperidin-1-yl)phenyl)amino)pyrimidin-4-yl)amino)-2,3-dihydrobenzofuran-5-yl)methanesulfonamide;
Compound No. 176: *N*-(4-chloro-2-((5-chloro-2-((2-methoxy-5-methyl-4-(4-(4-methylpiperazin-1-yl)piperidin-1-yl)phenyl)amino)pyrimidin-4-yl)amino)-5-fluorophenyl)methanesulfonamide;
Compound No. 177: *N*-(2-((5-bromo-2-((2-chloro-5-methyl-4-(4-(4-methylpiperazin-1-yl)piperidin-1-yl)phenyl)amino)pyrimidin-4-yl)amino)-4-chloro-5-fluorophenyl)methanesulfonamide;
Compound No. 178: *N*-(2-((5-bromo-2-((5-chloro-2-methoxy-4-(4-(4-methylpiperazin-1-yl)piperidin-1-yl)phenyl)amino)pyrimidin-4-yl)amino)-4-chloro-5-fluorophenyl)methanesulfonamide;
Compound No. 179: *N*-(4-chloro-2-((5-chloro-2-((2-chloro-4-(4-(3-(dimethylamino)pyrrolidin-1-yl)piperidin-1-yl)-5-methylphenyl)amino)pyrimidin-4-yl)amino)phenyl)-*N*-methylmethanesulfonamide;
Compound No. 180: *N*-(4-chloro-2-((5-chloro-2-((2-chloro-5-methyl-4-(4-(4-methyl-1,4-diazepan-1-yl)piperidin-1-yl)phenyl)amino)pyrimidin-4-yl)amino)phenyl)-*N*-methylmethanesulfonamide;
Compound No. 181: *N*-(4-chloro-2-((5-chloro-2-((5-chloro-2-methoxy-4-(4-(4-methylpiperazin-1-yl)piperidin-1-yl)phenyl)amino)pyrimidin-4-yl)amino)-5-fluorophenyl)methanesulfonamide;
Compound No. 182: *N*-(4-chloro-2-((5-chloro-2-((2-chloro-5-methyl-4-(4-(4-methylpiperazin-1-yl)piperidin-1-yl)phenyl)amino)pyrimidin-4-yl)amino)-5-fluorophenyl)methanesulfonamide;
Compound No. 183: *N*-(6-((5-bromo-2-((5-chloro-2-methoxy-4-(4-(4-methylpiperazin-1-yl)piperidin-1-yl)phenyl)amino)pyrimidin-4-yl)amino)-2,3-dihydrobenzofuran-5-yl)methanesulfonamide;
Compound No. 184: *N*-(6-((5-bromo-2-((5-ethyl-2-methoxy-4-(4-(4-methylpiperazin-1-yl)piperidin-1-yl)phenyl)amino)pyrimidin-4-yl)amino)-2,3-dihydrobenzofuran-5-yl)methanesulfonamide;
Compound No. 185: 5-chloro-*N*⁴-(5-chloro-2-(1*H*-1,2,3-triazol-5-yl)phenyl)-*N*²-(2-chloro-5-methyl-4-(4-(4-methylpiperazin-1-yl)piperidin-1-yl)phenyl)pyrimidine-2,4-diamine;
Compound No. 186: 5-chloro-*N*⁴-(5-chloro-2-(1*H*-1,2,3-triazol-5-yl)phenyl)-*N*²-(5-chloro-2-methoxy-4-(4-(4-methylpiperazin-1-yl)piperidin-1-yl)phenyl)pyrimidine-2,4-diamine;
Compound No. 187: 5-bromo-*N*⁴-(5-chloro-2-(1*H*-1,2,3-triazol-5-yl)phenyl)-*N*²-(2-methoxy-5-methyl-4-(4-(4-methylpiperazin-1-yl)piperidin-1-yl)phenyl)pyrimidine-2,4-diamine;
Compound No. 188: *N*-(6-((5-bromo-2-((2-methoxy-5-methyl-4-(4-(4-methyl-1,4-diazepan-1-yl)piperidin-1-yl)phenyl)amino)pyrimidin-4-yl)amino)-2,3-dihydrobenzofuran-5-yl)methanesulfonamide;
Compound No. 189: *N*-(6-((5-bromo-2-((5-chloro-2-methoxy-4-(4-(4-methyl-1,4-diazepan-1-yl)piperidin-1-yl)phenyl)amino)pyrimidin-4-yl)amino)-2,3-dihydrobenzofuran-5-yl)methanesulfonamide;
Compound No. 190: (6-((5-chloro-2-((5-ethyl-2-methoxy-4-(4-(4-methylpiperazin-1-yl)piperidin-1-yl)phenyl)amino)pyrimidin-4-yl)amino)quinoxalin-5-yl)dimethylphosphine oxide;
Compound No. 191: *N*-(2-((5-bromo-2-((5-ethyl-2-methoxy-4-(4-(4-methylpiperazin-1-yl)piperidin-1-yl)phenyl)amino)pyrimidin-4-yl)amino)-4-chlorophenyl)methanesulfonamide;
Compound No. 192: (6-((5-bromo-2-((5-ethyl-2-methoxy-4-(4-(4-methylpiperazin-1-yl)piperidin-1-yl)phenyl)amino)pyrimidin-4-yl)amino)quinoxalin-5-yl)dimethylphosphine oxide;
Compound No. 193: (6-((5-bromo-2-((2-chloro-5-methyl-4-(4-(4-methylpiperazin-1-yl)piperidin-1-yl)phenyl)amino)pyrimidin-4-yl)amino)quinoxalin-5-yl)dimethylphosphine oxide;
Compound No. 194: (6-((5-bromo-2-((5-chloro-2-methoxy-4-(4-(4-methylpiperazin-1-yl)piperidin-1-yl)phenyl)amino)pyrimidin-4-yl)amino)quinoxalin-5-yl)dimethylphosphine oxide;
Compound No. 195: 5-bromo-*N*⁴-(5-chloro-2-(1*H*-1,2,3-triazol-5-yl)phenyl)-*N*²-(5-chloro-2-methoxy-4-(4-(4-methylpiperazin-1-yl)piperidin-1-yl)phenyl)pyrimidine-2,4-diamine;
Compound No. 196: *N*-(6-((5-bromo-2-((4-(3-(dimethylamino)-[1,4'-bipiperidin]-1'-yl)-2-methoxy-5-methylphenyl)amino)pyrimidin-4-yl)amino)-2,3-dihydrobenzofuran-5-yl)methanesulfonamide;
Compound No. 197: *N*-(6-((5-chloro-2-((2-chloro-4-(3-(dimethylamino)-[1,4'-bipiperidin]-1'-yl)-5-methylphenyl)amino)pyrimidin-4-yl)amino)-2,3-dihydrobenzofuran-5-yl)methanesulfonamide;
Compound No. 198: *N*-(4-chloro-2-((5-chloro-2-((2,5-dichloro-4-(4-(4-methylpiperazin-1-yl)piperidin-1-yl)phenyl)amino)pyrimidin-4-yl)amino)phenyl)methanesulfonamide;
Compound No. 199: *N*-(2-((5-chloro-2-((2-methoxy-5-methyl-4-(4-(4-methylpiperazin-1-yl)piperidin-1-yl)phenyl)amino)pyrimidin-4-yl)amino)-4-methoxyphenyl)methanesulfonamide;
Compound No. 200: *N*-(2-((5-chloro-2-((2-chloro-5-methyl-4-(4-(4-methylpiperazin-1-yl)piperidin-1-yl)phenyl)amino)pyrimidin-4-yl)amino)-4-methoxyphenyl)methanesulfonamide;
Compound No. 201: *N*-2-((5-chloro-2-((5-chloro-2-methoxy-4-(4-(4-methylpiperazin-1-yl)piperidin-1-yl)phenyl)amino)pyrimidin-4-yl)amino)-4-methoxyphenyl)methanesulfonamide;
Compound No. 202: *N*-(2-((5-chloro-2-((2,5-dichloro-4-(4-(4-methylpiperazin-1-yl)piperidin-1-yl)phenyl)amino)pyrimidin-4-yl)amino)-4-methoxyphenyl)methanesulfonamide;
Compound No. 203: *N*-(2-((5-chloro-2-((5-ethyl-2-methoxy-4-(4-(4-methylpiperazin-1-yl)piperidin-1-yl)phenyl)amino)pyrimidin-4-yl)amino)-4-methoxyphenyl)methanesulfonamide;
Compound No. 204: *N*-(2-((5-chloro-2-((2-methoxy-5-methyl-4-(4-(4-methyl-1,4-diazepan-1-yl)piperidin-1-yl)phenyl)amino)pyrimidin-4-yl)amino)-4-methoxyphenyl)methanesulfonamide;
Compound No. 205: *N*-(6-((5-chloro-2-((2,5-dichloro-4-(4-(4-methylpiperazin-1-yl)piperidin-1-yl)phenyl)amino)pyrimidin-4-yl)amino)-2,3-dihydrobenzofuran-5-yl)methanesulfonamide;
Compound No. 206: *N*-(6-((5-chloro-2-((5-ethyl-2-methoxy-4-(4-(4-methylpiperazin-1-yl)piperidin-1-yl)phenyl)amino)pyrimidin-4-yl)amino)-2,3-dihydrobenzofuran-5-yl)methanesulfonamide;
Compound No. 207: *N*-(6-((5-chloro-2-((2-methoxy-5-methyl-4-(4-(piperazin-1-yl)piperidin-1-yl)phenyl)amino)pyrimidin-4-yl)amino)-2,3-dihydrobenzofuran-5-yl)methanesulfonamide;
Compound No. 208: *N*-(6-((5-chloro-2-((4-(4-(4-cyclopropylpiperazin-1-yl)piperidin-1-yl)-2-methoxy-5-methylphenyl)amino)pyrimidin-4-yl)amino)-2,3-dihydrobenzofuran-5-yl)methanesulfonamide;
Compound No. 209: *N*-(6-((5-chloro-2-((4-(4-(3-(dimethylamino)pyrrolidin-1-yl)piperidin-1-yl)-2-methoxy-5-methylphenyl)amino)pyrimidin-4-yl)amino)-2,3-dihydrobenzofuran-5-yl)methanesulfonamide;
Compound No. 210: *N*-(6-((5-chloro-2-((2-chloro-4-(4-(3-(dimethylamino)pyrrolidin-1 -yl)piperidin-1 -yl)-5-methylphenyl)amino)pyrimidin-4-yl)amino)-2,3-dihydrobenzofuran-5-yl)methanesulfonamide;
Compound No. 211: *N*-(6-((5-bromo-2-((4-(4-(3-(dimethylamino)pyrrolidin-1-yl)piperidin-1-yl)-2-methoxy-5-methylphenyl)amino)pyrimidin-4-yl)amino)-2,3-dihydrobenzofuran-5-yl)methanesulfonamide;
Compound No. 212: *N*-(6-((5-bromo-2-((4-(4-(4-cyclopropylpiperazin-1-yl)piperidin-1-yl)-2-methoxy-5-methylphenyl)amino)pyrimidin-4-yl)amino)-2,3-dihydrobenzofuran-5-yl)methanesulfonamide;
Compound No. 213: *N*-(6-((5-bromo-2-((2-chloro-4-(4-(3-(dimethylamino)pyrrolidin-1 -yl)piperidin-1 -yl)-5-methylphenyl)amino)pyrimidin-4-yl)amino)-2,3-dihydrobenzofuran-5-yl)methanesulfonamide;
Compound No. 214: *N*-(6-((5-bromo-2-((2-chloro-4-(3-(dimethylamino)-[1,4'-bipiperidin]-1'-yl)-5-methylphenyl)amino)pyrimidin-4-yl)amino)-2,3-dihydrobenzofuran-5-yl)methanesulfonamide;
Compound No. 215: *N*-6-((5-bromo-2-((2-methoxy-5-methyl-4-(4-(piperazin-1-yl)piperidin-1-yl)phenyl)amino)pyrimidin-4-yl)amino)-2,3-dihydrobenzofuran-5-yl)methanesulfonamide;
Compound No. 216: *N*-(6-((5-bromo-2-((5-chloro-4-(3-(dimethylamino)-[1,4'-bipiperidin]-1'-yl)-2-methoxyphenyl)amino)pyrimidin-4-yl)amino)-2,3-dihydrobenzofuran-5-yl)methanesulfonamide;
Compound No. 217: *N*-(6-((5-bromo-2-((2,5-dichloro-4-(4-(4-methylpiperazin-1-yl)piperidin-1-yl)phenyl)amino)pyrimidin-4-yl)amino)-2,3-dihydrobenzofuran-5-yl)methanesulfonamide;
Compound No. 218: *N*-(6-((5-chloro-2-((4-(3-(dimethylamino)-[1,4'-bipiperidin]-1'-yl)-2-methoxy-5-methylphenyl)amino)pyrimidin-4-yl)amino)-2,3-dihydrobenzofuran-5-yl)methanesulfonamide;
Compound No. 219: *N*-(6-((5-chloro-2-((5-chloro-4-(3-(dimethylamino)-[1,4'-bipiperidin]-1'-yl)-2-methoxyphenyl)amino)pyrimidin-4-yl)amino)-2,3-dihydrobenzofuran-5-yl)methanesulfonamide;
Compound No. 220: *N*-(6-((5-chloro-2-((2-methoxy-4-(4-(4-methylpiperazin-1-yl)piperidin-1-yl)phenyl)amino)pyrimidin-4-yl)amino)-2,3-dihydrobenzofuran-5-yl)methanesulfonamide;
Compound No. 221: *N*-(6-((5-chloro-2-((2-chloro-4-(4-(4-methylpiperazin-1-yl)piperidin-1-yl)phenyl)amino)pyrimidin-4-yl)amino)-2,3-dihydrobenzofuran-5-yl)methanesulfonamide;
Compound No. 222: *N*-(6-((5-chloro-2-((5-fluoro-2-methoxy-4-(4-(4-methylpiperazin-1-yl)piperidin-1-yl)phenyl)amino)pyrimidin-4-yl)amino)-2,3-dihydrobenzofuran-5-yl)methanesulfonamide;
Compound No. 223: *N*-(6-((5-chloro-2-((2-(4-(4-methylpiperazin-1-yl)piperidin-1-yl)quinolin-6-yl)amino)pyrimidin-4-yl)amino)-2,3-dihydrobenzofuran-5-yl)methanesulfonamide;
Compound No. 224: *N*-(6-((5-chloro-2-((2-methoxy-5-methyl-4-(4-((1-methyl-1H-pyrazol-5-yl)amino)piperidin-1-yl)phenyl)amino)pyrimidin-4-yl)amino)-2,3-dihydrobenzofuran-5-yl)methanesulfonamide;
Compound No. 225: *N*-(6-((5-chloro-2-((2-methoxy-5-methyl-4-(4-(4-(trifluoromethyl)piperazin-1-yl)piperidin-1-yl)phenyl)amino)pyrimidin-4-yl)amino)-2,3-dihydrobenzofuran-5-yl)methanesulfonamide;
Compound No. 226: *N*-(4-chloro-2-((5-chloro-2-((2,5-dichloro-4-(4-(4-methylpiperazin-1-yl)piperidin-1-yl)phenyl)amino)pyrimidin-4-yl)amino)phenyl)-*N-*methylmethanesulfonamide;
Compound No. 227: *N*-(2-((5-bromo-2-((2-chloro-4-(4-(3-(dimethylamino)pyrrolidin-1-yl)piperidin-1-yl)-5-methylphenyl)amino)pyrimidin-4-yl)amino)-4-chlorophenyl)-*N*-methylmethanesulfonamide;
Compound No. 228: *N*-(2-((5-bromo-2-((2,5-dichloro-4-(4-(4-methylpiperazin-1-yl)piperidin-1-yl)phenyl)amino)pyrimidin-4-yl)amino)-4-chlorophenyl)-*N-*methylmethanesulfonamide;
Compound No. 229: *N*-(2-((5-chloro-2-((5-chloro-2-methoxy-4-(4-(4-methyl-1,4-diazepan-1-yl)piperidin-1-yl)phenyl)amino)pyrimidin-4-yl)amino)-4-methoxyphenyl)methanesulfonamide;
Compound No. 230: *N*-(2-((5-chloro-2-((2-chloro-4-(4-(3-(dimethylamino)pyrrolidin-1-yl)piperidin-1-yl)-5-methylphenyl)amino)pyrimidin-4-yl)amino)-4-methoxyphenyl)methanesulfonamide;
Compound No. 231: *N*-(2-((5-chloro-2-((2-methoxy-4-(4-(4-methylpiperazin-1-yl)piperidin-1-yl)phenyl)amino)pyrimidin-4-yl)amino)-4-methoxyphenyl)methanesulfonamide;
Compound No. 232: *N*-(2-((5-chloro-2-((4-(3-(dimethylamino)-[1,4'-bipiperidin]-1'-yl)-2-methoxy-5-methylphenyl)amino)pyrimidin-4-yl)amino)-4-methoxyphenyl)methanesulfonamide;
Compound No. 233: *N*-(2-((5-chloro-2-((4-(4-(4-cyclopropylpiperazin-1-yl)piperidin-1-yl)-2-methoxy-5-methylphenyl)amino)pyrimidin-4-yl)amino)-4-methoxyphenyl)methanesulfonamide;
Compound No. 234: *N*-(2-((5-chloro-2-((2-methoxy-5-methyl-4-(4-(trifluoromethyl)-[1,4'-bipiperidin]-1'-yl)phenyl)amino)pyrimidin-4-yl)amino)-4-methoxyphenyl)methanesulfonamide;
Compound No. 235: *N*-(2-((5-chloro-2-((4-(4-(3-(dimethylamino)pyrrolidin-1-yl)piperidin-1-yl)-2-methoxy-5-methylphenyl)amino)pyrimidin-4-yl)amino)-4-methoxyphenyl)methanesulfonamide;
Compound No. 236: *N*-(2-((5-chloro-2-((2-chloro-4-(3-(dimethylamino)-[1,4'-bipiperidin]-1'-yl)-5-methylphenyl)amino)pyrimidin-4-yl)amino)-4-methoxyphenyl)methanesulfonamide;
Compound No. 237: *N*-(2-((5-chloro-2-((5-chloro-4-(3-(dimethylamino)-[1,4'-bipiperidin]-1'-yl)-2-methoxyphenyl)amino)pyrimidin-4-yl)amino)-4-methoxyphenyl)methanesulfonamide;
Compound No. 238: *N*-6-((5-bromo-2-((2-methoxy-6-(4-methyl-1,4-diazepan-1-yl)pyridin-3-yl)amino)pyrimidin-4-yl)amino)-2,3-dihydrobenzofuran-5-yl)methanesulfonamide;
Compound No. 239: *N*-(6-((5-bromo-2-((5-fluoro-6-(4-(4-methylpiperazin-1-yl)piperidin-1-yl)pyridin-3-yl)amino)pyrimidin-4-yl)amino)-2,3-dihydrobenzofuran-5-yl)methanesulfonamide;
Compound No. 240: *N*-(6-((5-bromo-2-((2-methoxy-5-methyl-6-(4-(4-methylpiperazin-1-yl)piperidin-1-yl)pyridin-3-yl)amino)pyrimidin-4-yl)amino)-2,3-dihydrobenzofuran-5-yl)methanesulfonamide;
Compound No. 241: *N*-(6-((5-chloro-2-((2-methoxy-5-methyl-6-(4-(4-methylpiperazin-1-yl)piperidin-1-yl)pyridin-3-yl)amino)pyrimidin-4-yl)amino)-2,3-dihydrobenzofuran-5-yl)methanesulfonamide;
Compound No. 242: *N*-(6-((5-chloro-2-((5-fluoro-6-(4-(4-methylpiperazin-1-yl)piperidin-1-yl)pyridin-3-yl)amino)pyrimidin-4-yl)amino)-2,3-dihydrobenzofuran-5-yl)methanesulfonamide;
Compound No. 243: *N*-(6-((5-bromo-2-((2-methoxy-4-(4-(4-methylpiperazin-1-yl)piperidin-1-yl)phenyl)amino)pyrimidin-4-yl)amino)-2,3-dihydrobenzofuran-5-yl)methanesulfonamide;
Compound No. 244: *N*-(6-((5-bromo-2-((3-fluoro-4-(4-(4-methylpiperazin-1-yl)piperidin-1-yl)phenyl)amino)pyrimidin-4-yl)amino)-2,3-dihydrobenzofuran-5-yl)methanesulfonamide;
Compound No. 245: *N*-(6-((5-bromo-2-((2-methoxy-5-methyl-4-(4-(trifluoromethyl)-[1,4'-bipiperidin]-1'-yl)phenyl)amino)pyrimidin-4-yl)amino)-2,3-dihydrobenzofuran-5-yl)methanesulfonamide;
Compound No. 246: *N*-(6-((5-bromo-2-((2-chloro-4-(4-(4-methylpiperazin-1-yl)piperidin-1-yl)phenyl)amino)pyrimidin-4-yl)amino)-2,3-dihydrobenzofuran-5-yl)methanesulfonylamide;
Compound No. 247: *N*-(6-((5-bromo-2-((2-(4-(4-methylpiperazin-1-yl)piperidin-1-yl)quinolin-6-yl)amino)pyrimidin-4-yl)amino)-2,3-dihydrobenzofuran-5-yl)methanesulfonamide;
Compound No. 248: *N*-(6-((5-chloro-2-((2-methoxy-5-methyl-4-(4-(4-methylpiperazin-1-yl)piperidin-1-yl)phenyl)amino)pyrimidin-4-yl)amino)-2,3-dihydrobenzofuran-5-yl)-*N*-methylmethanesulfonamide;
Compound No. 249: *N*-6-((5-chloro-2-((5-chloro-2-methoxy-4-(4-(4-methylpiperazin-1-yl)piperidin-1-yl)phenyl)amino)pyrimidin-4-yl)amino)-2,3-dihydrobenzofuran-5-yl)-*N*-methylmethanesulfonamide;
Compound No. 250: *N*-(6-((5-bromo-2-((2-methoxy-5-methyl-4-(4-(4-methylpiperazin-1-yl)piperidin-1-yl)phenyl)amino)pyrimidin-4-yl)amino)-2,3-dihydrobenzofuran-5-yl)-*N*-methylmethanesulfonamide;
Compound No. 251: *N*-(6-((5-bromo-2-((2-chloro-5-methyl-4-(4-(4-methylpiperazin-1-yl)piperidin-1-yl)phenyl)amino)pyrimidin-4-yl)amino)-2,3-dihydrobenzofuran-5-yl)-*N*-methylmethanesulfonamide;
Compound No. 252: *N*-(6-((5-bromo-2-((5-chloro-2-methoxy-4-(4-(4-methylpiperazin-1-yl)piperidin-1-yl)phenyl)amino)pyrimidin-4-yl)amino)-2,3-dihydrobenzofuran-5-yl)-*N*-methylmethanesulfonamide;
Compound No. 253: *N*-(6-((5-bromo-2-((2,5-dichloro-4-(4-(4-methylpiperazin-1-yl)piperidin-1-yl)phenyl)amino)pyrimidin-4-yl)amino)-2,3-dihydrobenzofuran-5-yl)-*N*-methylmethanesulfonamide;
Compound No. 254: *N*-(6-((5-bromo-2-((5-ethyl-2-methoxy-4-(4-(4-methylpiperazin-1-yl)piperidin-1-yl)phenyl)amino)pyrimidin-4-yl)amino)-2,3-dihydrobenzofuran-5-yl)-*N*-methylmethanesulfonamide;
Compound No. 255: *N*-(6-((5-bromo-2-((2-methoxy-5-methyl-4-(4-(4-(trifluoromethyl)piperazin-1-yl)piperidin-1-yl)phenyl)amino)pyrimidin-4-yl)amino)-2,3-dihydrobenzofuran-5-yl)-*N*-methylmethanesulfonamide;
Compound No. 256: *N*-(6-((5-bromo-2-((5-fluoro-2-methoxy-4-(4-(4-methylpiperazin-1-yl)piperidin-1-yl)phenyl)amino)pyrimidin-4-yl)amino)-2,3-dihydrobenzofuran-5-yl)-*N*-methylmethanesulfonamide;
Compound No. 257: *N*-(6-((5-bromo-2-((5-fluoro-2-methoxy-4-(4-(4-methyl-1,4-diazepan-1-yl)piperidin-1-yl)phenyl)amino)pyrimidin-4-yl)amino)-2,3-dihydrobenzofuran-5-yl)-*N*-methylmethanesulfonamide;
Compound No. 258: *N*-(6-((5-bromo-2-((4-(3-(dimethylamino)-[1,4'-bipiperidin]-1'-yl)-2-methoxy-5-methoxyphenyl)amino)pyrimidin-4-yl)amino)-2,3-dihydrobenzofuran-5-yl)-*N*-methylmethanesulfonamide;
Compound No. 259: *N*-(6-((5-chloro-2-((2,5-dichloro-4-(4-(4-methylpiperazin-1-yl)piperidin-1-yl)phenyl)amino)pyrimidin-4-yl)amino)-2,3-dihydrobenzofuran-5-yl)-*N*-methylmethanesulfonamide;
Compound No. 260: *N*-(2-((5-chloro-2-((2-methoxy-5-methyl-4-(4-(4-methylpiperazin-1-yl)piperidin-1-yl)phenyl)amino)pyrimidin-4-yl)amino)-4-methoxyphenyl)-*N*-methylmethanesulfonamide;
Compound No. 261: *N*-(6-((5-bromo-2-((4-(4-(4-cyclopropylpiperazin-1-yl)piperidin-1-yl)-2-methoxy-5-methylphenyl)amino)pyrimidin-4-yl)amino)-2,3-dihydrobenzofuran-5-yl)-*N*-methylmethanesulfonamide;
Compound No. 262: *N*-(6-((5-bromo-2-((4-(4-(3-(dimethylamino)pyrrolidin-1-yl)piperidin-1-yl)-2-methoxy-5-methylphenyl)amino)pyrimidin-4-yl)amino)-2,3-dihydrobenzofuran-5-yl)-*N*-methylmethanesulfonamide;
Compound No. 263: *N*-(6-((5-chloro-2-((2-chloro-5-methyl-4-(4-(4-methylpiperazin-1-yl)piperidin-1-yl)phenyl)amino)pyrimidin-4-yl)amino)-2,3-dihydrobenzofuran-5-yl)-*N*-methylmethanesulfonamide;
Compound No. 264: *N*-(6-((5-chloro-2-((5-chloro-2-methoxy-4-(4-(4-methyl-1,4-diazepan-1-yl)piperidin-1-yl)phenyl)amino)pyrimidin-4-yl)amino)-2,3-dihydrobenzofuran-5-yl)-*N*-methylmethanesulfonamide;
Compound No. 265: *N*-(6-((5-chloro-2-((2-methoxy-5-methyl-4-(4-(4-methyl-1,4-diazepan-1-yl)piperidin-1-yl)phenyl)amino)pyrimidin-4-yl)amino)-2,3-dihydrobenzofuran-5-yl)-*N*-methylmethanesulfonamide;
Compound No. 266: *N*-(6-((5-chloro-2-((4-(4-(4-cyclopropylpiperazin-1-yl)piperidin-1-yl)-2-methoxy-5-methylphenyl)amino)pyrimidin-4-yl)amino)-2,3-dihydrobenzofuran-5-yl)-*N*-methylmethanesulfonamide;
Compound No. 267: *N*-(6-((5-chloro-2-((4-(3-(dimethylamino)-[1,4'-bipiperidin]-1'-yl)-2-methoxy-5-methylphenyl)amino)pyrimidin-4-yl)amino)-2,3-dihydrobenzofuran-5-yl)-*N*-methylmethanesulfonamide;
Compound No. 268: *N*-(6-((5-chloro-2-((2,5-difluoro-4-(4-(4-methylpiperazin-1-yl)piperidin-1-yl)phenyl)amino)pyrimidin-4-yl)amino)-2,3-dihydrobenzofuran-5-yl)methanesulfonamide;
Compound No. 269: *N*-(5-((5-bromo-2-((2,5-difluoro-4-(4-(4-methylpiperazin-1-yl)piperidin-1-yl)phenyl)amino)pyrimidin-4-yl)amino)-2,3-dihydrobenzofuran-4-yl)methanesulfonamide;
Compound No. 270: *N*-(6-((2-((5-bromo-2-methoxy-4-(4-(4-methylpiperazin-1-yl)piperidin-1-yl)phenyl)amino)-5-chloropyrimidin-4-yl)amino)-2,3-dihydrobenzofuran-5-yl)methanesulfonamide;
Compound No. 271: *N*-(6-((2-((5-bromo-2-methoxy-4-(4-(4-methylpiperazin-1-yl)piperidin-1-yl)phenyl)amino)-5-bromopyrimidin-4-yl)amino)-2,3-dihydrobenzofuran-5-yl)methanesulfonamide;
Compound No. 272: 5-chloro-*N*²-(2-methoxy-5-methyl-4-(4-(4-methylpiperazin-1-yl)piperidin-1-yl)phenyl)-*N*⁴-(1-methylindolin-6-yl)pyrimidine-2,4-diamine;
Compound No. 273: 5-chloro-*N*²-(5-chloro-2-methoxy-4-(4-(4-methylpiperazin-1-yl)piperidin-1-yl)phenyl)-*N*⁴-(1-methylindolin-6-yl)pyrimidine-2,4-diamine;
Compound No. 274: 5-chloro-*N*²-(2-chloro-5-methyl-4-(4-(4-methylpiperazin-1-yl)piperidin-1-yl)phenyl)-*N*⁴-(1-methylindolin-6-yl)pyrimidine-2,4-diamine;
Compound No. 275: 5-chloro-*N*²-(2-methoxy-5-methyl-4-(4-(4-methyl-1,4-diazepan-1-yl)piperidin-1-yl)phenyl)-*N*⁴-(1-methylindolin-6-yl)pyrimidine-2,4-diamine;
Compound No. 276: *N*-(6-((5-chloro-2-((2-methoxy-5-methyl-4-(4-(4-methylpiperazin-1-yl)piperidin-1-yl)phenyl)amino)pyrimidin-4-yl)amino)-1-methylindolin-5-yl)methanesulfonamide;
Compound No. 277: 5-chloro-*N*²-(2-chloro-5-methyl-4-(4-(4-methylpiperazin-1-yl)piperidin-1-yl)phenyl)-*N*⁴-(indolin-6-yl)pyrimidine-2,4-diamine;
Compound No. 278: 5-chloro-*N*²-(5-chloro-2-methoxy-4-(4-(4-methylpiperazin-1-yl)piperidin-1-yl)phenyl)-*N*⁴-(indolin-6-yl)pyrimidine-2,4-diamine;
Compound No. 279: 5-chloro-*N*⁴-(indolin-6-yl)-*N*²-(2-methoxy-4-(4-(4-methylpiperazin-1-yl)piperidin-1-yl)phenyl)pyrimidine-2,4-diamine;
Compound No. 280: *N*-(6-((2-((5-bromo-4-(3-(dimethylamino)-[1,4'-bipiperidin]-1'-yl)-2-methoxyphenyl)amino)-5-chloropyrimidin-4-yl)amino)-2,3-dihydrobenzofuran-5-yl)methanesulfonamide;
Compound No. 281: *N*-(6-((5-bromo-2-((5-bromo-4-(3-(dimethylamino)-[1,4'-bipiperidin]-1'-yl)-2-methoxyphenyl)amino)pyrimidin-4-yl)amino)-2,3-dihydrobenzofuran-5-yl)methanesulfonamide;
Compound No. 282: *N*-(6-((2-((5-bromo-2-methoxy-4-(4-(4-methyl-1,4-diazepan-1-yl)piperidin-1-yl)phenyl)amino)-5-chloropyrimidin-4-yl)amino)-2,3-dihydrobenzofuran-5-yl)methanesulfonamide; and
Compound No. 283: *N*-(6-((2-((5-bromo-4-(4-(3-(dimethylamino)pyrrolidin-1-yl)piperidin-1-yl)-2-methoxyphenyl)amino)-5-chloropyrimidin-4-yl)amino)-2,3-dihydrobenzofuran-5-yl)methanesulfonamide.

In one aspect of the present invention, the pharmaceutically acceptable salt is a salt of an inorganic or inorganic acid selected from the group consisting of hydrochloric acid, hydrobromic acid, sulfuric acid, phosphoric acid, nitric acid, acetic acid, glycolic acid, lactic acid, pyruvic acid, malonic acid, succinic acid, glutaric acid, fumaric acid, malic acid, mandelic acid, tartaric acid, citric acid, ascorbic acid, palmitic acid, maleic acid, hydroxymaleic acid, benzoic acid, hydroxybenzoic acid, phenylacetic acid, cinnamic acid, salicylic acid, methanesulfonic acid, benzenesulfonic acid, and toluenesulfonic acid.

A further aspect of the present invention provides a pharmaceutical composition for preventing, ameliorating or treating cancer including the compound according to any one of the aspects of the present invention as an active ingredient.

In another aspect of the present invention, the cancer is caused by an EGFR mutation.

In another aspect of the present invention, the pharmaceutical composition is applied to a patient with an EGFR mutation.

In another aspect of the present invention, the cancer is selected from the group consisting of glioblastoma, triple-negative breast cancer, colorectal cancer, lung cancer, head and neck cancer, and combinations thereof.

In another aspect of the present invention, the cancer is lung cancer.

In another aspect of the present invention, the pharmaceutical composition is administered to a patient with exon 19 deletion/T790M/C797S triple mutation or L858R/T790M/C797S triple mutation as an EGFR-related mutation.

In another aspect of the present invention, the pharmaceutical composition is administered to a patient with exon 19 deletion/T790M double mutation or L858R/T790M double mutation as an EGFR-related mutation.

In another aspect of the present invention, the pharmaceutical composition is administered to a patient with an exon 19 deletion mutation or L858R mutation as an EGFR-related mutation.

The pharmaceutical composition may be applied to laboratory animals such as mice, rabbits, rats, guinea pigs, and hamsters, and primates, including humans, preferably primates, including humans, more preferably humans, but is not limited thereto.

As used herein, the term "treat", "treating", and "treatment" is meant to include alleviation or amelioration of symptoms, diminishment of extent of disease, delay or slowing of disease progression, amelioration, palliation or stabilization of the disease state, partial or complete remission, prolonged survival or other beneficial therapeutic results.

As used herein, the treatment of cancer refers to the treatment of all cancer cells. The cancer also includes angiogenesis of endothelial cells and their mitosis (solid tumors, tumor metastasis, and benign tumors). Examples of cancers include, but are not limited to, breast cancer, ovarian cancer, cervical cancer, prostate cancer, testicular cancer, genitourinary tract cancer, esophageal cancer, laryngeal cancer, glioblastoma, stomach cancer, skin cancer, keratoacanthoma, lung cancer, squamous cell carcinoma, large cell carcinoma, small cell carcinoma, lung adenocarcinoma, bone cancer, colon cancer, adenoma, pancreatic cancer, adenocarcinoma, thyroid cancer, follicular carcinoma, undifferentiated cancer, papillary carcinoma, seminoma, melanoma, sarcoma, bladder cancer, liver cancer, biliary tract cancer, kidney cancer, myeloid diseases, lymphatic diseases, Hodgkin's disease, hairy cell cancer, oral cancer, pharynx cancer, lip cancer, tongue cancer, small intestine cancer, colon-rectal cancer, colorectal cancer, rectal cancer, brain cancer, central nervous system cancer, leukemia, hemangioma, trachoma, and pyogenic granuloma.

The content of the compound represented by Formula 10, 11 or 12, pharmaceutically acceptable salt thereof or hydrate thereof as an active ingredient can be appropriately adjusted according to the choice of those skilled in the art depending on the purpose and method of use of the pharmaceutical composition according to the present invention.

For example, the pharmaceutical composition may include 0.1 to 10% by weight, preferably 0.5 to 5% by weight of the compound represented by Formula 10, 11 or 12, pharmaceutically acceptable salt thereof or hydrate thereof, based on its total weight.

The compound represented by Formula 10, 11 or 12, pharmaceutically acceptable salt thereof or hydrate thereof may be present alone or together with one or more other pharmacologically acceptable carriers, excipients, diluents or auxiliary agents in the pharmaceutical composition.
Examples of the pharmaceutically acceptable carriers, excipients or diluents include, but are not limited to, lactose, dextrose, sucrose, sorbitol, mannitol, xylitol, erythritol, maltitol, starch, gum acacia, alginate, gelatin, calcium phosphate, calcium silicate, cellulose, methyl cellulose, microcrystalline cellulose, polyvinylpyrrolidone, water, methyl hydroxybenzoate, propyl hydroxybenzoate, talc, magnesium stearate, mineral oil, dextrin, calcium carbonate, propylene glycol, liquid paraffin, and physiological saline. The pharmaceutically acceptable carriers, excipients or diluents may be those known in the art. The pharmaceutical composition may further include one or more additives selected from fillers, extenders, binders, disintegrants, anticoagulants, lubricating agents, wetting agents, pH adjusting agents, nutrients, vitamins, electrolytes, alginic acid and its salts, pectic acid and its salts, protective colloids, glycerin, flavoring agents, emulsifying agents, and preservatives known in the art.

The compound represented by Formula 10, 11 or 12 or pharmaceutically acceptable salt thereof according to the present invention can be co-administered with one or more other anticancer agents for treating cancer or tumor to enhance the therapeutic effect of the anticancer agents.

The pharmaceutical composition can be orally or parenterally. For example, the pharmaceutical composition may be administered via various routes, including oral, transdermal, subcutaneous, intravenous or intramuscular routes. The composition may be prepared into various formulations depending on the method of its use. The composition may be prepared into formulations by methods well known in the art to provide rapid-release, sustained or delayed release of the active ingredient after administration to mammals. In general, solid preparations for oral administration include tablets, troches, soft or hard capsules, pills, powders, and granules and may be formulated with one or more excipients, for example, starch, calcium carbonate, sucrose, lactose or gelatin. In addition to simple excipients, lubricating agents such as magnesium stearate and talc may also be used. Liquid preparations for oral application are suspensions, solutions for internal use, emulsions, and syrups. Such liquid preparations may include various excipients, for example, wetting agents, sweetening agents, flavoring agents, and preservatives, in addition to water and liquid paraffin, which are commonly used simple diluents. Formulations for parenteral administration include creams, lotions, ointments, plasters, liquids, solutions, aerosols, fluidextracts, elixirs, infusions, sachets, patches, and injections. Injectable formulations are preferably in the form of isotonic aqueous solutions or suspensions.

The pharmaceutical composition may further contain one or more adjuvants such as sterilizers, preservatives, stabilizers, hydrating agents, emulsion accelerators, salts for osmotic pressure adjustment, and/or buffers, and one or more other therapeutically useful substances. The pharmaceutical composition may be formulated by a general mixing or granulation technique. Any suitable technique known in the art may be used to formulate the pharmaceutical composition.

The dose of the pharmaceutical composition can be determined taking into consideration the mode of administration, the user's age and sex, the severity of the disease, the patient's condition, the rate of absorption of the active ingredient in the body, the inactivation rate, and the type of concomitant drugs. The pharmaceutical composition can be administered in a single dose or in divided doses. The active ingredient of the pharmaceutical composition can be administered to a mammal, including a human, in an amount of 0.001 to 100 mg/kg body weight, preferably 0.01 to 35 mg/kg body weight, on a daily basis once or in divided doses per day via an oral or parenteral route.

Another embodiment of the present invention provides a method for treating cancer including administering a therapeutically effective amount of the compound represented by Formula 10, 11 or 12, pharmaceutically acceptable salt thereof or hydrate thereof.

Preferably, the method may further include identifying a patient in need of prevention or treatment of the cancer prior to the administration.

As used herein, the term "therapeutically effective amount" refers to the amount of an active ingredient effective to prevent or treat cancer in a mammal. The therapeutically effective amount may be determined depending on various factors, including the type and severity of the disease, the types and contents of the active ingredient and other ingredients of the composition, the type of the formulation, the patient's age, weight, general health, sex, and diet, the time and route of administration, the blood clearance of the composition, the duration of treatment, and the type of concomitant drugs. As described above, the active ingredient is preferably administered in an amount of 0.001 to 100 mg/kg body weight, preferably 0.01 to 35 mg/kg body weight, on a daily basis once or in divided doses per day via an oral or parenteral route.

The present invention also provides a method for preparing a compound represented by Formula 1. 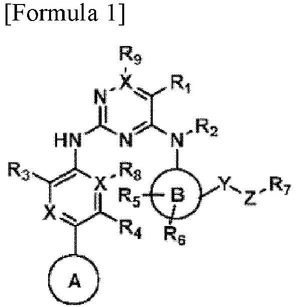
wherein R₁ is hydrogen, -Cl or -Br,
R₂ is hydrogen, C₁-C₅ alkyl, -SO₂CH₃ or -SO₂CF₃,
R₃, R₄, R₈, and R₉ are each independently selected from hydrogen, halo, amino, C₁-C₅ haloalkyl, alkoxy, and C₁-C₅ alkyl,
R₅ and R₆ are each independently selected from hydrogen, halo, -CF₃, C₁-C₅ haloalkyl, and C₁-C₅ alkyl,
R₇ is selected from -CF₃, C₁-C₅ haloalkyl, and C₁-C₅ alkyl,
A is C₃-C₁₀ heterocycloalkyl, C₆-C₁₃ heterobicycloalkyl or C₃-C₁₅ linear or branched heteroalkyl, with the proviso that the heterocycloalkyl, heterobicycloalkyl, and linear or branched heteroalkyl are optionally substituted with one or more substituents selected from halo, hydroxyl, alkanol, and C₁-C₅ alkyl,
X is carbon or nitrogen,
Y is selected from -N-, -NH-, -CH₂-, and -CO-,
Z is selected from -NH-, -SO₂-, and -SO-(CH₃)₂, and
B is C₆ aryl, heteroaryl or C₃-C₁₀ heterobicycloaryl.

In one aspect of the present invention, B is a monocyclic aryl group or a substituted or unsubstituted bicyclic aromatic group containing one or more heteroatoms selected from N, S, and O. Examples of bicyclic heteroaryl groups include, but are not limited to, indolyl, benzothiophenyl, benzofuranyl, benzimidazolyl, benzoxazolyl, benzisoxazolyl, benzthiazolyl, benzthiadiazolyl, benztriazolyl, quinolinyl, isoquinolinyl, purinyl, furopyridinyl, oxochromene, dioxoisoindolinyl, pyrazolopyridinyl, pyrazolo[1,5-a]pyridinyl, isoquinoxalinyl, and their similar groups.

The pyrimidine compound represented by Formula 1 can be prepared by coupling a commercially available pyrimidine compound represented by Formula 2 with an amine represented by Formula 3, as depicted in Reaction Scheme 1: 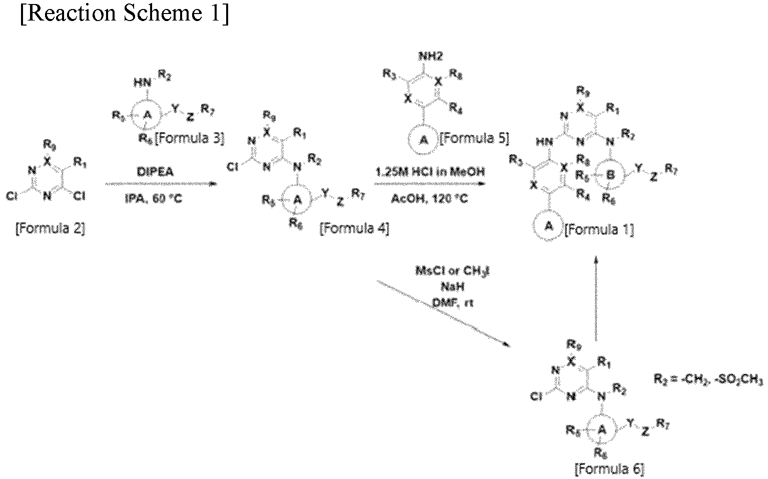 wherein R₁, R₂, R₃, R₄, R₅, R₆, R₇, R₈, R₉, X, Y, Z, A, and B are as defined in Formula 1.

First, as depicted in Reaction Scheme 1, the commercially available pyrimidine compound represented by Formula 2 is subjected to a coupling reaction with the commercially available amine represented by Formula 3 to give the compound represented by Formula 4. This reaction involves stirring at a temperature of 60 °C. The reaction solvent may be an organic solvent such as tetrahydrofuran, dioxane, *N*,*N-*dimethylformamide, *N*,*N*-dimethyl sulfoxide, 2-butanol or 2-pentanol.

Subsequently, the compound of Formula 4 is allowed to react with commercially available sulfonyl chloride or methyl iodide under basic conditions such as sodium hydride in a solvent such as *N*,*N*-dimethylformamide at room temperature. As a result of the reaction, the compound of Formula 4 is substituted with a sulfonyl or methyl group to give the compound of Formula 6.

The compound of Formula 4 or 6 and the amine represented by Formula 5 are stirred at 120 °C in the presence of a 1.25 M methanolic hydrochloric acid solution and acetic acid to obtain the compound of Formula 1.

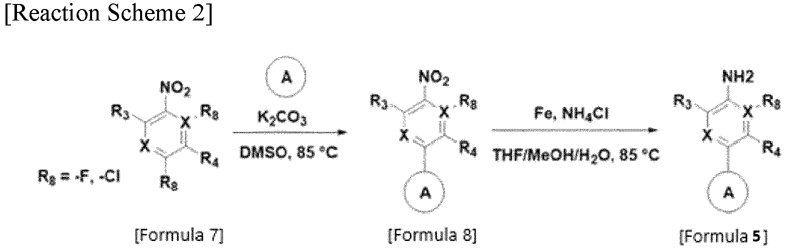

Reaction Scheme 2 shows reactions for preparing the amine intermediate of Formula 5. In Reaction Scheme 2, R₃, R₄, R₈, X, and A are as defined in Formula 1 and R₈ is fluoro or chloro.

As depicted in Reaction Scheme 2, the commercially available nitro compound represented by Formula 7 is subjected to a coupling reaction with A to give the compound of Formula 8.

Subsequently, the compound of Formula 8, iron, and ammonium chloride are stirred in a mixed solvent of tetrahydrofuran, methanol, and water at 85 °C. As a result of the reaction, the nitro group of the compound of Formula 8 is reduced to an amino group to give the compound of Formula 5.

The present invention also provides a pharmaceutical composition including the pyrimidine compound represented by Formula 1, 10, 11 or 12, pharmaceutically acceptable salt thereof, solvate thereof, hydrate thereof or enantiomer thereof as an active ingredient.

The compound of the present invention can be used as an active ingredient of a pharmaceutical composition for preventing and treating a disease caused by abnormal cell metabolism and glucose metabolism due to its outstanding ability to inhibit the activity of the EGFR protein kinase. Therefore, the compound of the present invention can be used as a prophylactic and therapeutic agent for diseases caused by abnormal cell metabolism and glucose metabolism, for example, metabolic diseases such as diabetes and obesity and a variety of tumor diseases selected from endometrial cancer, bladder cancer, stomach cancer, lung cancer, liver cancer, colorectal cancer, small intestine cancer, pancreatic cancer, brain cancer, bone cancer, melanoma, breast cancer, sclerosing adenosis, head and neck cancer, esophageal cancer, thyroid cancer, parathyroid cancer, kidney cancer, sarcoma, prostate cancer, urethral cancer, blood cancers such as leukemia, multiple myeloma, and myelodysplastic syndrome, lymphomas such as Hodgkin's disease and non-Hodgkin's lymphoma, and fibroadenoma.

Thus, the present invention provides a pharmaceutical composition including the pyrimidine compound represented by Formula 1, 10, 11 or 12, pharmaceutically acceptable salt thereof, solvate thereof or hydrate thereof as an active ingredient and a prophylactic and therapeutic agent for a disease caused by abnormal cell metabolism and glucose metabolism.

The pharmaceutical composition of the present invention contains, as an active ingredient, at least one selected from the pyrimidine compound represented by Formula 1, 10, 11 or 12, pharmaceutically acceptable salt thereof, solvate thereof, and hydrate thereof. The pharmaceutical composition of the present invention may be formulated with pharmaceutically acceptable carriers, reinforcing agents, and excipients into preparations known in the field of pharmaceuticals, for example, preparations for oral or parenteral administration such as tablets, capsules, troches, liquids, and suspensions.

Examples of excipients that can be used in the pharmaceutical composition of the present invention include sweetening agents, binders, solubilizers, dissolution aids, wetting agents, emulsifiers, isotonic agents, adsorbents, disintegrants, antioxidants, preservatives, lubricants, fillers, and flavoring agents. For example, the excipients may be lactose, dextrose, sucrose, mannitol, sorbitol, cellulose, glycine, silica, talc, stearic acid, stearin, magnesium stearate, magnesium aluminosilicate, starch, gelatin, gum tragacanth, alginic acid, sodium alginate, methyl cellulose, sodium carboxymethyl cellulose, agar, water, ethanol, polyethylene glycol, polyvinylpyrrolidone, sodium chloride, calcium chloride, orange essence, strawberry essence, and vanilla flavor.

The dose of the compound according to the present invention to a human may vary depending on the patient's age, body weight, and sex, dosage form, the patients' health condition, and the severity of the disease and is generally 0.01 to 1,000 mg/day for an adult patient weighing 70 kg. The compound of the present invention may be administered in an amount of 0.01 to 1,000 mg/day for an adult weighing 70 kg once or in divided doses per day at regular time intervals according to the judgment of the physician or pharmacist.

### Mode for Carrying out the Invention

The present invention will be explained in more detail with reference to the following examples, including formulation examples and experimental examples. However, these examples are merely illustrative and the scope of the present invention is not limited thereto.

### [EXAMPLES]

### Example 1: Preparation of N-(2-((5-chloro-2-((6-(4-ethylpiperazin-1-yl)-2-methoxypyridin-3-yl)amino)-4-yl)amino)phenyl)methanesulfonamide

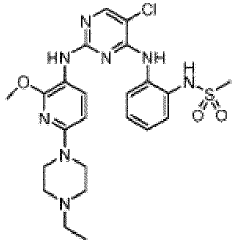

### Step 1) Preparation of N-(2-((2,5-dichloropyrimidin-4-yl)amino)phenyl)methanesulfonamide

5.0 g (26.9 mmol) of *N*-(2-aminophenyl)methanesulfonamide and 3.37 ml (35.0 mmol) of 2,4,5-trichloropyrimidine were diluted with 54 ml of isopropyl alcohol, and 9.3 ml (53.8 mmol) of *N*,*N-*diisopropylethylamine was added thereto. The mixture was stirred at 60 °C for 12 h. After completion of the reaction, the reaction mixture was cooled to room temperature, diluted with ethyl acetate, and washed with distilled water. The resulting residue was purified by column chromatography (50% ethyl acetate/hexane) to afford 4.66 g (yield: 52%) of the title compound.

### Step 2) Preparation of 6-(4-ethylpiperazin-1-yl)-2-methoxypyridin-3-amine

1.0 g (5.38 mmol) of 6-chloro-2-methoxy-3-nitropyridine and 0.82 ml (5.38 mmol) of 1-ethylpiperazine were diluted with 10 ml of DMSO, and 1.48 g (10.8 mmol) of potassium carbonate was added thereto. The mixture was stirred at 85 °C for 5 h. The reaction mixture was extracted with dichloromethane and distilled water. The organic layer was dried over magnesium sulfate. The resulting residue was dissolved in 10 ml of a mixture of tetrahydrofuran, methanol, and water in an 18:1:1 volume ratio without further purification. To the solution were added 0.3 g (2.7 mmol) of an iron powder and 0.16 g (2.7 mmol) of ammonium chloride, followed by stirring at 85 °C for 3 h. After completion of the reaction, the reaction mixture was washed with dichloromethane on a filter filled with celite, filtered under reduced pressure, and concentrated to afford 1.2 g (yield: 94%) of the title compound.

### Step 3) Preparation of N-(2-((5-chloro-2-((6-(4-ethylpiperazin-1-yl)-2-methoxypyridin-3-yl)amino)-4-yl)amino)phenyl)methanesulfonamide

100 mg (0.3 mmol) of the compound prepared in step 1) and 57 mg (0.24 mmol) of the compound prepared in step 2) were dissolved in 1.5 ml of a mixture of acetic acid and a 1.25 M HCl/methanol solution in a 1:1 volume ratio, sealed, and stirred at 120 °C for 3 h. After completion of the reaction, the reaction mixture was cooled to room temperature and extracted with a mixture of isopropyl alcohol and chloroform in a 1:4 volume ratio and a saturated aqueous solution of sodium bicarbonate. Thereafter, the organic layer was dried over anhydrous sodium sulfate, filtered under reduced pressure, and distilled under reduced pressure. The resulting residue was purified by column chromatography (10% methanol/dichloromethane) to afford 30 mg (yield: 20%) of the title compound. [M+H]⁺ = 534.

### Example 2: Preparation of A-(2-((5-chloro-2-((2-methoxy-5-methyl-4-(4-(4-methylpiperazin-1-yl)piperidin-1-yl)phenyl)amino)pyrimidin-4-yl)amino)phenyl)methanesulfonamide

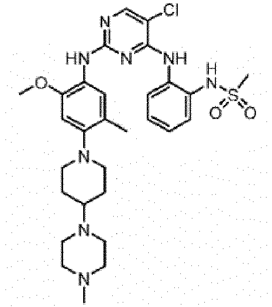

The title compound was prepared from the compound prepared in step 1) of Example 1 and the corresponding aniline in the same manner as in Example 1. [M+H]⁺ = 614.

### Example 3: Preparation of N-(2-((5-chloro-2-((6-(4-(4-methylpiperazin-1-yl)piperidin-1-yl)pyridin-3-yl)amino)pyrimidin-4-yl)amino)phenyl)methanesulfonamide

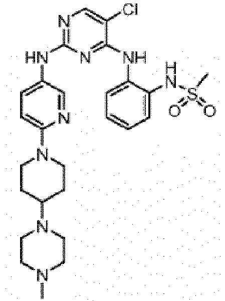

The title compound was prepared from the compound prepared in step 1) of Example 1 and the corresponding aniline in the same manner as in Example 1. [M+H]⁺ = 571.

### Example 4: Preparation of N-(2-((5-chloro-2-((6-(4-(4-methylpiperazin-1-yl)piperidin-1-yl)-2-(2,2,2-trifluoroethoxy)pyridin-3-yl)amino)pyrimidin-4-yl)amino)phenyl)methanesulfonamide

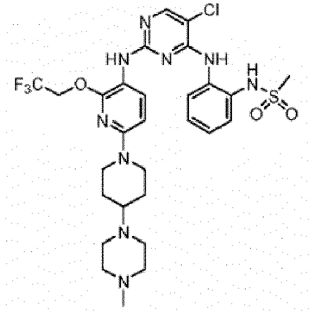

The title compound was prepared from the compound prepared in step 1) of Example 1 and the corresponding aniline in the same manner as in Example 1. [M+H]⁺ = 669.

### Example 5: Preparation of N-(2-((5-chloro-2-((3-fluoro-4-(4-(4-methylpiperazin-1-yl)piperidin-1-yl)phenyl)amino)pyrimidin-4-yl)amino)phenyl)methanesulfonamide

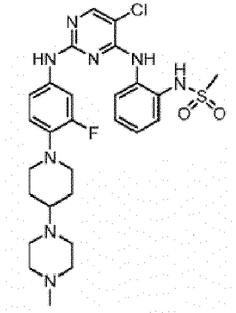

The title compound was prepared from the compound prepared in step 1) of Example 1 and the corresponding aniline in the same manner as in Example 1. [M+H]⁺ = 558.

### Example 6: Preparation of N-(2-((5-chloro-2-((6-(1,1-dioxythiomorpholino)-2-methoxypyridin-3-yl)amino)pyrimidin-4-yl)amino)phenyl)methanesulfonamide

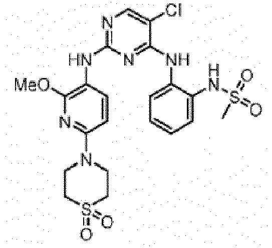

The title compound was prepared from the compound prepared in step 1) of Example 1 and the corresponding aniline in the same manner as in Example 1. [M+H]⁺ = 553.

### Example 7: Preparation of N-(2-((5-chloro-2-((5-chloro-2-methoxy-4-(4-(4-methylpiperazin-1-yl)piperidin-1-yl)phenyl)amino)pyrimidin-4-yl)amino)phenyl)methanesulfonamide

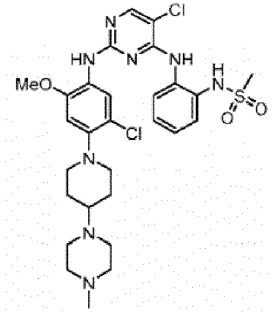

The title compound was prepared from the compound prepared in step 1) of Example 1 and the corresponding aniline in the same manner as in Example 1. [M+H]⁺ = 634.

### Example 8: Preparation of N-(5-chloro-2-((6-(3-(dimethylamino)pyrrolidin-1-yl)-2-methoxypyridin-3-yl)amino)pyrimidin-4-yl)-A-(2-(methylsulfonamido)phenyl)methanesulfonamide

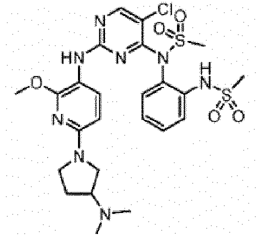

### Step 1) Preparation of N-(2,5-dichloropyrimidin-4-yl)-N-(2-(methylsulfonamido)phenyl)methanesulfonamide

1.0 g (3.0 mmol) of the compound prepared in step 1) of Example 1 was diluted with 6 ml of *N*,*N*-dimethylformamide, and 138 mg (6.0 mmol) of 60% sodium hydride was slowly added thereto at 0 °C. The mixture was stirred at room temperature for 30 min. To the reaction mixture was slowly added 0.27 ml (3.6 mmol) of methanesulfonyl chloride at 0 °C, followed by stirring at room temperature for 2 h. After completion of the reaction, the reaction mixture was diluted with ethyl acetate and washed with distilled water and saturated brine. The organic layer was separated, dried over anhydrous sodium sulfate, filtered under reduced pressure, and distilled under reduced pressure. The resulting residue was purified by chromatography (50% ethyl acetate/hexane) to afford 1.95 g (yield: 65%) of the title compound.

### Step 2) Preparation of N-(5-chloro-2-((6-(3-(dimethylamino)pyrrolidin-1-yl)-2-methoxypyridin-3-yl)amino)pyrimidin-4-yl)-N-(2-(methylsulfonamido)phenyl)methanesulfonamide

The title compound was prepared in the same manner as in Example 1, except that the compound prepared in step 1) was used and *N*,*N*-dimethylpyrrolidin-3-amine were used in step 2) of Example 1. [M+H]⁺ = 610.

### Example 9: Preparation of N-(5-chloro-2-((2-methoxy-5-methyl-4-(4-(4-methylpiperazin-1-yl)piperidin-1-yl)phenyl)amino)pyrimidin-4-yl)-N-(2-(methylsulfonamido)phenyl)methanesulfonamide

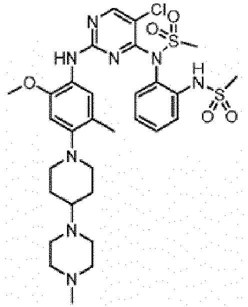

The title compound was prepared from the compound prepared in step 1) of Example 8 and the corresponding aniline in the same manner as in Example 1. [M+H]⁺ = 692.

### Example 10: Preparation of N-(5-chloro-2-((2-methoxy-6-(4-(4-methylpiperazin-1-yl)piperidin-1-yl)pyridin-3-yl)amino)pyrimidin-4-yl)-N-(2-(methylsulfonamido)phenyl)methanesulfonamide

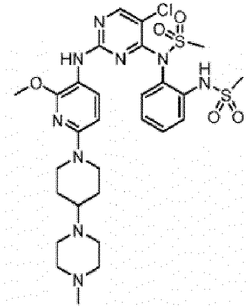

The title compound was prepared from the compound prepared in step 1) of Example 8 and the corresponding aniline in the same manner as in Example 1. [M+H]⁺ = 679.

### Example 11: Preparation of N-(2-((5-chloro-2-((2-methoxy-5-methyl-4-(4-(4-methylpiperazin-1-yl)piperidin-1-yl)phenyl)amino)pyrimidin-4-yl)(methyl)amino)phenyl)-N-methylsulfonamide

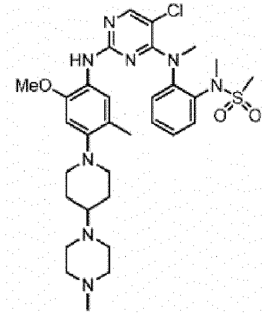

### Step 1) Preparation of N-(2-((2,5-dichloropyrimidin-4-yl)(methyl)amino)phenyl)-N-methylmethanesulfonamide

1.0 g (3.0 mmol) of the compound prepared in step 1) of Example 1 was diluted with 6 ml of *N*,*N*-dimethylformamide, and 138 mg (6.0 mmol) of 60% sodium hydride was slowly added thereto at 0 °C. The mixture was stirred at room temperature for 30 min. To the reaction mixture was slowly added 0.22 ml (3.6 mmol) of methyl iodide at 0 °C, followed by stirring at room temperature for 2 h. After completion of the reaction, the reaction mixture was diluted with ethyl acetate and washed with distilled water and saturated brine. The organic layer was separated, dried over anhydrous sodium sulfate, filtered under reduced pressure, and distilled under reduced pressure. The resulting residue was purified by chromatography (50% ethyl acetate/hexane) to afford 600 mg (yield: 53%) of the title compound.

### Step 2) Preparation of N-(2-((5-chloro-2-((2-methoxy-5-methyl-4-(4-(4-methylpiperazin-1-yl)piperidin-1-yl)phenyl)amino)pyrimidin-4-yl)(methyl)amino)phenyl)-N-methylsulfonamide

The title compound was prepared in the same manner as in Example 1, except that the compound prepared in step 1) was used and 1-chloro-5-methoxy-2-methyl-4-nitrobenzene and 1-methyl-4-piperidin-4-yl-piperazine hydrochloride were used in step 2) of Example 1. [M+H]⁺ = 679.

### Example 12: Preparation of N-(2-((5-chloro-2-((2-methoxy-5-methyl-4-(4-(4-methylpiperazin-1-yl)piperidin-1-yl)phenyl)amino)pyrimidin-4-yl)amido)-5-fluorophenyl)methanesulfonamide

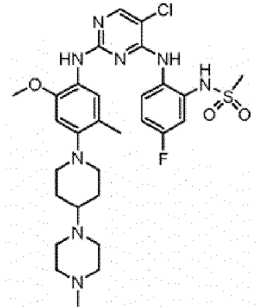

### Step 1) Preparation of N (2-((2,5-dichloropyrimidin-4-yl)amino)-5-fluorophenyl)methanesulfonylamide

The title compound was prepared from *N*-(2-amino-5-fluorophenyl)methanesulfonamide in the same manner as in step 1) of Example 1.

### Step 2) Preparation of N-(2-((5-chloro-2-((2-methoxy-5-methyl-4-(4-(4-methylpiperazin-1-yl)piperidin-1-yl)phenyl)amino)pyrimidin-4-yl)amido)-5-fluorophenyl)methanesulfonamide

The title compound was prepared in the same manner as in Example 1, except that the compound prepared in step 1) was used and 1-chloro-5-methoxy-2-methyl-4-nitrobenzene and 1-methyl-4-piperidin-4-yl-piperazine hydrochloride were used in step 2) of Example 1.

### Example 13: Preparation of N-(2-((5-chloro-2-((6-morpholinopyridin-3-yl)amino)pyrimidin-4-yl)amido)-5-fluorophenyl)methanesulfonamide

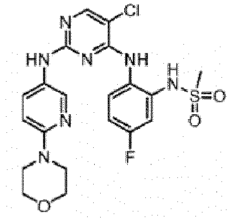

The title compound was prepared from the compound prepared in step 1) of Example 12 and the corresponding aniline in the same manner as in Example 1. [M+H]⁺ = 493.

### Example 14: Preparation of N-(2-((5-chloro-2-((6-(4-(4-methylpiperazin-1-yl)piperidin-1-yl)pyridin-3-yl)amido)pyrimidin-4-yl)amino)-5-fluorophenyl)methanesulfonamide

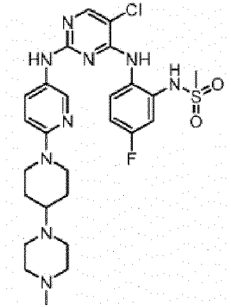

The title compound was prepared from the compound prepared in step 1) of Example 12 and the corresponding aniline in the same manner as in Example 1. [M+H]⁺ = 589.

### Example 15: Preparation of N-(2-((5-chloro-2-((2-methoxy-5-methyl-6-(4-(4-methylpiperazin-1-yl)piperidin-1-yl)pyridin-3-yl)amino)pyrimidin-4-yl)amino)-5-fluorophenyl)methanesulfonamide

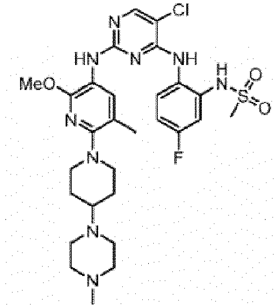

The title compound was prepared from the compound prepared in step 1) of Example 12 and the corresponding aniline in the same manner as in Example 1. [M+H]⁺ = 633.

### Example 16: Preparation of N-(2-((5-chloro-2-((5-fluoro-6-(4-(4-methylpiperazin-1-yl)piperidin-1-yl)pyridin-3-yl)amino)pyrimidin-4-yl)amino)-5-fluorophenyl)methanesulfonamide

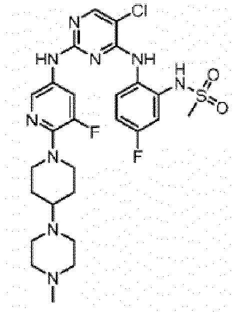

The title compound was prepared from the compound prepared in step 1) of Example 12 and the corresponding aniline in the same manner as in Example 1. [M+H]⁺ = 607.

### Example 17: Preparation of N-(2-((5-chloro-2-((2-methoxy-6-(4-(4-methylpiperazin-1-yl)piperidin-1-yl)pyridin-3-yl)amino)pyrimidin-4-yl)amino)-5-fluorophenyl)methanesulfonamide

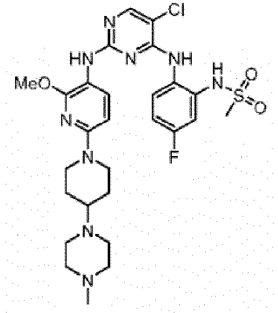

The title compound was prepared from the compound prepared in step 1) of Example 12 and the corresponding aniline in the same manner as in Example 1. [M+H]⁺ = 619.

### Example 18: Preparation of N-(2-((5-chloro-2-((6-(4-(2-hydroxymethyl)piperazin-1-yl)-2-methoxypyridin-3-yl)amino)pyrimidin-4-yl)amino)-5-fluorophenyl)methanesulfonamide

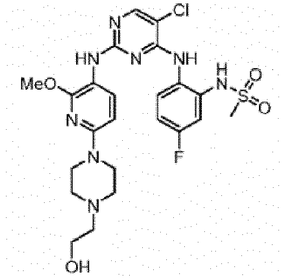

The title compound was prepared from the compound prepared in step 1) of Example 12 and the corresponding aniline in the same manner as in Example 1. [M+H]⁺ = 566.

### Example 19: Preparation of N-(2-((2-((6-(4-acetylpiperazin-1-yl)-2-methoxypyridin-3-yl)amino)-5-chloropyrimidin-4-yl)amino)-5-fluorophenyl)methanesulfonamide

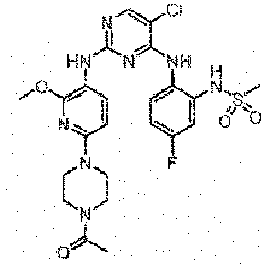

The title compound was prepared from the compound prepared in step 1) of Example 12 and the corresponding aniline in the same manner as in Example 1. [M+H]⁺ = 565.

### Example 20: Preparation of N-(2-((5-chloro-2-((2-methoxy-6-((3S,5R)-3,4,5-trimethylpiperazin-1-yl)pyridin-3-yl)amino)pyrimidin-4-yl)amino)-5-fluorophenyl)methanesulfonamide

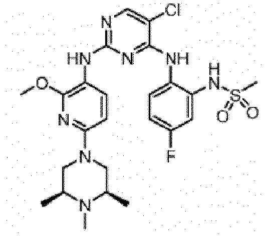

The title compound was prepared from the compound prepared in step 1) of Example 12 and the corresponding aniline in the same manner as in Example 1. [M+H]⁺ = 565.

### Example 21: Preparation of N-(2-((5-chloro-((6-(4-hydroxypiperidin-1-yl)-2-methoxypyridin-3-yl)amino)pyrimidin-4-yl)amino)-5-fluorophenyl)methanesulfonamide

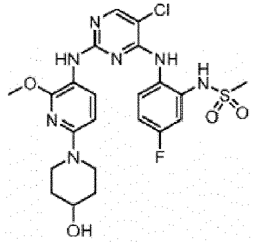

The title compound was prepared from the compound prepared in step 1) of Example 12 and the corresponding aniline in the same manner as in Example 1. [M+H]⁺ = 538.

### Example 22: Preparation of N-(2-((5-chloro-2-((2-methoxy-6-(4-(oxetan-3-yl)piperazin-1-yl)pyridin-3-yl)amino)pyrimidin-4-yl)amino)-5-fluorophenyl)methanesulfonamide

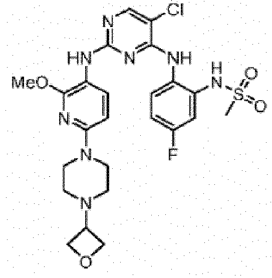

The title compound was prepared from the compound prepared in step 1) of Example 12 and the corresponding aniline in the same manner as in Example 1. [M+H]⁺ = 578.

### Example 23: Preparation of N-(2-((5-chloro-2-((6-(3-(dimethylamino)pyrrolidin-1-yl)-2-methoxypyridin-3-yl)amino)pyrimidin-4-yl)amino)-5-fluorophenyl)methanesulfonamide

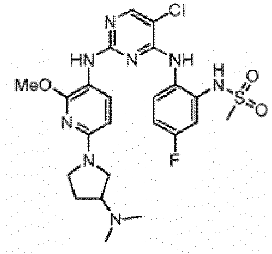

The title compound was prepared from the compound prepared in step 1) of Example 12 and the corresponding aniline in the same manner as in Example 1. [M+H]⁺ = 550.

### Example 24: Preparation of N-(2-((5-bromo-2-((2-methoxy-5-methyl-4-(4-(4-methylpiperazin-1-yl)piperidin-1-yl)phenyl)amino)pyrimidin-4-yl)amido)-5-fluorophenyl)methanesulfonamide

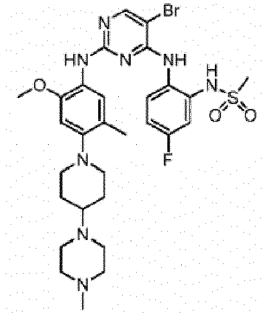

### Step 1) Preparation of N-(2-((5-bromo-2-chloropyrimidin-4-yl)amino)-5-fluorophenyl)methanesulfonamide

The title compound was prepared from 5-bromo-2,4-dichloropyrimidine and *N-*(2-amino-5-fluorophenyl)methanesulfonamide in the same manner as in step 1 of Example 1.

### Step 2) Preparation of N-(2-((5-bromo-2-((2-methoxy-5-methyl-4-(4-(4-methylpiperazin-1-yl)piperidin-1-yl)phenyl)amino)pyrimidin-4-yl)amido)-5-fluorophenyl)methanesulfonamide

The title compound was prepared in the same manner as in Example 1, except that the compound prepared in step 1) was used and 1-chloro-5-methoxy-2-methyl-4-nitrobenzene and 1-methyl-4-piperidin-4-yl-piperazine hydrochloride were used in step 2) of Example 1.

¹H NMR (400 MHz, DMSO-d6) δ 8.64 (s, 1H), 8.12 (s, 1H), 7.93-7.73 (m, 2H), 7.43 (s, 1H), 7.14 (dd, J = 10.9, 2.8 Hz, 1H), 6.79-6.58 (m, 2H), 3.76 (s, 3H), 3.06 (d, J = 11.1 Hz, 2H), 2.84 (s, 3H), 2.78-2.26 (m, 14H), 2.04 (s, 3H), 1.93-1.80 (m, 2H), 1.68-1.50 (m, 2H). [M+H]⁺ = 677.

### Example 25: Preparation of N-(2-((5-bromo-2-((5-fluoro-6-(4-(4-methylpiperazin-1-yl)piperidin-1-yl)pyridin-3-yl)amino)pyrimidin-4-yl)amino)-5-fluorophenyl)methanesulfonamide

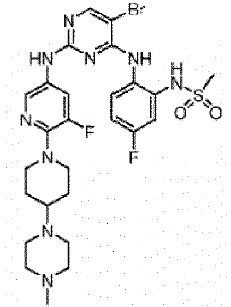

The title compound was prepared from the compound prepared in step 1) of Example 24 and the corresponding aniline in the same manner as in Example 1. [M+H]⁺ = 652.

### Example 26: Preparation of N (2-((5-bromo-2-((5-chloro-2-methoxy-4-(4-(4-methylpiperazin-1-yl)piperidin-1-yl)phenyl)amino)pyrimidin-4-yl)amino)-5-fluorophenyl)methanesulfonamide

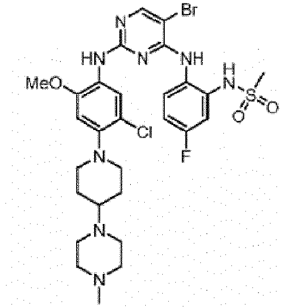

The title compound was prepared from the compound prepared in step 1) of Example 24 and the corresponding aniline in the same manner as in Example 1. [M+H]⁺ = 697.

### Example 27: Preparation of N-(2-((5-bromo-2-((2-chloro-4-(4-(4-methylpiperazin-1-yl)piperidin-1-yl)phenyl)amino)pyrimidin-4-yl)amino)-5-fluorophenyl)methanesulfonamide

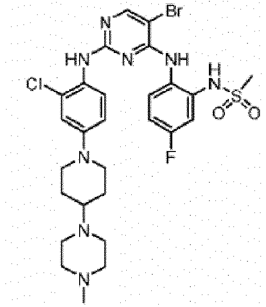

The title compound was prepared from the compound prepared in step 1) of Example 24 and the corresponding aniline in the same manner as in Example 1. [M+H]⁺ = 667.

### Example 28: Preparation of N-(2-((5-bromo-2-((2-methoxy-6-(4-(4-methylpiperazin-1-yl)piperidin-1-yl)pyridin-3-yl)amino)pyrimidin-4-yl)amino)-5-fluorophenyl)methanesulfonamide

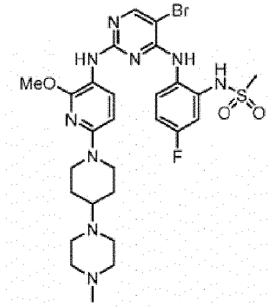

The title compound was prepared from the compound prepared in step 1) of Example 24 and the corresponding aniline in the same manner as in Example 1. [M+H]⁺ = 664.

### Example 29: Preparation of N-(2-((5-bromo-2-((2-methoxy-6-morpholinopyridin-3-yl)amino)pyrimidin-4-yl)amino)-5-fluorophenyl)methanesulfonamide

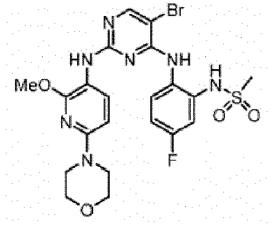

The title compound was prepared from the compound prepared in step 1) of Example 24 and the corresponding aniline in the same manner as in Example 1. [M+H]⁺ = 568.

### Example 30: Preparation of N-(2-((5-bromo-2-((6-(4-cyclopropylpiperazin-1-yl)-2-methoxypyridin-3-yl)amino)pyrimidin-4-yl)amino)-5-fluorophenyl)methanesulfonamide

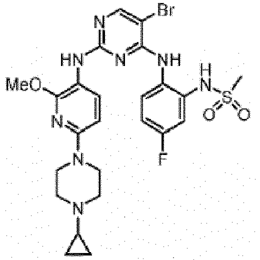

The title compound was prepared from the compound prepared in step 1) of Example 24 and the corresponding aniline in the same manner as in Example 1. [M+H]⁺ = 607.

### Example 31: Preparation of N-(2-((5-bromo-2-((2-methoxy-6-(4-morpholinopiperidin-1-yl)pyridin-3-yl)amino)pyrimidin-4-yl)amino)-5-fluorophenyl)methanesulfonamide

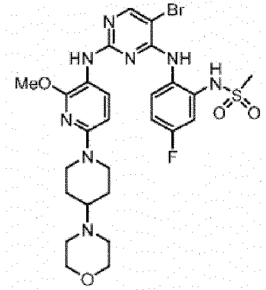

The title compound was prepared from the compound prepared in step 1) of Example 24 and the corresponding aniline in the same manner as in Example 1. [M+H]⁺ = 651.

### Example 32: Preparation of N-(2-((5-bromo-2-((6-(4,4-dimethylpiperidin-1-yl)-2-methoxypyridin-3-yl)amino)pyrimidin-4-yl)amino)-5-fluorophenyl)methanesulfonamide

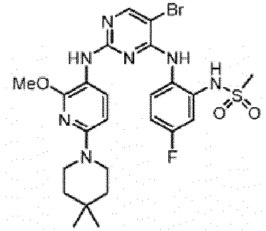

The title compound was prepared from the compound prepared in step 1) of Example 24 and the corresponding aniline in the same manner as in Example 1. [M+H]⁺ = 594.

### Example 33: Preparation of N-(2-((5-Bromo-2-((6-(4,4-difluoropiperidin-1-yl)-2-methoxypyridin-3-yl)amino)pyrimidin-4-yl)amino)-5-fluorophenyl)methanesulfonamide

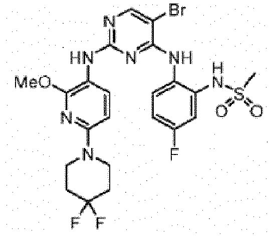

The title compound was prepared the compound prepared in step 1) of Example 24 and the corresponding aniline in the same manner as in Example 1. [M+H]⁺ = 602.

### Example 34: Preparation of N-(2-((5-bromo-2-((2-chloro-6-(4-(4-methylpiperazin-1-yl)piperidin-1-yl)-5-(trifluoromethyl)pyridin-3-yl)amino)pyrimidin-4-yl)amino)-5-fluorophenyl)methanesulfonamide

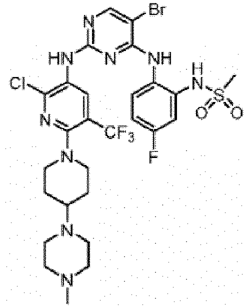

The title compound was prepared from the compound prepared in step 1) of Example 24 and the corresponding aniline in the same manner as in Example 1. [M+H]⁺ = 736.

### Example 35: Preparation of N-(2-((5-bromo-2-((2-(4-(4-methylpiperazin-1-yl)piperidin-1-yl)quinolin-6-yl)amino)pyrimidin-4-yl)amino)-5-fluorophenyl)methanesulfonamide

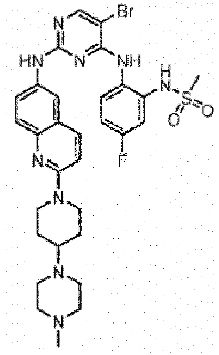

The title compound was prepared from the compound prepared in step 1) of Example 24 and the corresponding aniline in the same manner as in Example 1. [M+H]⁺ = 684.

### Example 36: Preparation of N-(2-((5-bromo-2-((2-methoxy-6-(4-(1-methylpiperidin-4-yl)piperazin-1-yl)pyridin-3-yl)amino)pyrimidin-4-yl)amino)-5-fluorophenyl)methanesulfonamide

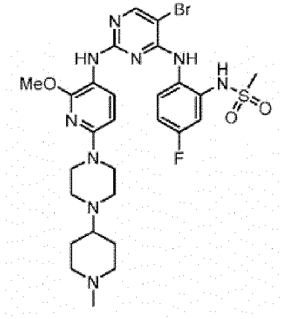

The title compound was prepared from the compound prepared in step 1) of Example 24 and the corresponding aniline in the same manner as in Example 1. [M+H]⁺ = 664.

### Example 37: Preparation of N-(2-((5-bromo-2-((2-methoxy-6-(4-(1-methyl-4-yl)piperazin-1-yl)-5-(trifluoromethyl)pyridin-3-yl)amino)pyrimidin-4-yl)amino)-5-fluorophenyl)methanesulfonamide

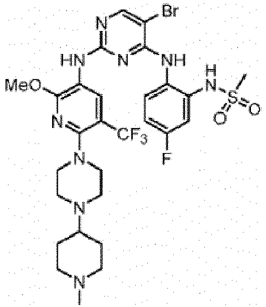

The title compound was prepared from the compound prepared in step 1) of Example 24 and the corresponding aniline in the same manner as in Example 1. [M+H]⁺ = 732.

### Example 38: Preparation of N-(2-((5-bromo-2-((2-methoxy-5-methyl-4-(4-(4-methyl-1,4-diazepan-1-yl)piperidin-1-yl)phenyl)amino)pyrimidin-4-yl)amino)-5-fluorophenyl)methanesulfonamide

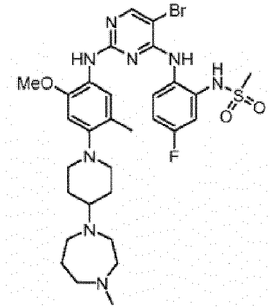

The title compound was prepared from the compound prepared in step 1) of Example 24 and the corresponding aniline in the same manner as in Example 1. [M+H]⁺ = 690.

### Example 39: Preparation of N-(2-((5-bromo-2-((4-(3-(dimethylamino)-[1,4'-bipiperidin]-1'-yl)-2-methoxy-5-methylphenyl)amino)pyrimidin-4-yl)amino)-5-fluorophenyl)methanesulfonamide

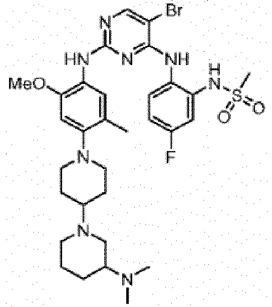

The title compound was prepared from the compound prepared in step 1) of Example 24 and the corresponding aniline in the same manner as in Example 1. [M+H]⁺ = 705.

### Example 40: Preparation of N-(2-((5-bromo-2-((4-(4-(3-(dimethylamino)pyrrolidin-1-yl)piperidin-1-yl)-2-methoxy-5-methylphenyl)amino)pyrimidin-4-yl)amino)-5-fluorophenyl)methanesulfonamide

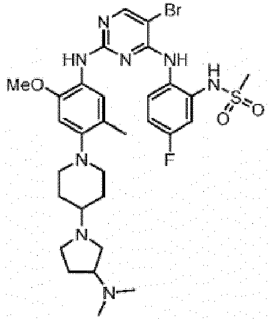

The title compound was prepared from the compound prepared in step 1) of Example 24 and the corresponding aniline in the same manner as in Example 1. [M+H]⁺ = 691.

### Example 41: Preparation of N-(2-((5-bromo-2-((2-methoxy-5-methyl-4-(4-((1-methyl-1H-pyrazol-5-yl)amino)piperidin-1-yl)phenyl)amino)pyrimidin-4-yl)amino)-5-fluorophenyl)methanesulfonamide

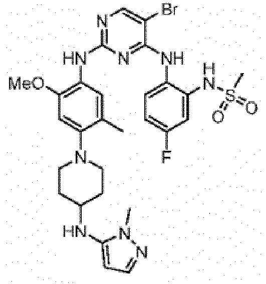

The title compound was prepared from the compound prepared in step 1) of Example 24 and the corresponding aniline in the same manner as in Example 1. [M+H]⁺ = 691.

### Example 42: Preparation of N-(2-((5-bromo-2-((4-(4-(4-cyclopropylpiperazin-1-yl)piperidin-1-yl)-2-methoxy-5-methylphenyl)amino)pyrimidin-4-yl)amino)-5-fluorophenyl)methanesulfonamide

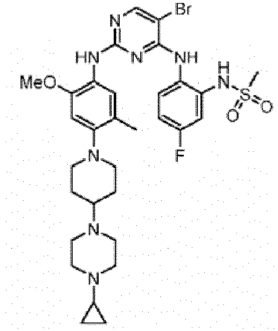

The title compound was prepared from the compound prepared in step 1) of Example 24 and the corresponding aniline in the same manner as in Example 1. [M+H]⁺ = 703.

### Example 43: Preparation of N-(2-((5-bromo-2-((2-chloro-5-methyl-4-(4-(4-methylpiperidin-1-yl)piperidin-1-yl)phenyl)amino)pyrimidin-4-yl)amino)-5-fluorophenyl)methanesulfonamide

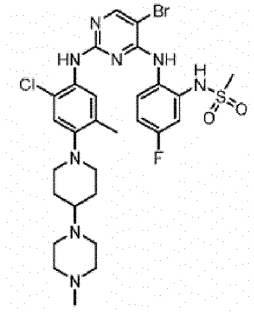

The title compound was prepared from the compound prepared in step 1) of Example 24 and the corresponding aniline in the same manner as in Example 1. [M+H]⁺ = 681.

### Example 44: Preparation of N-(2-((5-bromo-2-((2-chloro-5-methyl-4-(4-(4-methyl-1,4-diazepan-1-yl)piperidin-1-yl)phenyl)amino)pyrimidin-4-yl)amino)-5-fluorophenyl)methanesulfonamide

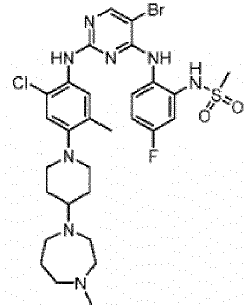

The title compound was prepared from the compound prepared in step 1) of Example 24 and the corresponding aniline in the same manner as in Example 1. [M+H]⁺ = 696.

### Example 45: Preparation of N-(2-((5-bromo-2-((2-chloro-4-(3-(dimethylamino)-[1,4'-bipiperidin]-1'-yl)-5-methylphenyl)amino)pyrimidin-4-yl)amino)fluorophenyl)methanesulfonamide

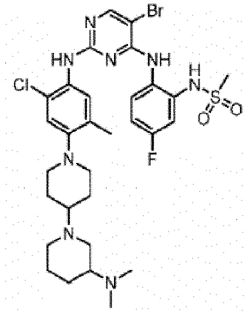

The title compound was prepared from the compound prepared in step 1) of Example 24 and the corresponding aniline in the same manner as in Example 1. [M+H]⁺ = 710.

### Example 46: Preparation of 5-chloro-N²-(2-methoxy-5-methyl-4-(4-(4-methylpiperazin-1-yl)piperidin-1-yl)phenyl)-N⁴-(2-(trifluoromethyl)phenyl)pyrimidine-2,4-diamine

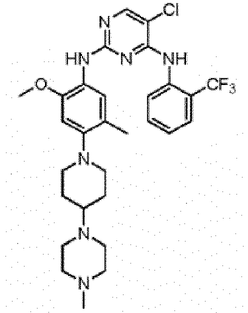

### Step 1) Preparation of 2,5-dichloro-N-(2-(trifluoromethyl)phenyl)pyrimidin-4-amine

The title compound was prepared from 2-(trifluoromethyl)aniline in the same manner as in step 1) of Example 1.

### Step 2) Preparation of 5-chloro-N²-(2-methoxy-5-methyl-4-(4-(4-methylpiperazin-1-yl)piperidin-1-yl)phenyl)-N⁴-(2-(trifluoromethyl)phenyl)pyrimidine-2,4-diamine

The title compound was prepared in the same manner as in Example 1, except that the compound prepared in step 1) was used and 1-chloro-5-methoxy-2-methyl-4-nitrobenzene and 1-methyl-4-piperidin-4-yl-piperazine hydrochloride were used in step 2) of Example 1.

### Example 47: Preparation of 5-chloro-N²-(2-methoxy-4-(4-(4-methylpiperazin-1-yl)piperidin-1-yl)phenyl)-N⁴-(2-(trifluoromethyl)phenyl)pyrimidine-2,4-diamine

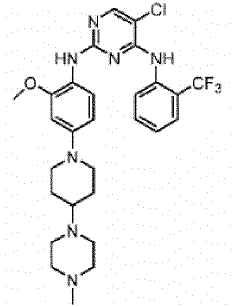

The title compound was prepared from the compound prepared in step 1) of Example 46 and the corresponding aniline in the same manner as in Example 1. [M+H]⁺ = 576.

### Example 48: Preparation of 5-chloro-N²-(4-(4-ethylpiperazin-1-yl)phenyl)-N⁴-(2-(trifluoromethyl)phenyl)pyrimidine-2,4-diamine

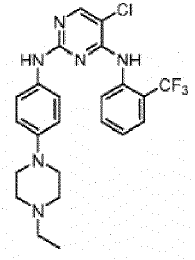

The title compound was prepared from the compound prepared in step 1) of Example 46 and the corresponding aniline in the same manner as in Example 1. [M+H]⁺ = 477.

### Example 49: Preparation of 5-chloro-N²-(6-morpholinopyridin-3-yl)-N⁴-(2-(trifluoromethyl)phenyl)pyrimidine-2,4-diamine

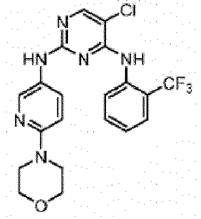

The title compound was prepared from the compound prepared in step 1) of Example 46 and the corresponding aniline in the same manner as in Example 1.

¹H NMR (400 MHz, CDCl3) δ 8.20 (d, J = 2.6 Hz, 1H), 8.16 (d, J = 8.4 Hz, 1H), 8.07 (s, 1H), 7.78 (dd, J = 9.0, 2.5 Hz, 1H), 7.67 (d, J = 7.7 Hz, 1H), 7.57-7.47 (m, 2H), 7.30-7.24 (m, 6H), 6.86 (s, 1H), 6.59 (d, J = 9.0 Hz), 1H), 3.89-3.77 (m, 4H), 3.52-3.28 (m, 4H). [M+H]⁺ = 451.

### Example 50: Preparation of 2-((5-chloro-2-((2-methoxy-5-methyl-4-(4-(4-methylpiperazin-1-yl)piperidin-1-yl)phenyl)amino)pyrimidin-4-yl)amino)-N-methylbenzenesulfonamide

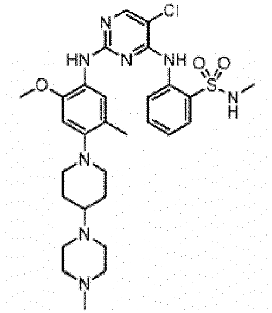

### Step 1) Preparation of 2-((2,5-dichloropyrimidin-4-yl)amino)-N-methylbenzenesulfonamide

The title compound was prepared from 2-amino-*N*-methylbenzenesulfonamide in the same manner as in step 1) of Example 1.

### Step 2) Preparation of 2-((5-chloro-2-((2-methoxy-5-methyl-4-(4-(4-methylpiperazin-1-yl)piperidin-1-yl)phenyl)amino)pyrimidin-4-yl)amino)-N-methylbenzenesulfonamide

The title compound was prepared in the same manner as in Example 1, except that the compound prepared in step 1) was used and 1-chloro-5-methoxy-2-methyl-4-nitrobenzene and 1-methyl-4-piperidin-4-yl-piperazine hydrochloride were used in step 2) of Example 1.

### Example 51: Preparation of 2-((5-chloro-2-((2-methoxy-4-(4-(4-methylpiperazin-1-yl)piperidin-1-yl)phenyl)amino)pyrimidin-4-yl)amino)-N-methylbenzenesulfonamide

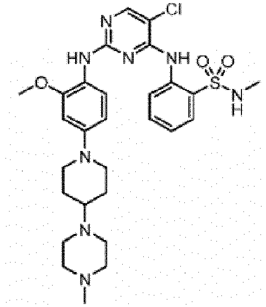

The title compound was prepared from the compound prepared in step 1) of Example 50 and the corresponding aniline in the same manner as in Example 1. [M+H]⁺ = 601.

### Example 52: Preparation of 2-((5-chloro-2-((6-(4-(4-methylpiperazin-1-yl)piperidin-1-yl)pyridin-3-yl)amino)pyrimidin-4-yl)amino)-N-methylbenzenesulfonamide

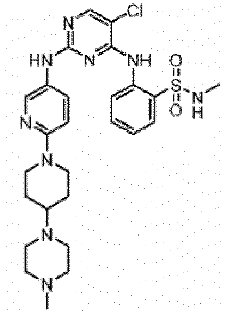

The title compound was prepared from the compound prepared in step 1) of Example 50 and the corresponding aniline in the same manner as in Example 1. [M+H]⁺ = 572.

### Example 53: Preparation of 2-((5-chloro-2-((6-morpholinopyridin-3-yl)amino)pyrimidin-4-yl)amino)-N-methylbenzenesulfonamide

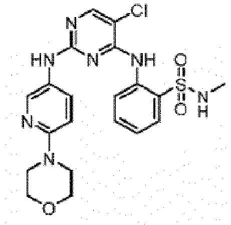

The title compound was prepared from the compound prepared in step 1) of Example 50 and the corresponding aniline in the same manner as in Example 1. ¹H NMR (400 MHz, DMSO) δ 9.28 (s, 2H), 8.52 (s, 1H), 8.30 (s, 1H), 8.22 (s, 1H), 7.81 (dd, J = 12.1, 5.4 Hz, 3H), 7.60 (t, J = 7.4 Hz, 1H), 7.28 (t, J = 7.6 Hz, 1H), 6.81 (d, J = 9.1 Hz, 1H), 3.83-3.47 (m, 4H), 3.43-3.18 (m, 4H), 2.44 (s, 3H). [M+H]⁺ = 476.

### Example 54: Preparation of 2-((5-chloro-2-((6-(4-ethylpiperazin-1-yl)-2-methoxypyridin-3-yl)amino)pyrimidin-4-yl)amino)-N-methylbenzenesulfonamide

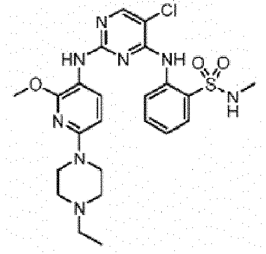

The title compound was prepared from the compound prepared in step 1) of Example 50 and the corresponding aniline in the same manner as in Example 1. [M+H]⁺ = 533.

### Example 55: Preparation of 2-((5-chloro-2-((2-methoxy-6-(4-(4-methylpiperazin-1-yl)piperidin-1-yl)pyridin-3-yl)amino)pyrimidin-4-yl)amino)-N-methylbenzenesulfonamide

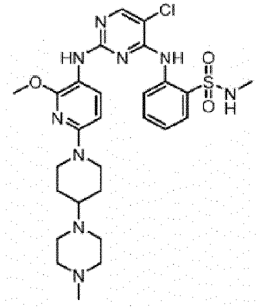

The title compound was prepared from the compound prepared in step 1) of Example 50 and the corresponding aniline in the same manner as in Example 1. [M+H]⁺ = 602.

### Example 56: Preparation of 2-((5-chloro-2-((6-(4-(2-hydroxyethyl)piperazin-1-yl)-2-methoxypyridin-3-yl)amino)pyrimidin-4-yl)amino)-N-methylbenzenesulfonamide

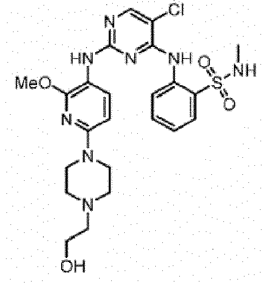

The title compound was prepared from the compound prepared in step 1) of Example 50 and the corresponding aniline in the same manner as in Example 1. [M+H]⁺ = 549.

### Example 57: Preparation of 2-((5-chloro-2-((6-(3-(dimethylamino)pyrrolidin-1-yl)-2-methoxypyridin-3-yl)amino)pyrimidin-4-yl)amino)-N-methylbenzenesulfonamide

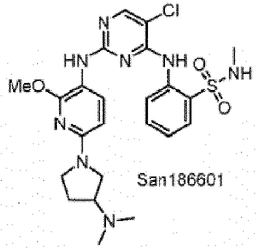

The title compound was prepared from the compound prepared in step 1) of Example 50 and the corresponding aniline in the same manner as in Example 1. [M+H]⁺ = 533.

### Example 58: Preparation of 2-((5-chloro-2-((2-methoxy-5-methyl-6-(4-(4-methylpiperazin-1-yl)piperidin-1-yl)pyridin-3-yl)amino)pyrimidin-4-yl)amino)-N-methylbenzenesulfonamide

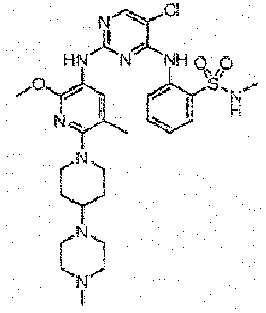

The title compound was prepared from the compound prepared in step 1) of Example 50 and the corresponding aniline in the same manner as in Example 1. [M+H]⁺ = 616.

### Example 59: Preparation of 2-((5-chloro-2-((2-methoxy-5-methyl-4-(4-(4-methylpiperazin-1-yl)piperidin-1-yl)phenyl)amino)pyrimidin-4-yl)amino)-N-cyclopropylbenzenesulfonamide

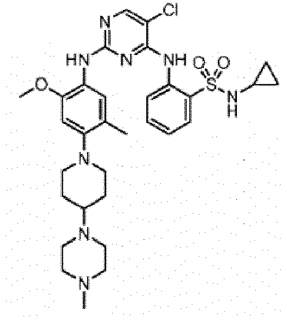

### Step 1) Preparation of N-cyclopropyl-2-((2,5-dichloropyrimidin-4-yl)amino)benzenesulfonamide

The title compound was prepared from 2-amino-*N-*cyclopropylbenzenesulfonamide in the same manner as in step 1) of Example 1.

### Step 2) Preparation of 2-((5-chloro-2-((2-methoxy-5-methyl-4-(4-(4-methylpiperazin-1-yl)piperidin-1-yl)phenyl)amino)pyrimidin-4-yl)amino)-N-cyclopropylbenzenesulfonamide

The title compound was prepared in the same manner as in Example 1, except that the compound prepared in step 1 was used and 1-chloro-5-methoxy-2-methyl-4-nitrobenzene and 1-methyl-4-piperidin-4-yl-piperazine hydrochloride were used in step 2) of Example 1.

### Example 60: Preparation of 2-((5-chloro-2-((3-fluoro-4-(4-(4-methylpiperazin-1-yl)piperidin-1-yl)phenyl)amino)pyrimidin-4-yl)amino)-N-cyclopropylbenzenesulfonamide

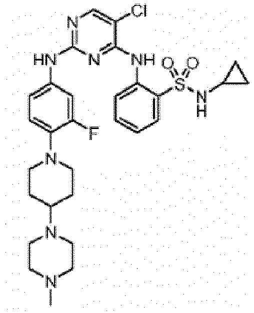

The title compound was prepared from the compound prepared in step 1) of Example 59 and the corresponding aniline in the same manner as in Example 1. [M+H]⁺ = 615.

### Example 61: Preparation of 2-((5-chloro-2-((2-methoxy-6-(4-(4-methylpiperazin-1-yl)piperadin-1-yl)pyridin-3-yl)amino)pyrimidin-4-yl)amino)-N-cyclopropylbenzenesulfonamide

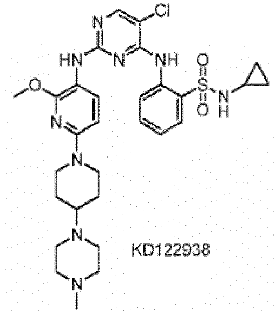

The title compound was prepared from the compound prepared in step 1) of Example 59 and the corresponding aniline in the same manner as in Example 1. [M+H]⁺ = 628.

### Example 62: Preparation of 5-chloro-N²-(2-methoxy-5-methyl-4-(4-(4-methylpiperazin-1-yl)piperidin-1-yl)phenyl)-N⁴-(2-((methylsulfonyl)methyl)phenyl)pyrimidine-2,4-diamine

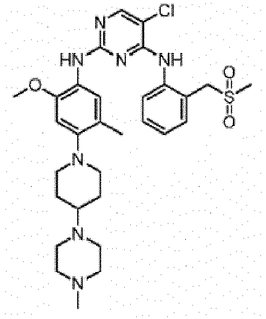

### Step 1) 2,5-dichloro-N-(2-((methylsulfonyl)methyl)phenyl)pyrimidin-4-amine

The title compound was prepared from 2-((methylsulfonyl)methyl)aniline in the same manner as in step 1) of Example 1.

### Step 2) Preparation of 5-chloro-N²-(2-methoxy-5-methyl-4-(4-(4-methylpiperazin-1-yl)piperidin-1-yl)phenyl)-N⁴-(2-((methylsulfonyl)methyl)phenyl)pyrimidine-2,4-diamine

The title compound was prepared in the same manner as in Example 1, except that the compound prepared in step 2) of Example 1 was used and 1-chloro-5-methoxy-2-methyl-4-nitrobenzene and 1-methyl-4-piperidin-4-yl-piperazine hydrochloride were used in step 2) of Example 1.

### Example 63: Preparation of 5-chloro-N²-(3-fluoro-4-(4-(4-methylpiperazin-1-yl)piperidin-1-yl)phenyl)-N⁴-(2-((methylsulfonyl)methyl)phenyl)pyrimidine-2,4-diamine

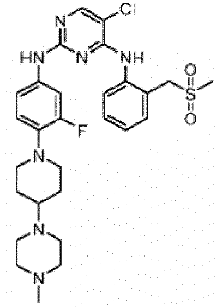

The title compound was prepared from the compound prepared in step 1) of Example 62 and the corresponding aniline in the same manner as in Example 1. [M+H]⁺ = 588.

### Example 64: Preparation of 5-chloro-N²-(2-methoxy-6-(4-(4-methylpiperazin-1-yl)piperidin-1-yl)pyridin-3-yl)-N⁴-(2-((methylsulfonyl)methyl)phenyl)pyrimidine-2,4-diamine

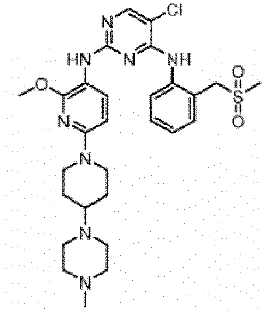

The title compound was prepared from the compound prepared in step 1) of Example 62 and the corresponding aniline in the same manner as in Example 1. [M+H]⁺ = 601.

### Example 65: Preparation of N-(4-chloro-2-((5-chloro-2-((2-methoxy-5-methyl-4-(4-(4-methylpiperazin-1-yl)piperidin-1-yl)phenyl)amino)pyrimidin-4-yl)amino)phenyl)acetamide

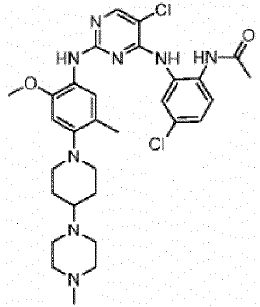

### Step 1) Preparation of N-(4-chloro-2-((2,5-dichloropyrimidin-4-yl)amino)phenyl)acetamide

The title compound was prepared from *N*-(2-amino-4-chlorophenyl)acetamide in the same manner as in step 1) of Example 1.

### Step 2) Preparation of N-(4-chloro-2-((5-chloro-2-((2-methoxy-5-methyl-4-(4-(4-methylpiperazin-1-yl)piperidin-1-yl)phenyl)amino)pyrimidin-4-yl)amino)phenyl)acetamide

The title compound was prepared in the same manner as in Example 1, except that the compound prepared in step 1) was used and 1-chloro-5-methoxy-2-methyl-4-nitrobenzene and 1-methyl-4-piperidin-4-yl-piperazine hydrochloride were used in step 2) of Example 1.

### Example 66: Preparation of N-(4-chloro-2-((5-chloro-2-((2-methoxy-5-methyl-6-(4-(4-methylpiperazin-1-yl)piperidin-1-yl)pyridin-3-yl)amino)pyrimidin-4-yl)amino)phenyl)acetamide

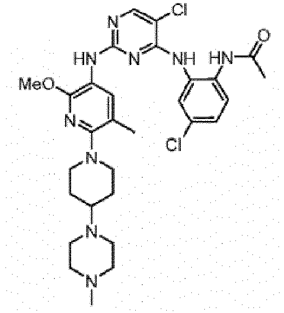

The title compound was prepared from the compound prepared in step 1) of Example 65 and the corresponding aniline in the same manner as in Example 1. [M+H]⁺ = 614.

### Example 67: Preparation of N-(4-chloro-2-((5-chloro-2-((3-fluoro-4-(4-(4-methylpiperazin-1-yl)piperidin-1-yl)phenyl)amino)pyrimidin-4-yl)amino)phenyl)acetamide

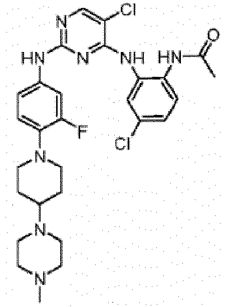

The title compound was prepared from the compound prepared in step 1) of Example 65 and the corresponding aniline in the same manner as in Example 1. [M+H]⁺ = 587.

### Example 68: Preparation of N-(2-((5-chloro-2-((2-methoxy-5-methyl-4-(4-(4-methylpiperazin-1-yl)piperidin-1-yl)phenyl)amino)pyrimidin-4-yl)amino)phenyl)acetamide

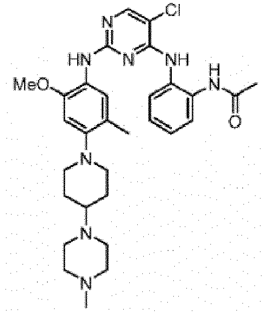

### Step 1) Preparation of N-(2-((2,5-dichloropyrimidin-4-yl)amino)phenyl)acetamide

The title compound was prepared from *N*-(2-aminophenyl)acetamide in the same manner as in step 1) of Example 1.

### Step 2) Preparation of N-(2-((5-chloro-2-((2-methoxy-5-methyl-4-(4-(4-methylpiperazin-1-yl)piperidin-1-yl)phenyl)amino)pyrimidin-4-yl)amino)phenyl)acetamide

The title compound was prepared in the same manner as in Example 1, except that the compound prepared in step 1) was used and 1-chloro-5-methoxy-2-methyl-4-nitrobenzene and 1-methyl-4-piperidin-4-yl-piperazine hydrochloride were used in step 2) of Example 1.

### Example 69: Preparation of N-(2-((5-chloro-2-((3-fluoro-4-(4-(4-methylpiperazin-1-yl)piperidin-1-yl)phenyl)amino)pyrimidin-4-yl)amino)phenyl)acetamide

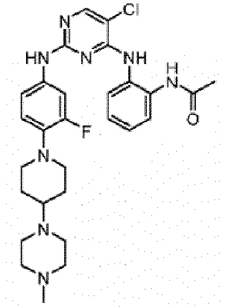

The title compound was prepared from the compound prepared in step 1) of Example 68 and the corresponding aniline in the same manner as in Example 1. [M+H]⁺ = 553.

### Example 70: Preparation of N-(2-((5-chloro-2-((5-fluoro-6-(4-(4-methylpiperazin-1-yl)piperidin-1-yl)pyridin-3-yl)-amino)pyrimidin-4-yl)amino)phenyl)acetamide

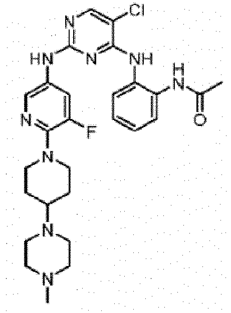

The title compound was prepared from the compound prepared in step 1) of Example 68 and the corresponding aniline in the same manner as in Example 1. [M+H]⁺ = 554.

### Example 71: Preparation of N-(4,5-dichloro-2-((5-chloro-2-((2-methoxy-5-methyl-4-(4-(4-methylpiperazin-1-yl)piperidin-1-yl)phenyl)amino)pyrimidin-4-yl)amino)phenyl)acetamide

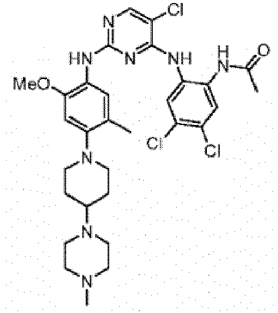

### Step 1) Preparation of N-(2-((2,5-dichloropyrimidin-4-yl)amino)phenyl)acetamide

5.0 g (22.8 mmol) of *N*-(2-amino-4,5-dichlorophenyl)acetamide and 3.37 ml (35.0 mmol) of 2,4,5-trichloropyrimidine were diluted with 54 ml of dimethyl sulfoxide, and 7.54 ml (53.8 mmol) of triethylamine and 841 mg (2.28 mmol) of tetrabutylammonium iodide were added thereto. The mixture was stirred at room temperature for 24 h. After completion of the reaction, the reaction mixture was cooled to room temperature, diluted with ethyl acetate, and washed with distilled water. The resulting residue was purified by column chromatography (50% ethyl acetate/hexane) to afford 5.01 g (yield: 60%) of the title compound.

### Step 2) Preparation of N-(4,5-dichloro-2-((5-chloro-2-((2-methoxy-5-methyl-4-(4-(4-methylpiperazin-1-yl)piperidin-1-yl)phenyl)amino)pyrimidin-4-yl)amino)phenyl)acetamide

The title compound was prepared in the same manner as in Example 1, except that the compound prepared in step 1) was used and 1-chloro-5-methoxy-2-methyl-4-nitrobenzene and 1-methyl-4-piperidin-4-yl-piperazine hydrochloride were used in step 2) of Example 1.

### Example 72: Preparation of N-(4,5-dichloro-2-((5-chloro-2-((2-methoxy-6-(4-(4-methylpiperazin-1-yl)piperidin-1-yl)pyridin-3-yl)amino)pyrimidin-4-yl)amino)phenyl)acetamide

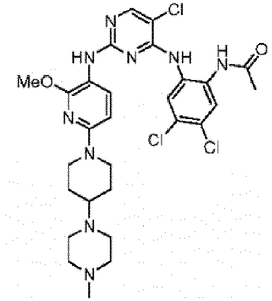

The title compound was prepared from the compound prepared in step 1) of Example 71 and the corresponding aniline in the same manner as in Example 1.

¹H NMR (400 MHz, DMSO-d6) δ 9.97 (brs, 1H), 8.37 (s, 1H), 8.10-7.88 (m, 3H), 7.69 (s, 1H), 7.51 (d, J = 8.5 Hz, 1H), 6.21 (d, J = 8.5 Hz, 1H), 4.19 (d, J = 12.7 Hz, 2H), 3.76 (s, 3H), 2.75 (t, J = 11.6 Hz, 3H), 2.64-2.30 (m, 11H), 2.09 (s, 3H), 1.87-1.75 (m, 2H), 1.46-1.32 (m, 2H). [M+H]⁺ = 639.

### Example 73: Preparation of N-(4,5-dichloro-2-((5-chloro-2-((5-fluoro-6-(4-(4-methylpiperazin-1-yl)piperidin-1-yl)pyridin-3-yl)amino)pyrimidin-4-yl)amino)phenyl)acetamide

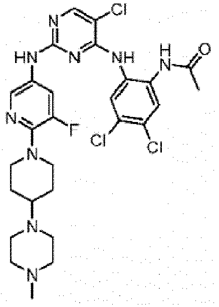

The title compound was prepared from the compound prepared in step 1) of Example 71 and the corresponding aniline in the same manner as in Example 1. [M+H]⁺ = 622.

### Example 74: Preparation of N-(4,5-dichloro-2-((5-chloro-2-((6-morpholinopyridin-3-yl)amino)pyrimidin-4-yl)amino)phenyl)acetamide

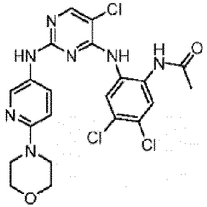

The title compound was prepared from the compound prepared in step 1) of Example 71 and the corresponding aniline in the same manner as in Example 1. [M+H]⁺ = 508.

### Example 75: Preparation of N-(4,5-dichloro-2-((5-chloro-2-((2-methoxy-4-(4-(4-methylpiperazin-1-yl)piperidin-1-yl)phenyl)amino)pyrimidin-4-yl)amino)phenyl)acetamide

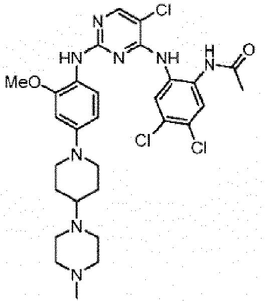

The title compound was prepared from the compound prepared in step 1) of Example 71 and the corresponding aniline in the same manner as in Example 1. [M+H]⁺ = 634.

### Example 76: Preparation of N-(4,5-dichloro-2-((5-chloro-2-((3-fluoro-4-(4-(4-methylpiperazin-1-yl)piperidin-1-yl)phenyl)amino)pyrimidin-4-yl)amino)phenyl)acetamide

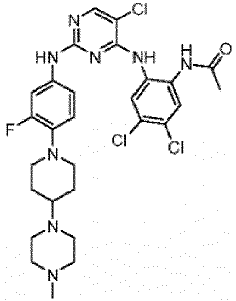

The title compound was prepared from the compound prepared in step 1) of Example 71 and the corresponding aniline in the same manner as in Example 1. [M+H]⁺ = 621.

### Example 77: Preparation of N-(4,5-dichloro-2-((5-chloro-2-((2-chloro-4-(4-(4-methylpiperazin-1-yl)piperidin-1-yl)phenyl)amino)pyrimidin-4-yl)amino)phenyl)acetamide

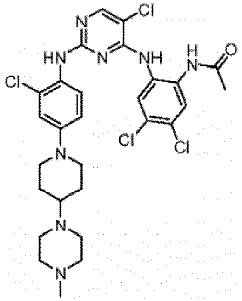

The title compound was prepared from the compound prepared in step 1) of Example 71 and the corresponding aniline in the same manner as in Example 1. [M+H]⁺ = 621.

### Example 78: Preparation of N-(4,5-dichloro-2-((5-chloro-2-((2-methoxy-5-methyl-4-(4-(4-methylpiperazin-1-yl)piperidin-1-yl)phenyl)amino)pyrimidin-4-yl)(methyl)amino)phenyl)-N-methylacetamide

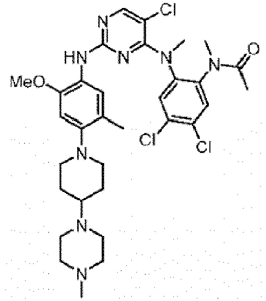

### Step 1) Preparation of N-(4,5-dichloro-2-((2,5-dichloropyrimidin-4-yl)(methyl)amino)phenyl)-N-methylacetamide

1.0 g (2.73 mmol) of the compound prepared in step 1) of Example 22 was diluted with 6 ml of *N*,*N*-dimethylformamide, and 138 mg (6.0 mmol) of 60% sodium hydride was slowly added thereto at 0 °C. The mixture was stirred at room temperature for 30 min. To the reaction mixture was slowly added 0.22 ml (3.6 mmol) of methyl iodide at 0 °C, followed by stirring at room temperature for 2 h. After completion of the reaction, the reaction mixture was diluted with ethyl acetate and washed with distilled water and saturated brine. The organic layer was separated, dried over anhydrous sodium sulfate, filtered under reduced pressure, and distilled under reduced pressure. The resulting residue was purified by chromatography (50% ethyl acetate/hexane) to afford 720 mg (yield: 66%) of the title compound.

### Step 2) Preparation of N-(4,5-dichloro-2-((5-chloro-2-((2-methoxy-5-methyl-4-(4-(4-methylpiperazin-1-yl)piperidin-1-yl)phenyl)amino)pyrimidin-4-yl)(methyl)amino)phenyl)-N-methylacetamide

The title compound was prepared in the same manner as in Example 1, except that the compound prepared in step 1) was used and 1-chloro-5-methoxy-2-methyl-4-nitrobenzene and 1-methyl-4-piperidin-4-yl-piperazine hydrochloride were used in step 2) of Example 1.

### Example 79: Preparation of N-(4,5-dichloro-2-((5-chloro-2-((2-methoxy-6-(4-(4-methylpiperazin-1-yl)piperidin-1-yl)pyridin-3-yl)amino)pyrimidin-4-yl)(methyl)amino)phenyl)-N-methylacetamide

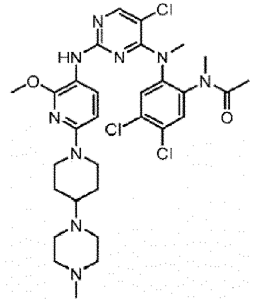

The title compound was prepared in the same manner as in Example 1, except that the compound prepared in step 1) of Example 78 was used and 6-chloro-2-methoxy-3-nitropyridine and 1-methyl-4-piperidin-4-yl-piperazine hydrochloride were used in step 2) of Example 1.

### Example 80: Preparation of N-(4-chloro-2-((5-chloro-2-((2-methoxy-5-methyl-4-(4-(4-methylpiperazin-1-yl)piperidin-1-yl)phenyl)amino)pyrimidin-4-yl)amino)phenyl)methanesulfonamide

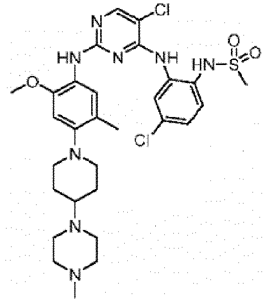

### Step 1) Preparation of N-(4-chloro-2-nitrophenyl)methanesulfonamide

5.0 g (29.0 mmol) of 4-chloro-2-nitroaniline was diluted with 60 ml of *N,N-*dimethylformamide, and 1.33 g (58.0 mmol) of 60% sodium hydride was slowly added thereto at 0 °C. The mixture was stirred at room temperature for 30 min. To the reaction mixture was slowly added 2.68 ml (34.8 mmol) of methanesulfonyl chloride at 0 °C, followed by stirring at room temperature for 2 h. After completion of the reaction, the reaction mixture was diluted with ethyl acetate and washed with distilled water and saturated brine. The organic layer was separated, dried over anhydrous sodium sulfate, filtered under reduced pressure, and distilled under reduced pressure. The resulting residue was purified by chromatography (20% ethyl acetate/hexane) to afford 5.1 g (yield: 70%) of the titled compound.

### Step 2) Preparation of N-(2-amino-4-chlorophenyl)methanesulfonamide

5.1 g (20.0 mmol) of the compound prepared in step 1) was dissolved in 100 ml of a mixture of tetrahydrofuran, methanol, and water in an 18:1:1 volume ratio. To the solution were added 11.2 g (200 mmol) of an iron powder and 10.8 g (200 mmol) of ammonium chloride, followed by stirring at 85 °C for 3 h. After completion of the reaction, the reaction mixture was washed with dichloromethane on a filter filled with celite, filtered under reduced pressure, and concentrated to afford 3.96 g (yield: 90%) of the title compound.

### Step 3) Preparation of N-(4-chloro-2-((2,5-dichloropyrimidin-4-yl)amino)phenyl)methanesulfonamide

The title compound was prepared in the same manner as in Example 21, except that the compound prepared in step 2) was used in step 2) of Example 21.

### Step 4) Preparation of N-(4-chloro-2-((5-chloro-2-((2-methoxy-5-methyl-4-(4-(4-methylpiperazin-1-yl)piperidin-1-yl)phenyl)amino)pyrimidin-4-yl)amino)phenyl)methanesulfonamide

The title compound was prepared in the same manner as in Example 1, except that the compound prepared in step 3) was used and 1-chloro-5-methoxy-2-methyl-4-nitrobenzene and 1-methyl-4-piperidin-4-yl-piperazine hydrochloride were used in step 2) of Example 1.

¹H NMR (400 MHz, DMSO-d6) δ 8.74 (brs, 1H), 8.13-8.07 (m, 2H), 8.04 (brs, 1H), 7.36-7.26 (m, 2H), 7.09 (dd, J = 8.6, 2.4 Hz, 1H), 6.68 (s, 1H), 3.76 (s, 3H), 3.14-3.02 (m, 2H), 2.88 (s, 3H), 2.77-2.24 (m, 14H), 2.06 (s, 3H), 1.94-1.80 (m, 2H), 1.64-1.48 (m, 2H).

### Example 81: Preparation of N-(4-chloro-2-((5-chloro-2-((2-methoxy-6-(4-(4-methylpiperazin-1-yl)piperidin-1-yl)pyridin-3-yl)amino)pyrimidin-4-yl)aminophenyl)methanesulfonamide

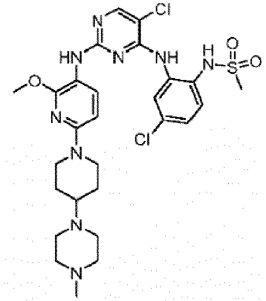

The title compound was prepared from the compound prepared in step 3) of Example 80 and the corresponding aniline in the same manner as in Example 1.

¹H NMR (400 MHz, DMSO-d6) δ 8.66 (s, 1H), 8.23 (brs, 1H), 8.17-8.04 (m, 2H), 7.52 (d, J = 8.4 Hz, 1H), 7.28 (d, J = 8.5 Hz, 1H), 7.05 (d, J = 6.9 Hz, 1H), 6.31 (d, J = 8.5 Hz, 1H), 4.25 (d, J = 13.0 Hz, 2H), 3.78 (s, 3H)), 2.94 (d, J = 19.2 Hz, 3H), 2.76 (t, J = 11.8 Hz, 3H), 2.68-2.42 (m, 8H), 2.32 (s, 3H), 1.90-1.79 (m, 2H), 1.48-1.33 (m, 2H). [M+H]⁺ = 636.

### Example 82: Preparation of N-(4-chloro-2-((5-chloro-2-((2-methoxy-4-(4-(4-methylpiperazin-1-yl)piperidin-1-yl)phenyl)amino)pyrimidin-4-yl)amino)phenyl)methanesulfonamide

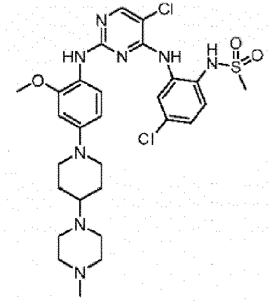

The title compound was prepared from the compound prepared in step 3) of Example 80 and the corresponding aniline in the same manner as in Example 1. ¹H NMR (400 MHz, DMSO-d6) δ 8.73 (brs, 1H), 8.14 (brs, 1H), 8.12-8.02 (m, 2H), 7.38 (d, J = 8.6 Hz, 1H), 7.29 (d, J = 8.5 Hz, 1H), 7.06 (dd, J = 8.5, 2.3 Hz, 1H), 6.61 (d, J = 1.9 Hz, 1H), 6.50-6.41 (m, 1H), 3.83-3.63 (m, 5H), 2.89 (s, 3H), 2.76-2.20 (m, 14H), 1.92-1.80 (m, 2H), 1.60-1.44 (m, 2H). [M+H]⁺ = 635.

### Example 83: Preparation of N-(4-chloro-2-((5-chloro-2-((2-methoxy-5-methyl-6-(4-(4-methylpiperazin-1-yl)piperidin-1-yl)pyridin-3-yl)amino)pyrimidin-4-yl)amino)phenyl)methanesulfonamide

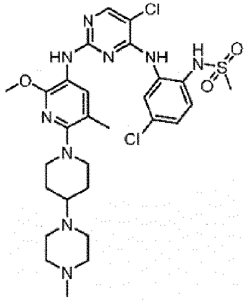

The title compound was prepared from the compound prepared in step 3) of Example 80 and the corresponding aniline in the same manner as in Example 1. [M+H]⁺ = 650.

### Example 84: Preparation of N-(4-chloro-2-((5-chloro-2-((3-fluoro-4-(4-(4-methylpiperazin-1-yl)piperidin-1-yl)phenyl)amino)pyrimidin-4-yl)amino)phenyl)methanesulfonamide

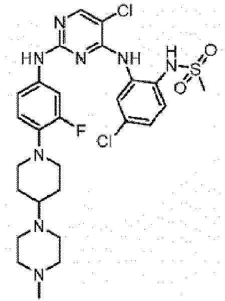

The title compound was prepared from the compound prepared in step 3) of Example 80 and the corresponding aniline in the same manner as in Example 1. [M+H]⁺ = 623.

### Example 85: Preparation of N-(2-((5-bromo-2-((2-methoxy-5-methyl-4-(4-(4-methylpiperazin-1-yl)piperidin-1-yl)phenyl)amino)pyrimidin-4-yl)amino-4-chlorophenyl)methanesulfonamide

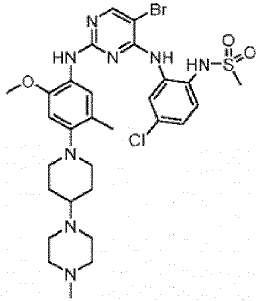

### Step 1) Preparation of N-(2-((5-bromo-2-chloro-4-yl)amino)-4-chlorophenyl)methanesulfonamide

The title compound was prepared in the same manner as in step 3) of Example 80, except that 5-bromo-2,4-dichloropyrimidine was used.

### Step 2)

The title compound was prepared in the same manner as in Example 1, except that the compound prepared in step 1) was used and 1-chloro-5-methoxy-2-methyl-4-nitrobenzene and 1-methyl-4-piperidin-4-yl-piperazine hydrochloride were used in step 2) of Example 1.

¹H NMR (400 MHz, DMSO-d6) δ 8.67 (brs, 1H), 8.19-8.11 (m, 2H), 8.07 (brs, 1H), 7.33-7.26 (m, 2H), 7.07 (dd, J = 8.5, 2.4 Hz, 1H), 6.69 (s, 1H), 3.76 (s, 3H), 3.14-3.03 (m, 2H), 2.90 (s, 3H), 2.78-2.24 (m, 14H), 2.09 (s, 3H), 1.92-1.81 (m, 2H), 1.64-1.49 (m, 2H).

### Example 86: Preparation of N-(2-((5-bromo-2-((2-methoxy-5-methyl-6-(4-(4-methylpiperazin-1-yl)piperidin-1-yl)pyridin-3-yl)amino)pyrimidin-4-yl)amino)-4-chlorophenyl)methanesulfonamide

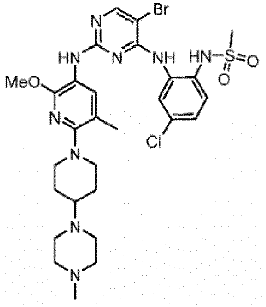

The title compound was prepared from the compound prepared in step 1) of Example 85 and the corresponding aniline in the same manner as in Example 1. [M+H]⁺ = 694.

### Example 87: Preparation of N-(2-((5-bromo-2-((3-fluoro-4-(4-(4-methylpiperazin-1-yl)piperidin-1-yl)phenyl)amino)pyrimidin-4-yl)amino)-4-chlorophenyl)methanesulfonamide

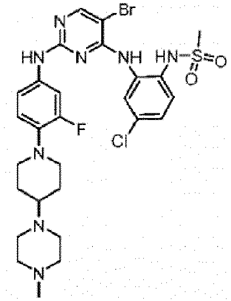

The title compound was prepared from the compound prepared in step 1) of Example 85 and the corresponding aniline in the same manner as in Example 1. [M+H]⁺ = 667.

### Example 88: Preparation of N-(2-((5-bromo-2-((5-chloro-2-methoxy-4-(4-(4-methylpiperazin-1-yl)piperidin-1-yl)phenyl)amino)pyrimidin-4-yl)amino)-4-chlorophenyl)methanesulfonamide

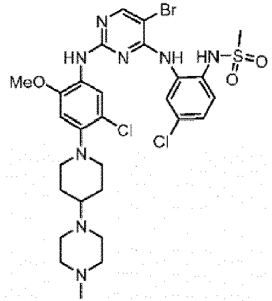

The title compound was prepared from the compound prepared in step 1) of Example 85 and the corresponding aniline in the same manner as in Example 1.

¹H NMR (400 MHz, DMSO-d6) δ 8.68 (brs, 1H), 8.25-8.20 (m, 2H), 8.04 (brs, 1H), 7.58 (s, 1H), 7.30 (t, J = 8.7 Hz, 1H), 7.10 (dd, J = 8.5, 2.4 Hz, 1H), 6.78 (s, 1H), 3.80 (s, 3H), 3.36-3.25 (m, 2H), 2.92 (s, 3H), 2.80-2.27 (m, 14H), 1.94-1.80 (m, 2H), 1.66-1.52 (m, 2H). [M+H]⁺ = 713.

### Example 89: Preparation of N-(2-((5-bromo-2-((2-methoxy-5-methyl-4-(4-(4-methyl-1,4-diazepan-1 -yl)piperidin-1 -yl)phenyl)amino)pyrimidin-4-yl)amino)-4-chlorophenyl)methanesulfonylamide

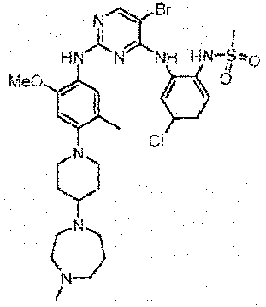

The title compound was prepared from the compound prepared in step 1) of Example 85 and the corresponding aniline in the same manner as in Example 1. [M+H]⁺ = 707.

### Example 90: Preparation of N-(2-((5-Bromo-2-((4-(3-(dimethylamino)-[1,4'-bipiperidin]-1'-yl)-2-methoxy-5-methylphenyl)amino)pyrimidin-4-yl)amino)-4-chlorophenyl)methanesulfonamide

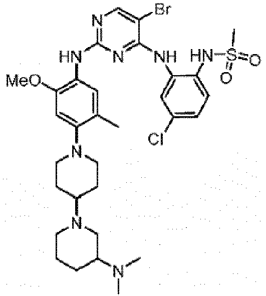

The title compound was prepared from the compound prepared in step 1) of Example 85 and the corresponding aniline in the same manner as in Example 1. [M+H]⁺ = 721.

### Example 91: Preparation of N-(2-((5-bromo-2-((4-(4-(3-(dimethylamino)pyrrolidin-1 -yl)piperidin-1 -yl)-2-methoxy-5-methylphenyl)amino)pyrimidin-4-yl)amino)-4-chlorophenyl)methanesulfonamide

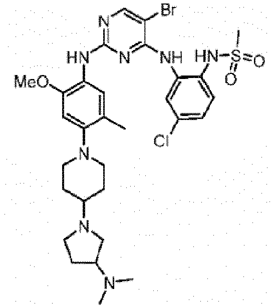

The title compound was prepared from the compound prepared in step 1) of Example 85 and the corresponding aniline in the same manner as in Example 1. [M+H]⁺ = 707.

### Example 92: Preparation of N-(2-((5-bromo-2-((5-fluoro-2-methoxy-4-(4-(4-methylpiperazin-1-yl)piperidin-1-yl)phenyl)amino)pyrimidin-4-yl)amino)-4-chlorophenyl)methanesulfonamide

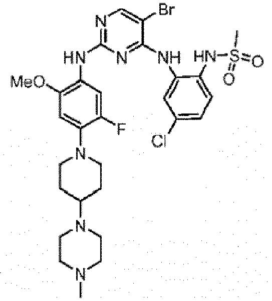

The title compound was prepared from the compound prepared in step 1) of Example 85 and the corresponding aniline in the same manner as in Example 1. [M+H]⁺ = 696.

### Example 93: Preparation of N-(2-((5-bromo-2-((2-chloro-5-methyl-4-(4-(4-methylpiperazin-1-yl)piperidin-1-yl)phenyl)amino)pyrimidin-4-yl)amino)-4-chlorophenyl)methanesulfonamide

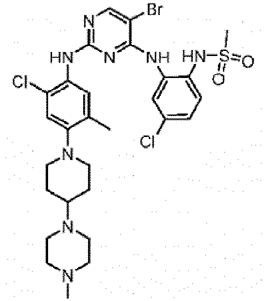

The title compound was prepared from the compound prepared in step 1) of Example 85 and the corresponding aniline in the same manner as in Example 1. [M+H]⁺ = 698.

### Example 94: Preparation of N-(2-((5-bromo-2-((2-methoxy-5-methyl-4-(4-(piperazin-1-yl)piperidin-1-yl)phenyl)amino)pyrimidin-4-yl)amino)-4-chlorophenyl)methanesulfonamide

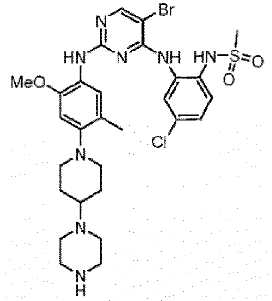

The title compound was prepared from the compound prepared in step 1) of Example 85 and the corresponding aniline in the same manner as in Example 1. [M+H]⁺ = 680.

### Example 95: Preparation of N-(2-((5-bromo-2-((5-chloro-2-methoxy-4-(4-(4-methyl-1,4-diazepan-1 -yl)piperidin-1 -yl)phenyl)amino)pyrimidin-4-yl)amino)-4-chlorophenyl)methanesulfonamide

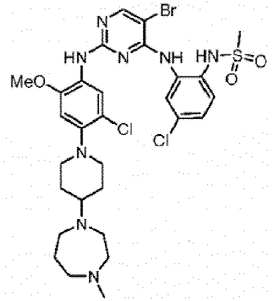

The title compound was prepared from the compound prepared in step 1) of Example 85 and the corresponding aniline in the same manner as in Example 1. [M+H]⁺ = 728.

### Example 96: Preparation of N-(2-((5-bromo-2-((2-chloro-4-(4-(3-(dimethylamino)pyrrolidin-1 -yl)piperidin-1 -yl)-5-methylphenyl)amino)pyrimidin-4-yl)amino)-4-chlorophenyl)methanesulfonamide

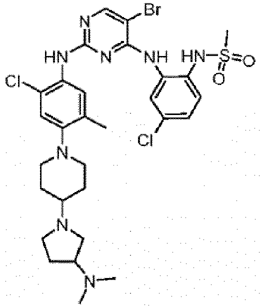

The title compound was prepared from the compound prepared in step 1) of Example 85 and the corresponding aniline in the same manner as in Example 1. [M+H]⁺ = 712.

### Example 97: Preparation of N-(2-((5-bromo-2-((2-chloro-5-methyl-4-(4-(4-methyl-1,4-diazepan-1 -yl)piperidin-1 -yl)phenyl)amino)pyrimidin-4-yl)amino)-4-chlorophenyl)methanesulfonamide

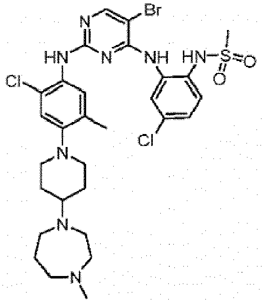

The title compound was prepared from the compound prepared in step 1) of Example 85 and the corresponding aniline in the same manner as in Example 1. [M+H]⁺ = 712.

### Example 98: Preparation of N-(2-((5-bromo-2-((2-chloro-4-(3-(dimethylamino)-[1,4'-bipiperidin]-1'-yl)-5-methylphenyl)amino)pyrimidin-4-yl)amino)-4-chlorophenyl)methanesulfonamide

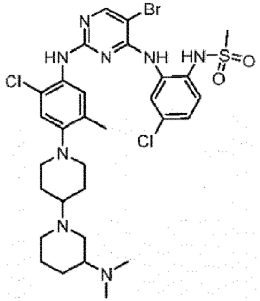

The title compound was prepared from the compound prepared in step 1) of Example 85 and the corresponding aniline in the same manner as in Example 1. [M+H]⁺ = 726.

### Example 99: Preparation of N-(4,5-dichloro-2-((5-chloro-2-((2-methoxy-5-methyl-4-(4-(4-methylpiperazin-1-yl)piperidin-1-yl)phenyl)amino)pyrimidin-4-yl)amino)phenyl)methanesulfonamide

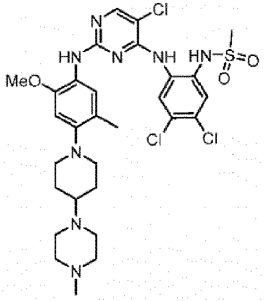

### Step 1) Preparation of N-(4,5-dichloro-2-((2,5-dichloropyrimidin-4-yl)amino)phenyl)methanesulfonamide

The title compound was prepared in the same manner as in step 3) of Example 80, except that 4,5-dichloro-2-nitroaniline was used in step 1) of Example 80.

### Step 2) Preparation of N-(4,5-dichloro-2-((5-chloro-2-((2-methoxy-5-methyl-4-(4-(4-methylpiperazin-1-yl)piperidin-1-yl)phenyl)amino)pyrimidin-4-yl)amino)phenyl)methanesulfonamide

The title compound was prepared in the same manner as in Example 1, except that the compound prepared in step 1) was used and 1-chloro-5-methoxy-2-methyl-4-nitrobenzene and 1-methyl-4-piperidin-4-yl-piperazine hydrochloride were used in step 2) of Example 1. [M+H]⁺ = 684.

### Example 100: Preparation of N-(4,5-dichloro-2-((5-chloro-2-((2-methoxy-5-methyl-6-(4-(4-methylpiperazin-1-yl)piperidin-1-yl)pyridin-3-yl)amino)pyrimidin-4-yl)amino)phenylmethanesulfonamide

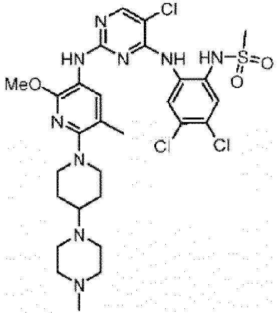

The title compound was prepared from the compound prepared in step 1) of Example 99 and the corresponding aniline in the same manner as in Example 1. [M+H]⁺ = 685.

### Example 101: Preparation of N-(4,5-dichloro-2-((5-chloro-2-((3-fluoro-4-(4-(4-methylpiperazin-1-yl)piperidin-1-yl)phenyl)amino)pyrimidin-4-yl)amino)phenyl)methanesulfonamide

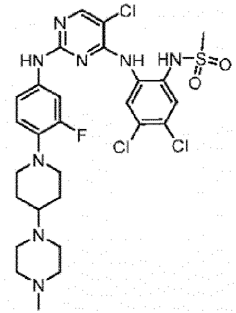

The title compound was prepared from the compound prepared in step 1) of Example 99 and the corresponding aniline in the same manner as in Example 1. [M+H]⁺ = 658.

### Example 102: Preparation of N-(4,5-dichloro-2-((5-chloro-2-((2-(4-(4-methylpiperazin-1-yl)piperidin-1-yl)quinolin-6-yl)amino)pyrimidin-4-yl)amino)phenyl)methanesulfonamide

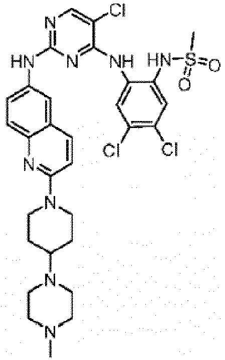

The title compound was prepared from the compound prepared in step 1) of Example 99 and the corresponding aniline in the same manner as in Example 1. [M+H]⁺ = 691.

### Example 103: Preparation of N-(4,5-dichloro-2-((5-chloro-2-((5-chloro-2-methoxy-4-(4-(4-methylpiperazin-1-yl)piperidin-1-yl)phenyl)amino)pyrimidin-4-yl)aminophenyl)methanesulfonamide

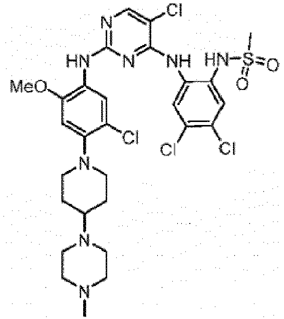

The title compound was prepared from the compound prepared in step 1) of Example 99 and the corresponding aniline in the same manner as in Example 1. [M+H]⁺ = 704.

### Example 104: Preparation of N-(4,5-dichloro-2-((5-chloro-2-((4-(4-((2-(dimethylamino)ethyl)(methyl)amino)piperidin-1-yl)-2-methoxy-5-methylphenyl)amino)pyrimidin-4-yl)amino)phenyl)methanesulfonamide

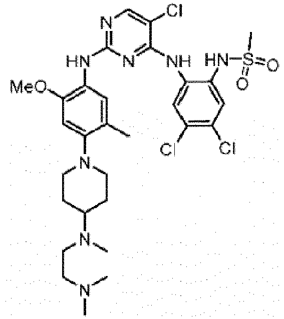

The title compound was prepared from the compound prepared in step 1) of Example 99 and the corresponding aniline in the same manner as in Example 1. [M+H]⁺ = 686.

### Example 105: Preparation of N-(4,5-dichloro-2-((5-chloro-2-((2-methoxy-5-methyl-4-(methyl(3-(4-methylpiperazin-1-yl)propyl)amino)phenyl)amino)pyrimidin-4-yl)amino)phenyl)methanesulfonamide

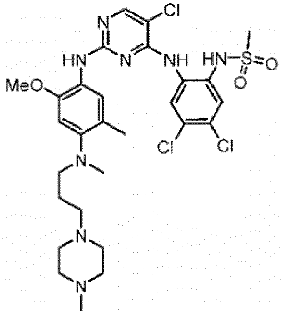

The title compound was prepared from the compound prepared in step 1) of Example 99 and the corresponding aniline in the same manner as in Example 1. [M+H]⁺ = 672.

### Example 106: Preparation of N-(2-((5-bromo-2-((2-methoxy-5-methyl-4-(4-(4-methylpiperazin-1-yl)piperidin-1-yl)phenyl)amino)pyrimidin-4-yl)amino)-4-methylphenyl)methanesulfonamide

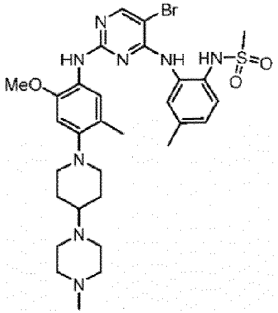

### Step 1) Preparation of N-(2-((5-bromo-2-chloropyrimidin-4-yl)amino)-4-methylphenyl)methanesulfonamide

The title compound was prepared in the same manner as in step 3) of Example 24, except that 4-methyl-2-nitroaniline was used in step 1) of Example 24 and 5-bromo-2,4-dichloropyrimidine was used in step 3) of Example 24.

### Step 2) Preparation of N-(2-((5-bromo-2-((2-methoxy-5-methyl-4-(4-(4-methylpiperazin-1-yl)piperidin-1-yl)phenyl)amino)pyrimidin-4-yl)amino)-4-methylphenyl)methanesulfonamide

The title compound was prepared in the same manner as in Example 1, except that the compound prepared in step 1) was used and 1-chloro-5-methoxy-2-methyl-4-nitrobenzene and 1-methyl-4-piperidin-4-yl-piperazine hydrochloride were used in step 2) of Example 1.

### Example 107: Preparation of N-(2-((5-bromo-2-((5-chloro-2-methoxy-4-(4-(4-methylpiperazin-1-yl)piperidin-1-yl)phenyl)amino)pyrimidin-4-yl)amino)-4-methylphenyl)methanesulfonamide

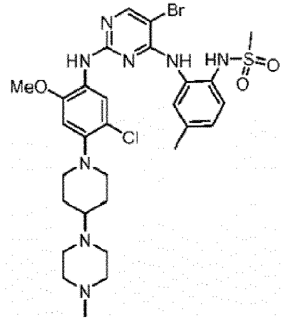

The title compound was prepared from the compound prepared in step 1) of Example 106 and the corresponding aniline in the same manner as in Example 1. [M+H]⁺ = 694.

### Example 108: Preparation of N-(2-((5-bromo-2-((5-fluoro-2-methoxy-4-(4-(4-methoxy-1 -yl)piperidin-1 -yl)phenyl)amino)pyrimidin-4-yl)amino)-4-methylphenyl)methanesulfonamide

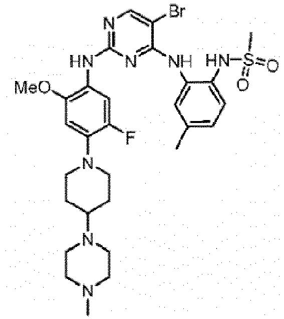

The title compound was prepared from the compound prepared in step 1) of Example 106 and the corresponding aniline in the same manner as in Example 1. [M+H]⁺ = 677.

### Example 109: Preparation of N-(2-((5-bromo-2-((2-chloro-5-methyl-4-(4-(4-methylpiperazin-1-yl)piperidin-1-yl)phenyl)amino)pyrimidin-4-yl)amino)-4-methylphenyl)methanesulfonamide

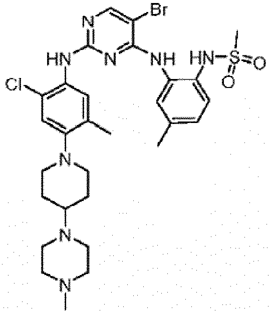

The title compound was prepared from the compound prepared in step 1) of Example 106 and the corresponding aniline in the same manner as in Example 1. [M+H]⁺ = 678.

### Example 110: Preparation of N-(5-chloro-2-((2-methoxy-5-methyl-4-(4-(4-methylpiperazin-1-yl)piperidin-1-yl)phenyl)amino)pyrimidin-4-yl)-N-(4-(methylsulfonamido)benzo[d]thiazol-5-yl)methanesulfonamide

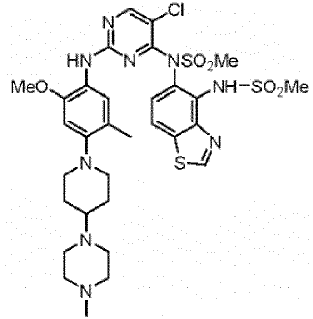

### Step 1) Preparation of N-(2,5-dichloropyrimidin-4-yl)-4-nitrobenzo[d]thiazol-5-amine

5.0 g (25.6 mmol) of 4-nitrobenzo[d]thiazol-5-amine was diluted with 50 ml of *N,N*-dimethylformamide, and 883 mg (38.4 mmol) of 60% sodium hydride was slowly added thereto at 0 °C. The mixture was stirred at room temperature for 30 min. To the reaction mixture was slowly added 3.5 ml (30.7 mmol) of 2,4,5-trichloropyrimidine at 0 °C, followed by stirring at room temperature for 2 h. After completion of the reaction, the reaction mixture was diluted with ethyl acetate and washed with distilled water and saturated brine. The organic layer was separated, dried over anhydrous sodium sulfate, filtered under reduced pressure, and distilled under reduced pressure. The resulting residue was purified by chromatography (40% ethyl acetate/hexane) to afford 6.84 g (yield: 52%).

### Step 2) Preparation of N⁵-(2,5-dichloropyrimidin-4-yl)benzo[d]thiazole-4,5-diamine

6.84 g (20.0 mmol) of the compound prepared in step 1) was diluted with 40 ml of a mixture of tetrahydrofuran, methanol, and water in an 18:1:1 volume ratio. To the dilution were added 11.2 g (200 mmol) of an iron powder and 10.8 g (200 mmol) of ammonium chloride, followed by stirring at 85 °C for 3 h. After completion of the reaction, the reaction mixture was washed with dichloromethane on a filter filled with celite, filtered under reduced pressure, and concentrated to afford 5.6 g (yield: 90%) of the title compound.

### Step 3) Preparation of N-(2,S-dichloropyrimidin-4-yl)-N-(4-(methylsulfonamido)benzo[d]thiazol-5-yl)methanesulfonamide

5.6 g (18.0 mmol) of the compound prepared in step 2) was diluted with 40 ml of *N,N*-dimethylformamide, and 5.0 ml (36.0 mmol) of triethylamine and 3.2 ml (45.0 mmol) of methanesulfonyl chloride were slowly added thereto at 0 °C. The mixture was stirred at room temperature for 2 h. After completion of the reaction, the reaction mixture was diluted with ethyl acetate and washed with distilled water and saturated brine. The organic layer was separated, dried over anhydrous sodium sulfate, filtered under reduced pressure, and distilled under reduced pressure. The resulting residue was purified by chromatography (50% ethyl acetate/hexane) to afford 7.02 g (yield: 83%) of the title compound.

### Step 4) Preparation of N-(5-chloro-2-((2-methoxy-5-methyl-4-(4-(4-methylpiperazin-1-yl)piperidin-1-yl)phenyl)amino)pyrimidin-4-yl)-N-(4-(methylsulfonamido)benzo[d]thiazol-5-yl)methanesulfonamide

The title compound was prepared in the same manner as in Example 1, except that the compound prepared in step 1) was used and 1-chloro-5-methoxy-2-methyl-4-nitrobenzene and 1-methyl-4-piperidin-4-yl-piperazine hydrochloride were used in step 2) of Example 1. [M+H]⁺ = 750.

### Example 111: Preparation of N-(5-chloro-2-((2-methoxy-6-(4-(4-methylpiperazin-1-yl)piperidin-1-yl)pyridin-3-yl)amino)pyrimidin-4-yl)-N-(4-(methylsulfonamido)benzo[d]thiazol-5-yl)methanesulfonamide

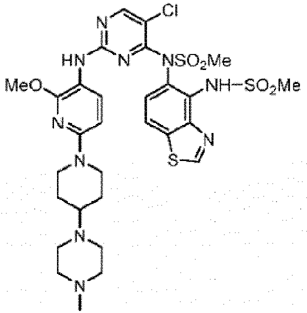

The title compound was prepared from the compound prepared in step 3) of Example 110 and the corresponding aniline in the same manner as in Example 1. [M+H]⁺ = 737.

### Example 112: Preparation of N-(5-chloro-2-((3-fluoro-4-(4-(4-methylpiperazin-1-yl)piperidin-1-yl)phenyl)amino)pyrimidin-4-yl)-N-(4-(methylsulfonamido)benzo[d]thiazol-5-yl)methanesulfonamide

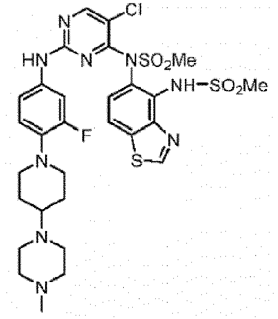

The title compound was prepared from the compound prepared in step 3) of Example 110 and the corresponding aniline in the same manner as in Example 1. [M+H]⁺ = 724.

### Example 113: Preparation of N-(5-chloro-2-((5-fluoro-6-(4-(4-methylpiperazin-1-yl)piperidin-1-yl)pyridin-3-yl)amino)pyrimidin-4-yl)-N-(4-(methylsulfonamido)benzo[d]thiazol-5-yl)methanesulfonamide

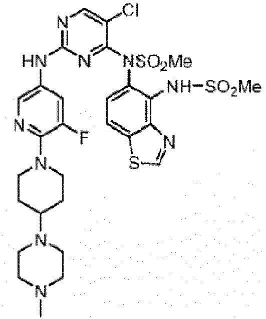

The title compound was prepared from the compound prepared in step 3) of Example 110 and the corresponding aniline in the same manner as in Example 1. [M+H]⁺ = 725.

### Example 114: Preparation of N-(5-chloro-2-((5-chloro-2-methoxy-4-(4-(4-methylpiperazin-1-yl)piperidin-1-yl)phenyl)amino)pyrimidin-4-yl)-N-(4-(methylsulfonamido)benzo[d]thiazol-5-yl)methanesulfonamide

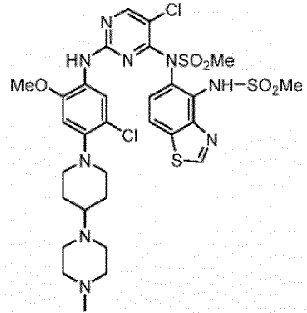

The title compound was prepared from the compound prepared in step 3) of Example 110 and the corresponding aniline in the same manner as in Example 1. [M+H]⁺ = 770.

### Example 115: Preparation of N-(6-((5-chloro-2-((2-methoxy-5-methyl-4-(4-(4-methylpiperazin-1-yl)piperidin-1-yl)phenyl)amino)pyrimidin-4-yl)amino)quinolin-5-yl)methanesulfonamide

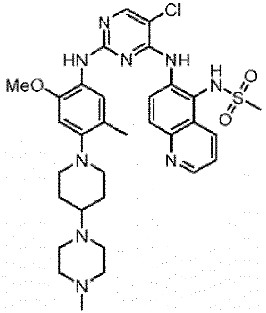

### Step 1) Preparation of N-(6-((2,5-dichloropyrimidin-4-yl)amino)quinolin-5-yl)methanesulfonamide

The title compound was prepared in the same manner as in Example 80, except that 6-nitroquinolin-5-amine and 2,4-dichloropyrimidine were used in step 1) of Example 80.

### Step 2)

The title compound was prepared in the same manner as in Example 1, except that the compound prepared in Example 1 was used and 1-chloro-5-methoxy-2-methyl-4-nitrobenzene and 1-methyl-4-piperidin-4-yl-piperazine hydrochloride were used in step 2) of Example 1.

### Example 116: Preparation of N-(6-((5-chloro-2-((2-methoxy-5-methyl-6-(4-(4-methylpiperazin-1-yl)piperidin-1-yl)pyridin-3-yl)amino)pyrimidin-4-yl)amino)quinolin-5-yl)methanesulfonamide

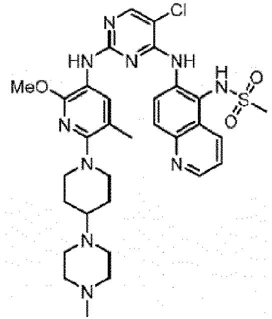

The title compound was prepared from the compound prepared in step 1) of Example 115 and the corresponding aniline in the same manner as in Example 1. [M+H]⁺ = 667.

### Example 117: Preparation of N-(6-((5-chloro-2-((2-methoxy-6-(4-morpholinopiperidin-1-yl)pyridin-3-yl)amino)pyrimidin-4-yl)amino)quinolin-5-yl)methanesulfonamide

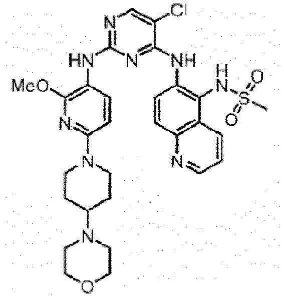

The title compound was prepared from the compound prepared in step 1) of Example 115 and the corresponding aniline in the same manner as in Example 1. [M+H]⁺ = 640.

### Example 118: Preparation of N-(6-((5-chloro-2-((5-chloro-2-methoxy-4-(4-(4-methoxypiperazin-1-yl)piperidin-1-yl)phenyl)amino)pyrimidin-4-yl)amino)quinolin-5-yl)methanesulfonamide

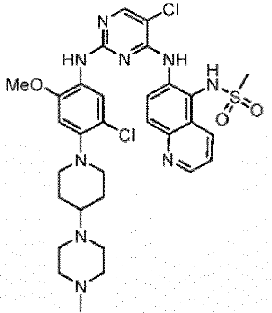

The title compound was prepared from the compound prepared in step 1) of Example 115 and the corresponding aniline in the same manner as in Example 1. [M+H]⁺ = 686.

### Example 119: Preparation of N-(6-((5-chloro-2-((2-methoxy-4-(4-methylpiperazin-1-yl)phenyl)amino)pyrimidin-4-yl)amino)quinolin-5-yl)methanesulfonamide

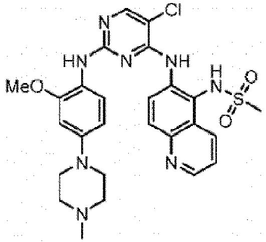

The title compound was prepared from the compound prepared in step 1) of Example 115 and the corresponding aniline in the same manner as in Example 1. [M+H]⁺ = 569.

### Example 120: Preparation of N-(2-((5-chloro-2-((2-methoxy-5-methyl-4-(4-(4-methylpiperazin-1-yl)piperidin-1-yl)phenyl)amino)pyrimidin-4-yl)(methyl)amino)phenyl)methanesulfonamide

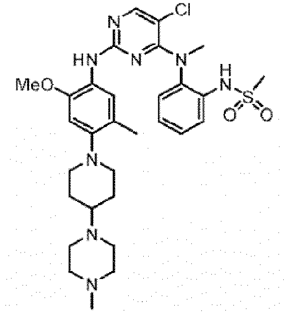

### Step 1) Preparation of 2,5-dichloro-N-methyl-N-(2-nitrophenyl)pyrimidin-4-amine

5.0 g (32.9 mmol) of *N*-methyl-2-nitroaniline was diluted with 50 ml of *N,N-*dimethylformamide, and 883 mg (38.4 mmol) of 60% sodium hydride was slowly added thereto at 0 °C. The mixture was stirred at room temperature for 30 min. To the reaction mixture was slowly added 4.6 ml (40.0 mmol) of 2,4,5-trichloropyrimidine at 0 °C, followed by stirring at room temperature for 2 h. After completion of the reaction, the reaction mixture was diluted with ethyl acetate and washed with distilled water and saturated brine. The organic layer was separated, dried over anhydrous sodium sulfate, filtered under reduced pressure, and distilled under reduced pressure. The resulting residue was purified by chromatography (20% ethyl acetate/hexane) to afford 5.98 g (yield: 61%) of the title compound.

### Step 2) Preparation of N¹-(2,5-dichloropyrimidin-4-yl)-N¹-methylbenzene-1,2-diamine

5.98 g (20.0 mmol) of the compound prepared in step 1) was dissolved in 40 ml of a mixture of tetrahydrofuran, methanol, and water in an 18:1:1 volume ratio. To the solution were added 11.8 g (200 mmol) of an iron powder and 10.8 g (200 mmol) of ammonium chloride, followed by stirring at 85 °C for 3 h. After completion of the reaction, the reaction mixture was extracted with dichloromethane on a filter filled with celite, filtered under reduced pressure, and concentrated to afford 4.8 g (yield: 90%) of the title compound.

### Step 3) Preparation of N-(2-((2,5-dichloropyrimidin-4-yl)(methyl)amino)phenyl)methanesulfonamide

The title compound was prepared from 4.8 g (18.0 mmol) of the compound prepared in step 2) in the same manner as in step 1) of Example 26.

### Step 4) Preparation of N-(2-((5-chloro-2-((2-methoxy-5-methyl-4-(4-(4-methylpiperazin-1-yl)piperidin-1-yl)phenyl)amino)pyrimidin-4-yl)(methyl)amino)phenyl)methanesulfonamide

The title compound was prepared in the same manner as in Example 1, except that the compound prepared in step 3) was used and 1-chloro-5-methoxy-2-methyl-4-nitrobenzene and 1-methyl-4-piperidin-4-yl-piperazine hydrochloride were used in step 2) of Example 1.

### Example 121: Preparation of N-(2-((5-chloro-2-((2-methoxy-5-methyl-6-(4-(4-methylpiperazin-1-yl)piperidin-1-yl)pyridin-3-yl)amino)pyrimidin-4-yl)(methyl)amino)phenyl)methanesulfonamide

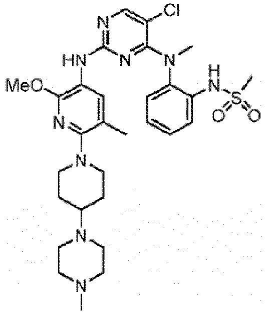

The title compound was prepared in the same manner as in Example 1, except that the compound prepared in step 3) of Example 120 was used and 2-chloro-6-methoxy-3-methyl-5-nitropyridine and 1-methyl-4-piperidin-4-yl-piperazine hydrochloride were used in step 2) of Example 1.

### Example 122: Preparation of (6-((5-chloro-2-((2-methoxy-5-methyl-6-(4-(4-methylpiperazin-1-yl)piperidin-1-yl)pyridin-3-yl)amino)pyrimidin-4-yl)amino)quinoxalin-5-yl)dimethylphosphine oxide

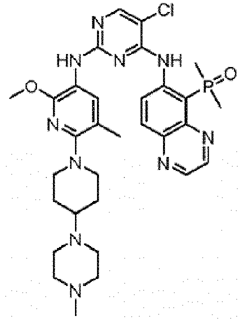

### Step 1) Preparation of 6-nitroquinoxaline

5.0 g of 4-nitrobenzene-1,2-diamine (32.7 mmol) was diluted with 70 ml of ethanol, and a 40% glyoxal solution (35 mmol) was added thereto. The mixture was stirred at 80 °C for 12 h. After completion of the reaction, the reaction mixture was cooled to room temperature, extracted with ethyl acetate and distilled water, filtered under reduced pressure, and concentrated to afford 4.9 g (yield: 86%) of the title compound.

### Step 2) Preparation of quinoxalin-6-amine

4.9 g (28.0 mmol) of the compound prepared in step 1) was dissolved in 60 ml of a mixture of tetrahydrofuran, methanol, and water in an 18:1:1 volume ratio. To the solution were added 11.8 g (200 mmol) of an iron powder and 10.8 g (200 mmol) of ammonium chloride, followed by stirring at 85 °C for 3 h. After completion of the reaction, the reaction mixture was cooled to room temperature, extracted with dichloromethane on a filter filled with celite, filtered under reduced pressure, and concentrated to afford 3.6 g (yield: 89%) of the title compound.

### Step 3) Preparation of 5-iodoquinoxalin-6-amine

3.6 g (25.0 mmol) of the compound prepared in step 2) was diluted with 60 ml of acetic acid, and 4.9 g (30.0 mmol) of iodine monochloride was added thereto. The mixture was stirred at room temperature for 2 h. After completion of the reaction, the reaction mixture was concentrated with a saturated sodium bicarbonate solution and dichloromethane to afford 4.9 g (yield: 72%) of the title compound.

### Step 4) Preparation of (6-aminoquinoxalin-5-yl)dimethylphosphine oxide

4.9 g (18.0 mmol) of the compound prepared in step 3), 1.68 g (21.6 mmol) of dimethylphosphine oxide, and 7.6 g (36.0 mmol) of tripotassium phosphate were diluted with 60 ml of a mixture of *N,N*-dimethylformamide and distilled water in a 5:1 volume ratio. To the dilution were added catalytic amounts of xantphos and palladium diacetate, followed by stirring at 120 °C for 24 h. After completion of the reaction, the reaction mixture was cooled to room temperature, extracted with dichloromethane on a filter filled with celite, filtered under reduced pressure, and concentrated to afford 2.2 g (yield: 56%) of the title compound.

### Step 5) Preparation of 6-((2,5-dichloropyrimidin-4-yl)amino)quinoxalin-5-yl)dimethylphosphine oxide

2.2 g (10.0 mmol) of the compound prepared in step 4) was diluted with 20 ml of *N,N*-dimethylformamide, and 440 mg (19.2 mmol) of 60% sodium hydride was slowly added thereto at 0 °C. The mixture was stirred at room temperature for 30 min. To the reaction mixture was slowly added 2.3 ml (20.0 mmol) of 2,4,5-trichloropyrimidine at 0 °C, followed by stirring at room temperature for 2 h. After completion of the reaction, the reaction mixture was diluted with ethyl acetate and washed with distilled water and saturated brine. The organic layer was separated, dried over anhydrous sodium sulfate, filtered under reduced pressure, and distilled under reduced pressure. The resulting residue was purified by chromatography (50% ethyl acetate/hexane) to afford 2.2 g (yield: 60%) of the title compound.

### Step 6) Preparation of (6-((5-chloro-2-((2-methoxy-5-methyl-6-(4-(4-methylpiperazin-1-yl)piperidin-1-yl)pyridin-3-yl)amino)pyrimidin-4-yl)amino)quinoxalin-5-yl)dimethylphosphine oxide

The title compound was prepared in the same manner as in Example 1, except that the compound prepared in step 5) was used and 2-chloro-6-methoxy-3-methyl-5-nitropyridine and 1-methyl-4-piperidin-4-yl-piperazine hydrochloride were used in step 2) of Example 1.

### Example 123: Preparation of (6-((5-chloro-2-((2-methoxy-6-(4-(4-methylpiperazin-1-yl)piperidin-1-yl)pyridin-3-yl)amino)pyrimidin-4-yl)amino)quinoxalin-5-yl)dimethylphosphine oxide

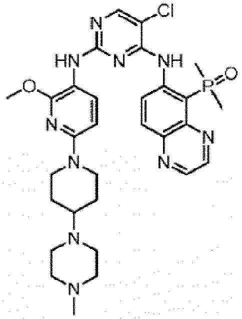

The title compound was prepared from the compound prepared in step 5) of Example 122 and the corresponding aniline in the same manner as in Example 1. [M+H]⁺ = 637.

### Example 124: Preparation of (6-((5-chloro-2-((5-fluoro-6-(4-(4-methylpiperazin-1-yl)piperidin-1-yl)pyridin-3-yl)amino)pyrimidin-4-yl)amino)quinoxalin-5-yl)dimethylphosphine oxide

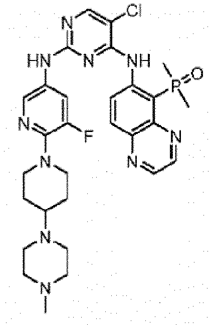

The title compound was prepared from the compound prepared in step 5) of Example 122 and the corresponding aniline in the same manner as in Example 1. [M+H]⁺ = 623.

### Example 125: Preparation of (6-((5-chloro-2-((3-fluoro-4-(4-(4-methylpiperazin-1-yl)piperidin-1-yl)phenyl)amino)pyrimidin-4-yl)amino)quinoxalin-5-yl)dimethylphosphine oxide

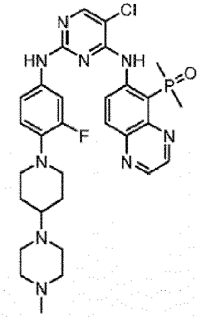

The title compound was prepared from the compound prepared in step 5) of Example 122 and the corresponding aniline in the same manner as in Example 1. [M+H]⁺ = 624.

### Example 126: Preparation of (6-((5-chloro-2-((2-methoxy-6-((3S,5R)-3,4,5-trimethylpiperazin-1-yl)pyridin-3-yl)amino)pyrimidin-4-yl)amino)quinoxalin-5-yl)dimethylphosphine oxide

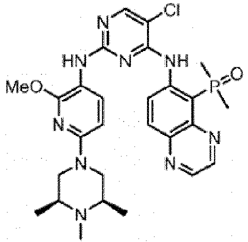

The title compound was prepared from the compound prepared in step 5) of Example 122 and the corresponding aniline in the same manner as in Example 1. [M+H]⁺ = 582.

### Example 127: Preparation of (6-((5-chloro-2-((6-(3-(dimethylamino)pyrrolidin-1-yl)-2-methoxypyridin-3-yl)amino)pyrimidin-4-yl)amino)quinoxalin-5-yl)dimethylphosphine oxide

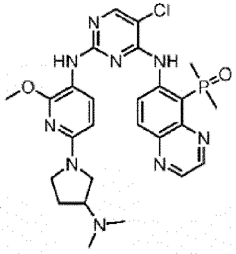

The title compound was prepared from the compound prepared in step 5) of Example 122 and the corresponding aniline in the same manner as in Example 1. [M+H]⁺ = 568.

### Example 128: Preparation of (6-((5-chloro-2-((2-methoxy-6-(piperazin-1-yl)pyridin-3-yl)amino)pyrimidin-4-yl)amino)quinoxalin-5-yl)dimethylphosphine oxide

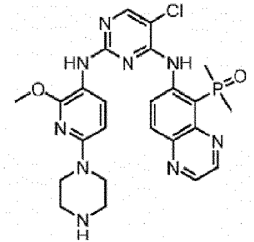

The title compound was prepared from the compound prepared in step 5) of Example 122 and the corresponding aniline in the same manner as in Example 1. [M+H]⁺ = 540.

### Example 129: Preparation of 4-(5-((5-chloro-4-((5-(dimethylphosphoryl)quinoxalin-6-yl)amino)pyrimidin-2-yl)amino)-6-methoxypyridin-2-yl)thiomorpholine 1,1-dioxide

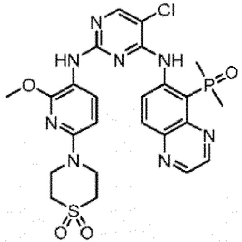

The title compound was prepared from the compound prepared in step 5) of Example 122 and the corresponding aniline in the same manner as in Example 1. [M+H]⁺ = 589.

### Example 130: Preparation of (6-((5-bromo-2-((2-methoxy-5-methyl-4-(4-(4-methylpiperazin-1-yl)piperidin-1-yl)phenyl)amino)pyrimidin-4-yl)amino)quinoxalin-5-yl)dimethylphosphine oxide

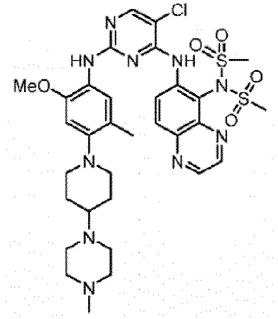

### Step 1) Preparation of 6-nitroquinoxalin-5-amine

5.0 g (28.5 mmol) of the compound prepared in step 1) of Example 122 was diluted with 60 ml methanol, and 5.8 g (60 mmol) of sodium t-butoxide and 4.1 g (60 mmol) of hydroxylamine hydrochloride were added thereto at 0 °C. The mixture was stirred at 65 °C for 4 h. After completion of the reaction, the reaction mixture was cooled to room temperature, extracted with dichloromethane, filtered under reduced pressure, and concentrated to afford 3.2 g (yield: 60%) of the title compound.

### Step 2) Preparation of N-(methylsulfonyl)-N-(6-nitroquinoxalin-5-yl)methanesulfonamide

3.2 g (17 mmol) of the compound prepared in step 1) was diluted with 30 ml of *N,N*-dimethylformamide, and 575 mg (25 mmol) of 60% sodium hydride was slowly added thereto at 0 °C. The mixture was stirred at room temperature for 30 min. To the reaction mixture was slowly added 2.7 ml (36.0 mmol) of methanesulfonyl chloride at 0 °C, followed by stirring at room temperature for 2 h. After completion of the reaction, the reaction mixture was diluted with ethyl acetate and washed with distilled water and saturated brine. The organic layer was separated, dried over anhydrous sodium sulfate, filtered under reduced pressure, and distilled under reduced pressure. The resulting residue was purified by chromatography (50% ethyl acetate/hexane) to afford 4.8 g (yield: 82%) of the title compound.

### Step 3) Preparation of N-(6-aminoquinoxalin-5-yl)-N-(methylsulfonyl)methanesulfonamide

4.8 g (14.0 mmol) of the compound prepared in step 2) was dissolved in 30 ml of a mixture of tetrahydrofuran, methanol, and water in an 18:1:1 volume ratio. To the solution were added 7.8 g (140 mmol) of an iron powder and 7.6 g (140 mmol) of ammonium chloride, followed by stirring at 85 °C for 3 h. After completion of the reaction, the reaction mixture was washed with dichloromethane on a filter filled with celite, filtered under reduced pressure, and concentrated to afford 3.16 g (yield: 71%) of the title compound.

### Step 4) Preparation of (6-((5-bromo-2-((2-methoxy-5-methyl-4-(4-(4-methylpiperazin-1-yl)piperidin-1-yl)phenyl)amino)pyrimidin-4-yl)amino)quinoxalin-5-yl)dimethylphosphine oxide

The title compound was prepared from the compound prepared in step 3) in the same manner as in steps 1) and 3) of Example 1. [M+H]⁺ = 745.

### Example 131: Preparation of N-(6-((5-chloro-2-((2-methoxy-5-methyl-4-(4-(4-methylpiperazin-1-yl)piperidin-1-yl)phenyl)amino)pyrimidin-4-yl)amino)quinoxalin-5-yl)methanesulfonamide

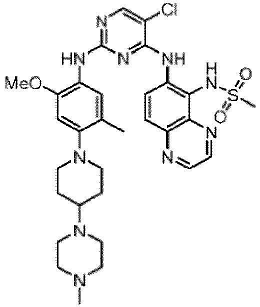

### Step 1) Preparation of N-(6-nitroquinoxalin-5-yl)methanesulfonamide

5.0 g (28.5 mmol) of the compound prepared in the same manner as in step 2) of Example 130 was diluted with 40 ml of 2 N sodium hydroxide/tetrahydrofuran. The dilution was stirred at room temperature for 4 h. After completion of the reaction, the reaction mixture was cooled to room temperature, extracted with dichloromethane, filtered under reduced pressure, and concentrated to afford 6.7 g (yield: 87%) of the title compound.

### Step 2) Preparation of N-(6-((5-chloro-2-((2-methoxy-5-methyl-4-(4-(4-methylpiperazin-1-yl)piperidin-1-yl)phenyl)amino)pyrimidin-4-yl)amino)quinoxalin-5-yl)methanesulfonamide

The title compound was prepared in the same manner as in steps 3) and 4) of Example 130.

¹H NMR (400 MHz, DMSO-d6) δ 9.92 (brs, 1H), 9.00 (d, J = 1.8 Hz, 1H), 8.96 (brs, 1H), 8.93 (d, J = 1.8 Hz, 1H), 8.71 (d, J = 8.5 Hz, 1H), 8.25-8.22 (m, 2H), 7.90 (d, J = 9.3 Hz, 1H), 7.38 (s, 1H), 6.72 (s, 1H), 3.76 (s, 3H), 3.16-2.96 (m, 5H), 2.74-2.27 (m, 11H), 2.21 (s, 3H), 2.02 (s, 3H), 1.93-1.80 (m, 2H), 1.65-1.48 (m, 2H). [M+H]⁺ = 667.

### Example 132: Preparation of N-(6-((5-chloro-2-((2-methoxy-6-(4-(4-methylpiperazin-1-yl)piperidin-1-yl)pyridin-3-yl)amino)pyrimidin-4-yl)amino)quinoxalin-5-yl)methanesulfonamide

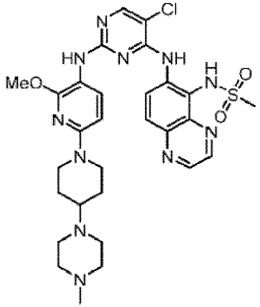

The title compound was prepared from the corresponding aniline in the same manner as in Example 131. [M+H]⁺ = 654.

### Example 133: Preparation of N-(6-((5-chloro-2-((2-methoxy-4-(4-(4-methylpiperazin-1-yl)piperidin-1-yl)phenyl)amino)pyrimidin-4-yl)amino)quinoxalin-5-yl)methanesulfonamide

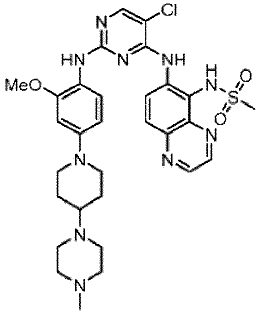

The title compound was prepared from the corresponding aniline in the same manner as in Example 131. [M+H]⁺ = 653.

### Example 134: Preparation of N-(6-((5-chloro-2-((2-methoxy-5-methyl-6-(4-(4-methylpiperazin-1-yl)piperidin-1-yl)pyridin-3-yl)amino)pyrimidin-4-yl)amino)quinoxalin-5-yl)methanesulfonamide

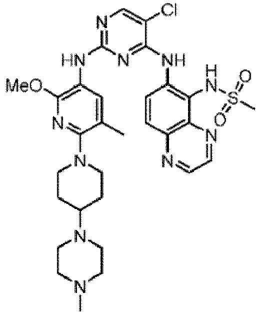

The title compound was prepared from the corresponding aniline in the same manner as in Example 131. [M+H]⁺ = 667.

### Example 135: Preparation of N-(6-((5-chloro-2-((2-methoxy-6-((3S,5R)-3,4,5-trimethylpiperazin-1-yl)pyridin-3-yl)amino)pyrimidin-4-yl)amino)quinoxalin-5-yl)methanesulfonamide

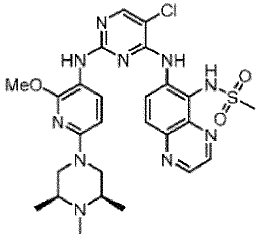

The title compound was prepared from the corresponding aniline in the same manner as in Example 131. [M+H]⁺ = 598.

### Example 136: Preparation of N-(6-((5-chloro-2-((5-chloro-2-methoxy-4-(4-(4-methylpiperazin-1-yl)piperidin-1-yl)phenyl)amino)pyrimidin-4-yl)amino)quinoxalin-5-yl)methanesulfonamide

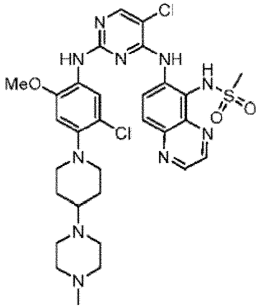

The title compound was prepared from the corresponding aniline in the same manner as in Example 131. [M+H]⁺ = 686.

### Example 137: Preparation of N-(2-((5-chloro-2-((5-chloro-2-methoxy-4-(4-(4-methylpiperazin-1-yl)piperidin-1-yl)phenyl)amino)pyrimidin-4-yl)amino)-4-(trifluoromethyl)phenyl)methanesulfonamide

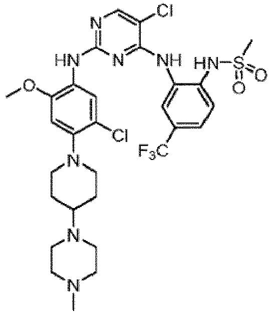

The title compound was prepared in the same manner as in Example 1, except that *N*-(2-amino-4-(trifluoromethyl)phenyl)methanesulfonamide and the corresponding aniline were used in steps 1) and 2), respectively. [M+H]⁺ = 703.

### Example 138: Preparation of N-(2-((5-chloro-2-((2-methoxy-5-methyl-4-(4-(4-methylpiperazin-1-yl)piperidin-1-yl)phenyl)amino)pyrimidin-4-yl)amino)-4-(trifluoromethyl)phenyl)methanesulfonamide

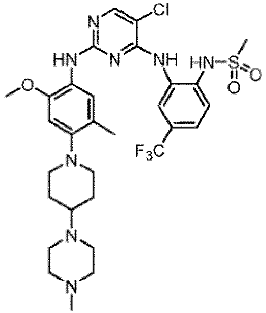

The title compound was prepared in the same manner as in Example 137, except that the corresponding aniline was used. [M+H]⁺ = 683.

### Example 139: Preparation of N-(2-((5-chloro-2-((4-(4-(4-cyclopropylpiperazin-1-yl)piperidin-1-yl)-2-methoxy-5-methylphenyl)amino)pyrimidin-4-yl)amino)-4-(trifluoromethyl)phenyl)methanesulfonamide

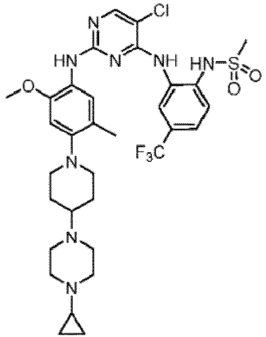

The title compound was prepared in the same manner as in Example 137, except that the corresponding aniline was used. [M+H]⁺ = 709.

### Example 140: Preparation of N-(6-((5-chloro-2-((2-methoxy-5-methyl-4-(4-(4-methylpiperazin-1-yl)piperidin-1-yl)phenyl)amino)pyrimidin-4-yl)amino)-2,3-dihydrobenzofuran-5-yl)methanesulfonamide

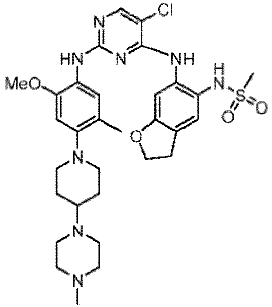

The title compound was prepared in the same manner as in Example 83, except that 6-nitro-2,3-dihydrobenzofuran-5-amine was used in step 1) of Example 80. [M+H]⁺ = 657.

### Example 141: Preparation of N-(2-((6-amino-5-chloro-2-((2-methoxy-5-methyl-4-(4-(4-methylpiperazin-1-yl)piperidin-1-yl)phenyl)amino)pyrimidin-4-yl)amino)-5-fluorophenyl)methanesulfonamide

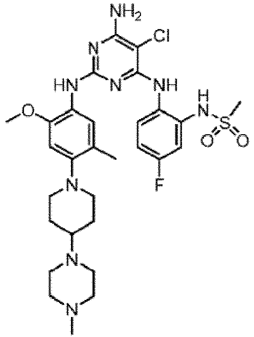

The title compound was prepared in the same manner as in Example 1, except that 2,5,6-trichloropyrimidin-4-amine was used in step 1) of Example 1. [M+H]⁺ = 648.

### Example 142: Preparation of ((2-((5-bromo-2-((2-methoxy-5-methyl-4-(4-(4-methylpiperazin-1-yl)piperidin-1-yl)phenyl)amino)pyrimidin-4-yl)amino)phenyl)imino)dimethyl-λ⁶-sulfanone

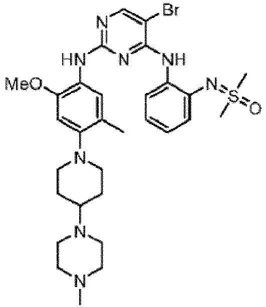

### Step 1) Preparation of N-(dimethyloxido-λ⁴-sulfanylidene)-2-nitro-benzenamine

5.0 g (40.3 mmol) of 1-oxo-1,7a-dihydrobenzo[c][1,2,5]oxadiazol-1-ium was diluted with 80 ml of dimethyl sulfoxide. The dilution was stirred at 120 °C for 12 h. After completion of the reaction, the reaction mixture was cooled to room temperature, diluted with ethyl acetate, and washed with distilled water. The resulting residue was purified by column chromatography (20% ethyl acetate/hexane) to afford 4.28 g (yield: 50%) of the title compound.

### Step 2) Preparation of dimethyl((2-nitrophenyl)imino)-l6-sulfanone

4.28 g (20 mmol) of the compound prepared in step 1) was dissolved in 40 ml of a mixture of tetrahydrofuran, methanol, and water in an 18:1:1 volume ratio. To the solution were added 0.3 g (2.7 mmol) of an iron powder and 0.16 g (2.7 mmol) of ammonium chloride, followed by stirring at 85 °C for 3 h. After completion of the reaction, the reaction mixture was washed with dichloromethane on a filter filled with celite, filtered under reduced pressure, and concentrated to afford 3.3 g (yield: 90%) of the title compound.

### Step 3) Preparation of ((2-((2,5-dichloropyrimidin-4-yl)amino)phenyl)imino)dimethyl-16-sulfanone

3.3 g (18 mmol) of the compound prepared in step 2) and 3.37 ml (35.0 mmol) of 5-bromo-2,4-dichloropyrimidine were diluted with 54 ml of isopropyl alcohol, and 9.3 ml (53.8 mmol) of *N*,*N*-diisopropylethylamine was added thereto. The mixture was stirred at 60 °C for 12 h. After completion of the reaction, the reaction mixture was cooled to room temperature, diluted with ethyl acetate, and washed with distilled water. The resulting residue was purified by column chromatography (50% ethyl acetate/hexane) to afford 3.31 g (yield: 55%) of the title compound.

### Step 4) Preparation of ((2-((5-bromo-2-((2-methoxy-5-methyl-4-(4-(4-methylpiperazin-1-yl)piperidin-1-yl)phenyl)amino)pyrimidin-4-yl)amino)phenyl)imino)dimethyl-λ⁶-sulfanone

The title compound was prepared from the compound prepared in step 3) and the corresponding aniline in the same manner as in Example 1.

[M+H]⁺ = 657

### Example 143: Preparation of ((2-((5-bromo-2-((5-chloro-2-methoxy-4-(4-(4-methylpiperazin-1-yl)piperidin-1-yl)phenyl)amino)pyrimidin-4-yl)amino)phenyl)imino)dimethyl-λ⁶-sulfanone

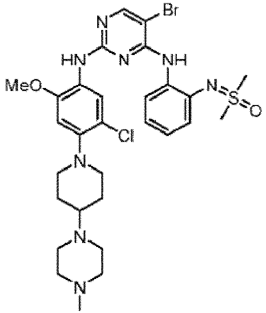

The title compound was prepared in the same manner as in steps 3) and 4) of Example 142. [M+H]⁺ = 678.

### Example 144: Preparation of ((2-((5-bromo-2-((2-chloro-5-methyl-4-(4-(4-methylpiperazin-1-yl)piperidin-1-yl)phenyl)amino)pyrimidin-4-yl)amino)phenyl)imino)dimethyl-λ⁶-sulfanone

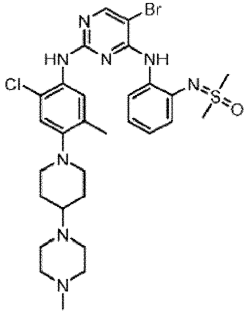

The title compound was prepared in the same manner as in steps 3) and 4) of Example 142. [M+H]⁺ = 662.

### Example 145: Preparation of N-(2-((5-bromo-2-((2-methoxy-6-(1-methylazepan-4-yl)pyridin-3-yl)amino)pyrimidin-4-yl)amino)-5-fluorophenyl)methanesulfonamide

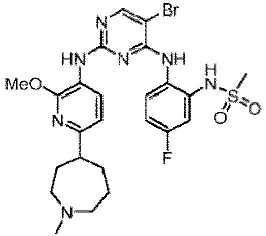

The title compound was prepared from the compound prepared in step 1) of Example 24 and the corresponding aniline in the same manner as in Example 1. [M+H]⁺ = 594.

### Example 146: Preparation of N-(2-((5-bromo-2-((5-fluoro-2-methoxy-4-(4-(4-methylpiperazin-1-yl)piperidin-1-yl)phenyl)amino)pyrimidin-4-yl)amino)-5-fluorophenyl)methanesulfonamide

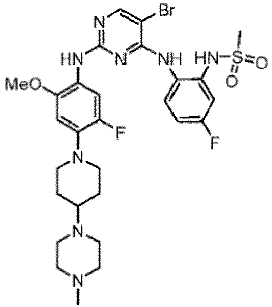

The title compound was prepared from the compound prepared in step 1) of Example 24 and the corresponding aniline in the same manner as in Example 1. [M+H]⁺ = 681.

### Example 147: Preparation of N-(2-((5-bromo-2-((2-methoxy-6-(4-(4-methylpiperazin-1-yl)piperidin-1-yl)pyridin-3-yl)amino)pyrimidin-4-yl)amino)-5-fluorophenyl)methanesulfonamide

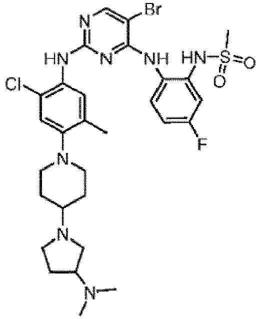

The title compound was prepared from the compound prepared in step 1) of Example 24 and the corresponding aniline in the same manner as in Example 1. [M+H]⁺ = 696.

### Example 148: Preparation of N-(2-((5-bromo-2-((2-methoxy-5-methyl-4-(4-(trifluoromethyl)-[1,4'-bipiperidin]-1'-yl)phenyl)amino)pyrimidin-4-yl)amino)-5-fluorophenyl)methanesulfonamide

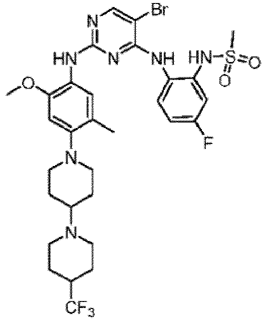

The title compound was prepared from the compound prepared in step 1) of Example 24 and the corresponding aniline in the same manner as in Example 1. [M+H]⁺ = 730.

### Example 149: Preparation of N-(2-((5-bromo-2-((5-chloro-4-(3-(dimethylamino)-[1,4'-bipiperidin]-1'-yl)-2-methoxyphenyl)amino)pyrimidin-4-yl)amino)-5-fluorophenyl)methanesulfonamide

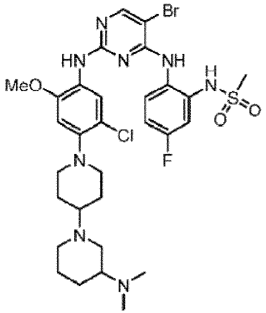

The title compound was prepared from the compound prepared in step 1) of Example 24 and the corresponding aniline in the same manner as in Example 1. [M+H]⁺ = 726.

### Example 150: Preparation of N-(2-((5-bromo-2-((2-methoxy-5-methyl-4-(4-(trifluoromethyl)-[1,4'-bipiperidin]-1'-yl)phenyl)amino)pyrimidin-4-yl)amino)-4-chlorophenyl)methanesulfonamide

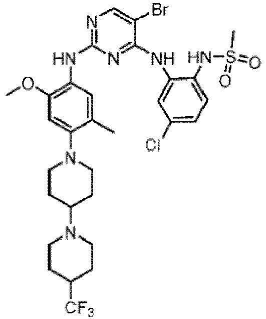

The title compound was prepared from the compound prepared in step 3) of Example 85 and the corresponding aniline in the same manner as in Example 1. [M+H]⁺ = 747.

### Example 151: Preparation of N-(4-chloro-2-((5-chloro-2-((5-chloro-2-methoxy-4-(4-(4-methylpiperazin-1-yl)piperidin-1-yl)phenyl)amino)pyrimidin-4-yl)amino)phenyl)methanesulfonamide

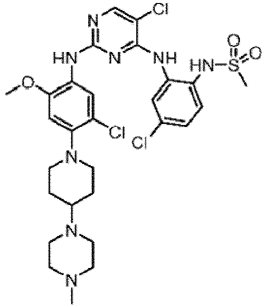

The title compound was prepared from the compound prepared in step 3) of Example 80 and the corresponding aniline in the same manner as in Example 1. [M+H]⁺ = 650.

### Example 152: Preparation of N-(4-chloro-2-((6-chloro-3-((-2-methoxy-5-methyl-4-(4-(4-methylpiperazin-1-yl)piperidin-1-yl)phenyl)amino)-1,2,4-triazin-5-yl)amino)phenyl)methanesulfonamide

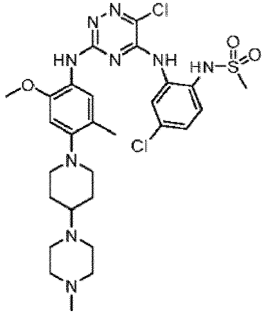

### Step 1) Preparation of N-(4-chloro-2-((3,6-dichloro-1,2,4-triazin-5-yl)amino)phenyl)methanesulfonamide

The title compound was prepared from 3,5,6-trichloro-1,2,4-triazine and *N*-(2-amino-4-chlorophenyl)methanesulfonamide in the same manner as in step 1) of Example 1.

### Step 2) Preparation of N-(4-chloro-2-((6-chloro-3-((-2-methoxy-5-methyl-4-(4-(4-methylpiperazin-1-yl)piperidin-1-yl)phenyl)amino)-1,2,4-triazin-5-yl)amino)phenyl)methanesulfonamide

The title compound was prepared in the same manner as in Example 1, except that the compound prepared in step 1) was used and 1-chloro-5-methoxy-2-methyl-4-nitrobenzene and 1-methyl-4-piperidin-4-yl-piperazine hydrochloride were used in step 2) of Example 1. [M+H]⁺ = 650.

### Example 153: Preparation of (4-chloro-2-((5-chloro-2-((2-methoxy-5-methyl-4-(4-(4-methylpiperazin-1-yl)piperidin-1-yl)phenyl)amino)pyrimidin-4-yl)amino)phenyl)dimethylphosphine oxide

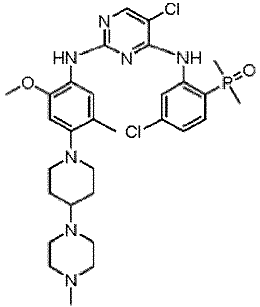

The title compound was prepared from (2-amino-4-chlorophenyl)dimethylphosphine oxide in the same manner as in step 1) of Example 1. [M+H]⁺ = 632.

### Example 154: Preparation of (4-chloro-2-((5-chloro-2-((2-chloro-5-methyl-4-(4-(4-methylpiperazin-1-yl)piperidin-1-yl)phenyl)amino)pyrimidin-4-yl)amino)phenyl)dimethylphosphine oxide

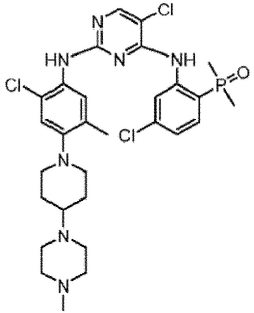

The title compound was prepared from the corresponding aniline in the same manner as in Example 153. [M+H]⁺ = 637.

### Example 155: Preparation of N-(2-((5-bromo-2-((2-methoxy-5-methyl-4-(4-(4-methylpiperazin-1-yl)piperidin-1-yl)phenyl)amino)pyrimidin-4-yl)amino)-4-chlorophenyl)-N-methylmethanesulfonamide

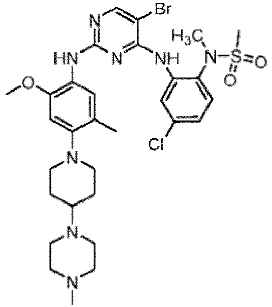

### Step 1) Preparation of N-(2-((5-bromo-2-chloropyrimidin-4-yl)amino)-4-chlorophenyl)-N-methylmethanesulfonamide

The title compound was prepared in the same manner as in step 1) of Example 1, except that *N-*(2-amino-4-chlorophenyl)-*N*-methylmethanesulfonamide was used in step 1) of Example 1 and 5-bromo-2,4-dichloropyrimidine was used in step 3) of Example 24.

### Step 2) Preparation of N-(2-((5-bromo-2-((2-methoxy-5-methyl-4-(4-(4-methylpiperazin-1-yl)piperidin-1-yl)phenyl)amino)pyrimidin-4-yl)amino)-4-chlorophenyl)-N-methylmethanesulfonamide

The title compound was prepared from the compound prepared in step 1) in the same manner as in step 2) of Example 1. [M+H]⁺ = 708.

### Example 156: Preparation of N-(2-((5-bromo-2-((5-chloro-2-methoxy-4-(4-(4-methylpiperazin-1-yl)piperidin-1-yl)phenyl)amino)pyrimidin-4-yl)amino)-4-chlorophenyl)-N-methylmethanesulfonamide

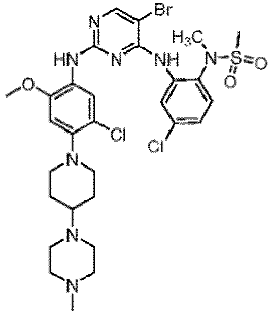

The title compound was prepared from the corresponding aniline in the same manner as in Example 155. [M+H]⁺ = 728.

### Example 157: Preparation of N-(4-chloro-2-((5-chloro-2-((2-chloro-4-(4-(3-(dimethylamino)pyrrolidin-1 -yl)piperidin-1 -yl)-5-methylphenyl)amino)pyrimidin-4-yl)amino)phenyl)methanesulfonamide

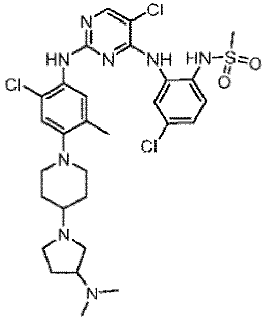

The title compound was prepared from the compound prepared in step 3) of Example 80 and the corresponding aniline in the same manner as in Example 1. [M+H]⁺ = 668.

### Example 158: Preparation of N-(4-chloro-2-((5-chloro-2-((2-chloro-5-methyl-4-(4-(4-methylpiperazin-1-yl)piperidin-1-yl)phenyl)amino)pyrimidin-4-yl)amino)phenyl)methanesulfonamide

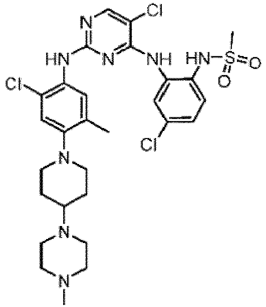

The title compound was prepared from the compound prepared in step 3) of Example 80 and the corresponding aniline in the same manner as in Example 1. [M+H]⁺ = 654.

### Example 159: Preparation of N-(4-chloro-2-((5-chloro-2-((5-chloro-2-methoxy-4-(4-(4-methylpiperazin-1-yl)piperidin-1-yl)phenyl)amino)pyrimidin-4-yl)amino)phenyl)methanesulfonamide

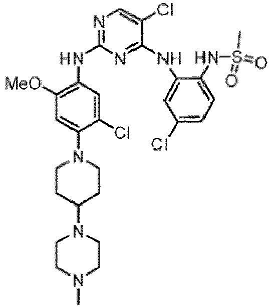

The title compound was prepared from the compound prepared in step 3) of Example 80 and the corresponding aniline in the same manner as in Example 1. [M+H]⁺ = 670.

### Example 160: Preparation of N-(2-((5-chloro-2-((2-methoxy-5-methyl-4-(4-(4-methylpiperazin-1-yl)piperidin-1-yl)phenyl)amino)pyrimidin-4-yl)amino)-4-isopropylphenyl)methanesulfonamide

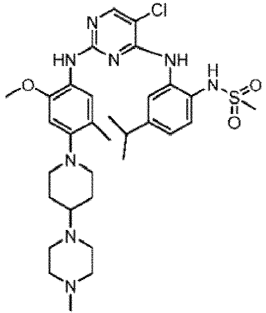

### Step 1) Preparation of N-(2-((2,5-dichloropyrimidin-4-yl)amino)-4-isopropylphenyl)methanesulfonamide

*N*-(2-amino-4-isopropylphenyl)methanesulfonamide (1 eq.) was diluted with isopropyl alcohol (0.3 M), and 2,4,5-trichloropyrimidine (2 eq.) and sodium bicarbonate (4 eq.) were added thereto. The mixture was stirred under a nitrogen atmosphere at 65 °C for 40 h. After completion of the reaction, the reaction mixture was cooled to room temperature, washed with dichloromethane on a filter filled with celite, filtered under reduced pressure, diluted with a mixture of isopropyl alcohol and chloroform in a 1:4 volume ratio, and washed with distilled water. Thereafter, the organic layer was dried over anhydrous sodium sulfate, filtered under reduced pressure, and distilled under reduced pressure. The resulting residue was purified by column chromatography (40% tetrahydrofuran/hexane) to afford the title compound (yield: 69%).

### Step 2) Preparation of N-(2-((5-chloro-2-((2-methoxy-5-methyl-4-(4-(4-methylpiperazin-1-yl)piperidin-1-yl)phenyl)amino)pyrimidin-4-yl)amino)-4-isopropylphenyl)methanesulfonamide

The compound (1 eq.) prepared in step 1) and the corresponding aniline (0.6 eq.) were dissolved in a mixture of acetic acid (0.3 M) and a 1.25 M HCl/methanol solution in a 1:1 volume ratio, sealed, and stirred at 120 °C for 3 h. After completion of the reaction, the reaction mixture was cooled to room temperature and extracted with a mixture of isopropyl alcohol and chloroform in a 1:4 volume ratio and a saturated aqueous solution of sodium bicarbonate. Thereafter, the organic layer was dried over anhydrous sodium sulfate, filtered under reduced pressure, and distilled under reduced pressure. The resulting residue was purified by column chromatography (10% methanol/dichloromethane) to afford the title compound (yield: 30%). [M+H]⁺ = 657.

### Example 161: Preparation of N-(2-((5-chloro-2-((5-chloro-2-methoxy-4-(4-(4-methylpiperazin-1-yl)piperidin-1-yl)phenyl)amino)pyrimidin-4-yl)amino)-4-isopropylphenyl)methanesulfonamide

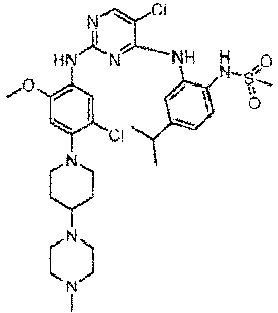

The title compound was prepared in the same manner as in step 2) of Example 160. [M+H]⁺ = 677.

### Example 162: Preparation of N-(4-chloro-2-((5-chloro-2-((2-chloro-5-methyl-4-(4-(4-m ethyl-1,4-diazepan-1 -yl)piperidin-1 -yl)phenyl)amino)pyrimidin-4-yl)amino)phenyl)methanesulfonamide

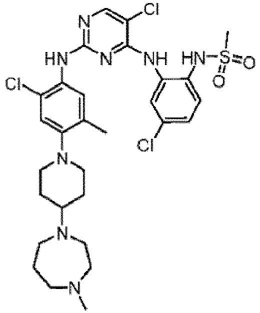

The title compound was prepared from the compound prepared in step 3) of Example 80 and the corresponding aniline in the same manner as in Example 1. [M+H]⁺ = 667.

### Example 163: Preparation of N-(4-chloro-2-((5-chloro-2-((5-chloro-2-methoxy-4-(4-(4-m ethyl-1,4-diazepan-1 -yl)piperidin-1 -yl)phenyl)amino)pyrimidin-4-yl)amino)phenyl)methanesulfonamide

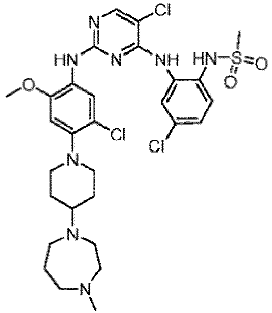

The title compound was prepared from the compound prepared in step 3) of Example 80 and the corresponding aniline in the same manner as in Example 1. [M+H]⁺ = 683.

### Example 164: Preparation of N-(2-((6-chloro-3-((2-methoxy-5-methyl-4-(4-(4-methylpiperazin-1-yl)piperidin-1-yl)phenyl)amino)-1,2,4-triazin-5-yl)amino)-5-fluorophenyl)methanesulfonamide

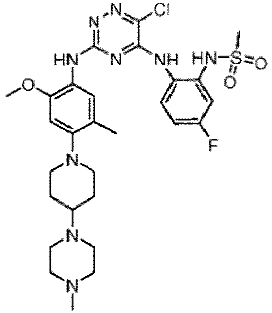

### Step 1) Preparation of N-(2-((3,6-dichloro-1,2,4-triazin-5-yl)amino)-5-fluorophenyl)methanesulfonamide

*N*-(2-amino-5-fluorophenyl)methanesulfonamide (1 eq.) was diluted with isopropyl alcohol (0.3 M), and 3,5,6-trichloro-1,2,4-triazine (2 eq.) and diisopropylethylamine (4 eq.) were added thereto. The mixture was stirred under a nitrogen atmosphere at 65 °C for 40 h. After completion of the reaction, the reaction mixture was cooled to room temperature, washed with dichloromethane on a filter filled with celite, filtered under reduced pressure, diluted with a mixture of isopropyl alcohol and chloroform in a 1:4 volume ratio, and washed with distilled water. Thereafter, the organic layer was dried over anhydrous sodium sulfate, filtered under reduced pressure, and distilled under reduced pressure. The resulting residue was purified by column chromatography (40% tetrahydrofuran/hexane) to afford the title compound (yield: 55%).

### Step 2) Preparation of N-(2-((6-chloro-3-((2-methoxy-5-methyl-4-(4-(4-methylpiperazin-1-yl)piperidin-1-yl)phenyl)amino)-1,2,4-triazin-5-yl)amino)-5-fluorophenyl)methanesulfonamide

The compound (1 eq.) prepared in step 1) and the corresponding aniline (0.6 eq.) were dissolved in a mixture of acetic acid (0.3 M) and a 1.25 M HCl/methanol solution in a 1:1 volume ratio, sealed, and stirred at 120 °C for 3 h. After completion of the reaction, the reaction mixture was cooled to room temperature and extracted with a mixture of isopropyl alcohol and chloroform in a 1:4 volume ratio and a saturated aqueous solution of sodium bicarbonate. Thereafter, the organic layer was dried over anhydrous sodium sulfate, filtered under reduced pressure, and distilled under reduced pressure. The resulting residue was purified by column chromatography (10% methanol/dichloromethane) to afford the title compound (yield: 34%).

[M+H]⁺ = 634.

### Example 165: Preparation of N-(6-((5-chloro-2-((2-methoxy-5-methyl-4-(4-(4-methyl-1,4-diazepan-1-yl)piperidin-1-yl)phenyl)amino)pyrimidin-4-yl)amino)-2,3-dihydrobenzofuran-5-yl)methanesulfonamide

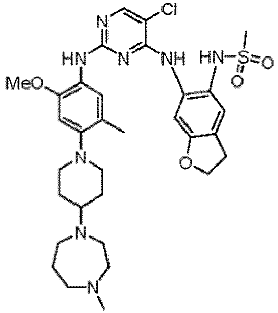

### Step 1) Preparation of N-(methylsulfonyl)-N-(6-nitro-2,3-dihydrobenzofuran-5-yl)methanesulfonamide

6-Nitro-2,3-dihydrobenzofuran-5-amine (1 eq.) was dissolved in dichloromethane (0.3 M), and triethylamine (4 eq.) was added thereto at 0 °C. To the mixture was slowly added methanesulfonyl chloride (MsCl, 3 eq.), followed by stirring at room temperature for 30 min. After completion of the reaction, the reaction mixture was diluted with hexane and further stirred for 15 minutes. The resulting yellow solid was collected by filtration under reduced pressure to afford the title compound.

### Step 2) Preparation of N-(6-nitro-2,3-dihydrobenzofuran-5-yl)methanesulfonylamide

The compound prepared in step 1) was dissolved in a mixture of tetrahydrofuran and a 4 N NaOH solution (1:1, 0.2 M). The solution was stirred at room temperature for 2 h. After completion of the reaction, the reaction mixture was distilled under reduced pressure and adjusted to pH 5-6 with a 1 N HCl solution at 0 °C. The resulting yellow solid was collected by filtration under reduced pressure to afford the title compound.

### Step 3) Preparation of N-(6-amino-2,3-dihydrobenzofuran-5-yl)methanesulfonamide

The compound prepared in step 2) was dissolved in a mixture of tetrahydrofuran, methanol, and water in an 18:1:1 volume ratio. To the solution were added an iron powder and ammonium chloride, followed by stirring at 85 °C for 3 h. After completion of the reaction, the reaction mixture was washed with dichloromethane on a filter filled with celite, filtered under reduced pressure, and concentrated to afford the title compound (yield: 91%).

### Step 4) Preparation of N-(6-((2,5-dichloropyrimidin-4-yl)amino)-2,3-dihydrobenzofuran-5-yl)methanesulfonamide

The compound (1 eq.) prepared in step 3) was diluted with isopropyl alcohol (0.3 M), and 2,4,5-trichloropyrimidine (2 eq.) and sodium bicarbonate (4 eq.) were added thereto. The mixture was stirred under a nitrogen atmosphere at 65 °C for 40 h. After completion of the reaction, the reaction mixture was cooled to room temperature, washed with dichloromethane on a filter filled with celite, filtered under reduced pressure, diluted with a mixture of isopropyl alcohol and chloroform in a 1:4 volume ratio, and washed with distilled water. Thereafter, the organic layer was dried over anhydrous sodium sulfate, filtered under reduced pressure, and distilled under reduced pressure. The resulting residue was purified by column chromatography (40% tetrahydrofuran/hexane) to afford the title compound (yield: 62%).

### Step 5) Preparation of N-(6-((5-chloro-2-((2-methoxy-5-methyl-4-(4-(4-methyl-1,4-diazepan-1-yl)piperidin-1-yl)phenyl)amino)pyrimidin-4-yl)amino)-2,3-dihydrobenzofuran-5-yl)methanesulfonamide

The compound (1 eq.) prepared in step 4) and the corresponding aniline (0.6 eq.) were dissolved in a mixture of acetic acid (0.3 M) and a 1.25 M HCl/methanol solution in a 1:1 volume ratio, sealed, and stirred at 120 °C for 3 h. After completion of the reaction, the reaction mixture was cooled to room temperature and extracted with a mixture of isopropyl alcohol and chloroform in a 1:4 volume ratio and a saturated aqueous solution of sodium bicarbonate. Thereafter, the organic layer was dried over anhydrous sodium sulfate, filtered under reduced pressure, and distilled under reduced pressure. The resulting residue was purified by column chromatography (10% methanol/dichloromethane) to afford the title compound (yield: 33%). [M+H]⁺ = 671.

### Example 166: Preparation of 5-chloro-N⁴-(5-chloro-2-(1H-1,2,3-triazol-4-yl)phenyl)-N²-(2-methoxy-5-methyl-4-(4-(4-methylpiperazin-1-yl)piperidin-1-yl)phenyl)pyrimidine-2,4-diamine

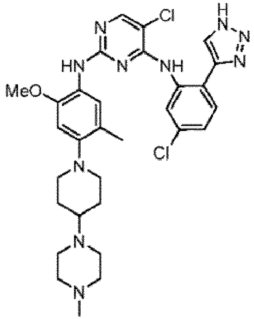

### Step 1) Preparation of 2,5-dichloro-N-(5-chloro-2-iodophenyl)pyrimidin-4-amine

5-Chloro-2-iodoaniline (1 eq.) was diluted with isopropyl alcohol (0.3 M), and 2,4,5-trichloropyrimidine (2 eq.) and sodium bicarbonate (4 eq.) were added thereto. The mixture was stirred under a nitrogen atmosphere at 65 °C for 40 h. After completion of the reaction, the reaction mixture was cooled to room temperature, washed with dichloromethane on a filter filled with celite, filtered under reduced pressure, diluted with a mixture of isopropyl alcohol and chloroform in a 1:4 volume ratio, and washed with distilled water. Thereafter, the organic layer was dried over anhydrous sodium sulfate, filtered under reduced pressure, and distilled under reduced pressure. The resulting residue was purified by column chromatography (40% tetrahydrofuran/hexane) to afford the title compound (yield: 62%).

### Step 2) Preparation of 5-chloro-N⁴-(5-chloro-2-idophenyl)-N²-(2-methoxy-5-methyl-4-(4-(4-methylpiperazin-1-yl))piperidin-1-yl)phenyl)pyrimidine-2,4-diamine

The compound (1 eq.) prepared in step 1) and the corresponding aniline (0.6 eq.) were dissolved in a mixture of acetic acid (0.3 M) and a 1.25 M HCl/methanol solution in a 1:1 volume ratio, sealed, and stirred at 120 °C for 3 h. After completion of the reaction, the reaction mixture was cooled to room temperature and extracted with a mixture of isopropyl alcohol and chloroform in a 1:4 volume ratio and a saturated aqueous solution of sodium bicarbonate. Thereafter, the organic layer was dried over anhydrous sodium sulfate, filtered under reduced pressure, and distilled under reduced pressure. The resulting residue was purified by column chromatography (10% methanol/dichloromethane) to afford the title compound (yield: 45%).

### Step 3) Preparation of 5-chloro-N⁴-(5-chloro-2-ethynylphenyl)-N²-(2-methoxy-5-methyl-4-(4-(4-methylpiperazin-1-yl)piperidin-1-yl)phenyl)pyrimidine-2,4-diamine

The compound prepared in step 2) (1 eq.), trimethylsilyl acetylene (1.5 eq.), Pd(PPh₃)Cl₂ (0.3 eq.), and CuI (0.2 eq.) were dissolved in a mixture of tetrahydrofuran and triethylamine in a 2:1 ratio. The solution was stirred at room temperature for 30 min. After completion of the reaction, the reaction mixture was filtered through a filter filled with celite under reduced pressure and distilled under reduced pressure. The resulting residue was diluted with methanol (0.3 M), and potassium carbonate (3 eq.) was added thereto. The mixture was stirred at room temperature for 5 min. After completion of the reaction, the reaction mixture was diluted with dichloromethane, washed with saturated brine, and purified by column chromatography (yield: 42%).

### Step 4) Preparation of 5-chloro-N⁴-(5-chloro-2-(1H-1,2,3-triazol-4-yl)phenyl)-N²-(2-methoxy-5-methyl-4-(4-(4-methylpiperazin-1-yl)piperidin-1-yl)phenyl)pyrimidine-2,4-diamine

The compound prepared in step 3) (1 eq.), trimethylsilyl azide (1.5 eq.), copper sulfate (0.1 eq.), and sodium ascorbate (0.2 eq.) were diluted with distilled water (0.3 M). The dilution was stirred at 50 °C for 3 h. After completion of the reaction, the reaction mixture was extracted with a mixture of isopropyl alcohol and chloroform in a 1:4 volume ratio and a saturated aqueous solution of sodium bicarbonate. The extract was purified by column chromatography to afford the title compound (yield: 33%). [M+H]⁺ = 623.

### Example 167: Preparation of N-(4-chloro-2-((5-chloro-2-((1-(3-(dimethylamino)propyl)-1H-pyrazol-4-yl)amino)pyrimidine-4-yl)amino)phenyl)methanesulfonamide

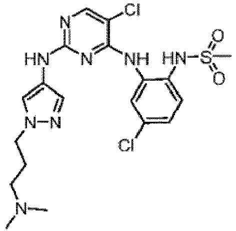

The title compound was prepared from the compound prepared in step 3) of Example 80 and the corresponding aniline in the same manner as in Example 1. [M+H]⁺ = 499.

### Example 168: Preparation of N-(6-((5-chloro-2-((5-chloro-2-methoxy-4-(4-(4-methylpiperazin-1-yl)piperidin-1-yl)phenyl)amino)pyrimidin-4-yl)amino)-2,3-dihydrobenzofuran-5-yl)methanesulfonamide

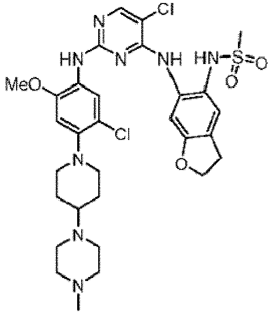

The title compound was prepared in the same manner as in step 5) of Example 165. ¹H NMR (300 MHz, DMSO) δ 8.45 (s, 1H), 8.14 (s, 1H), 8.09 (s, 1H), 7.70 (s, 1H), 7.43 (s, 1H), 7.20 (s, 1H), 6.77 (s, 1H), 4.56 (t, J = 8.6 Hz, 2H), 3.81 (s, 3H), 3.37-3.22 (m, 4H), 3.18 (t, J = 8.6 Hz, 3H), 2.9 (s, 3H), 2.64 (t, J = 11.0 Hz, 3H), 2.43-2.22 (m, 5H), 2.16 (s, 3H), 1.91-1.79 (m, 2H), 1.67-1.48 (m, 2H). [M+H]⁺ = 677.

### Example 169: Preparation of N-(6-((5-bromo-2-((2-methoxy-5-methyl-4-(4-(4-methylpiperazin-1-yl)piperidin-1-yl)phenyl)amino)pyrimidin-4-yl)amino)-2,3-dihydrobenzofuran-5-yl)methanesulfonamide

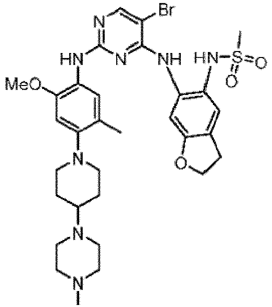

### Step 1) Preparation of N-(6-((5-bromo-2-chloropyrimidin-4-yl)amino)-2,3-dihydrobenzofuran-5-yl)methanesulfonamide

The compound (1 eq.) prepared in step 3) of Example 165 was diluted with isopropyl alcohol (0.3 M), and 5-bromo-2,4-dichloropyrimidine (2 eq.) and sodium bicarbonate (4 eq.) were added thereto. The mixture was stirred under a nitrogen atmosphere at 65 °C for 40 h. After completion of the reaction, the reaction mixture was cooled to room temperature, washed with dichloromethane on a filter filled with celite, filtered under reduced pressure, diluted with a mixture of isopropyl alcohol and chloroform in a 1:4 volume ratio, and washed with distilled water. Thereafter, the organic layer was dried over anhydrous sodium sulfate, filtered under reduced pressure, and distilled under reduced pressure. The resulting residue was purified by column chromatography (40% tetrahydrofuran/hexane) to afford the title compound (yield: 60%).

### Step 2) Preparation of N-(6-((5-bromo-2-((2-methoxy-5-methyl-4-(4-(4-methylpiperazin-1-yl)piperidin-1-yl)phenyl)amino)pyrimidin-4-yl)amino)-2,3-dihydrobenzofuran-5-yl)methanesulfonamide

The compound (1 eq.) prepared in step 1) and the corresponding aniline (0.6 eq.) were dissolved in a mixture of acetic acid (0.3 M) and a 1.25 M HCl/methanol solution in a 1:1 volume ratio, sealed, and stirred at 120 °C for 3 h. After completion of the reaction, the reaction mixture was cooled to room temperature and extracted with a mixture of isopropyl alcohol and chloroform in a 1:4 volume ratio and a saturated aqueous solution of sodium bicarbonate. Thereafter, the organic layer was dried over anhydrous sodium sulfate, filtered under reduced pressure, and distilled under reduced pressure. The resulting residue was purified by column chromatography (10% methanol/dichloromethane) to afford the title compound (yield: 34%). ¹H NMR (300 MHz, DMSO) δ 8.32 (s, 1H), 8.16 (s, 1H), 8.03 (s, 1H), 7.52 (s, 1H), 7.39 (s, 1H), 7.20 (s, 1H), 6.68 (s, 1H), 4.55 (t, J = 8.7 Hz, 2H), 3.77 (s, 3H), 3.22-3.09 (m, 6H), 2.96 (s, 3H), 2.89-2.76 (m, 6H)), 2.62 (t, J = 11.0 Hz, 3H), 2.50 (s, 3H), 2.08 (s, 3H), 1.96-1.85 (m, 2H), 1.69-1.57 (m, 2H). [M+H]⁺ = 701.

### Example 170: Preparation of N-(4-chloro-2-((5-chloro-2-((5-chloro-2-methoxy-4-(4-(4-methylpiperazin-1-yl)piperidin-1-yl)phenyl)amino)pyrimidin-4-yl)amino)phenyl)-N-methylmethanesulfonamide

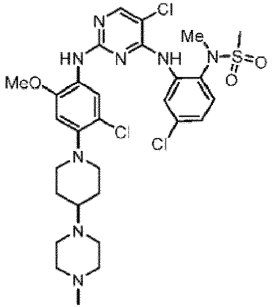

### Step 1) Preparation of N-(4-chloro-2-((2,5-dichloropyrimidin-4-yl)amino)phenyl)-N-methylmethanesulfonamide

The title compound was prepared in the same manner as in step 1) of Example 1, except that *N*-(2-amino-4-chlorophenyl)-*N*-methylmethanesulfonamide was used.

### Step 2) Preparation of N-(4-chloro-2-((5-chloro-2-((5-chloro-2-methoxy-4-(4-(4-methylpiperazin-1-yl)piperidin-1-yl)phenyl)amino)pyrimidin-4-yl)amino)phenyl)-N-methylmethanesulfonamide

The compound (1 eq.) prepared in step 1) and the corresponding aniline (0.6 eq.) were dissolved in a mixture of acetic acid (0.3 M) and a 1.25 M HCl/methanol solution in a 1:1 volume ratio, sealed, and stirred at 120 °C for 3 h. After completion of the reaction, the reaction mixture was cooled to room temperature and extracted with a mixture of isopropyl alcohol and chloroform in a 1:4 volume ratio and a saturated aqueous solution of sodium bicarbonate. Thereafter, the organic layer was dried over anhydrous sodium sulfate, filtered under reduced pressure, and distilled under reduced pressure. The resulting residue was purified by column chromatography (10% methanol/dichloromethane) to afford the title compound (yield: 32%). [M+H]⁺ = 683.

### Example 171: Preparation of N-(4-chloro-2-((5-chloro-2-((2-chloro-5-methyl-4-(4-(4-methylpiperazin-1-yl)piperidin-1-yl)phenyl)amino)pyrimidin-4-yl)amino)phenyl)-N-methylmethanesulfonamide

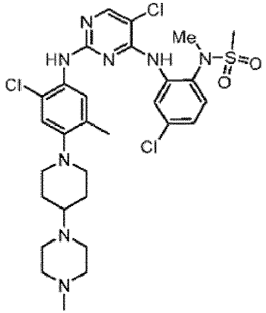

The title compound was prepared in the same manner as in step 2) of Example 170. [M+H]⁺ = 667.

### Example 172: Preparation of N-(6-((5-chloro-2-((2-chloro-5-methyl-4-(4-(4-methyl-1,4-diazepan-1-yl)piperidin-1-yl)phenyl)amino)pyrimidin-4-yl)amino)-2,3-dihydrobenzofuran-5-yl)methanesulfonamide

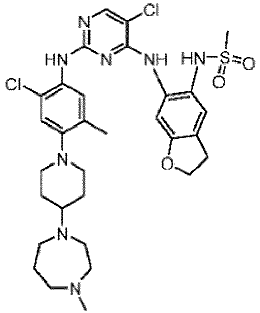

The title compound was prepared in the same manner as in step 5) of Example 165. [M+H]⁺ = 675.

### Example 173: Preparation of N-(6-((5-chloro-2-((2-chloro-5-methyl-4-(4-(4-methylpiperazin-1-yl)piperidin-1-yl)phenyl)amino)pyrimidin-4-yl)amino)-2,3-dihydrobenzofuran-5-yl)methanesulfonamide

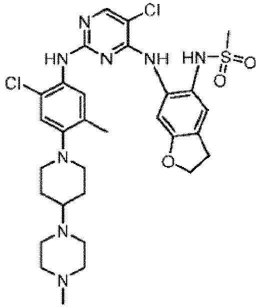

The title compound was prepared in the same manner as in step 5) of Example 165. [M+H]⁺ = 661.

### Example 174: Preparation of N-(6-((5-chloro-2-((5-chloro-2-methoxy-4-(4-(4-methyl-1,4-diazepan-1-yl)piperidin-1-yl)phenyl)amino)pyrimidin-4-yl)amino)-2,3-dihydrobenzofuran-5-yl)methanesulfonamide

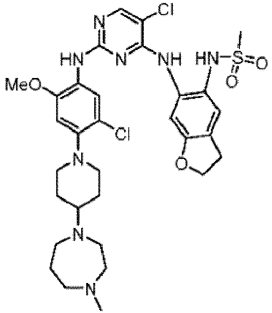

The title compound was prepared in the same manner as in step 5) of Example 165. ¹H NMR (300 MHz, DMSO) δ 8.48 (s, 1H), 8.14 (s, 1H), 8.09 (s, 1H), 7.69 (s, 1H), 7.44 (s, 1H), 7.20 (s, 1H), 6.77 (s, 1H), 4.55 (t, J = 8.7 Hz, 2H), 3.81 (s, 3H), 3.22-3.12 (m, 3H), 2.93 (s, 3H), 2.82-2.64 (m, 6H)), 2.64-2.52 (m, 6H), 2.28 (s, 3H), 1.84-1.68 (m, 4H), 1.65-1.53 (m, 2H) [M+H]⁺ = 691.

### Example 175: Preparation of N-(6-((5-bromo-2-((2-chloro-5-methyl-4-(4-(4-methylpiperazin-1-yl)piperidin-1-yl)phenyl)amino)pyrimidin-4-yl)amino)-2,3-dihydrobenzofuran-5-yl)methanesulfonamide

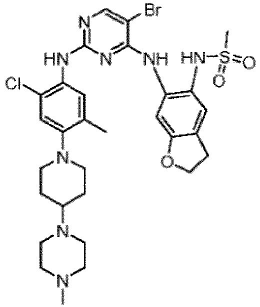

The title compound was prepared in the same manner as in step 2) of Example 169. [M+H]⁺ = 706.

### Example 176: Preparation of N-(4-chloro-2-((5-chloro-2-((2-methoxy-5-methyl-4-(4-(4-methylpiperazin-1-yl)piperidin-1-yl)phenyl)amino)pyrimidin-4-yl)amino)-5-fluorophenyl)methanesulfonamide

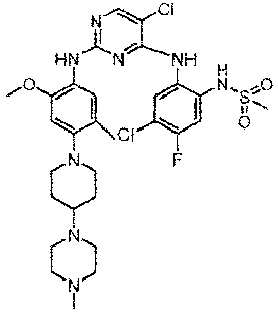

### Step 1) Preparation of N-(4-chloro-2-((2,5-dichloropyrimidin-4-yl)amino)-5-fluorophenyl)methanesulfonamide

The title compound was prepared in the same manner as in step 1) of Example 1, except that *N-*(2-amino-4-chloro-5-fluorophenyl)methanesulfonamide was used.

### Step 2) Preparation of N-(4-chloro-2-((5-chloro-2-((2-methoxy-5-methyl-4-(4-(4-methylpiperazin-1-yl)piperidin-1-yl)phenyl)amino)pyrimidin-4-yl)amino)-5-fluorophenyl)methanesulfonamide

The compound (1 eq.) prepared in step 1) and the corresponding aniline (0.6 eq.) were dissolved in a mixture of acetic acid (0.3 M) and a 1.25 M HCl/methanol solution in a 1:1 volume ratio, sealed, and stirred at 120 °C for 3 h. After completion of the reaction, the reaction mixture was cooled to room temperature and extracted with a mixture of isopropyl alcohol and chloroform in a 1:4 volume ratio and a saturated aqueous solution of sodium bicarbonate. Thereafter, the organic layer was dried over anhydrous sodium sulfate, filtered under reduced pressure, and distilled under reduced pressure. The resulting residue was purified by column chromatography (10% methanol/dichloromethane) to afford the title compound (yield: 35%). ¹H NMR (300 MHz, DMSO) δ 8.94 (s, 1H), 8.09 (s, 1H), 8.06 (s, 1H), 8.00 (s, 1H), 7.33 (s, 1H), 7.24 (s, 1H), 7.21 (s, 1H), 6.69 (s, 1H), 3.76 (s, 3H), 3.16-3.03 (m, 3H), 2.89-2.78 (m, 7H), 2.78 (s, 3H), 2.69-2.55 (m, 3H), 2.50 (s, 3H), 2.08 (s, 3H), 1.96-1.82 (m, 2H), 1.70-1.50 (m, 2H). [M+H]⁺ = 667.

### Example 177: Preparation of N-(2-((5-bromo-2-((2-chloro-5-methyl-4-(4-(4-methylpiperazin-1-yl)piperidin-1-yl)phenyl)amino)pyrimidin-4-yl)amino)-4-chloro-5-fluorophenyl)methanesulfonamide

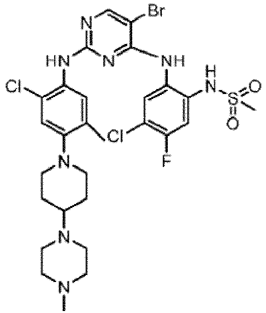

### Step 1) Preparation of N-(2-((5-bromo-2-chloropyrimidin-4-yl)amino)-4-chloro-5-fluorophenyl)methanesulfonamide

The title compound was prepared in the same manner as in step 1) of Example 1, except that *N-*(2-amino-4-chloro-5-fluorophenyl)methanesulfonamide and 5-bromo-2,4-dichloropyrimidine were used.

### Step 2) Preparation of N-(2-((5-bromo-2-((2-chloro-5-methyl-4-(4-(4-methylpiperazin-1-yl)piperidin-1-yl)phenyl)amino)pyrimidin-4-yl)amino)-4-chloro-5-fluorophenyl)methanesulfonamide

The compound (1 eq.) prepared in step 1) and the corresponding aniline (0.6 eq.) were dissolved in a mixture of acetic acid (0.3 M) and a 1.25 M HCl/methanol solution in a 1:1 volume ratio, sealed, and stirred at 120 °C for 3 h. After completion of the reaction, the reaction mixture was cooled to room temperature and extracted with a mixture of isopropyl alcohol and chloroform in a 1:4 volume ratio and a saturated aqueous solution of sodium bicarbonate. Thereafter, the organic layer was dried over anhydrous sodium sulfate, filtered under reduced pressure, and distilled under reduced pressure. The resulting residue was purified by column chromatography (10% methanol/dichloromethane) to afford the title compound (yield: 33%). [M+H]⁺ = 716.

### Example 178: Preparation of N-(2-((5-bromo-2-((5-chloro-2-methoxy-4-(4-(4-methylpiperazin-1-yl)piperidin-1-yl)phenyl)amino)pyrimidin-4-yl)amino)-4-chloro-5-fluorophenyl)methanesulfonamide

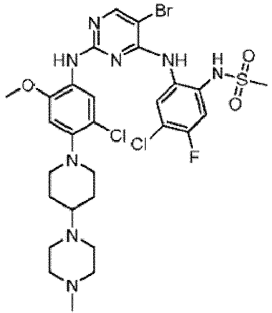

The title compound was prepared in the same manner as in step 2) of Example 177. [M+H]⁺ = 732.

### Example 179: Preparation of N-(4-chloro-2-((5-chloro-2-((2-chloro-4-(4-(3-(dimethylamino)pyrrolidin-1-yl)piperidin-1-yl)-5-methylphenyl)amino)pyrimidin-4-yl)amino)phenyl)-N-methylmethanesulfonamide

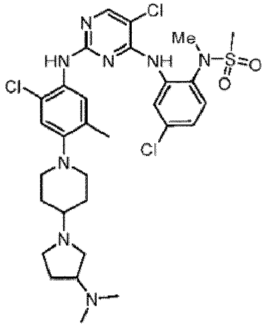

The title compound was prepared in the same manner as in step 2) of Example 170. [M+H]⁺ = 682.

### Example 180: Preparation of N-(4-chloro-2-((5-chloro-2-((2-chloro-5-methyl-4-(4-(4-methyl-1,4-diazepan-1-yl)piperidin-1-yl)phenyl)amino)pyrimidin-4-yl)amino)phenyl)-N-methylmethanesulfonamide

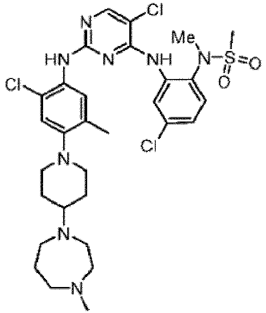

The title compound was prepared in the same manner as in step 2) of Example 170. [M+H]⁺ = 682.

### Example 181: Preparation of N-(4-chloro-2-((5-chloro-2-((5-chloro-2-methoxy-4-(4-(4-methylpiperazin-1-yl)piperidin-1-yl)phenyl)amino)pyrimidin-4-yl)amino)-5-fluorophenyl)methanesulfonamide

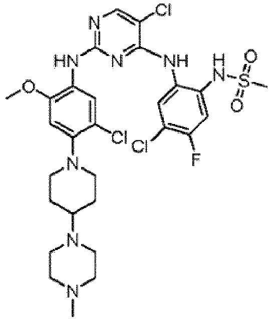

The title compound was prepared in the same manner as in step 2) of Example 176. [M+H]⁺ = 688.

### Example 182: Preparation of N-(4-chloro-2-((5-chloro-2-((2-chloro-5-methyl-4-(4-(4-methylpiperazin-1-yl)piperidin-1-yl)phenyl)amino)pyrimidin-4-yl)amino)-5-fluorophenyl)methanesulfonamide

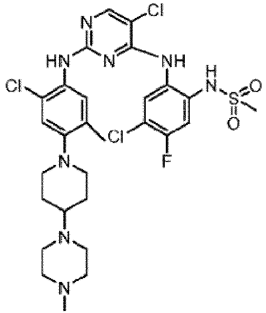

The title compound was prepared in the same manner as in step 2) of Example 176. [M+H]⁺ = 671.

### Example 183: Preparation of N-(6-((5-bromo-2-((5-chloro-2-methoxy-4-(4-(4-methylpiperazin-1-yl)piperidin-1-yl)phenyl)amino)pyrimidin-4-yl)amino)-2,3-dihydrobenzofuran-5-yl)methanesulfonamide

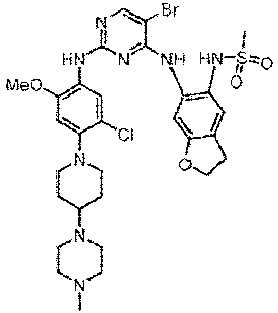

The title compound was prepared in the same manner as in step 2) of Example 169. [M+H]⁺ = 772.

### Example 184: Preparation of N-(6-((5-bromo-2-((5-ethyl-2-methoxy-4-(4-(4-methylpiperazin-1-yl)piperidin-1-yl)phenyl)amino)pyrimidin-4-yl)amino)-2,3-dihydrobenzofuran-5-yl)methanesulfonamide

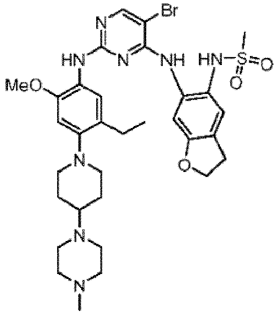

The title compound was prepared in the same manner as in step 2) of Example 169. [M+H]⁺ = 715.

### Example 185: Preparation of 5-chloro-N⁴-(5-chloro-2-(1H-1,2,3-triazol-5-yl)phenyl)-N²-(2-chloro-5-methyl-4-(4-(4-methylpiperazin-1-yl)piperidin-1-yl)phenyl)pyrimidine-2,4-diamine

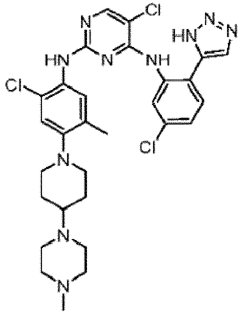

The title compound was prepared from the corresponding iodo compound in the same manner as in step 4) of Example 166. [M+H]⁺ = 627.

### Example 186: Preparation of 5-chloro-N⁴-(5-chloro-2-(1H-1,2,3-triazol-5-yl)phenyl)-N²-(5-chloro-2-methoxy-4-(4-(4-methylpiperazin-1-yl)piperidin-1-yl)phenyl)pyrimidine-2,4-diamine

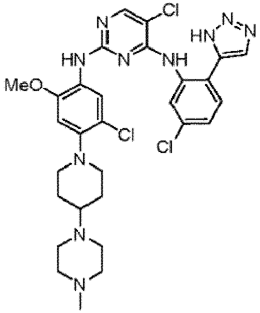

The title compound was prepared from the corresponding iodo compound in the same manner as in step 4) of Example 166. [M+H]⁺ = 643.

### Example 187: Preparation of 5-bromo-N⁴-(5-chloro-2-(1H-1,2,3-triazol-5-yl)phenyl)-N²-(2-methoxy-5-methyl-4-(4-(4-methylpiperazin-1-yl)piperidin-1-yl)phenyl)pyrimidine-2,4-diamine

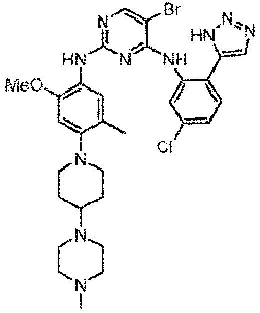

The title compound was prepared from the corresponding iodo compound in the same manner as in step 4) of Example 166. [M+H]⁺ = 668.

### Example 188: Preparation of N-(6-((5-bromo-2-((2-methoxy-5-methyl-4-(4-(4-methyl-1,4-diazepan-1-yl)piperidin-1-yl)phenyl)amino)pyrimidin-4-yl)amino)-2,3-dihydrobenzofuran-5-yl)methanesulfonamide

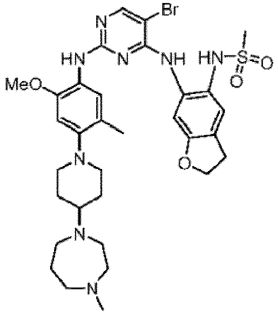

The title compound was prepared in the same manner as in step 2) of Example 169. [M+H]⁺ = 716.

### Example 189: Preparation of N-(6-((5-bromo-2-((5-chloro-2-methoxy-4-(4-(4-methyl-1,4-diazepan-1-yl)piperidin-1-yl)phenyl)amino)pyrimidin-4-yl)amino)-2,3-dihydrobenzofuran-5-yl)methanesulfonamide

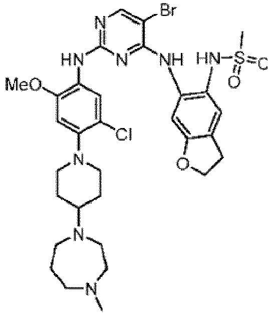

The title compound was prepared in the same manner as in step 2) of Example 169. [M+H]⁺ = 736.

### Example 190: Preparation of (6-((5-chloro-2-((5-ethyl-2-methoxy-4-(4-(4-methylpiperazin-1-yl)piperidin-1-yl)phenyl)amino)pyrimidin-4-yl)amino)quinoxalin-5-yl)dimethylphosphine oxide

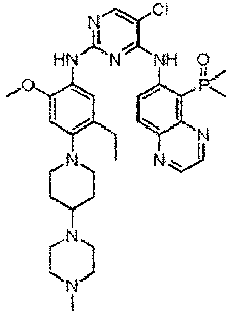

The title compound was prepared in the same manner as in step 6) of Example 122. [M+H]⁺ = 664.

### Example 191: Preparation of N-(2-((5-bromo-2-((5-ethyl-2-methoxy-4-(4-(4-methylpiperazin-1-yl)piperidin-1-yl)phenyl)amino)pyrimidin-4-yl)amino)-4-chlorophenyl)methanesulfonamide

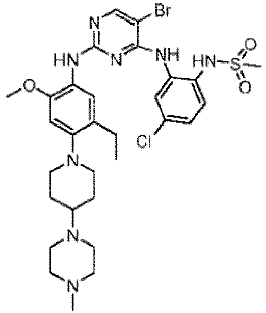

The title compound was prepared from the compound prepared in step 3) of Example 80 and the corresponding aniline in the same manner as in Example 1. [M+H]⁺ = 708.

### Example 192: Preparation of (6-((5-bromo-2-((5-ethyl-2-methoxy-4-(4-(4-methylpiperazin-1-yl)piperidin-1-yl)phenyl)amino)pyrimidin-4-yl)amino)quinoxalin-5-yl)dimethylphosphine oxide

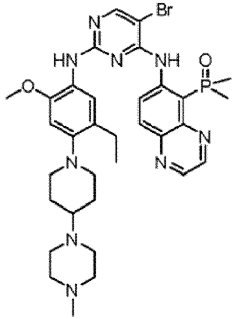

### Step 1) Preparation of (6-((5-bromo-2-chloro-4-yl)amino)quinoxalin-5-yl)dimethylphosphine oxide

The compound (1 eq.) prepared in step 4) of Example 122 was diluted with *N*,*N-*dimethylformamide (0.3 M), and 60% sodium hydride (1.5 eq.) was slowly added thereto at 0 °C. The mixture was stirred at room temperature for 30 min. To the reaction mixture was slowly added 5-bromo5-trichloropyrimidine (2 eq.) at 0°C, followed by stirring at room temperature for 2 h. After completion of the reaction, the reaction mixture was diluted with ethyl acetate and washed with distilled water and saturated brine. The organic layer was separated, dried over anhydrous sodium sulfate, filtered under reduced pressure, and distilled under reduced pressure. The resulting residue was purified by chromatography (50% ethyl acetate/hexane) to afford the title compound (yield: 60%).

### Step 2) Preparation of (6-((5-bromo-2-((5-ethyl-2-methoxy-4-(4-(4-methylpiperazin-1-yl)piperidin-1-yl)phenyl)amino)pyrimidin-4-yl)amino)quinoxalin-5-yl)dimethylphosphine oxide

The compound (1 eq.) prepared in step 1) and the corresponding aniline (0.6 eq.) were dissolved in a mixture of acetic acid (0.3 M) and a 1.25 M HCl/methanol solution in a 1:1 volume ratio, sealed, and stirred at 120 °C for 3 h. After completion of the reaction, the reaction mixture was cooled to room temperature and extracted with a mixture of isopropyl alcohol and chloroform in a 1:4 volume ratio and a saturated aqueous solution of sodium bicarbonate. Thereafter, the organic layer was dried over anhydrous sodium sulfate, filtered under reduced pressure, and distilled under reduced pressure. The resulting residue was purified by column chromatography (10% methanol/dichloromethane) to afford the title compound (yield: 33%). [M+H]⁺ = 709.

### Example 193: Preparation of (6-((5-bromo-2-((2-chloro-5-methyl-4-(4-(4-methylpiperazin-1-yl)piperidin-1-yl)phenyl)amino)pyrimidin-4-yl)amino)quinoxalin-5-yl)dimethylphosphine oxide

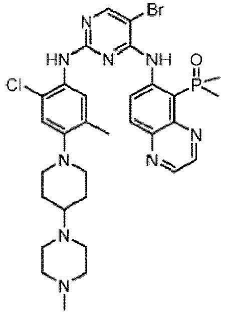

The title compound was prepared in the same manner as in step 2) of Example 192. [M+H]⁺ = 699.

### Example 194: Preparation of (6-((5-bromo-2-((5-chloro-2-methoxy-4-(4-(4-methylpiperazin-1-yl)piperidin-1-yl)phenyl)amino)pyrimidin-4-yl)amino)quinoxalin-5-yl)dimethylphosphine oxide

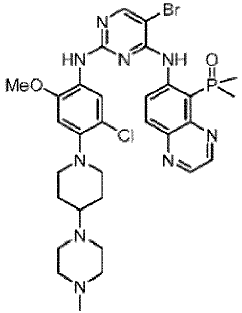

The title compound was prepared in the same manner as in step 2) of Example 192. [M+H]⁺ = 715.

### Example 195: Preparation of 5-bromo-N⁴-(5-chloro-2-(1H-1,2,3-triazol-5-yl)phenyl)-N²-(5-chloro-2-methoxy-4-(4-(4-methylpiperazin-1-yl)piperidin-1-yl)phenyl)pyrimidine-2,4-diamine

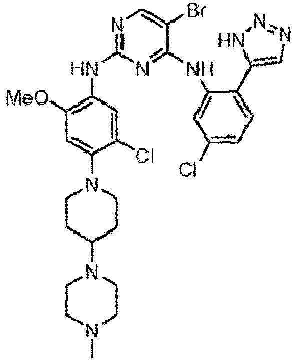

The title compound was prepared from the corresponding iodo compound in the same manner as in step 4) of Example 166. [M+H]⁺ = 688.

### Example 196: Preparation of N-(6-((5-bromo-2-((4-(3-(dimethylamino)-[1,4'-bipiperidin]-1'-yl)-2-methoxy-5-methylphenyl)amino)pyrimidin-4-yl)amino)-2,3-dihydrobenzofuran-5-yl)methanesulfonamide

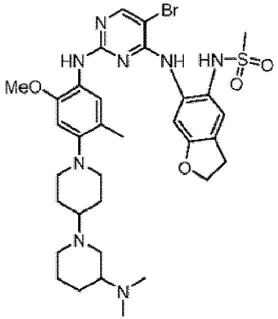

The title compound was prepared in the same manner as in step 2) of Example 169. [M+H]⁺ = 730.

### Example 197: Preparation of N-(6-((5-chloro-2-((2-chloro-4-(3-(dimethylamino)-[1,4'-bipiperidin]-1'-yl)-5-methylphenyl)amino)pyrimidin-4-yl)amino)-2,3-dihydrobenzofuran-5-yl)methanesulfonamide

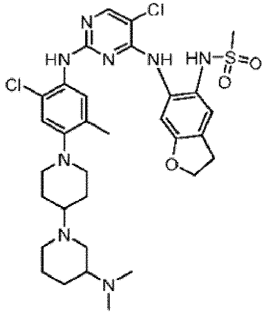

The title compound was prepared in the same manner as in step 5) of Example 165. [M+H]⁺ = 689.

### Example 198: Preparation of N-(4-chloro-2-((5-chloro-2-((2,5-dichloro-4-(4-(4-methylpiperazin-1-yl)piperidin-1-yl)phenyl)amino)pyrimidin-4-yl)amino)phenyl)methanesulfonamide

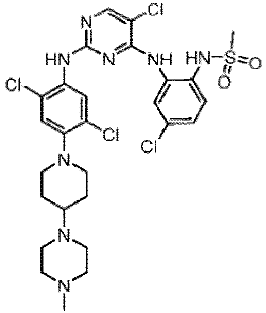

The title compound was prepared from the compound prepared in step 3) of Example 80 and the corresponding aniline in the same manner as in Example 1. [M+H]⁺ = 673.

### Example 199: Preparation of N-(2-((5-chloro-2-((2-methoxy-5-methyl-4-(4-(4-methylpiperazin-1-yl)piperidin-1-yl)phenyl)amino)pyrimidin-4-yl)amino)-4-methoxyphenyl)methanesulfonamide

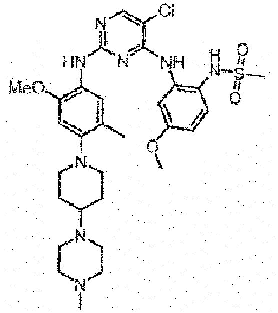

### Step 1) Preparation of N-(2-amino-4-methoxyphenyl)methanesulfonamide

The title compound was prepared in the same manner as in steps 1) to 3) of Example 165, except that 4-methoxy-2-nitroaniline was used in step 1) of Example 165.

### Step 2) Preparation of N-(2-((2,5-dichloropyrimidin-4-yl)amino)-4-methoxyphenyl)methanesulfonamide

The compound (1 eq.) prepared in step 1) was diluted with isopropyl alcohol (0.3 M), and 2,4,5-trichloropyrimidine (2 eq.) and sodium bicarbonate (4 eq.) were added thereto. The mixture was stirred under a nitrogen atmosphere at 65 °C for 40 h. After completion of the reaction, the reaction mixture was cooled to room temperature, washed with dichloromethane on a filter filled with celite, filtered under reduced pressure, diluted with a mixture of isopropyl alcohol and chloroform in a 1:4 volume ratio, and washed with distilled water. Thereafter, the organic layer was dried over anhydrous sodium sulfate, filtered under reduced pressure, and distilled under reduced pressure. The resulting residue was purified by column chromatography (40% tetrahydrofuran/hexane) to afford the title compound (yield: 60%).

### Step 3) Preparation of N-(2-((5-chloro-2-((2-methoxy-5-methyl-4-(4-(4-methylpiperazin-1-yl)piperidin-1-yl)phenyl)amino)pyrimidin-4-yl)amino)-4-methoxyphenyl)methanesulfonamide

The compound (1 eq.) prepared in step 2) and the corresponding aniline (0.6 eq.) were dissolved in a mixture of acetic acid (0.3 M) and a 1.25 M HCl/methanol solution in a 1:1 volume ratio, sealed, and stirred at 120 °C for 3 h. After completion of the reaction, the reaction mixture was cooled to room temperature and extracted with a mixture of isopropyl alcohol and chloroform in a 1:4 volume ratio and a saturated aqueous solution of sodium bicarbonate. Thereafter, the organic layer was dried over anhydrous sodium sulfate, filtered under reduced pressure, and distilled under reduced pressure. The resulting residue was purified by column chromatography (10% methanol/dichloromethane) to afford the title compound (yield: 30%). [M+H]⁺ = 645.

### Example 200: Preparation of N-(2-((5-chloro-2-((2-chloro-5-methyl-4-(4-(4-methylpiperazin-1-yl)piperidin-1-yl)phenyl)amino)pyrimidin-4-yl)amino)-4-methoxyphenyl)methanesulfonamide

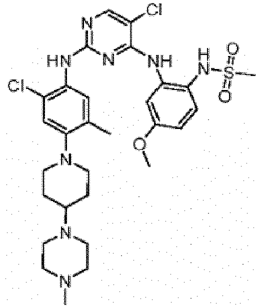

The title compound was prepared in the same manner as in step 3) of Example 199. [M+H]⁺ = 649.

### Example 201: Preparation of N-(2-((5-chloro-2-((5-chloro-2-methoxy-4-(4-(4-methylpiperazin-1-yl)piperidin-1-yl)phenyl)amino)pyrimidin-4-yl)amino)-4-methoxyphenyl)methanesulfonamide

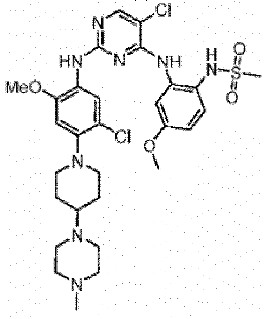

The title compound was prepared in the same manner as in step 3) of Example 199. [M+H]⁺ = 665.

### Example 202: Preparation of N-(2-((5-chloro-2-((2,5-dichloro-4-(4-(4-methylpiperazin-1-yl)piperidin-1-yl)phenyl)amino)pyrimidin-4-yl)amino)-4-methoxyphenyl)methanesulfonamide

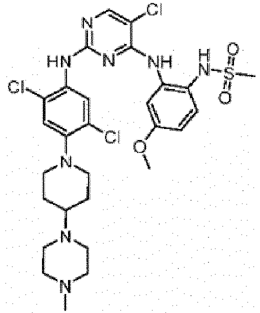

The title compound was prepared in the same manner as in step 3) of Example 199. [M+H]⁺ = 669.

### Example 203: Preparation of N-(2-((5-chloro-2-((5-ethyl-2-methoxy-4-(4-(4-methylpiperazin-1-yl)piperidin-1-yl)phenyl)amino)pyrimidin-4-yl)amino)-4-methoxyphenyl)methanesulfonamide

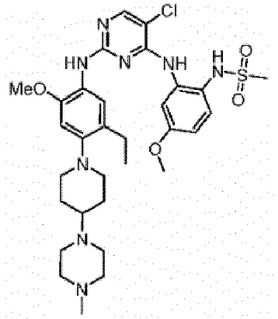

The title compound was prepared in the same manner as in step 3) of Example 199. [M+H]⁺ = 659.

### Example 204: Preparation of N-(2-((5-chloro-2-((2-methoxy-5-methyl-4-(4-(4-methyl-1,4-diazepan-1 -yl)piperidin-1 -yl)phenyl)amino)pyrimidin-4-yl)amino)-4-methoxyphenyl)methanesulfonamide

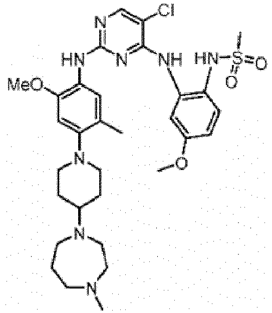

The title compound was prepared in the same manner as in step 3) of Example 199. [M+H]⁺ = 659.

### Example 205: Preparation of N-(6-((5-chloro-2-((2,5-dichloro-4-(4-(4-methylpiperazin-1-yl)piperidin-1-yl)phenyl)amino)pyrimidin-4-yl)amino)-2,3-dihydrobenzofuran-5-yl)methanesulfonamide

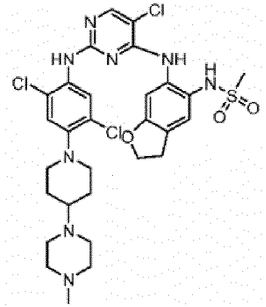

The title compound was prepared in the same manner as in step 5) of Example 165. [M+H]⁺ = 681.

### Example 206: Preparation of N-(6-((5-chloro-2-((5-ethyl-2-methoxy-4-(4-(4-methylpiperazin-1-yl)piperidin-1-yl)phenyl)amino)pyrimidin-4-yl)amino)-2,3-dihydrobenzofuran-5-yl)methanesulfonamide

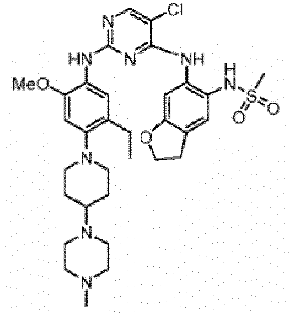

The title compound was prepared in the same manner as in step 5) of Example 165. [M+H]⁺ = 671.

### Example 207: Preparation of N-(6-((5-chloro-2-((2-methoxy-5-methyl-4-(4-(piperazin-1-yl)piperidin-1-yl)phenyl)amino)pyrimidin-4-yl)amino)-2,3-dihydrobenzofuran-5-yl)methanesulfonamide

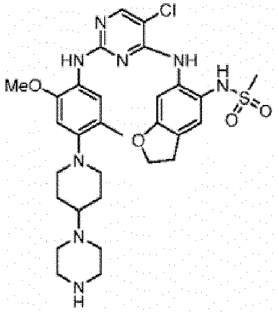

The title compound was prepared in the same manner as in step 5) of Example 165. [M+H]⁺ = 643.

### Example 208: Preparation of N-(6-((5-chloro-2-((4-(4-(4-cyclopropylpiperazin-1-yl)piperidin-1-yl)-2-methoxy-5-methylphenyl)amino)pyrimidin-4-yl)amino)-2,3-dihydrobenzofuran-5-yl)methanesulfonamide

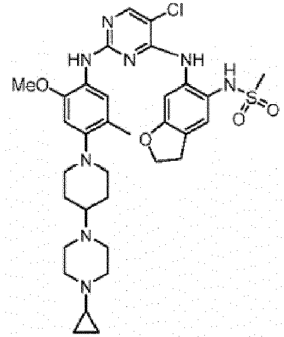

The title compound was prepared in the same manner as in step 5) of Example 165. [M+H]⁺ = 683.

### Example 209: Preparation of N-(6-((5-chloro-2-((4-(4-(3-(dimethylamino)pyrrolidin-1 -yl)piperidin-1 -yl)-2-methoxy-5-methylphenyl)amino)pyrimidin-4-yl)amino)-2,3-dihydrobenzofuran-5-yl)methanesulfonamide

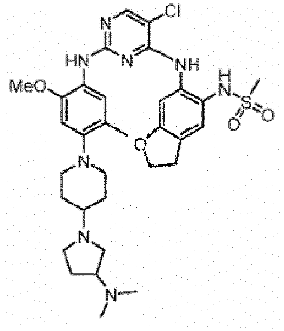

The title compound was prepared in the same manner as in step 5) of Example 165. [M+H]⁺ = 671.

### Example 210: Preparation of N-(6-((5-chloro-2-((2-chloro-4-(4-(3-(dimethylamino)pyrrolidin-1 -yl)piperidin-1 -yl)-5-methylphenyl)amino)pyrimidin-4-yl)amino)-2,3-dihydrobenzofuran-5-yl)methanesulfonamide

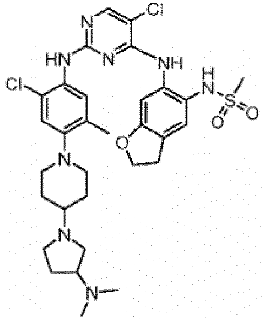

The title compound was prepared in the same manner as in step 5) of Example 165. [M+H]⁺ = 675.

### Example 211: Preparation of N-(6-((5-bromo-2-((4-(4-(3-(dimethylamino)pyrrolidin-1 -yl)piperidin-1 -yl)-2-methoxy-5-methylphenyl)amino)pyrimidin-4-yl)amino)-2,3-dihydrobenzofuran-5-yl)methanesulfonamide

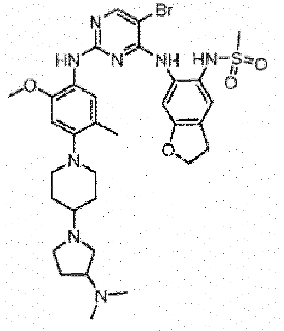

The title compound was prepared in the same manner as in step 2) of Example 169. [M+H]⁺ = 716.

### Example 212: Preparation of N-(6-((5-bromo-2-((4-(4-(4-cyclopropylpiperazin-1-yl)piperidin-1-yl)-2-methoxy-5-methylphenyl)amino)pyrimidin-4-yl)amino)-2,3-dihydrobenzofuran-5-yl)methanesulfonamide

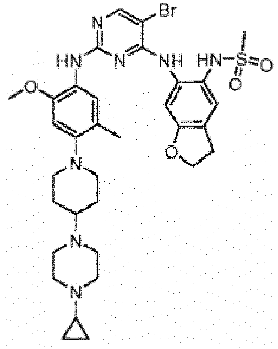

The title compound was prepared in the same manner as in step 2) of Example 169. [M+H]⁺ = 728.

### Example 213: Preparation of N-(6-((5-bromo-2-((2-chloro-4-(4-(3-(dimethylamino)pyrrolidin-1 -yl)piperidin-1 -yl)-5-methylphenyl)amino)pyrimidin-4-yl)amino)-2,3-dihydrobenzofuran-5-yl)methanesulfonamide

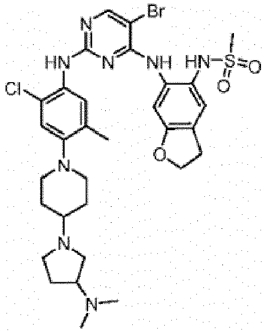

The title compound was prepared in the same manner as in step 2) of Example 169. [M+H]⁺ = 720.

### Example 214: Preparation of N-(6-((5-bromo-2-((2-chloro-4-(3-(dimethylamino)-[1,4'-bipiperidin]-1'-yl)-5-methylphenyl)amino)pyrimidin-4-yl)amino)-2,3-dihydrobenzofuran-5-yl)methanesulfonamide

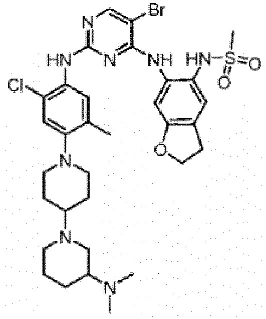

The title compound was prepared in the same manner as in step 2) of Example 169. [M+H]⁺ = 734.

### Example 215: Preparation of N-(6-((5-bromo-2-((2-methoxy-5-methyl-4-(4-(piperazin-1-yl)piperidin-1-yl)phenyl)amino)pyrimidin-4-yl)amino)-2,3-dihydrobenzofuran-5-yl)methanesulfonamide

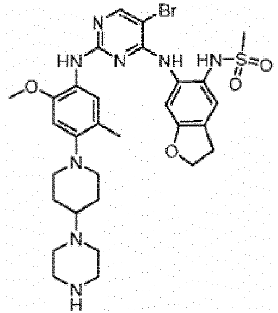

The title compound was prepared in the same manner as in step 2) of Example 169. [M+H]⁺ = 688.

### Example 216: Preparation of N-(6-((5-bromo-2-((5-chloro-4-(3-(dimethylamino)-[1,4'-bipiperidin]-1'-yl)-2-methoxyphenyl)amino)pyrimidin-4-yl)amino)-2,3-dihydrobenzofuran-5-yl)methanesulfonamide

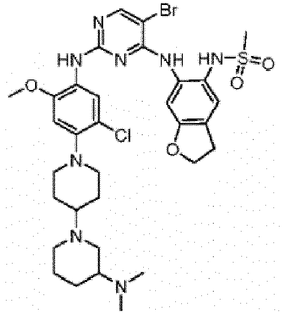

The title compound was prepared in the same manner as in step 2) of Example 169. [M+H]⁺ = 750.

### Example 217: Preparation of N-(6-((5-bromo-2-((2,5-dichloro-4-(4-(4-methylpiperazin-1-yl)piperidin-1-yl)phenyl)amino)pyrimidin-4-yl)amino)-2,3-dihydrobenzofuran-5-yl)methanesulfonamide

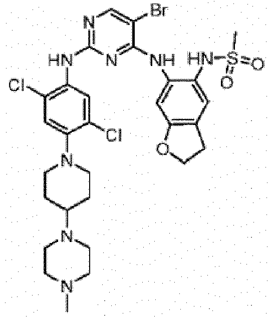

The title compound was prepared in the same manner as in step 2) of Example 169. [M+H]⁺ = 726.

### Example 218: Preparation of N-(6-((5-chloro-2-((4-(3-(dimethylamino)-[1,4'-bipiperidin]-1'-yl)-2-methoxy-5-methylphenyl)amino)pyrimidin-4-yl)amino)-2,3-dihydrobenzofuran-5-yl)methanesulfonamide

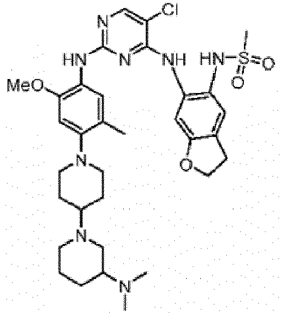

The title compound was prepared in the same manner as in step 5) of Example 165. [M+H]⁺ = 685.

### Example 219: Preparation of N-(6-((5-chloro-2-((5-chloro-4-(3-(dimethylamino)-[1,4'-bipiperidin]-1'-yl)-2-methoxyphenyl)amino)pyrimidin-4-yl)amino)-2,3-dihydrobenzofuran-5-yl)methanesulfonamide

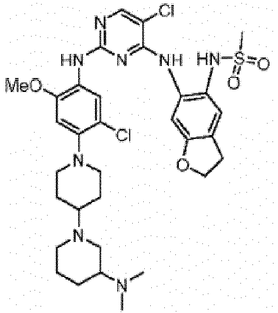

The title compound was prepared in the same manner as in step 5) of Example 165. [M+H]⁺ = 705.

### Example 220: Preparation of N-(6-((5-chloro-2-((2-methoxy-4-(4-(4-methylpiperazin-1-yl)piperidin-1-yl)phenyl)amino)pyrimidin-4-yl)amino)-2,3-dihydrobenzofuran-5-yl)methanesulfonamide

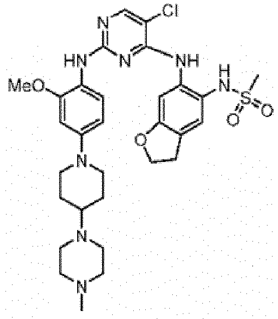

The title compound was prepared in the same manner as in step 5) of Example 165. [M+H]⁺ = 643.

### Example 221: Preparation of N-(6-((5-chloro-2-((2-chloro-4-(4-(4-methylpiperazin-1-yl)piperidin-1-yl)phenyl)amino)pyrimidin-4-yl)amino)-2,3-dihydrobenzofuran-5-yl)methanesulfonamide

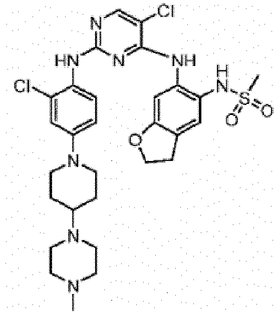

The title compound was prepared in the same manner as in step 5) of Example 165. [M+H]⁺ = 647.

### Example 222: Preparation of N-(6-((5-chloro-2-((5-fluoro-2-methoxy-4-(4-(4-methylpiperazin-1-yl)piperidin-1-yl)phenyl)amino)pyrimidin-4-yl)amino)-2,3-dihydrobenzofuran-5-yl)methanesulfonamide

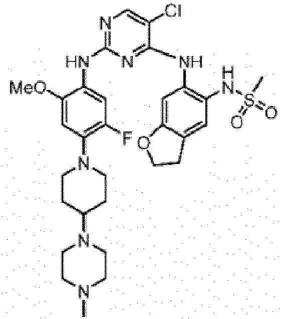

The title compound was prepared in the same manner as in step 5) of Example 165. [M+H]⁺ = 661.

### Example 223: Preparation of N-(6-((5-chloro-2-((2-(4-(4-methylpiperazin-1-yl)piperidin-1-yl)quinolin-6-yl)amino)pyrimidin-4-yl)amino)-2,3-dihydrobenzofuran-5-yl)methanesulfonamide

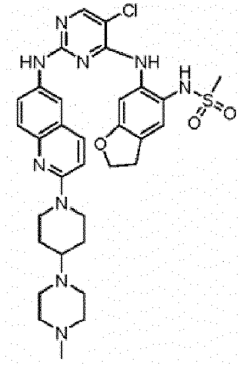

The title compound was prepared in the same manner as in step 5) of Example 165. [M+H]⁺ = 664.

### Example 224: Preparation of N-(6-((5-chloro-2-((2-methoxy-5-methyl-4-(4-((1-methyl-1H-pyrazol-5-yl)amino)piperidin-1-yl)phenyl)amino)pyrimidin-4-yl)amino)-2,3-dihydrobenzofuran-5-yl)methanesulfonamide

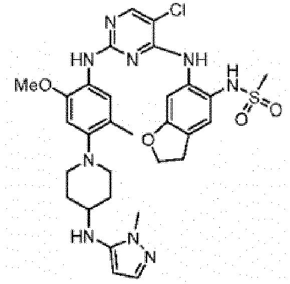

The title compound was prepared in the same manner as in step 5) of Example 165. [M+H]⁺ = 654.

### Example 225: Preparation of N-(6-((5-chloro-2-((2-methoxy-5-methyl-4-(4-(4-(trifluoromethyl)piperazin-1-yl)piperidin-1-yl)phenyl)amino)pyrimidin-4-yl)amino)-2,3-dihydrobenzofuran-5-yl)methanesulfonamide

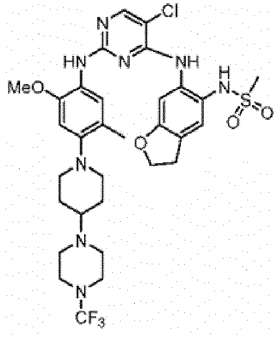

The title compound was prepared in the same manner as in step 5) of Example 165. [M+H]⁺ = 711.

### Example 226: Preparation of N-(4-chloro-2-((5-chloro-2-((2,5-dichloro-4-(4-(4-methylpiperazin-1-yl)piperidin-1-yl)phenyl)amino)pyrimidin-4-yl)amino)phenyl)-N-methylmethanesulfonamide

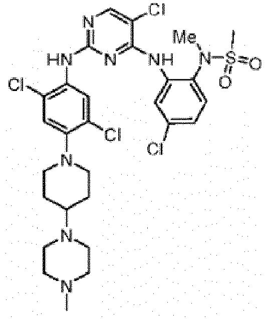

The title compound was prepared in the same manner as in step 2) of Example 170. [M+H]⁺ = 688.

### Example 227: Preparation of N-(2-((5-bromo-2-((2-chloro-4-(4-(3-(dimethylamino)pyrrolidin-1-yl)piperidin-1-yl)-5-methylphenyl)amino)pyrimidin-4-yl)amino)-4-chlorophenyl)-N-methylmethanesulfonamide

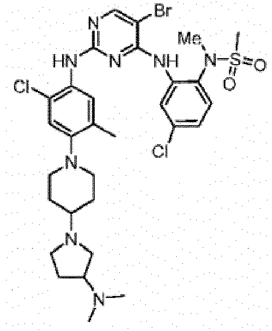

The title compound was prepared from the corresponding aniline in the same manner as in Example 170, except that 5-bromo-2,4-dichloropyrimidine was used in step 2) of Example 170. [M+H]⁺ = 726.

### Example 228: Preparation of N-(2-((5-bromo-2-((2,5-dichloro-4-(4-(4-methylpiperazin-1-yl)piperidin-1-yl)phenyl)amino)pyrimidin-4-yl)amino)-4-chlorophenyl)-N-methylmethanesulfonamide

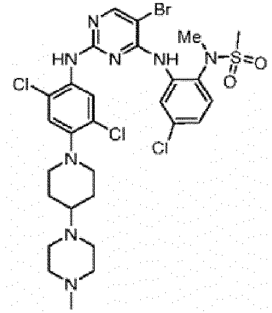

The title compound was prepared from the corresponding aniline in the same manner as in Example 170, except that 5-bromo-2,4-dichloropyrimidine was used in step 2) of Example 170. [M+H]⁺ = 732.

### Example 229: Preparation of N-(2-((5-chloro-2-((5-chloro-2-methoxy-4-(4-(4-methyl-1,4-diazepan-1 -yl)piperidin-1 -yl)phenyl)amino)pyrimidin-4-yl)amino)-4-methoxyphenyl)methanesulfonamide

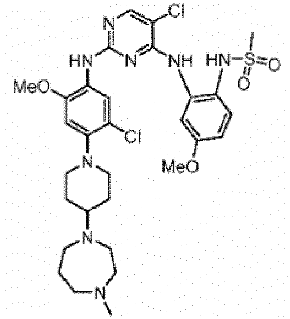

The title compound was prepared in the same manner as in step 3) of Example 199. [M+H]⁺ = 679.

### Example 230: Preparation of N-(2-((5-chloro-2-((2-chloro-4-(4-(3-(dimethylamino)pyrrolidin-1-yl)piperidin-1-yl)-5-methylphenyl)amino)pyrimidin-4-yl)amino)-4-methoxyphenyl)methanesulfonamide

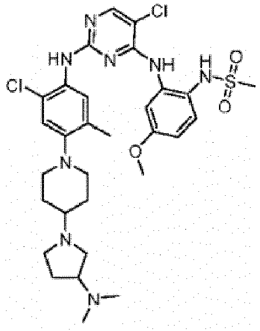

The title compound was prepared in the same manner as in step 3) of Example 199. [M+H]⁺ = 663.

### Example 231: Preparation of N-(2-((5-chloro-2-((2-methoxy-4-(4-(4-methylpiperazin-1-yl)piperidin-1-yl)phenyl)amino)pyrimidin-4-yl)amino)-4-methoxyphenyl)methanesulfonamide

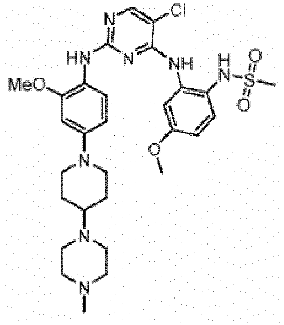

The title compound was prepared in the same manner as in step 3) of Example 199. [M+H]⁺ = 631.

### Example 232: Preparation of N-(2-((5-chloro-2-((4-(3-(dimethylamino)-[1,4'-bipiperidin]-1'-yl)-2-methoxy-5-methylphenyl)amino)pyrimidin-4-yl)amino)-4-methoxyphenyl)methanesulfonamide

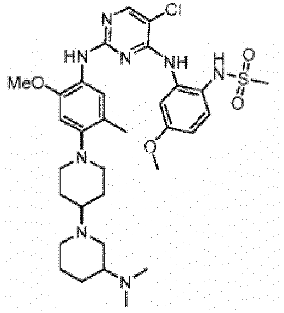

The title compound was prepared in the same manner as in step 3) of Example 199. [M+H]⁺ = 673.

### Example 233: Preparation of N-(2-((5-chloro-2-((4-(4-(4-cyclopropylpiperazin-1-yl)piperidin-1-yl)-2-methoxy-5-methylphenyl)amino)pyrimidin-4-yl)amino)-4-methoxyphenyl)methanesulfonamide

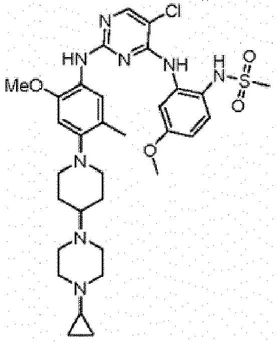

The title compound was prepared in the same manner as in step 3) of Example 199. [M+H]⁺ = 671.

### Example 234: Preparation of N-(2-((5-chloro-2-((2-methoxy-5-methyl-4-(4-(trifluoromethyl)-[1,4'-bipiperidin]-1'-yl)phenyl)amino)pyrimidin-4-yl)amino)-4-methoxyphenyl)methanesulfonamide

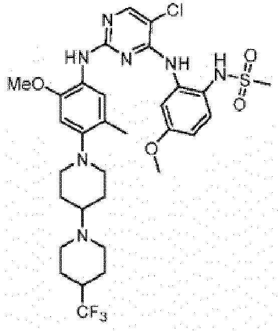

The title compound was prepared in the same manner as in step 3) of Example 199. [M+H]⁺ = 698.

### Example 235: Preparation of N-(2-((5-chloro-2-((4-(4-(3-(dimethylamino)pyrrolidin-1-yl)piperidin-1-yl)-2-methoxy-5-methylphenyl)amino)pyrimidin-4-yl)amino)-4-methoxyphenyl)methanesulfonamide

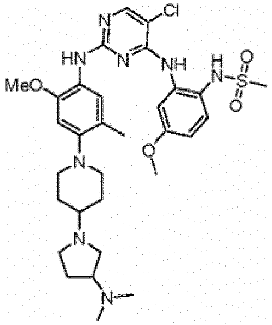

The title compound was prepared in the same manner as in step 3) of Example 199. [M+H]⁺ = 659.

### Example 236: Preparation of N-(2-((5-chloro-2-((2-chloro-4-(3-(dimethylamino)-[1,4'-bipiperidin]-1'-yl)-5-methylphenyl)amino)pyrimidin-4-yl)amino)-4-methoxyphenyl)methanesulfonamide

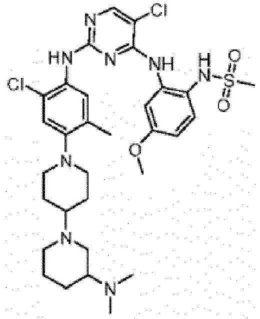

The title compound was prepared in the same manner as in step 3) of Example 199. [M+H]⁺ = 677.

### Example 237: Preparation of N-(2-((5-chloro-2-((5-chloro-4-(3-(dimethylamino)-[1,4'-bipiperidin]-1'-yl)-2-methoxyphenyl)amino)pyrimidin-4-yl)amino)-4-methoxyphenyl)methanesulfonamide

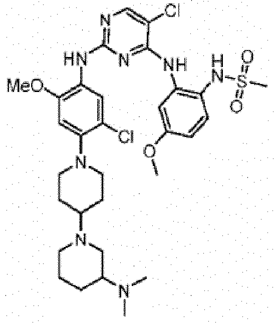

The title compound was prepared in the same manner as in step 3) of Example 199. [M+H]⁺ = 693.

### Example 238: Preparation of N-(6-((5-bromo-2-((2-methoxy-6-(4-methyl-1,4-diazepan-1-yl)pyridin-3-yl)amino)pyrimidin-4-yl)amino)-2,3-dihydrobenzofuran-5-yl)methanesulfonamide

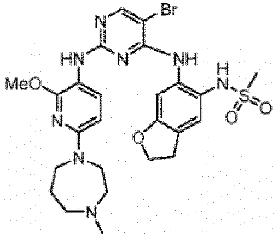

The title compound was prepared in the same manner as in step 2) of Example 169. [M+H]⁺ = 620.

### Example 239: Preparation of N-(6-((5-bromo-2-((5-fluoro-6-(4-(4-methylpiperazin-1-yl)piperidin-1-yl)pyridin-3-yl)amino)pyrimidin-4-yl)amino)-2,3-dihydrobenzofuran-5-yl)methanesulfonamide

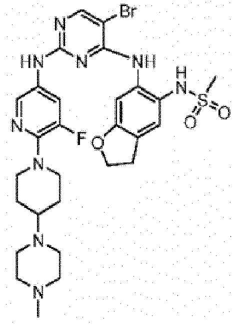

The title compound was prepared in the same manner as in step 2) of Example 169. [M+H]⁺ = 677.

### Example 240: Preparation of N-(6-((5-bromo-2-((2-methoxy-5-methyl-6-(4-(4-methylpiperazin-1-yl)piperidin-1-yl)pyridin-3-yl)amino)pyrimidin-4-yl)amino)-2,3-dihydrobenzofuran-5-yl)methanesulfonamide

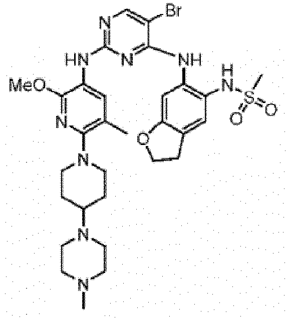

The title compound was prepared in the same manner as in step 2) of Example 169. [M+H]⁺ = 703.

### Example 241: Preparation of N-(6-((5-chloro-2-((2-methoxy-5-methyl-6-(4-(4-methylpiperazin-1-yl)piperidin-1-yl)pyridin-3-yl)amino)pyrimidin-4-yl)amino)-2,3-dihydrobenzofuran-5-yl)methanesulfonamide

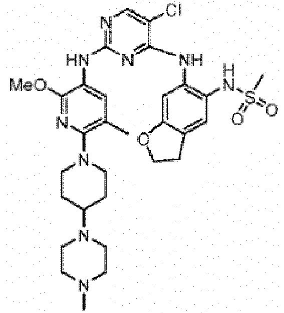

The title compound was prepared in the same manner as in step 5) of Example 165. [M+H]⁺ = 658.

### Example 242: Preparation of N-(6-((5-chloro-2-((5-fluoro-6-(4-(4-methylpiperazin-1-yl)piperidin-1-yl)pyridin-3-yl)amino)pyrimidin-4-yl)amino)-2,3-dihydrobenzofuran-5-yl)methanesulfonamide

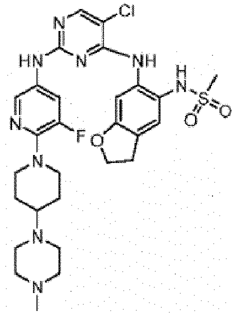

The title compound was prepared in the same manner as in step 5) of Example 165. [M+H]⁺ = 632.

### Example 243: Preparation of N-(6-((5-bromo-2-((2-methoxy-4-(4-(4-methylpiperazin-1-yl)piperidin-1-yl)phenyl)amino)pyrimidin-4-yl)amino)-2,3-dihydrobenzofuran-5-yl)methanesulfonamide

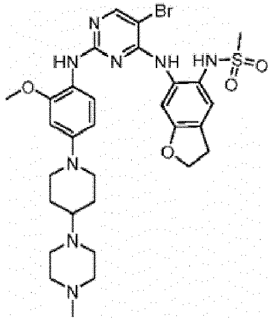

The title compound was prepared in the same manner as in step 2) of Example 169. [M+H]⁺ = 688.

### Example 244: Preparation of N-(6-((5-bromo-2-((3-fluoro-4-(4-(4-methylpiperazin-1-yl)piperidin-1-yl)phenyl)amino)pyrimidin-4-yl)amino)-2,3-dihydrobenzofuran-5-yl)methanesulfonamide

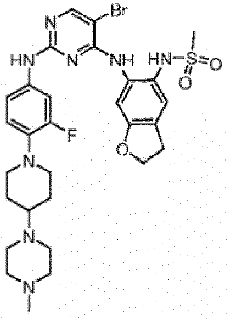

The title compound was prepared in the same manner as in step 2) of Example 169. [M+H]⁺ = 676.

### Example 245: Preparation of N-(6-((5-bromo-2-((2-methoxy-5-methyl-4-(4-(trifluoromethyl)-[1,4'-bipiperidin]-1'-yl)phenyl)amino)pyrimidin-4-yl)amino)-2,3-dihydrobenzofuran-5-yl)methanesulfonamide

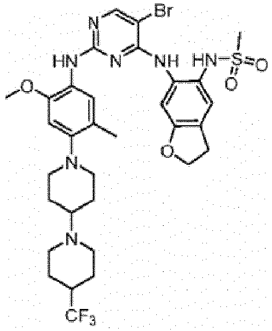

The title compound was prepared in the same manner as in step 2) of Example 169. [M+H]⁺ = 755.

### Example 246: Preparation of N-(6-((5-bromo-2-((2-chloro-4-(4-(4-methylpiperazin-1-yl)piperidin-1-yl)phenyl)amino)pyrimidin-4-yl)amino)-2,3-dihydrobenzofuran-5-yl)methanesulfonylamide

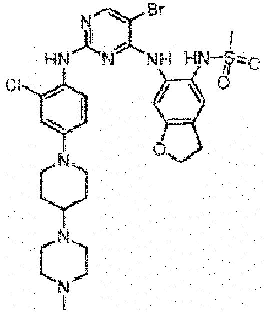

The title compound was prepared in the same manner as in step 2) of Example 169. [M+H]⁺ = 692.

### Example 247: Preparation of N-(6-((5-bromo-2-((2-(4-(4-methylpiperazin-1-yl)piperidin-1-yl)quinolin-6-yl)amino)pyrimidin-4-yl)amino)-2,3-dihydrobenzofuran-5-yl)methanesulfonamide

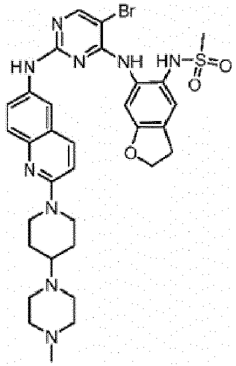

The title compound was prepared in the same manner as in step 2) of Example 169. [M+H]⁺ = 709.

### Example 248: Preparation of N-(6-((5-chloro-2-((2-methoxy-5-methyl-4-(4-(4-methylpiperazin-1-yl)piperidin-1-yl)phenyl)amino)pyrimidin-4-yl)amino)-2,3-dihydrobenzofuran-5-yl)-N-methylmethanesulfonamide

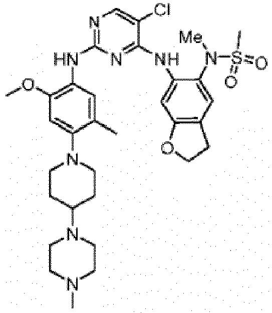

### Step 1) Preparation of N-methyl-N-(6-nitro-2,3-dihydrobenzofuran-5-yl)methanesulfonamide

The compound prepared in step 3) of Example 165 was diluted with acetone (0.3 M), and a 4 N NaOH solution (10 eq.) was added thereto. To the mixture was slowly added dimethyl sulfate (1.8 eq.) at 0 °C, followed by stirring at room temperature for 2 h. After completion of the reaction, the reaction mixture was distilled under reduced pressure and adjusted to pH 5-6 with a 1 N HCl solution at 0 °C. The resulting yellow solid was collected by filtration under reduced pressure and used in the next reaction without further purification.

### Step 2) Preparation of N-(6-amino-2,3-dihydrobenzofuran-5-yl)-N-methylsulfonamide

The compound prepared in step 1) was dissolved in a mixture of tetrahydrofuran, methanol, and water in an 18:1:1 volume ratio. To the solution were added an iron powder and ammonium chloride, followed by stirring at 85 °C for 3 h. After completion of the reaction, the reaction mixture was washed with dichloromethane on a filter filled with celite, filtered under reduced pressure, and concentrated to afford the title compound (yield: 95%, step 2).

### Step 3) Preparation of N-(6-((2,5-dichloropyrimidin-4-yl)amino)-2,3-dihydrobenzofuran-5-yl)-N-methylmethanesulfonamide

The compound (1 eq.) prepared in step 2) was diluted with isopropyl alcohol (0.3 M), and 2,4,5-trichloropyrimidine (2 eq.) and sodium bicarbonate (4 eq.) were added thereto. The mixture was stirred under a nitrogen atmosphere at 65 °C for 40 h. After completion of the reaction, the reaction mixture was cooled to room temperature, washed with dichloromethane on a filter filled with celite, filtered under reduced pressure, diluted with a mixture of isopropyl alcohol and chloroform in a 1:4 volume ratio, and washed with distilled water. Thereafter, the organic layer was dried over anhydrous sodium sulfate, filtered under reduced pressure, and distilled under reduced pressure. The resulting residue was purified by column chromatography (40% tetrahydrofuran/hexane) to afford the title compound (yield: 60%).

### Step 4) Preparation of N-(6-((5-chloro-2-((2-methoxy-5-methyl-4-(4-(4-methylpiperazin-1-yl)piperidin-1-yl)phenyl)amino)pyrimidin-4-yl)amino)-2,3-dihydrobenzofuran-5-yl)-N-methylmethanesulfonamide

The compound (1 eq.) prepared in step 3) and the corresponding aniline (0.6 eq.) were dissolved in a mixture of acetic acid (0.3 M) and a 1.25 M HCl/methanol solution in a 1:1 volume ratio, sealed, and stirred at 120 °C for 3 h. After completion of the reaction, the reaction mixture was cooled to room temperature and extracted with a mixture of isopropyl alcohol and chloroform in a 1:4 volume ratio and a saturated aqueous solution of sodium bicarbonate. Thereafter, the organic layer was dried over anhydrous sodium sulfate, filtered under reduced pressure, and distilled under reduced pressure. The resulting residue was purified by column chromatography (10% methanol/dichloromethane) to afford the title compound (yield: 35%). [M+H]⁺ = 671.

### Example 249: Preparation of N-(6-((5-chloro-2-((5-chloro-2-methoxy-4-(4-(4-methylpiperazin-1-yl)piperidin-1-yl)phenyl)amino)pyrimidin-4-yl)amino)-2,3-dihydrobenzofuran-5-yl)-N-methylmethanesulfonamide

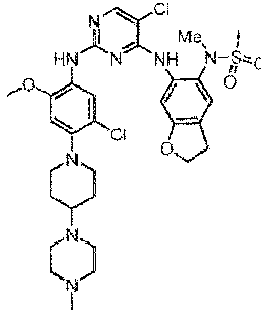

The title compound was prepared in the same manner as in step 4) of Example 248. [M+H]⁺ = 691.

### Example 250: Preparation of N-(6-((5-bromo-2-((2-methoxy-5-methyl-4-(4-(4-methylpiperazin-1-yl)piperidin-1-yl)phenyl)amino)pyrimidin-4-yl)amino)-2,3-dihydrobenzofuran-5-yl)-N-methylmethanesulfonamide

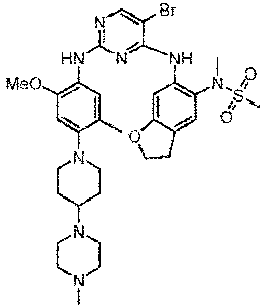

### Step 1) Preparation of N-(6-((5-bromo-2-chloropyrimidin-4-yl)amino)-2,3-dihydrobenzofuran-5-yl)-N-methylmethanesulfonamide

The compound (1 eq.) prepared in step 2) of Example 248 was diluted with isopropyl alcohol (0.3 M), and 5-bromo-2,4-dichloropyrimidine (2 eq.) and sodium bicarbonate (4 eq.) were added thereto. The mixture was stirred under a nitrogen atmosphere at 65 °C for 40 h. After completion of the reaction, the reaction mixture was cooled to room temperature, washed with dichloromethane on a filter filled with celite, filtered under reduced pressure, diluted with a mixture of isopropyl alcohol and chloroform in a 1:4 volume ratio, and washed with distilled water. Thereafter, the organic layer was dried over anhydrous sodium sulfate, filtered under reduced pressure, and distilled under reduced pressure. The resulting residue was purified by column chromatography (40% tetrahydrofuran/hexane) to afford the title compound (yield: 52%).

### Step 2) Preparation of N-(6-((5-bromo-2-((2-methoxy-5-methyl-4-(4-(4-methylpiperazin-1-yl)piperidin-1-yl)phenyl)amino)pyrimidin-4-yl)amino)-2,3-dihydrobenzofuran-5-yl)-N-methylmethanesulfonamide

The compound (1 eq.) prepared in step 1) and the corresponding aniline (0.6 eq.) were dissolved in a mixture of acetic acid (0.3 M) and a 1.25 M HCl/methanol solution in a 1:1 volume ratio, sealed, and stirred at 120 °C for 3 h. After completion of the reaction, the reaction mixture was cooled to room temperature and extracted with a mixture of isopropyl alcohol and chloroform in a 1:4 volume ratio and a saturated aqueous solution of sodium bicarbonate. Thereafter, the organic layer was dried over anhydrous sodium sulfate, filtered under reduced pressure, and distilled under reduced pressure. The resulting residue was purified by column chromatography (10% methanol/dichloromethane) to afford the title compound (yield: 29%). ¹H NMR (300 MHz, DMSO) δ 8.22 (s, 1H), 8.19 (s, 1H), 8.17 (s, 1H), 7.71 (s, 1H), 7.44 (s, 1H), 7.32 (s, 1H), 6.70 (s, 1H), 4.55 (t, J = 8.7 Hz, 2H), 3.76 (s, 3H), 3.23-3.13 (m, 4H), 3.14 (s, 3H), 3.10 (s, 3H), 2.68-2.54 (m, 5H), 2.45-2.23 (m, 6H), 2.18 (s, 3H), 2.12 (s, 3H), 1.91-1.80 (m, 2H), 1.67-1.50 (m, 2H). [M+H]⁺ = 716.

### Example 251: Preparation of N-(6-((5-bromo-2-((2-chloro-5-methyl-4-(4-(4-methylpiperazin-1-yl)piperidin-1-yl)phenyl)amino)pyrimidin-4-yl)amino)-2,3-dihydrobenzofuran-5-yl)-N-methylmethanesulfonamide

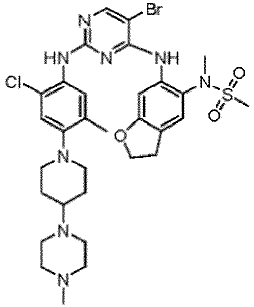

The title compound was prepared in the same manner as in step 2) of Example 250. [M+H]⁺ = 720.

### Example 252: Preparation of N-(6-((5-bromo-2-((5-chloro-2-methoxy-4-(4-(4-methylpiperazin-1-yl)piperidin-1-yl)phenyl)amino)pyrimidin-4-yl)amino)-2,3-dihydrobenzofuran-5-yl)-N-methylmethanesulfonamide

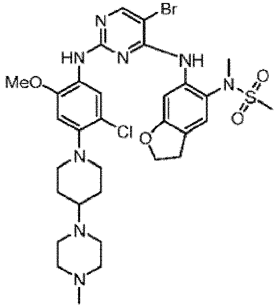

The title compound was prepared in the same manner as in step 2) of Example 250. [M+H]⁺ = 736.

### Example 253: Preparation of N-(6-((5-bromo-2-((2,5-dichloro-4-(4-(4-methylpiperazin-1-yl)piperidin-1-yl)phenyl)amino)pyrimidin-4-yl)amino)-2,3-dihydrobenzofuran-5-yl)-N-methylmethanesulfonamide

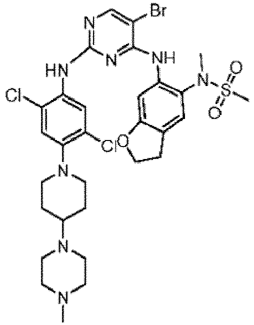

The title compound was prepared in the same manner as in step 2) of Example 250. [M+H]⁺ = 740.

### Example 254: Preparation of N-(6-((5-bromo-2-((5-ethyl-2-methoxy-4-(4-(4-methylpiperazin-1-yl)piperidin-1-yl)phenyl)amino)pyrimidin-4-yl)amino)-2,3-dihydrobenzofuran-5-yl)-N-methylmethanesulfonamide

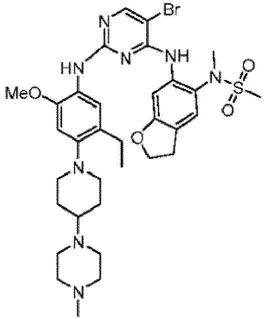

The title compound was prepared in the same manner as in step 2) of Example 250. [M+H]⁺ = 730.

### Example 255: Preparation of N-(6-((5-bromo-2-((2-methoxy-5-methyl-4-(4-(4-(trifluoromethyl)piperazin-1-yl)piperidin-1-yl)phenyl)amino)pyrimidin-4-yl)amino)-2,3-dihydrobenzofuran-5-yl)-N-methylmethanesulfonamide

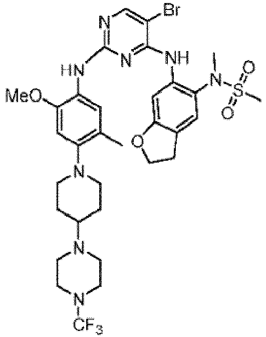

The title compound was prepared in the same manner as in step 2) of Example 250. [M+H]⁺ = 770.

### Example 256: Preparation of N-(6-((5-bromo-2-((5-fluoro-2-methoxy-4-(4-(4-methylpiperazin-1-yl)piperidin-1-yl)phenyl)amino)pyrimidin-4-yl)amino)-2,3-dihydrobenzofuran-5-yl)-N-methylmethanesulfonamide

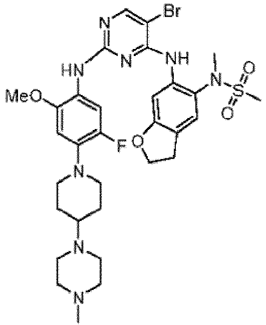

The title compound was prepared in the same manner as in step 2) of Example 250. [M+H]⁺ = 720.

### Example 257: Preparation of N-(6-((5-bromo-2-((5-fluoro-2-methoxy-4-(4-(4-methyl-1,4-diazepan-1-yl)piperidin-1-yl)phenyl)amino)pyrimidin-4-yl)amino)-2,3 - dihydrobenzofuran-5-yl)-N-methylmethanesulfonamide

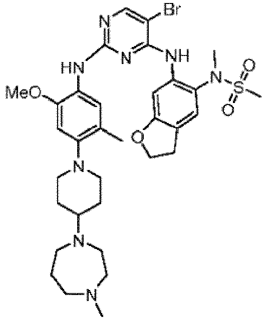

The title compound was prepared in the same manner as in step 2) of Example 250. [M+H]⁺ = 730.

### Example 258: Preparation of N-(6-((5-bromo-2-((4-(3-(dimethylamino)-[1,4'-bipiperidin]-1'-yl)-2-methoxy-5-methoxyphenyl)amino)pyrimidin-4-yl)amino)-2,3-dihydrobenzofuran-5-yl)-N-methylmethanesulfonamide

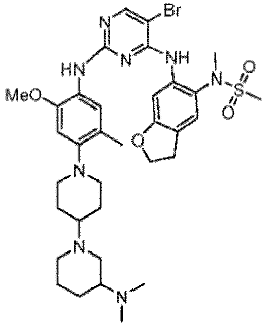

The title compound was prepared in the same manner as in step 2) of Example 250. [M+H]⁺ = 744.

### Example 259: Preparation of N-(6-((5-chloro-2-((2,5-dichloro-4-(4-(4-methylpiperazin-1-yl)piperidin-1-yl)phenyl)amino)pyrimidin-4-yl)amino)-2,3-dihydrobenzofuran-5-yl)-N-methylmethanesulfonamide

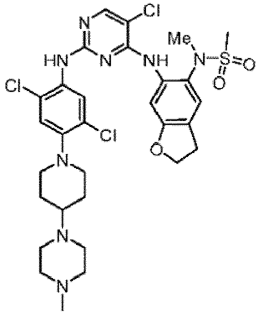

The title compound was prepared in the same manner as in step 4) of Example 248. [M+H]⁺ = 696.

### Example 260: Preparation of N-(2-((5-chloro-2-((2-methoxy-5-methyl-4-(4-(4-methylpiperazin-1-yl)piperidin-1-yl)phenyl)amino)pyrimidin-4-yl)amino)-4-methoxyphenyl)-N-methylmethanesulfonamide

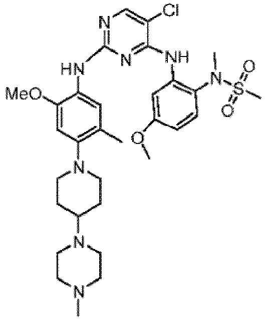

### Step 1) Preparation of N-(4-methoxy-2-nitrophenyl)-N-methylmethanesulfonamide

The compound (1 eq.) prepared in step 1) of Example 229 was diluted with acetone, and a 4 N NaOH solution (10 eq.) was added thereto. To the mixture was slowly added dimethyl sulfate (1.8 eq.) at 0 °C, followed by stirring at room temperature for 2 h. After completion of the reaction, the reaction mixture was distilled under reduced pressure and adjusted to pH 5-6 with a 1 N HCl solution at 0 °C. The resulting yellow solid was collected by filtration under reduced pressure and used in the next reaction without further purification.

### Step 2) Preparation of N-(2-amino-4-methoxyphenyl)-N-methylmethanesulfonamide

The compound prepared in step 1) was dissolved in a mixture of tetrahydrofuran, methanol, and water in an 18:1:1 volume ratio. To the solution were added an iron powder and ammonium chloride, followed by stirring at 85 °C for 3 h. After completion of the reaction, the reaction mixture was washed with dichloromethane on a filter filled with celite, filtered under reduced pressure, and concentrated to afford the title compound (yield: 95%).

### Step 3) Preparation of N-(2-((2,5-dichloropyrimidin-4-yl)(methyl)amino)-4-methoxyphenyl)methanesulfonamide

The compound (1 eq.) prepared in step 2) was diluted with isopropyl alcohol (0.3 M), and 2,4,5-trichloropyrimidine (2 eq.) and sodium bicarbonate (4 eq.) were added thereto. The mixture was stirred under a nitrogen atmosphere at 65 °C for 40 h. After completion of the reaction, the reaction mixture was cooled to room temperature, washed with dichloromethane on a filter filled with celite, filtered under reduced pressure, diluted with a mixture of isopropyl alcohol and chloroform in a 1:4 volume ratio, and washed with distilled water. Thereafter, the organic layer was dried over anhydrous sodium sulfate, filtered under reduced pressure, and distilled under reduced pressure. The resulting residue was purified by column chromatography (40% tetrahydrofuran/hexane) to afford the title compound (yield: 60%).

### Step 4) Preparation of N-(2-((5-chloro-2-((2-methoxy-5-methyl-4-(4-(4-methylpiperazin-1-yl)piperidin-1-yl)phenyl)amino)pyrimidin-4-yl)amino)-4-methoxyphenyl)-N-methylmethanesulfonamide

The compound (1 eq.) prepared in step 3) and the corresponding aniline (0.6 eq.) were dissolved in a mixture of acetic acid (0.3 M) and a 1.25 M HCl/methanol solution in a 1:1 volume ratio, sealed, and stirred at 120 °C for 3 h. After completion of the reaction, the reaction mixture was cooled to room temperature and extracted with a mixture of isopropyl alcohol and chloroform in a 1:4 volume ratio and a saturated aqueous solution of sodium bicarbonate. Thereafter, the organic layer was dried over anhydrous sodium sulfate, filtered under reduced pressure, and distilled under reduced pressure. The resulting residue was purified by column chromatography (10% methanol/dichloromethane) to afford the title compound (yield: 35%). [M+H]⁺ = 659.

### Example 261: Preparation of N-(6-((5-bromo-2-((4-(4-(4-cyclopropylpiperazin-1-yl)piperidin-1-yl)-2-methoxy-5-methylphenyl)amino)pyrimidin-4-yl)amino)-2,3-dihydrobenzofuran-5-yl)-N-methylmethanesulfonamide

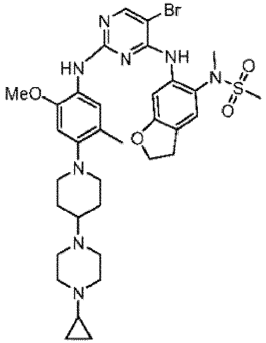

The title compound was prepared from the compound prepared in step 2) of Example 250 and the corresponding aniline in the same manner as in Example 1. [M+H]⁺ = 742.

### Example 262: Preparation of N-(6-((5-bromo-2-((4-(4-(3-(dimethylamino)pyrrolidin-1 -yl)piperidin-1 -yl)-2-methoxy-5-methylphenyl)amino)pyrimidin-4-yl)amino)-2,3-dihydrobenzofuran-5-yl)-N-methylmethanesulfonamide

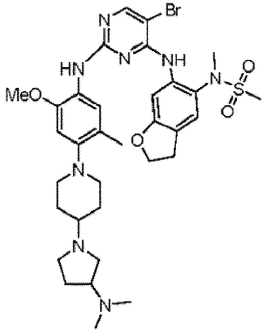

The title compound was prepared from the compound prepared in step 2) of Example 250 and the corresponding aniline in the same manner as in Example 1. [M+H]⁺ = 730.

### Example 263: Preparation of N-(6-((5-chloro-2-((2-chloro-5-methyl-4-(4-(4-methylpiperazin-1-yl)piperidin-1-yl)phenyl)amino)pyrimidin-4-yl)amino)-2,3-dihydrobenzofuran-5-yl)-N-methylmethanesulfonamide

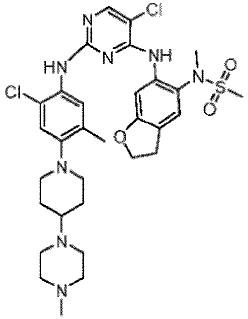

The title compound was prepared in the same manner as in step 4) of Example 248. [M+H]⁺ = 675.

### Example 264: Preparation of N-(6-((5-chloro-2-((5-chloro-2-methoxy-4-(4-(4-methyl-1,4-diazepan-1-yl)piperidin-1-yl)phenyl)amino)pyrimidin-4-yl)amino)-2,3 - dihydrobenzofuran-5-yl)-N-methylmethanesulfonamide

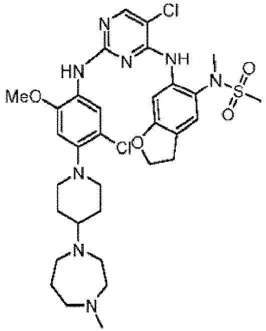

The title compound was prepared in the same manner as in step 4) of Example 248. [M+H]⁺ = 705.

### Example 265: Preparation of N-(6-((5-chloro-2-((2-methoxy-5-methyl-4-(4-(4-methyl-1,4-diazepan-1-yl)piperidin-1-yl)phenyl)amino)pyrimidin-4-yl)amino)-2,3 - dihydrobenzofuran-5-yl)-N-methylmethanesulfonamide

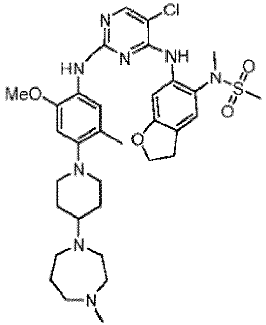

The title compound was prepared in the same manner as in step 4) of Example 248. [M+H]⁺ = 685.

### Example 266: Preparation of N-(6-((5-chloro-2-((4-(4-(4-cyclopropylpiperazin-1-yl)piperidin-1-yl)-2-methoxy-5-methylphenyl)amino)pyrimidin-4-yl)amino)-2,3-dihydrobenzofuran-5-yl)-N-methylmethanesulfonamide

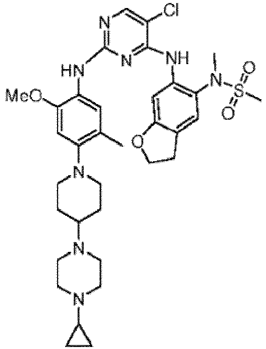

The title compound was prepared in the same manner as in step 4) of Example 248. [M+H]⁺ = 697.

### Example 267: Preparation of N-(6-((5-chloro-2-((4-(3-(dimethylamino)-[1,4'-bipiperidin]-1'-yl)-2-methoxy-5-methylphenyl)amino)pyrimidin-4-yl)amino)-2,3-dihydrobenzofuran-5-yl)-N-methylmethanesulfonamide

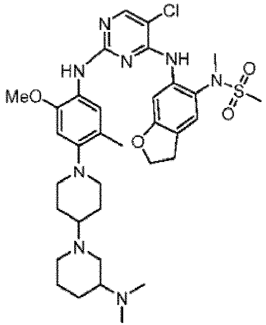

The title compound was prepared in the same manner as in step 4) of Example 248. [M+H]⁺ = 699.

### Example 268: Preparation of N-(6-((5-chloro-2-((2,5-difluoro-4-(4-(4-methylpiperazin-1-yl)piperidin-1-yl)phenyl)amino)pyrimidin-4-yl)amino)-2,3-dihydrobenzofuran-5-yl)methanesulfonamide

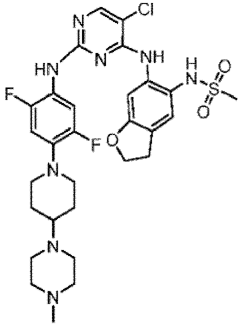

The title compound was prepared in the same manner as in step 5) of Example 165. [M+H]⁺ = 649.

### Example 269: Preparation of N-(5-((5-bromo-2-((2,5-difluoro-4-(4-(4-methylpiperazin-1-yl)piperidin-1-yl)phenyl)amino)pyrimidin-4-yl)amino)-2,3-dihydrobenzofuran-4-yl)methanesulfonamide

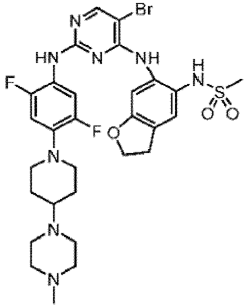

The title compound was prepared in the same manner as in step 2) of Example 169. [M+H]⁺ = 649.

### Example 270: Preparation of N-(6-((2-((5-Bromo-2-methoxy-4-(4-(4-methylpiperazin-1-yl)piperidin-1-yl)phenyl)amino)-5-chloropyrimidin-4-yl)amino)-2,3-dihydrobenzofuran-5-yl)methanesulfonamide

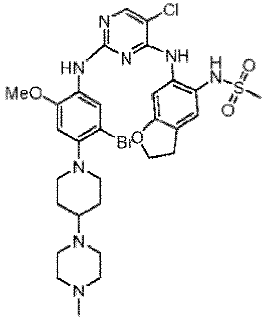

The title compound was prepared in the same manner as in step 5) of Example 165. ¹H NMR (300 MHz, DMSO) δ 8.43 (s, 1H), 8.14 (d, J = 1.8 Hz, 2H), 7.82 (s, 1H), 7.43 (s, 1H), 7.19 (s, 1H), 6.80 (s, 1H), 4.56 (t, J = 8.7 Hz, 2H), 3.81 (s, 3H), 3.18 (t, J = 8.7 Hz, 3H), 2.95 (s, 3H), 2.65 (t, J = 10.8 Hz, 3H), 2.59-2.50 (m, 4H), 2.43-2.25 (m, 5H), 2.18 (s, 3H), 1.93-1.79 (m, 2H), 1.68-1.50 (m, 2H). [M+H]⁺ = 722.

### Example 271: Preparation of N-(6-((2-((5-bromo-2-methoxy-4-(4-(4-methylpiperazin-1-yl)piperidin-1-yl)phenyl)amino)-5-bromopyrimidin-4-yl)amino)-2,3-dihydrobenzofuran-5-yl)methanesulfonamide

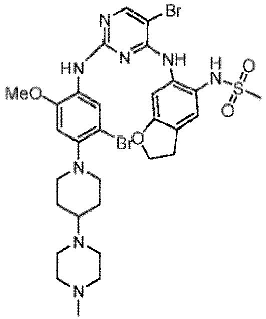

The title compound was prepared in the same manner as in step 2) of Example 169. [M+H]⁺ = 766.

### Example 272: Preparation of 5-chloro-N²-(2-methoxy-5-methyl-4-(4-(4-methylpiperazin-1-yl)piperidin-1-yl)phenyl)-N⁴-(1-methylindolin-6-yl)pyrimidine-2,4-diamine

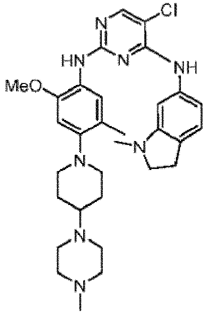

### Step 1) Preparation of 1-methyl-6-nitroindoline

6-Nitroindoline (1 eq.) was diluted with acetone (0.3 M), and potassium carbonate (4 eq.) was added thereto. To the mixture was added methyl iodide (2 eq.), followed by stirring at 50 °C for 12 h. After completion of the reaction, the reaction mixture was distilled under reduced pressure and purified by column chromatography to afford the title compound (yield: 68%).

### Step 2) Preparation of 1-methylindolin-6-amine

The compound prepared in step 2) was dissolved in a mixture of tetrahydrofuran, methanol, and water in an 18:1:1 volume ratio. To the solution were added an iron powder and ammonium chloride, followed by stirring at 85 °C for 3 h. After completion of the reaction, the reaction mixture was washed with dichloromethane on a filter filled with celite, filtered under reduced pressure, and concentrated to afford the title compound (yield: 70%).

### Step 3) Preparation of N-(2,5-dichloropyrimidin-4-yl)-1-methylindolin-6-amine

The compound (1 eq.) prepared in step 3) was diluted with isopropyl alcohol (0.3 M), and 2,4,5-trichloropyrimidine (2 eq.) and sodium bicarbonate (4 eq.) were added thereto. The mixture was stirred under a nitrogen atmosphere at 65 °C for 40 h. After completion of the reaction, the reaction mixture was cooled to room temperature, washed with dichloromethane on a filter filled with celite, filtered under reduced pressure, diluted with a mixture of isopropyl alcohol and chloroform in a 1:4 volume ratio, and washed with distilled water. Thereafter, the organic layer was dried over anhydrous sodium sulfate, filtered under reduced pressure, and distilled under reduced pressure. The resulting residue was purified by column chromatography (40% tetrahydrofuran/hexane) to afford the title compound (yield: 61%).

### Step 4) Preparation of 5-chloro-N²-(2-methoxy-5-methyl-4-(4-(4-methylpiperazin-1-yl)piperidin-1-yl)phenyl)-N⁴-(1-methylindolin-6-yl)pyrimidine-2,4-diamine

The compound (1 eq.) prepared in step 4) and the corresponding aniline (0.6 eq.) were dissolved in a mixture of acetic acid (0.3 M) and a 1.25 M HCl/methanol solution in a 1:1 volume ratio, sealed, and stirred at 120 °C for 3 h. After completion of the reaction, the reaction mixture was cooled to room temperature and extracted with a mixture of isopropyl alcohol and chloroform in a 1:4 volume ratio and a saturated aqueous solution of sodium bicarbonate. Thereafter, the organic layer was dried over anhydrous sodium sulfate, filtered under reduced pressure, and distilled under reduced pressure. The resulting residue was purified by column chromatography (10% methanol/dichloromethane) to afford the title compound (yield: 35%). [M+H]⁺ = 577.

### Example 273: Preparation of 5-chloro-N²-(5-chloro-2-methoxy-4-(4-(4-methylpiperazin-1-yl)piperidin-1-yl)phenyl)-N⁴-(1-methylindolin-6-yl)pyrimidine-2,4-diamine

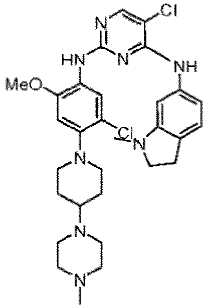

The title compound was prepared in the same manner as in step 4) of Example 272. [M+H]⁺ = 597.

### Example 274: Preparation of 5-chloro-N²-(2-chloro-5-methyl-4-(4-(4-methylpiperazin-1-yl)piperidin-1-yl)phenyl)-N⁴-(1-methylindolin-6-yl)pyrimidine-2,4-diamine

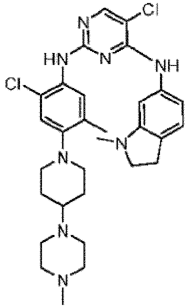

The title compound was prepared in the same manner as in step 4) of Example 272. [M+H]⁺ = 581.

### Example 275: Preparation of 5-chloro-N²-(2-methoxy-5-methyl-4-(4-(4-methyl-1,4-diazepan-1-yl)piperidin-1-yl)phenyl)-N⁴-(1-methylindolin-6-yl)pyrimidine-2,4-diamine

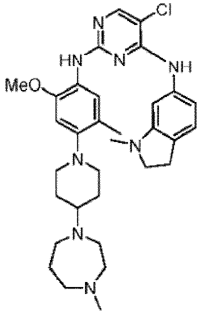

The title compound was prepared in the same manner as in step 4) of Example 272. [M+H]⁺ = 591.

### Example 276: Preparation of N-(6-((5-chloro-2-((2-methoxy-5-methyl-4-(4-(4-methylpiperazin-1-yl)piperidin-1-yl)phenyl)amino)pyrimidin-4-yl)amino)-1-methylindolin-5-yl)methanesulfonamide

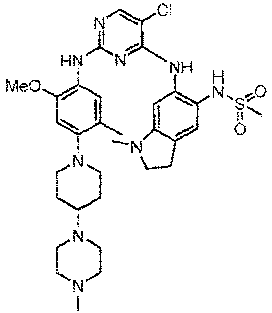

### Step 1) Preparation of 5-bromo-6-nitroindoline

5-Bromoindoline (4.5 g, 22.7 mmol) was dissolved in sulfuric acid (98%, 20 mL), and potassium nitrate (2.35 g, 23 mmol) was slowly added thereto at 0 °C. The mixture was stirred at 0-10 °C for 4 h. After completion of the reaction, the reaction mixture was slowly added to ice-water and adjusted to pH 8 with a saturated solution of sodium carbonate. The resulting solid was collected by filtration under reduced pressure and used in the next reaction without further purification.

### Step 2) Preparation of 5-bromo-1-methyl-6-nitroindoline

The compound (2.2 g, 9 mmol) prepared in step 1) was diluted with acetone (30 mL), and potassium carbonate (5 g, 36 mmol) was added thereto. To the mixture was added methyl iodide (0.9 mL, 14.4 mmol), followed by stirring at 50 °C for 12 h. After completion of the reaction, the reaction mixture was distilled under reduced pressure and purified by column chromatography to afford the title compound (1.57 g, yield: 68%).

### Step 3) Preparation of N-(1-methyl-6-nitroindolin-5-yl)methanesulfonamide

The compound (200 mg, 0.78 mmol) prepared in step 2), methanesulfonamide (220 mg, 3 eq.), cesium carbonate (1 g, 4 eq.), xantphos (90 mg, 0.2 eq.), and Pd(OAc)₂ (20 mg, 0.1 eq.) were diluted with dioxane, degassed for 10 min, and stirred at 100 °C for 24 h. After completion of the reaction, the reaction mixture was filtered through a filter filled with celite under reduced pressure and washed with ethyl acetate, distilled water, and saturated brine. The resulting residue was purified by column chromatography to afford the title compound (88 mg, yield: 42%).

### Step 4) Preparation of N-(6-amino-1-methylindolin-5-yl)methanesulfonamide

The compound prepared in step 3) was dissolved in a mixture of tetrahydrofuran, methanol, and water in an 18:1:1 volume ratio. To the solution were added an iron powder and ammonium chloride, followed by stirring at 85 °C for 3 h. After completion of the reaction, the reaction mixture was washed with dichloromethane on a filter filled with celite, filtered under reduced pressure, and concentrated to afford the title compound (yield: 61%).

### Step 5) Preparation of N-(6-((2,5-dichloro-4-yl)amino)-1-methylindolin-5-yl)methanesulfonamide

The compound (1 eq.) prepared in step 4) was diluted with isopropyl alcohol (0.3 M), and 2,4,5-trichloropyrimidine (2 eq.) and sodium bicarbonate (4 eq.) were added thereto. The mixture was stirred under a nitrogen atmosphere at 65 °C for 40 h. After completion of the reaction, the reaction mixture was cooled to room temperature, washed with dichloromethane on a filter filled with celite, filtered under reduced pressure, diluted with a mixture of isopropyl alcohol and chloroform in a 1:4 volume ratio, and washed with distilled water. Thereafter, the organic layer was dried over anhydrous sodium sulfate, filtered under reduced pressure, and distilled under reduced pressure. The resulting residue was purified by column chromatography (40% tetrahydrofuran/hexane) to afford the title compound (yield: 61%).

### Step 6) Preparation of N-(6-((5-chloro-2-((2-methoxy-5-methyl-4-(4-(4-methylpiperazin-1-yl)piperidin-1-yl)phenyl)amino)pyrimidin-4-yl)amino)-1-methylindolin-5-yl)methanesulfonamide

The compound (1 eq.) prepared in step 5) and the corresponding aniline (0.6 eq.) were dissolved in a mixture of acetic acid (0.3 M) and a 1.25 M HCl/methanol solution in a 1:1 volume ratio, sealed, and stirred at 120 °C for 3 h. After completion of the reaction, the reaction mixture was cooled to room temperature and extracted with a mixture of isopropyl alcohol and chloroform in a 1:4 volume ratio and a saturated aqueous solution of sodium bicarbonate. Thereafter, the organic layer was dried over anhydrous sodium sulfate, filtered under reduced pressure, and distilled under reduced pressure. The resulting residue was purified by column chromatography (10% methanol/dichloromethane) to afford the title compound (yield: 35%). [M+H]⁺ = 670.

### Example 277: Preparation of 5-chloro-N²-(2-chloro-5-methyl-4-(4-(4-methylpiperazin-1-yl)piperidin-1-yl)phenyl)-N⁴-(indolin-6-yl)pyrimidine-2,4-diamine

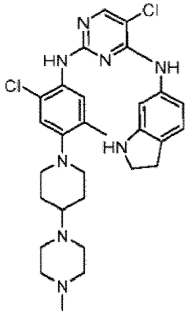

### Step 1) Preparation of tert-butyl 6-nitroindole-1-carboxylate

6-Nitroindoline (1 eq.) was diluted with dichloromethane (0.3 M), and di-tert-butyl dicarbonate (2 eq.) and DMAP (0.1 eq.) were added thereto. The mixture was stirred at room temperature for 12 h. After completion of the reaction, the reaction mixture was diluted with dichloromethane, washed with saturated brine, filtered under reduced pressure, and distilled under reduced pressure. The resulting residue was used in the next reaction without further purification.

### Step 2) Preparation of tert-butyl 6-aminoindoline-1-carboxylate

The compound prepared in step 1) was dissolved in a mixture of tetrahydrofuran, methanol, and water in an 18:1:1 volume ratio. To the solution were added an iron powder and ammonium chloride, followed by stirring at 85 °C for 3 h. After completion of the reaction, the reaction mixture was washed with dichloromethane on a filter filled with celite, filtered under reduced pressure, and concentrated to afford the title compound (yield: 75%).

### Step 3) Preparation of tert-butyl 6-((2,5-dichloropyrimidin-4-yl)amino)indoline-1-carboxylate

The compound (1 eq.) prepared in step 2) was diluted with isopropyl alcohol (0.3 M), and 2,4,5-trichloropyrimidine (2 eq.) and sodium bicarbonate (4 eq.) were added thereto. The mixture was stirred under a nitrogen atmosphere at 65 °C for 40 h. After completion of the reaction, the reaction mixture was cooled to room temperature, washed with dichloromethane on a filter filled with celite, filtered under reduced pressure, diluted with a mixture of isopropyl alcohol and chloroform in a 1:4 volume ratio, and washed with distilled water. Thereafter, the organic layer was dried over anhydrous sodium sulfate, filtered under reduced pressure, and distilled under reduced pressure. The resulting residue was purified by column chromatography (40% tetrahydrofuran/hexane) to afford the title compound (yield: 65%).

### Step 4) Preparation of 5-chloro-N²-(2-chloro-5-methyl-4-(4-(4-methylpiperazin-1-yl)piperidin-1-yl)phenyl)-N⁴-(indolin-6-yl)pyrimidine-2,4-diamine

The compound (1 eq.) prepared in step 5) and the corresponding aniline (0.6 eq.) were dissolved in a mixture of acetic acid (0.3 M) and a 1.25 M HCl/methanol solution in a 1:1 volume ratio, sealed, and stirred at 120 °C for 3 h. After completion of the reaction, the reaction mixture was cooled to room temperature and extracted with a mixture of isopropyl alcohol and chloroform in a 1:4 volume ratio and a saturated aqueous solution of sodium bicarbonate. Thereafter, the organic layer was dried over anhydrous sodium sulfate, filtered under reduced pressure, and distilled under reduced pressure. The resulting residue was purified by column chromatography (10% methanol/dichloromethane) to afford the title compound (yield: 35%). [M+H]⁺ = 567.

### Example 278: Preparation of 5-chloro-N²-(5-chloro-2-methoxy-4-(4-(4-methylpiperazin-1-yl)piperidin-1-yl)phenyl)-N⁴-(indolin-6-yl)pyrimidine-2,4-diamine

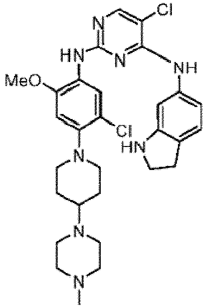

The title compound was prepared in the same manner as in step 4) of Example 277. [M+H]⁺ = 583.

### Example 279: Preparation of 5-chloro-N⁴-(indolin-6-yl)-N²-(2-methoxy-4-(4-(4-methylpiperazin-1-yl)piperidin-1-yl)phenyl)pyrimidine-2,4-diamine

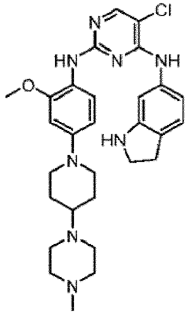

The title compound was prepared in the same manner as in step 4) of Example 277. [M+H]⁺ = 549.

### Example 280: Preparation of N-(6-((2-((5-bromo-4-(3-(dimethylamino)-[1,4'-bipiperidin]-1'-yl)-2-methoxyphenyl)amino)-5-chloropyrimidin-4-yl)amino)-2,3-dihydrobenzofuran-5-yl)methanesulfonamide

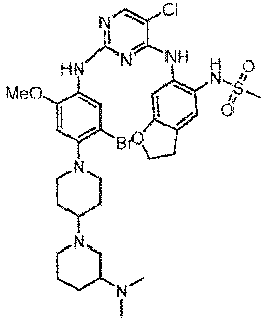

The title compound was prepared in the same manner as in step 5) of Example 165. [M+H]⁺ = 750.

### Example 281: Preparation of N-(6-((5-bromo-2-((5-bromo-4-(3-(dimethylamino)-[1,4'-bipiperidin]-1'-yl)-2-methoxyphenyl)amino)pyrimidin-4-yl)amino)-2,3-dihydrobenzofuran-5-yl)methanesulfonamide

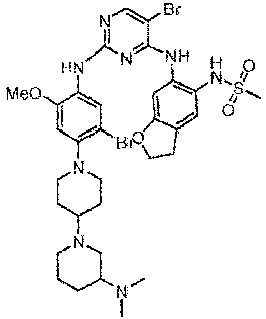

The title compound was prepared in the same manner as in step 2) of Example 169. [M+H]⁺ = 794.

### Example 282: Preparation of N-(6-((2-((5-bromo-2-methoxy-4-(4-(4-methyl-1,4-diazepan-1-yl)piperidin-1-yl)phenyl)amino)-5-chloropyrimidin-4-yl)amino)-2,3-dihydrobenzofuran-5-yl)methanesulfonamide

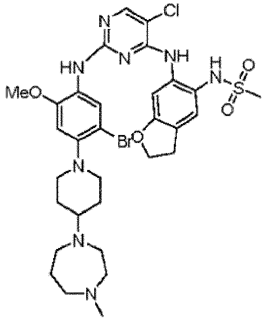

The title compound was prepared in the same manner as in step 5) of Example 165. [M+H]⁺ = 735.

### Example 283: Preparation of N-(6-((2-((5-bromo-4-(4-(3-(dimethylamino)pyrrolidin-1 -yl)piperidin-1 -yl)-2-methoxyphenyl)amino)-5-chloropyrimidin-4-yl)amino)-2,3-dihydrobenzofuran-5-yl)methanesulfonamide

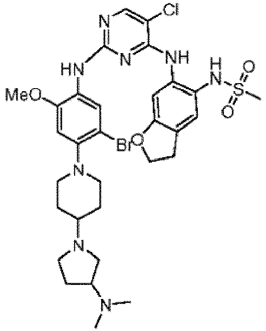

The title compound was prepared in the same manner as in step 5) of Example 165. [M+H]⁺ = 735.

The novel compound represented by Formula 1 according to the present invention can be formulated in various forms depending on its purpose. The following exemplifies some methods for preparing formulations containing the compound represented by Formula 1 according to the present invention as an active ingredient, but the present invention is not limited thereto.

### [Formulation Examples]

### Formulation Example 1: Tablets (direct compression)

5.0 mg of the active ingredient was sieved, mixed with 14.1 mg of lactose, 0.8 mg of Crospovidone USNF, and 0.1 mg of magnesium stearate, and compressed into tablets.

### Formulation Example 2: Tablets (wet granulation)

5.0 mg of the active ingredient was sieved and mixed with 16.0 mg of lactose and 4.0 mg of starch. To the mixture was added an appropriate amount of a solution of 0.3 mg of Polysorbate 80 in pure water. The resulting mixture was atomized, dried, sieved, mixed with 2.7 mg of colloidal silicon dioxide and 2.0 mg of magnesium stearate, and compressed into tablets.

### Formulation Example 3: Powder and Capsules

5.0 mg of the active ingredient was sieved and mixed with 14.8 mg of lactose, 10.0 mg of polyvinylpyrrolidone, and 0.2 mg of magnesium stearate. The mixture was filled in hard No. 5 gelatin capsules using a suitable device.

### Formulation Example 4: Injection

An injection containing 100 mg of the active ingredient, 180 mg of mannitol, 26 mg of Na₂HPO₄ . 12H₂O, and 2974 mg of distilled water was prepared.

### [Experimental Examples]

### Experimental Example 1. Measurement of inhibitory activity against EGFR kinase

In this experimental example, a determination was made as to whether the compounds prepared in Examples 1-283 have the ability to inhibit the growth of Ba/F3 cells expressing EGFR-wt, del19/T790M/C797S (EGFR-DTC), L858R/T790M/C797S (EGFR-LTC), del19/T790M (EGFR-DT), and L858R/T790M (EGFR-LT) variants.

The parental Ba/F3 cell line is a population of cells that are not introduced with EGFR variants. Specifically, 100 µl of a cell line expressing each variant was plated in a 96-well plate at a density of 100,000 cells/ml. 10-point, 4-fold serial dilutions of each compound were prepared, starting at 2 mM. After 4 h, 0.5 µl of the compound (0.5% DMSO) was added, followed by incubation at 37 °C and 5% CO₂ for 72 h. After incubation, the number of viable cells was measured using a Celltiter glo assay kit (Promega) and the GI50 of the compound was determined. The term "GI50" refers to the molar concentration (µM) of the compound required for 50% growth inhibition of cells. The results are summarized in Table 1.

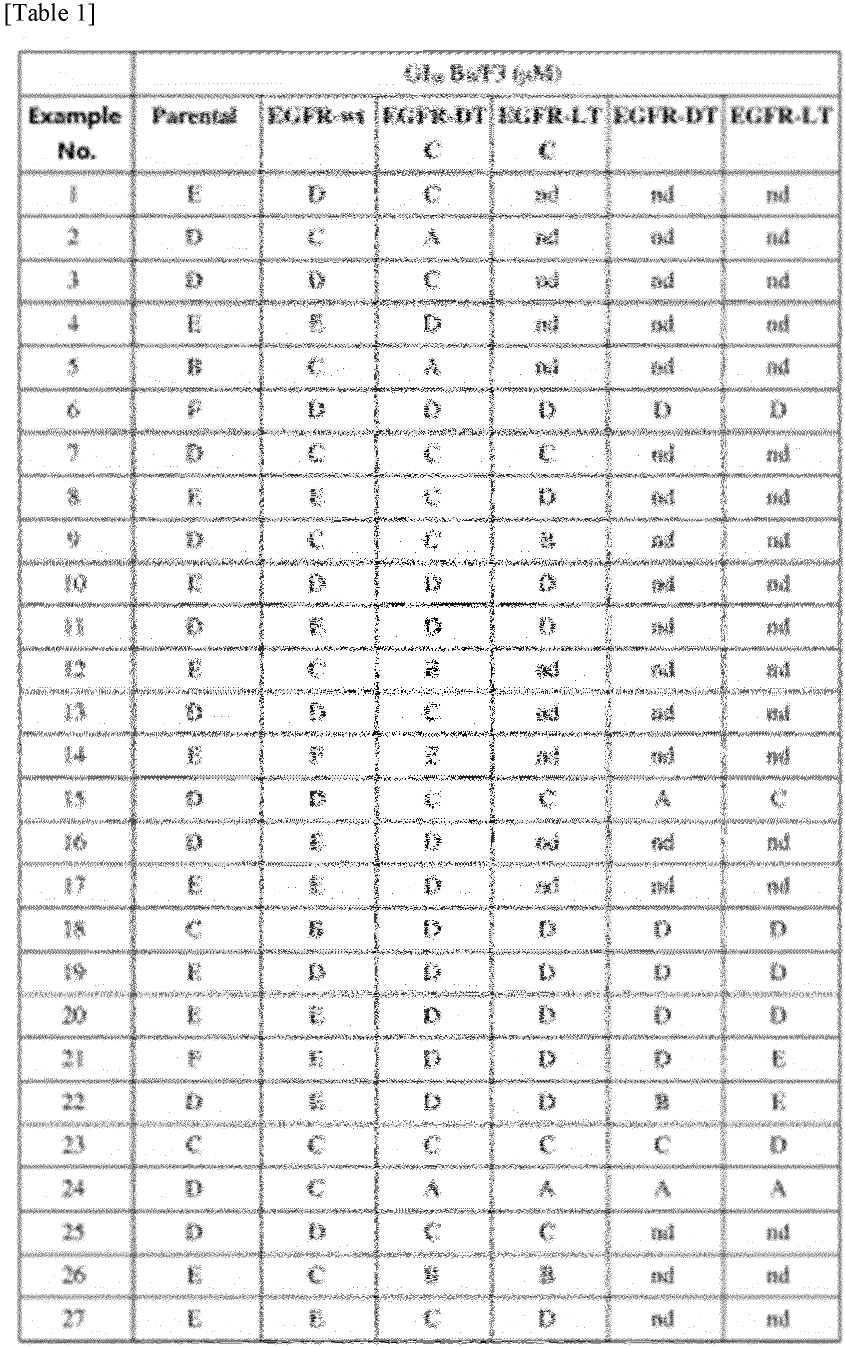

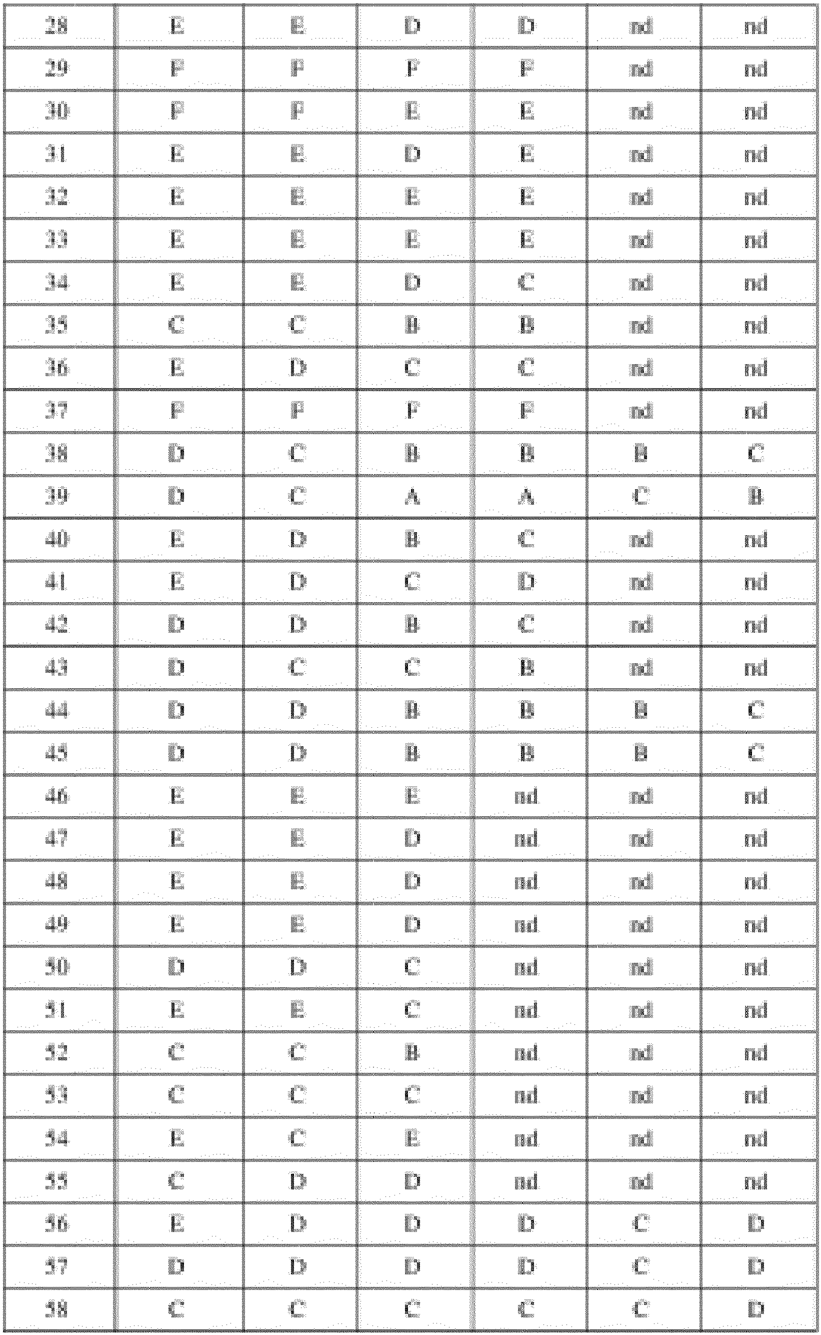

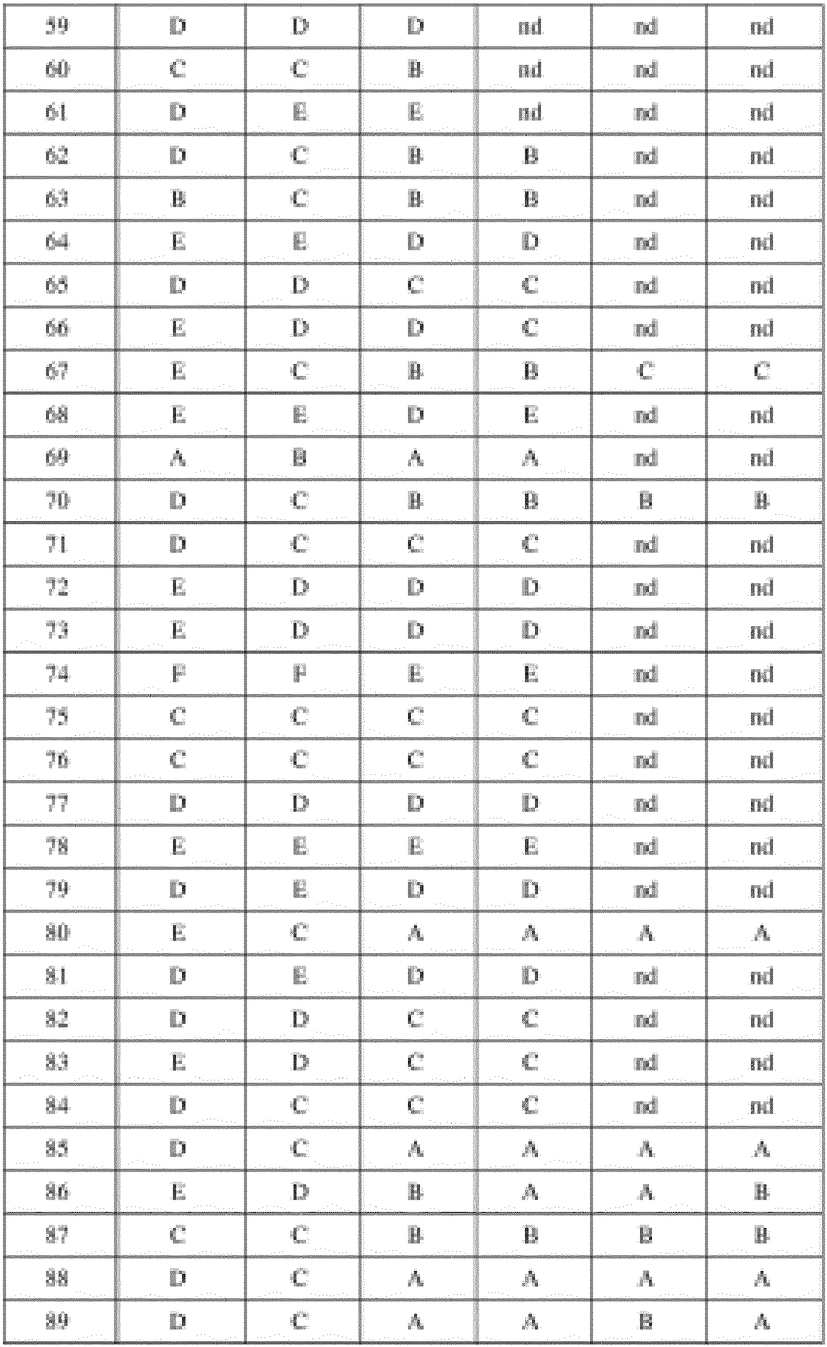

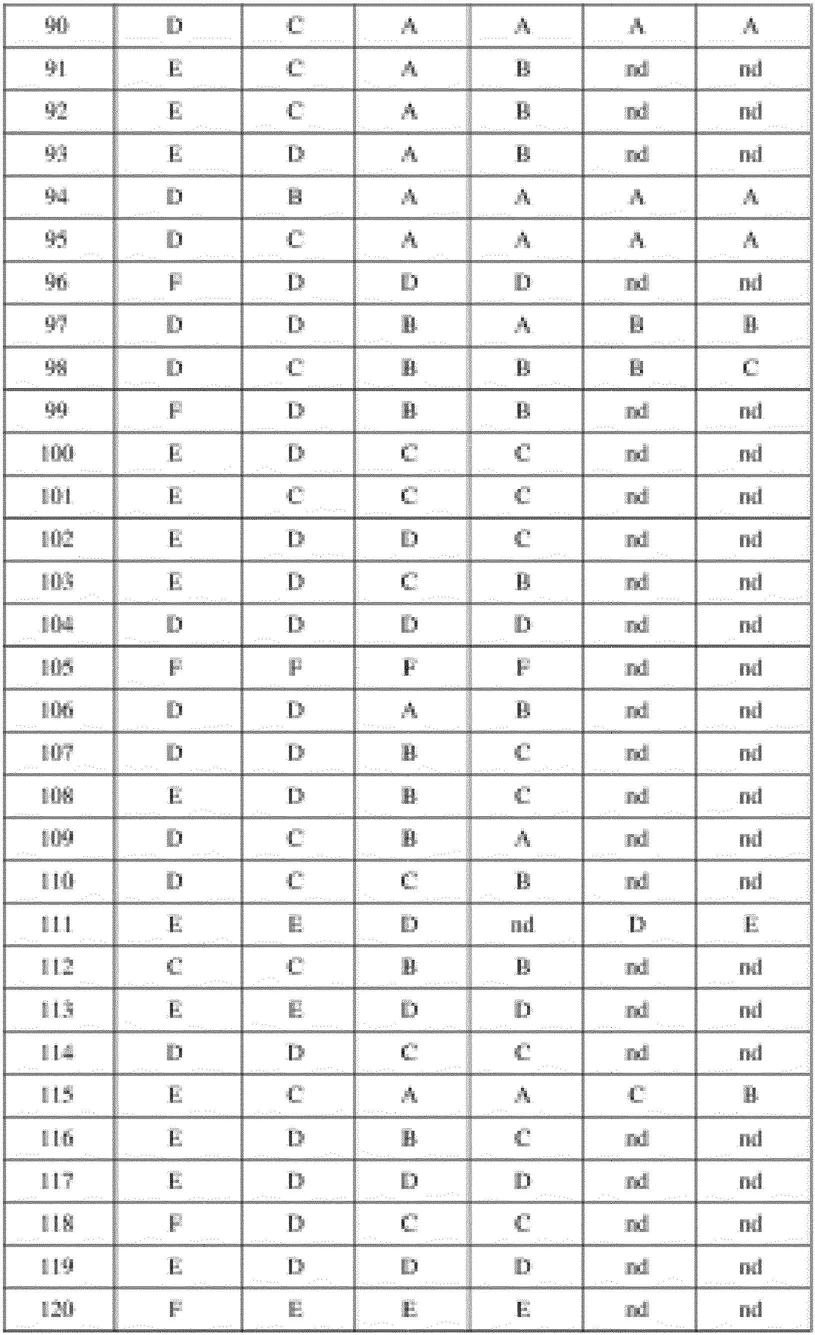

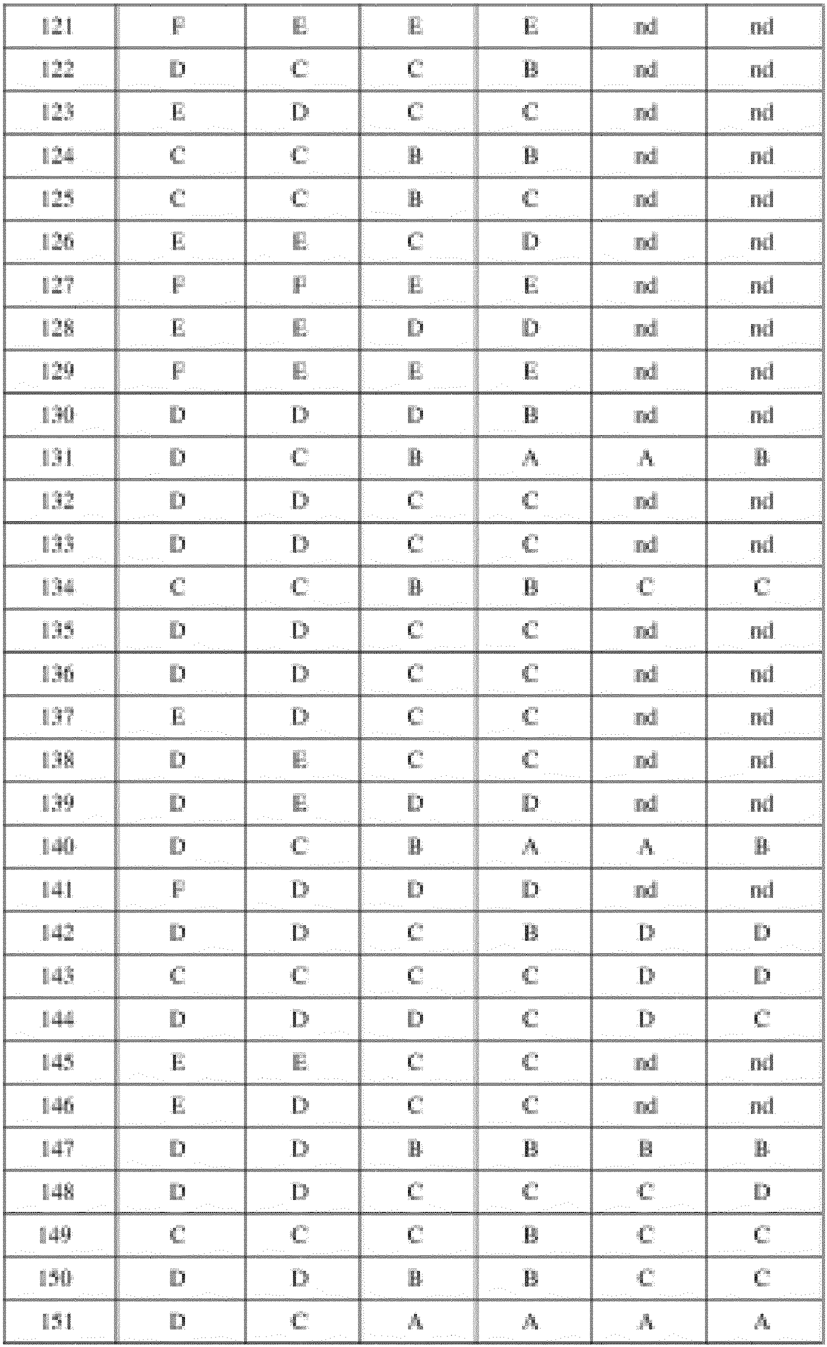

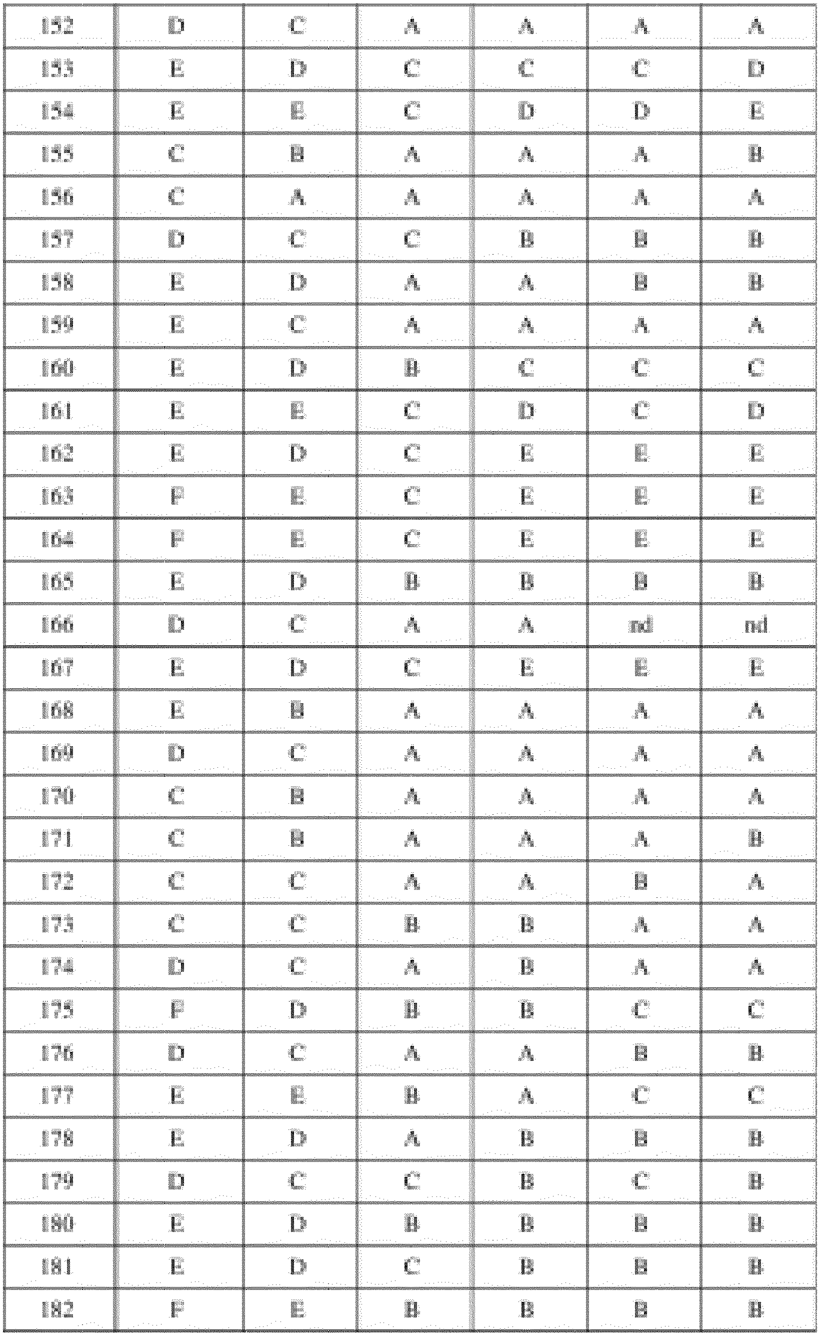

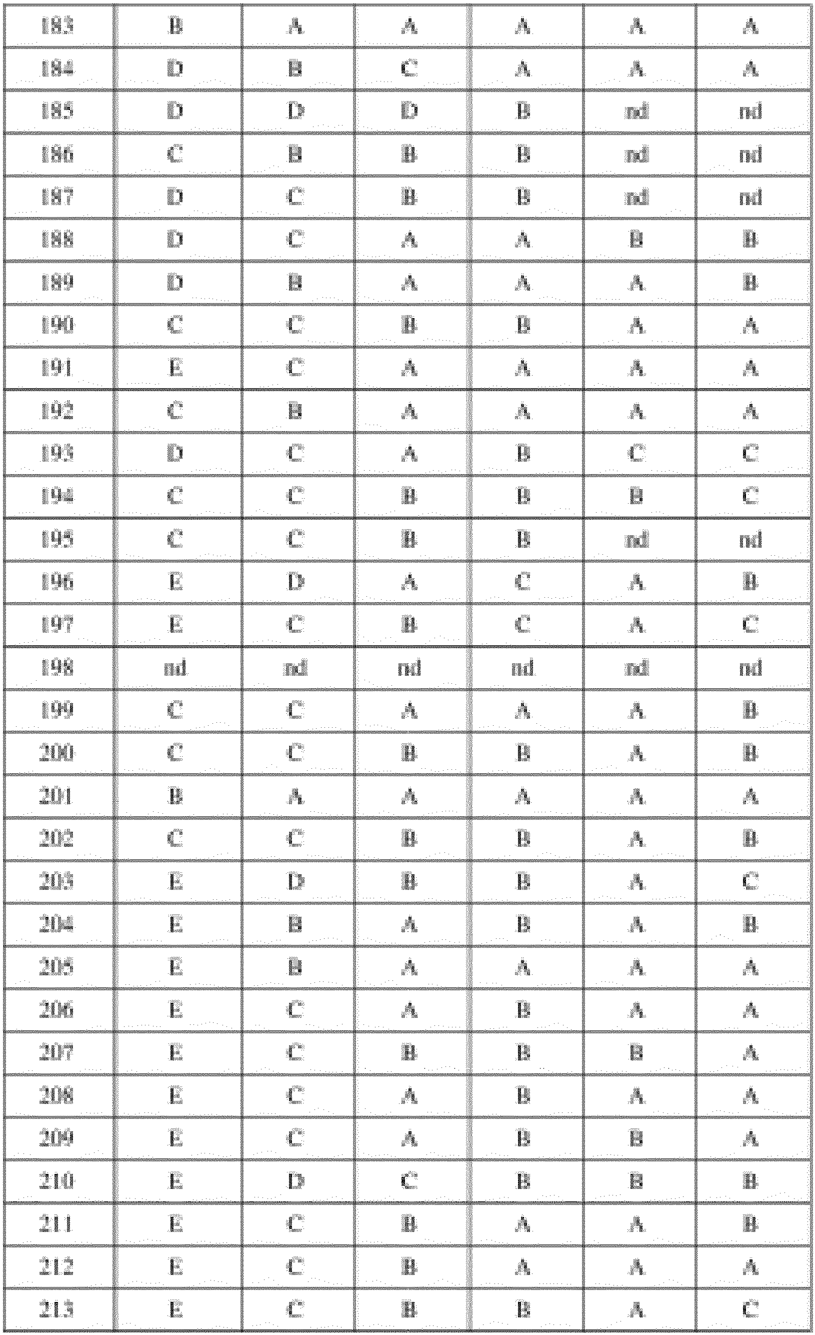

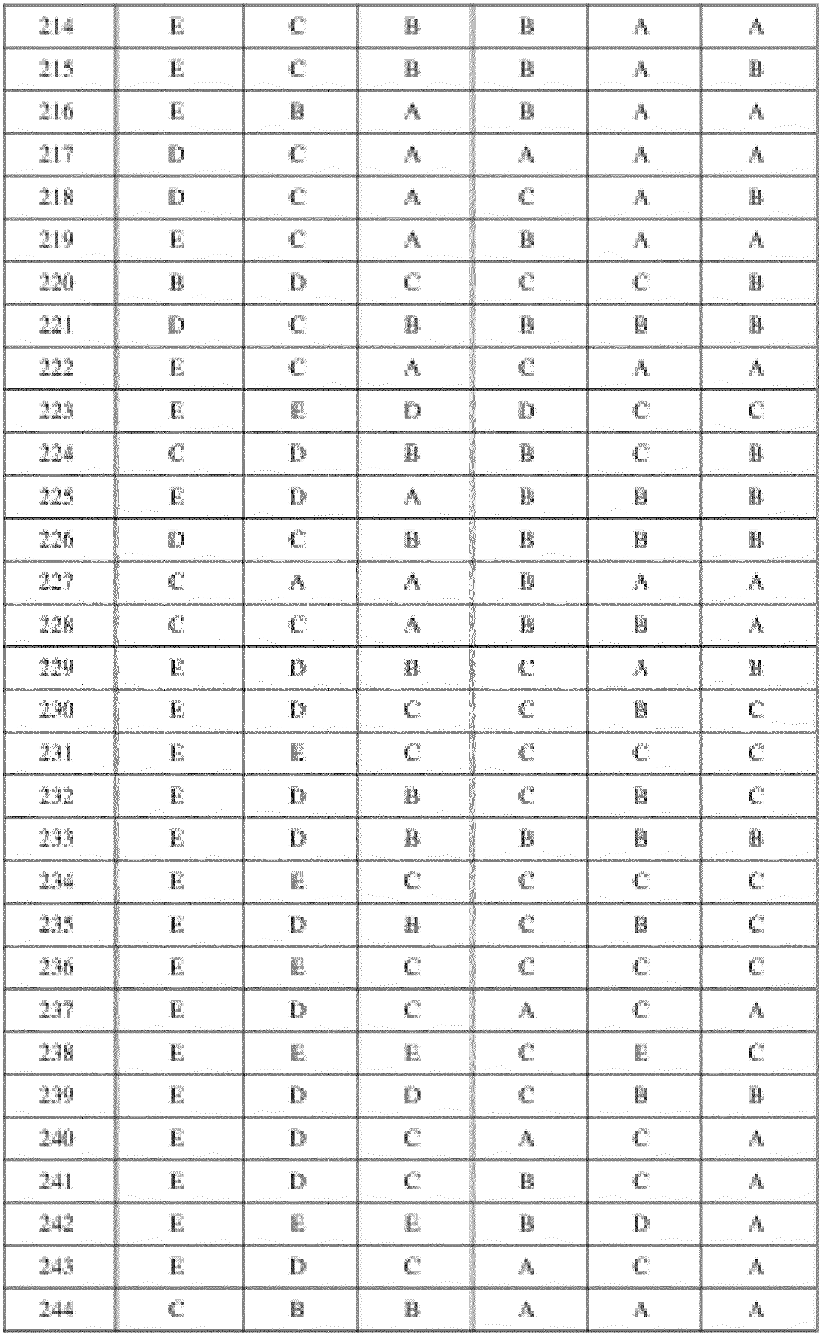

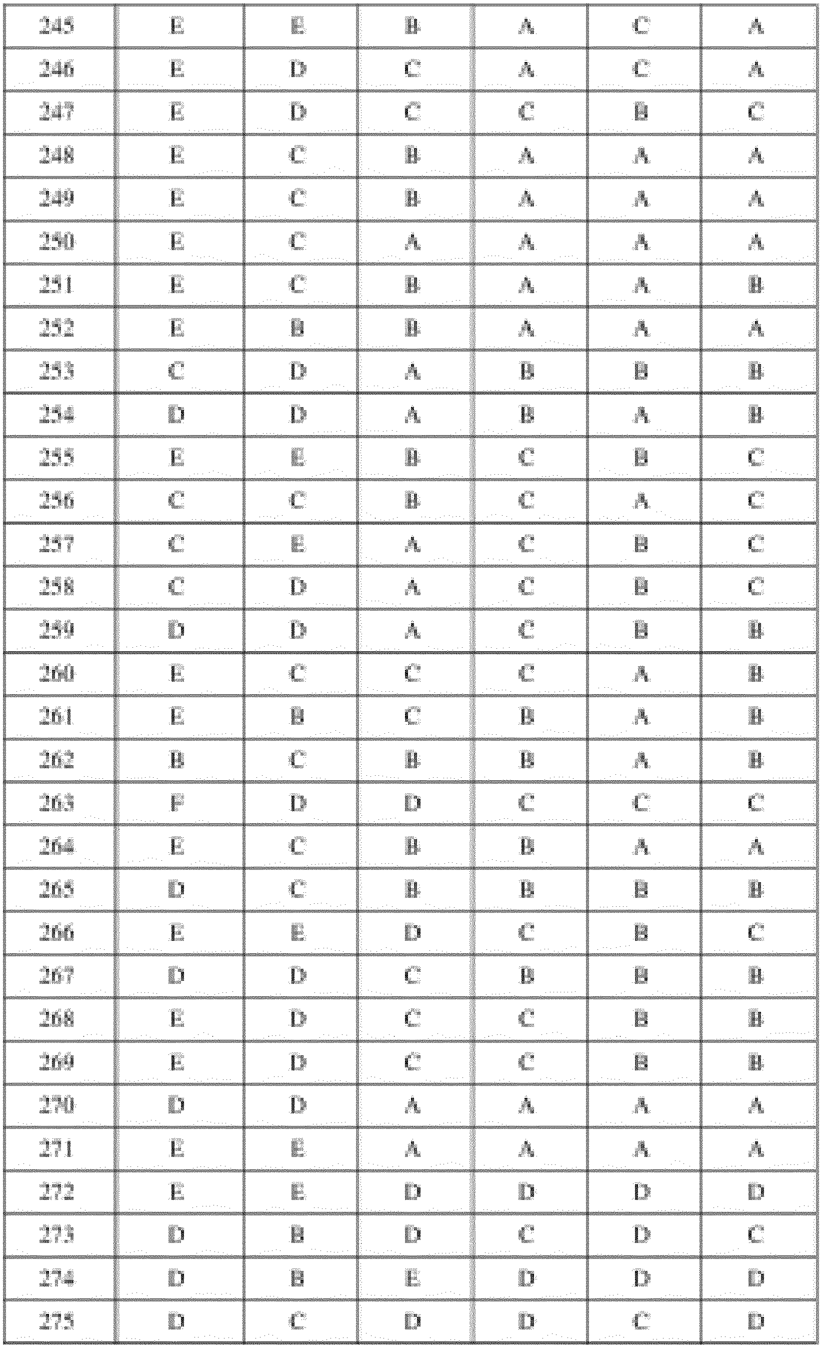

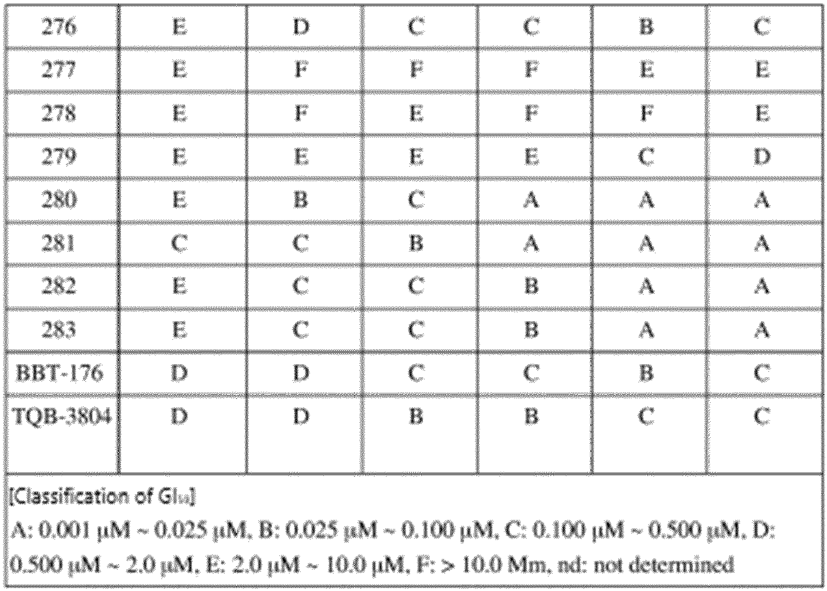

### Industrial Applicability

As is apparent from the above, due to its inhibitory activity against the EGFR protein kinase, the pyrimidine compound represented by Formula 1 or pharmaceutically acceptable salt thereof according to the present invention is useful as a prophylactic and therapeutic agent for diseases resulting from abnormal cell metabolism and glucose metabolism caused by the EGFR protein kinase, for example, metabolic diseases such as diabetes and obesity and tumor diseases selected from the group consisting of endometrial cancer, bladder cancer, stomach cancer, lung cancer, liver cancer, colorectal cancer, small intestine cancer, pancreatic cancer, brain cancer, bone cancer, melanoma, breast cancer, sclerosing adenosis, head and neck cancer, esophageal cancer, thyroid cancer, parathyroid cancer, kidney cancer, sarcoma, prostate cancer, urethral cancer, blood cancers such as leukemia, multiple myeloma, and myelodysplastic syndrome, lymphomas such as Hodgkin's disease and non-Hodgkin's lymphoma, and fibroadenoma.

## Claims

1. A compound selected from a pyrimidine derivative represented by Formula 10, 11 or 12: 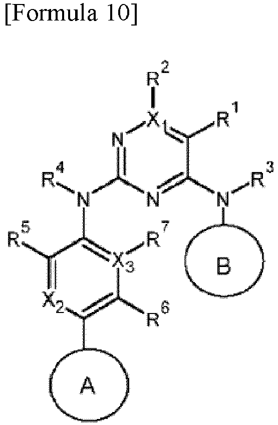
wherein R₁ is hydrogen, hydroxyl, halo, C₁-C₁₃ alkyl or C₁-C₆ alkoxy,
R₂ is hydrogen, halo, C₁-C₁₃ alkyl, C₃-C₁₀ cyclyl, amino (-NR₈R₉) or nitro (-N(O)₂),
R₃ and R₄ are each independently hydrogen, C₁-C₁₃ alkyl, C₃-C₁₀ cyclyl, sulfido (-SR₈), sulfonyl (-S(O)₂R₈) or phosphoryl (-P(O)R₈R₉),
R₅, R₆, and R₇ are each independently hydrogen, hydroxyl, halo, C₁-C₁₃ alkyl, C₁-C₆ alkoxy, C₃-C₁₀ cyclyl, C₃-C₁₀ heterocyclyl, -C(O)-(C₁-C₁₃ alkyl), amino (-NR₈R₉), nitro (-N(O)₂), amido (-(C=O)NR₈R₉), carboxyl (-C(O)OH), nitrile (-CN), ureido (-NR₈(C=O)NR₉-), sulfonamido (-NHS(O)₂R₈), sulfido (-SR₈), sulfonyl (-S(O)₂R₈) or phosphoryl (-P(O)R₈R₉),
X₁, X₂, and X₃ are each independently carbon or nitrogen,
A is C₁-C₁₃ alkyl, amino (-NR₈R₉), C₆-C₁₀ aryl, C₃-C₁₀ cyclyl, C₃-C₁₀ heteroaryl or C₃-C₁₀ heterocyclyl or together with the carbon atom to which R₆ is attached forms a 3- to 7-membered saturated ring optionally containing at least one of N, O, S, NH, C=N, C=O, -NHC(O)-, -NHC(O)NH-, -NHS(O)₂-, and SO₂ and optionally substituted with at least one of hydrogen, C₁-C₁₃ alkyl, C₆-C₁₀ aryl, C₃-C₁₀ heteroaryl, hydroxyl, halo, and cyano,
B is C₆-C₁₀ aryl, C₃-C₁₀ cyclyl, C₃-C₁₀ heteroaryl or C₃-C₁₀ heterocyclyl, provided that
when X₁ and X₃ are each independently nitrogen, R₂ or R₇ is nothing,
the C₁-C₆ alkoxy, C₁-C₁₃ alkyl or C₃-C₁₀ cyclyl comprises one or more substituents selected from the group consisting of hydrogen, hydroxyl, halo, C₁-C₁₃ alkyl, C₁-C₆ alkoxy, amino (-NR₈R₉), nitro (-N(O)₂), amido (-(C=O)NR₈R₉), carboxyl (-C(O)OH), nitrile (-CN), ureido (-NR₈(C=O)NR₉₋), sulfonamido (-NHS(O)z-), sulfido (-S-), sulfonyl (-S(O)₂-), phosphoryl (-P(O)R₈R₉),
C₆-C₁₀ aryl, C₃-C₁₀ heteroaryl, and C₃-C₁₀ heterocyclyl, the C₆-C₁₀ aryl, C₃-C₁₀ heteroaryl or C₃-C₁₀ heterocyclyl comprises one or more substituents selected from the group consisting of hydrogen, hydroxyl, halo, carbonyl (-(C=O)R₈R₉), C₁-C₃ alkyl unsubstituted or substituted with halo or C₃-C₁₀ heterocyclyl, C₁-C₃ alkoxy unsubstituted or substituted with halo or C₃-C₁₀ heterocyclyl, C₆-C₁₀ phenoxy, amino (-NR₈R₉), nitro (-N(O)z), amido (-(C=O)NR₈R₉), carboxyl (-C(O)OH), nitrile (-CN), ureido (-NR₈(C=O)NR₉₋), sulfonamido (-NHS(O)z-), sulfido (-S-), sulfonyl (-S(O)₂-), phosphoryl (-P(O)R₈R₉), C₆-C₁₀ aryl, C₃-C₁₀ heteroaryl, and C₃-C₁₀ heterocyclyl,
R₈ and R₉ are each independently hydrogen, C₁-C₆ alkyl, C₁-C₆ alkenyl, C₁-C₆ alkynyl, C₆-C₁₀ aryl, C₃-C₁₀ heteroaryl or C₃-C₁₀ heterocyclyl, with the proviso that R₈ together with the nitrogen or carbon atom to which R₉ is attached optionally forms a 3-to 7-membered saturated ring optionally containing at least one of N, O, S, NH, C=N, C=O, -NHC(O)-, -NHC(O)NH-, -NHS(O)z-, and SO₂ and optionally substituted with at least one of hydrogen, C₁-C₁₃ alkyl, C₆-C₁₀ aryl, C₃-C₁₀ heteroaryl, hydroxyl, halo, and cyano, and
the C₃-C₁₀ heteroaryl and C₃-C₁₀ heterocyclyl comprise at least one heteroatom selected from the group consisting of N, O, and S, 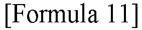 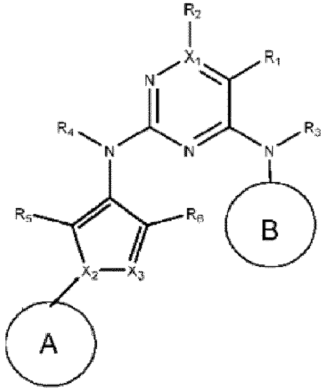
wherein R₁ to R₆, X₁ to X₃, A, and B are as defined in Formula 10, 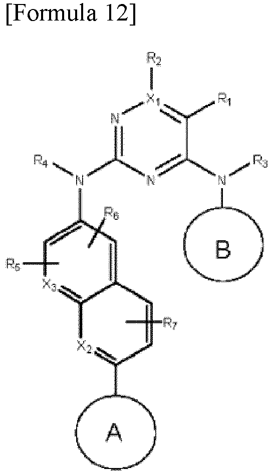
wherein R₁ to R₇, X₁ to X₃, A, and B are as defined in Formula 10; a pharmaceutically acceptable salt thereof; a hydrate thereof; and a stereoisomer thereof.

2. The compound according to claim 1, wherein R₁ is hydrogen, halo or C₁-C₃ alkyl, R₂ is hydrogen, C₁-C₃ alkyl or amino (-NR₈R₉), R₃ is hydrogen, C₁-C₃ alkyl, sulfido (-SR₈) or sulfonyl (-S(O)₂R₈), R₄ is hydrogen or C₁-C₃ alkyl, R₅ is hydrogen, hydroxyl, halo, C₁-C₃ alkyl or C₁-C₆ alkoxy, R₆ is hydrogen, halo or C₁-C₃ alkyl, and R₇ is hydrogen or C₁-C₃ alkyl.

3. The compound according to claim 1, wherein A is amino (-NR₈R₉), C₃-C₁₀ heteroaryl or C₃-C₁₀ heterocyclyl or together with the carbon atom to which R₆ is attached forms a 3- to 7-membered saturated ring optionally containing at least one of N, O, S, NH, C=N, C=O, -NHC(O)-, -NHC(O)NH-, -NHS(O)₂-, and SO₂ and optionally substituted with at least one of hydrogen, C₁-C₁₃ alkyl, C₆-C₁₀ aryl, C₃-C₁₀ heteroaryl, hydroxyl, halo, and cyano and B is C₆-C₁₀ aryl or C₃-C₁₀ heteroaryl.

4. The compound according to claim 1, wherein R₁ is halo, R₂ is hydrogen or amino (-NR₈R₉), R₃ is hydrogen, C₁-C₃ alkyl or sulfonyl (-S(O)₂R₈), R₄ is hydrogen, R₅ is hydrogen, halo or C₁-C₆ alkoxy, R₆ is hydrogen, halo or C₁-C₃ alkyl, R₇ is hydrogen, A is selected from the group consisting of piperazine, piperidine, morpholine, pyrrolidine, and imidazole, and B is selected from the group consisting of benzene, thiazole, thiophene, pyrazole, benzothiophene, pyridazine, pyrazine, imidazole, oxadiazole, triazole, furan, pyrimidine, oxazole, pyrrole, pyridine, oxadiazole, triazine, thiadiazole, isoxazole, and tetrazole.

5. The compound according to claim 1, wherein R₁ is Cl or Br, R₂ is hydrogen or -NH₂, R₃ is hydrogen, -CH₃ or -S(O)₂CH₃, R₄ is hydrogen, R₅ is hydrogen, Cl, Br, -OCH₃, -OCH₂CH₃ or -OCH₂CF₃, R₆ is hydrogen, Cl, Br, F, -CH₃ or -CF₃, R₇ is hydrogen, A is selected from the group consisting of piperazine, piperidine, morpholine, and pyrrolidine, and B is selected from the group consisting of benzene, benzothiazole, quinoline, and quinoxaline.

6. The compound according to claim 1, wherein A is selected from

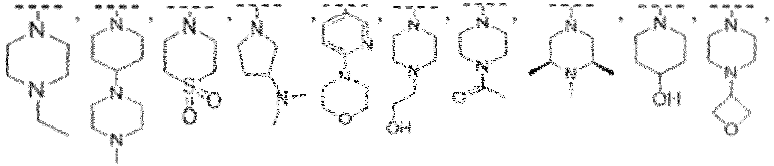

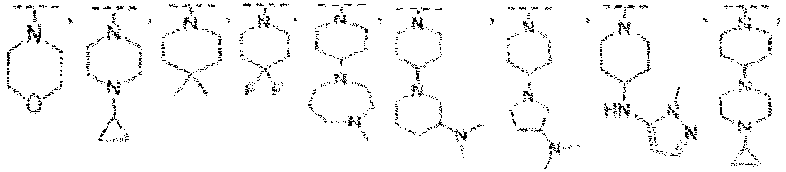

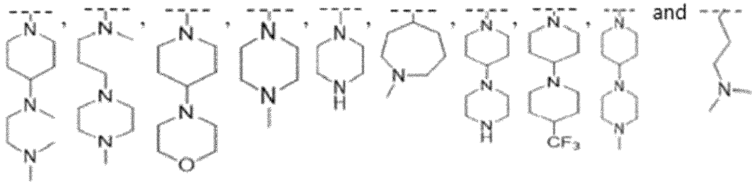

7. The compound according to claim 1, wherein B is selected from

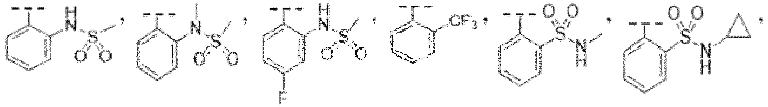

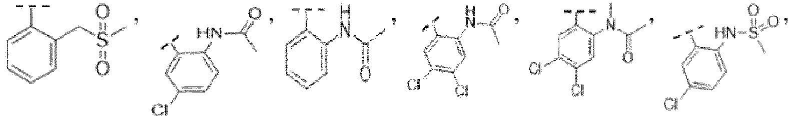

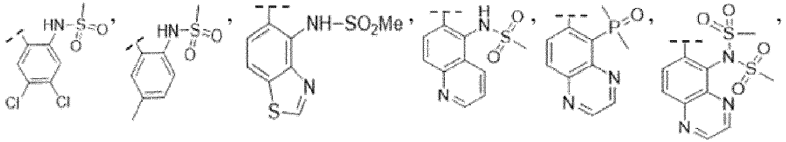

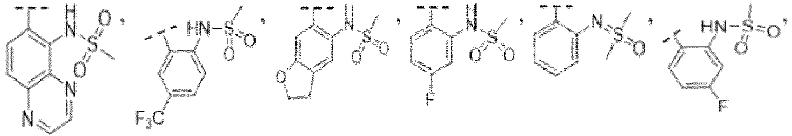

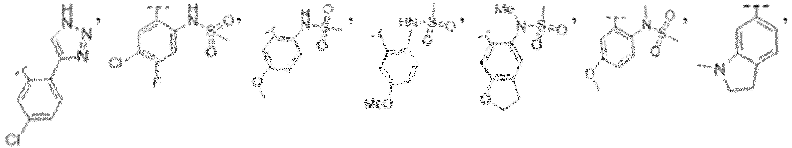

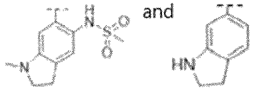

8. The compound according to claim 1, wherein R₁ is Cl or Br, R₂ is hydrogen or -NH₂, R₃ is hydrogen, -CH₃ or -S(O)₂CH₃, R₄ is hydrogen, R₅ is hydrogen, Cl, Br, -OCH₃, -OCH₂CH₃ or -OCH₂CF₃, R₆ is hydrogen, Cl, Br, F -CH₃ or -CF₃, R₇ is hydrogen, A is selected from the group consisting of 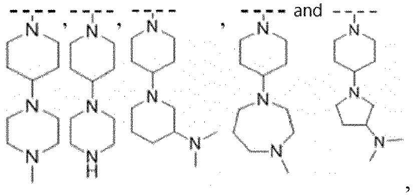 and B is selected from the group consisting of

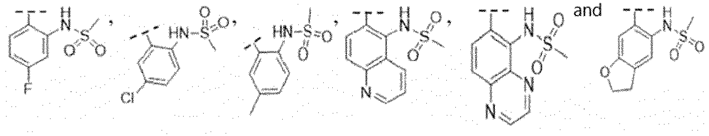

9. The compound according to claim 1, wherein the compound is selected from the group consisting of the following compounds Nos. 1 to 283:
Compound No. 1: *N*-(2-((5-chloro-2-((6-(4-ethylpiperazin-1-yl)-2-methoxypyridin-3-yl)amino)-4-yl)amino)phenyl)methanesulfonamide;
Compound No. 2: *N*-(2-((5-chloro-2-((2-methoxy-5-methyl-4-(4-(4-methylpiperazin-1-yl)piperidin-1-yl)phenyl)amino)pyrimidin-4-yl)amino)phenyl)methanesulfonamide;
Compound No. 3: *N*-(2-((5-chloro-2-((6-(4-(4-methylpiperazin-1-yl)piperidin-1-yl)pyridin-3-yl)amino)pyrimidin-4-yl)amino)phenyl)methanesulfonamide;
Compound No. 4: *N*-(2-((5-chloro-2-((6-(4-(4-methylpiperazin-1-yl)piperidin-1-yl)-2-(2,2,2-trifluoroethoxy)pyridin-3-yl)amino)pyrimidin-4-yl)amino)phenyl)methanesulfonamide;
Compound No. 5: *N*-(2-((5-chloro-2-((3-fluoro-4-(4-(4-methylpiperazin-1-yl)piperidin-1-yl)phenyl)amino)pyrimidin-4-yl)amino)phenyl)methanesulfonamide;
Compound No. 6: *N*-(2-((5-chloro-2-((6-(1,1-dioxythiomorpholino)-2-methoxypyridin-3-yl)amino)pyrimidin-4-yl)amino)phenyl)methanesulfonamide;
Compound No. 7: *N*-(2-((5-chloro-2-((5-chloro-2-methoxy-4-(4-(4-methylpiperazin-1-yl)piperidin-1-yl)phenyl)amino)pyrimidin-4-yl)amino)phenyl)methanesulfonamide;
Compound No. 8: *N*-(5-chloro-2-((6-(3-(dimethylamino)pyrrolidin-1-yl)-2-methoxypyridin-3-yl)amino)pyrimidin-4-yl)-N-(2-(methylsulfonamido)phenyl)methanesulfonamide;
Compound No. 9: *N*-(5-chloro-2-((2-methoxy-5-methyl-4-(4-(4-methylpiperazin-1-yl)piperidin-1-yl)phenyl)amino)pyrimidin-4-yl)-*N*-(2-(methylsulfonamido)phenyl)methanesulfonamide;
Compound No. 10: *N*-(5-chloro-2-((2-methoxy-6-(4-(4-methylpiperazin-1-yl)piperidin-1-yl)pyridin-3-yl)amino)pyrimidin-4-yl)-*N*-(2-(methylsulfonamido)phenyl)methanesulfonamide;
Compound No. 11: *N*-(2-((5-chloro-2-((2-methoxy-5-methyl-4-(4-(4-methylpiperazin-1-yl)piperidin-1-yl)phenyl)amino)pyrimidin-4-yl)(methyl)amino)phenyl)-*N*-methylsulfonamide;
Compound No. 12: *N*-(2-((5-chloro-2-((2-methoxy-5-methyl-4-(4-(4-methylpiperazin-1-yl)piperidin-1-yl)phenyl)amino)pyrimidin-4-yl)amido)-5-fluorophenyl)methanesulfonamide;
Compound No. 13: *N*-(2-((5-chloro-2-((6-morpholinopyridin-3-yl)amino)pyrimidin-4-yl)amido)-5-fluorophenyl)methanesulfonamide;
Compound No. 14: *N*-(2-((5-chloro-2-((6-(4-(4-methylpiperazin-1-yl)piperidin-1-yl)pyridin-3-yl)amido)pyrimidin-4-yl)amino)-5-fluorophenyl)methanesulfonamide;
Compound No. 15: *N*-(2-((5-chloro-2-((2-methoxy-5-methyl-6-(4-(4-methylpiperazin-1-yl)piperidin-1-yl)pyridin-3-yl)amino)pyrimidin-4-yl)amino)-5-fluorophenyl)methanesulfonamide;
Compound No. 16: *N*-(2-((5-chloro-2-((5-fluoro-6-(4-(4-methylpiperazin-1-yl)piperidin-1-yl)pyridin-3-yl)amino)pyrimidin-4-yl)amino)-5-fluorophenyl)methanesulfonamide;
Compound No. 17: *N*-(2-((5-chloro-2-((2-methoxy-6-(4-(4-methylpiperazin-1-yl)piperidin-1-yl)pyridin-3-yl)amino)pyrimidin-4-yl)amino)-5-fluorophenyl)methanesulfonamide;
Compound No. 18: *N*-(2-((5-chloro-2-((6-(4-(2-hydroxymethyl)piperazin-1-yl)-2-methoxypyridin-3-yl)amino)pyrimidin-4-yl)amino)-5-fluorophenyl)methanesulfonamide;
Compound No. 19: *N*-(2-((2-((6-(4-acetylpiperazin-1-yl)-2-methoxypyridin-3-yl)amino)-5-chloropyrimidin-4-yl)amino)-5-fluorophenyl)methanesulfonamide;
Compound No. 20: *N*-(2-((5-chloro-2-((2-methoxy-6-((3S,5R)-3,4,5-trimethylpiperazin-1-yl)pyridin-3-yl)amino)pyrimidin-4-yl)amino)-5-fluorophenyl)methanesulfonamide;
Compound No. 21: *N*-(2-((5-chloro-((6-(4-hydroxypiperidin-1-yl)-2-methoxypyridin-3-yl)amino)pyrimidin-4-yl)amino)-5-fluorophenyl)methanesulfonamide;
Compound No. 22: *N*-(2-((5-chloro-2-((2-methoxy-6-(4-(oxetan-3-yl)piperazin-1-yl)pyridin-3-yl)amino)pyrimidin-4-yl)amino)-5-fluorophenyl)methanesulfonamide;
Compound No. 23: *N*-(2-((5-chloro-2-((6-(3-(dimethylamino)pyrrolidin-1-yl)-2-methoxypyridin-3-yl)amino)pyrimidin-4-yl)amino)-5-fluorophenyl)methanesulfonamide;
Compound No. 24: *N*-(2-((5-bromo-2-((2-methoxy-5-methyl-4-(4-(4-methylpiperazin-1-yl)piperidin-1-yl)phenyl)amino)pyrimidin-4-yl)amido)-5-fluorophenyl)methanesulfonamide;
Compound No. 25: *N*-(2-((5-bromo-2-((5-fluoro-6-(4-(4-methylpiperazin-1-yl)piperidin-1-yl)pyridin-3-yl)amino)pyrimidin-4-yl)amino)-5-fluorophenyl)methanesulfonamide;
Compound No. 26: *N*-(2-((5-bromo-2-((5-chloro-2-methoxy-4-(4-(4-methylpiperazin-1-yl)piperidin-1-yl)phenyl)amino)pyrimidin-4-yl)amino)-5-fluorophenyl)methanesulfonamide;
Compound No. 27: *N*-(2-((5-bromo-2-((2-chloro-4-(4-(4-methylpiperazin-1-yl)piperidin-1-yl)phenyl)amino)pyrimidin-4-yl)amino)-5-fluorophenyl)methanesulfonamide;
Compound No. 28: *N*-(2-((5-bromo-2-((2-methoxy-6-(4-(4-methylpiperazin-1-yl)piperidin-1-yl)pyridin-3-yl)amino)pyrimidin-4-yl)amino)-5-fluorophenyl)methanesulfonamide;
Compound No. 29: *N*-(2-((5-bromo-2-((2-methoxy-6-morpholinopyridin-3-yl)amino)pyrimidin-4-yl)amino)-5-fluorophenyl)methanesulfonamide;
Compound No. 30: *N*-2-((5-bromo-2-((6-(4-cyclopropylpiperazin-1-yl)-2-methoxypyridin-3-yl)amino)pyrimidin-4-yl)amino)-5-fluorophenyl)methanesulfonamide;
Compound No. 31: *N*-(2-((5-bromo-2-((2-methoxy-6-(4-morpholinopiperidin-1-yl)pyridin-3-yl)amino)pyrimidin-4-yl)amino)-5-fluorophenyl)methanesulfonamide;
Compound No. 32: *N*-(2-((5-bromo-2-((6-(4,4-dimethylpiperidin-1-yl)-2-methoxypyridin-3-yl)amino)pyrimidin-4-yl)amino)-5-fluorophenyl)methanesulfonamide;
Compound No. 33: *N*-(2-((5-bromo-2-((6-(4,4-difluoropiperidin-1-yl)-2-methoxypyridin-3-yl)amino)pyrimidin-4-yl)amino)-5-fluorophenyl)methanesulfonamide;
Compound No. 34: *N*-(2-((5-bromo-2-((2-chloro-6-(4-(4-methylpiperazin-1-yl)piperidin-1-yl)-5-(trifluoromethyl)pyridin-3-yl)amino)pyrimidin-4-yl)amino)-5-fluorophenyl)methanesulfonamide;
Compound No. 35: *N*-(2-((5-bromo-2-((2-(4-(4-methylpiperazin-1-yl)piperidin-1-yl)quinolin-6-yl)amino)pyrimidin-4-yl)amino)-5-fluorophenyl)methanesulfonamide;
Compound No. 36: *N*-(2-((5-bromo-2-((2-methoxy-6-(4-(1-methylpiperidin-4-yl)piperazin-1-yl)pyridin-3-yl)amino)pyrimidin-4-yl)amino)-5-fluorophenyl)methanesulfonamide;
Compound No. 37: *N*-(2-((5-bromo-2-((2-methoxy-6-(4-(1-methyl-4-yl)piperazin-1-yl)-5-(trifluoromethyl)pyridin-3-yl)amino)pyrimidin-4-yl)amino)-5-fluorophenyl)methanesulfonamide;
Compound No. 38: *N*-(2-((5-bromo-2-((2-methoxy-5-methyl-4-(4-(4-methyl-1,4-diazepan-1-yl)piperidin-1-yl)phenyl)amino)pyrimidin-4-yl)amino)-5-fluorophenyl)methanesulfonamide;
Compound No. 39: *N*-(2-((5-bromo-2-((4-(3-(dimethylamino)-[1,4'-bipiperidin]-1'-yl)-2-methoxy-5-methylphenyl)amino)pyrimidin-4-yl)amino)-5-fluorophenyl)methanesulfonamide;
Compound No. 40: *N*-2-((5-bromo-2-((4-(4-(3-(dimethylamino)pyrrolidin-1-yl)piperidin-1-yl)-2-methoxy-5-methylphenyl)amino)pyrimidin-4-yl)amino)-5-fluorophenyl)methanesulfonamide;
Compound No. 41: *N*-(2-((5-bromo-2-((2-methoxy-5-methyl-4-(4-((1-methyl-1H-pyrazol-5-yl)amino)piperidin-1-yl)phenyl)amino)pyrimidin-4-yl)amino)-5-fluorophenyl)methanesulfonamide;
Compound No. 42: *N* (2-((5-bromo-2-((4-(4-(4-cyclopropylpiperazin-1-yl)piperidin-1-yl)-2-methoxy-5-methylphenyl)amino)pyrimidin-4-yl)amino)-5-fluorophenyl)methanesulfonamide;
Compound No. 43: *N*-(2-((5-bromo-2-((2-chloro-5-methyl-4-(4-(4-methylpiperidin-1-yl)piperidin-1-yl)phenyl)amino)pyrimidin-4-yl)amino)-5-fluorophenyl)methanesulfonamide;
Compound No. 44: *N*-(2-((5-bromo-2-((2-chloro-5-methyl-4-(4-(4-methyl-1,4-diazepan-1-yl)piperidin-1-yl)phenyl)amino)pyrimidin-4-yl)amino)-5-fluorophenyl)methanesulfonamide;
Compound No. 45: *N*-(2-((5-bromo-2-((2-chloro-4-(3-(dimethylamino)-[1,4'-bipiperidin]-1'-yl)-5-methylphenyl)amino)pyrimidin-4-yl)amino)fluorophenyl)methanesulfonamide;
Compound No. 46: 5-chloro-*N*²-(2-methoxy-5-methyl-4-(4-(4-methylpiperazin-1-yl)piperidin-1-yl)phenyl)-*N*⁴-(2-(trifluoromethyl)phenyl)pyrimidine-2,4-diamine;
Compound No. 47: 5-chloro-*N*²-(2-methoxy-4-(4-(4-methylpiperazin-1-yl)piperidin-1-yl)phenyl)-*N*⁴-(2-(trifluoromethyl)phenyl)pyrimidine-2,4-diamine;
Compound No. 48: 5-chloro-*N*²-(4-(4-ethylpiperazin-1-yl)phenyl)-*N*⁴-(2-(trifluoromethyl)phenyl)pyrimidine-2,4-diamine;
Compound No. 49: 5-chloro-*N*²-(6-morpholinopyridin-3-yl)-*N*⁴-(2-(trifluoromethyl)phenyl)pyrimidine-2,4-diamine;
Compound No. 50: 2-((5-chloro-2-((2-methoxy-5-methyl-4-(4-(4-methylpiperazin-1-yl)piperidin-1-yl)phenyl)amino)pyrimidin-4-yl)amino)-*N-*methylbenzenesulfonamide;
Compound No. 51: 2-((5-chloro-2-((2-methoxy-4-(4-(4-methylpiperazin-1-yl)piperidin-1-yl)phenyl)amino)pyrimidin-4-yl)amino)-*N*-methylbenzenesulfonamide;
Compound No. 52: 2-((5-chloro-2-((6-(4-(4-methylpiperazin-1-yl)piperidin-1-yl)pyridin-3-yl)amino)pyrimidin-4-yl)amino)-*N*-methylbenzenesulfonamide;
Compound No. 53: 2-((5-chloro-2-((6-morpholinopyridin-3-yl)amino)pyrimidin-4-yl)amino)-N-methylbenzenesulfonamide;
Compound No. 54: 2-((5-chloro-2-((6-(4-ethylpiperazin-1-yl)-2-methoxypyridin-3-yl)amino)pyrimidin-4-yl)amino)-*N*-methylbenzenesulfonamide;
Compound No. 55: 2-((5-chloro-2-((2-methoxy-6-(4-(4-methylpiperazin-1-yl)piperidin-1-yl)pyridin-3-yl)amino)pyrimidin-4-yl)amino)-*N-*methylbenzenesulfonamide;
Compound No. 56: 2-((5-chloro-2-((6-(4-(2-hydroxyethyl)piperazin-1-yl)-2-methoxypyridin-3-yl)amino)pyrimidin-4-yl)amino)-*N*-methylbenzenesulfonamide;
Compound No. 57: 2-((5-chloro-2-((6-(3-(dimethylamino)pyrrolidin-1-yl)-2-methoxypyridin-3-yl)amino)pyrimidin-4-yl)amino)-*N*-methylbenzenesulfonamide;
Compound No. 58: 2-((5-chloro-2-((2-methoxy-5-methyl-6-(4-(4-methylpiperazin-1-yl)piperidin-1-yl)pyridin-3-yl)amino)pyrimidin-4-yl)amino)-*N-*methylbenzenesulfonamide;
Compound No. 59: 2-((5-chloro-2-((2-methoxy-5-methyl-4-(4-(4-methylpiperazin-1-yl)piperidin-1-yl)phenyl)amino)pyrimidin-4-yl)amino)-*N-*cyclopropylbenzenesulfonamide;
Compound No. 60: 2-((5-chloro-2-((3-fluoro-4-(4-(4-methylpiperazin-1-yl)piperidin-1-yl)phenyl)amino)pyrimidin-4-yl)amino)-N-cyclopropylbenzenesulfonamide;
Compound No. 61: 2-((5-chloro-2-((2-methoxy-6-(4-(4-methylpiperazin-1-yl)piperadin-1-yl)pyridin-3-yl)amino)pyrimidin-4-yl)amino)-*N-*cyclopropylbenzenesulfonamide;
Compound No. 62: 5-chloro-*N*²-(2-methoxy-5-methyl-4-(4-(4-methylpiperazin-1-yl)piperidin-1-yl)phenyl)-*N*⁴-(2-((methylsulfonyl)methyl)phenyl)pyrimidine-2,4-diamine;
Compound No. 63: 5-chloro-*N*²-(3-fluoro-4-(4-(4-methylpiperazin-1-yl)piperidin-1-yl)phenyl)-N⁴-(2-((methylsulfonyl)methyl)phenyl)pyrimidine-2,4-diamine;
Compound No. 64: 5-chloro-*N*²-(2-methoxy-6-(4-(4-methylpiperazin-1-yl)piperidin-1-yl)pyridin-3-yl)-*N*⁴-(2-((methylsulfonyl)methyl)phenyl)pyrimidine-2,4-diamine;
Compound No. 65: *N*-(4-chloro-2-((5-chloro-2-((2-methoxy-5-methyl-4-(4-(4-methylpiperazin-1-yl)piperidin-1-yl)phenyl)amino)pyrimidin-4-yl)amino)phenyl)acetamide;
Compound No. 66: *N*-(4-chloro-2-((5-chloro-2-((2-methoxy-5-methyl-6-(4-(4-methylpiperazin-1-yl)piperidin-1-yl)pyridin-3-yl)amino)pyrimidin-4-yl)amino)phenyl)acetamide;
Compound No. 67: *N*-(4-chloro-2-((5-chloro-2-((3-fluoro-4-(4-(4-methylpiperazin-1-yl)piperidin-1-yl)phenyl)amino)pyrimidin-4-yl)amino)phenyl)acetamide;
Compound No. 68: *N*-(2-((5-chloro-2-((2-methoxy-5-methyl-4-(4-(4-methylpiperazin-1-yl)piperidin-1-yl)phenyl)amino)pyrimidin-4-yl)amino)phenyl)acetamide;
Compound No. 69: *N*-(2-((5-chloro-2-((3-fluoro-4-(4-(4-methylpiperazin-1-yl)piperidin-1-yl)phenyl)amino)pyrimidin-4-yl)amino)phenyl)acetamide;
Compound No. 70: *N*-(2-((5-chloro-2-((5-fluoro-6-(4-(4-methylpiperazin-1-yl)piperidin-1-yl) pyridin-3-yl)-amino)pyrimidin-4-yl)amino)phenyl)acetamide;
Compound No. 71: *N*-(4,5-dichloro-2-((5-chloro-2-((2-methoxy-5-methyl-4-(4-(4-methylpiperazin-1-yl)piperidin-1-yl)phenyl)amino)pyrimidin-4-yl)amino)phenyl)acetamide;
Compound No. 72: *N*-(4,5-dichloro-2-((5-chloro-2-((2-methoxy-6-(4-(4-methylpiperazin-1-yl)piperidin-1-yl)pyridin-3-yl)amino)pyrimidin-4-yl)amino)phenyl)acetamide;
Compound No. 73: *N*-(4,5-dichloro-2-((5-chloro-2-((5-fluoro-6-(4-(4-methylpiperazin-1-yl)piperidin-1-yl)pyridin-3-yl)amino)pyrimidin-4-yl)amino)phenyl)acetamide;
Compound No. 74: *N*-(4,5-dichloro-2-((5-chloro-2-((6-morpholinopyridin-3-yl)amino)pyrimidin-4-yl)amino)phenyl)acetamide;
Compound No. 75: *N*-(4,5-dichloro-2-((5-chloro-2-((2-methoxy-4-(4-(4-methylpiperazin-1-yl)piperidin-1-yl)phenyl)amino)pyrimidin-4-yl)amino)phenyl)acetamide;
Compound No. 76: *N*-(4,5-dichloro-2-((5-chloro-2-((3-fluoro-4-(4-(4-methylpiperazin-1-yl)piperidin-1-yl)phenyl)amino)pyrimidin-4-yl)amino)phenyl)acetamide;
Compound No. 77: *N*-(4,5-dichloro-2-((5-chloro-2-((2-chloro-4-(4-(4-methylpiperazin-1-yl)piperidin-1-yl)phenyl)amino)pyrimidin-4-yl)amino)phenyl)acetamide;
Compound No. 78: *N*-(4,5-dichloro-2-((5-chloro-2-((2-methoxy-5-methyl-4-(4-(4-methylpiperazin-1-yl)piperidin-1-yl)phenyl)amino)pyrimidin-4-yl)(methyl)amino)phenyl)-*N*-methylacetamide;
Compound No. 79: *N*-(4,5-dichloro-2-((5-chloro-2-((2-methoxy-6-(4-(4-methylpiperazin-1-yl)piperidin-1-yl)pyridin-3-yl)amino)pyrimidin-4-yl)(methyl)amino)phenyl)-*N*-methylacetamide;
Compound No. 80: *N*-(4-chloro-2-((5-chloro-2-((2-methoxy-5-methyl-4-(4-(4-methylpiperazin-1-yl)piperidin-1-yl)phenyl)amino)pyrimidin-4-yl)amino)phenyl)methanesulfonamide;
Compound No. 81: *N*-(4-chloro-2-((5-chloro-2-((2-methoxy-6-(4-(4-methylpiperazin-1-yl)piperidin-1-yl)pyridin-3-yl)amino)pyrimidin-4-yl)aminophenyl)methanesulfonamide;
Compound No. 82: *N*-(4-chloro-2-((5-chloro-2-((2-methoxy-4-(4-(4-methylpiperazin-1-yl)piperidin-1-yl)phenyl)amino)pyrimidin-4-yl)amino)phenyl)methanesulfonamide;
Compound No. 83: *N*-(4-chloro-2-((5-chloro-2-((2-methoxy-5-methyl-6-(4-(4-methylpiperazin-1-yl)piperidin-1-yl)pyridin-3-yl)amino)pyrimidin-4-yl)amino)phenyl)methanesulfonamide;
Compound No. 84: *N*-(4-chloro-2-((5-chloro-2-((3-fluoro-4-(4-(4-methylpiperazin-1-yl)piperidin-1-yl)phenyl)amino)pyrimidin-4-yl)amino)phenyl)methanesulfonamide;
Compound No. 85: *N*-(2-((5-bromo-2-((2-methoxy-5-methyl-4-(4-(4-methylpiperazin-1-yl)piperidin-1-yl)phenyl)amino)pyrimidin-4-yl)amino-4-chlorophenyl)methanesulfonamide;
Compound No. 86: *N*-(2-((5-bromo-2-((2-methoxy-5-methyl-6-(4-(4-methylpiperazin-1-yl)piperidin-1-yl)pyridin-3-yl)amino)pyrimidin-4-yl)amino)-4-chlorophenyl)methanesulfonamide;
Compound No. 87: *N*-(2-((5-bromo-2-((3-fluoro-4-(4-(4-methylpiperazin-1-yl)piperidin-1-yl)phenyl)amino)pyrimidin-4-yl)amino)-4-chlorophenyl)methanesulfonamide;
Compound No. 88: *N*-(2-((5-bromo-2-((5-chloro-2-methoxy-4-(4-(4-methylpiperazin-1-yl)piperidin-1-yl)phenyl)amino)pyrimidin-4-yl)amino)-4-chlorophenyl)methanesulfonamide;
Compound No. 89: *N*-(2-((5-bromo-2-((2-methoxy-5-methyl-4-(4-(4-methyl-1,4-diazepan-1-yl)piperidin-1-yl)phenyl)amino)pyrimidin-4-yl)amino)-4-chlorophenyl)methanesulfonylamide;
Compound No. 90: *N*-(2-((5-bromo-2-((4-(3-(dimethylamino)-[1,4'-bipiperidin]-1'-yl)-2-methoxy-5-methylphenyl)amino)pyrimidin-4-yl)amino)-4-chlorophenyl)methanesulfonamide;
Compound No. 91: *N*-(2-((5-bromo-2-((4-(4-(3-(dimethylamino)pyrrolidin-1-yl)piperidin-1-yl)-2-methoxy-5-methylphenyl)amino)pyrimidin-4-yl)amino)-4-chlorophenyl)methanesulfonamide;
Compound No. 92: *N*-(2-((5-bromo-2-((5-fluoro-2-methoxy-4-(4-(4-methylpiperazin-1-yl)piperidin-1-yl)phenyl)amino)pyrimidin-4-yl)amino)-4-chlorophenyl)methanesulfonamide;
Compound No. 93: *N*-(2-((5-bromo-2-((2-chloro-5-methyl-4-(4-(4-methylpiperazin-1-yl)piperidin-1-yl)phenyl)amino)pyrimidin-4-yl)amino)-4-chlorophenyl)methanesulfonamide;
Compound No. 94: *N*-(2-((5-bromo-2-((2-methoxy-5-methyl-4-(4-(piperazin-1-yl)piperidin-1-yl)phenyl)amino)pyrimidin-4-yl)amino)-4-chlorophenyl)methanesulfonamide;
Compound No. 95: *N*-(2-((5-bromo-2-((5-chloro-2-methoxy-4-(4-(4-methyl-1,4-diazepan-1-yl)piperidin-1-yl)phenyl)amino)pyrimidin-4-yl)amino)-4-chlorophenyl)methanesulfonamide;
Compound No. 96: *N*-(2-((5-bromo-2-((2-chloro-4-(4-(3-(dimethylamino)pyrrolidin-1 -yl)piperidin-1 -yl)-5-methylphenyl)amino)pyrimidin-4-yl)amino)-4-chlorophenyl)methanesulfonamide;
Compound No. 97: *N*-(2-((5-bromo-2-((2-chloro-5-methyl-4-(4-(4-methyl-1,4-diazepan-1-yl)piperidin-1-yl)phenyl)amino)pyrimidin-4-yl)amino)-4-chlorophenyl)methanesulfonamide;
Compound No. 98: *N*-(2-((5-bromo-2-((2-chloro-4-(3-(dimethylamino)-[1,4'-bipiperidin]-1'-yl)-5-methylphenyl)amino)pyrimidin-4-yl)amino)-4-chlorophenyl)methanesulfonamide;
Compound No. 99: *N*-(4,5-dichloro-2-((5-chloro-2-((2-methoxy-5-methyl-4-(4-(4-methylpiperazin-1-yl)piperidin-1-yl)phenyl)amino)pyrimidin-4-yl)amino)phenyl)methanesulfonamide;
Compound No. 100: *N*-(4,5-dichloro-2-((5-chloro-2-((2-methoxy-5-methyl-6-(4-(4-methylpiperazin-1 -yl)piperi din-1 -yl)pyri din-3 -yl)amino)pyrimidin-4-yl)amino)phenylmethanesulfonamide;
Compound No. 101: *N*-(4,5-dichloro-2-((5-chloro-2-((3-fluoro-4-(4-(4-methylpiperazin-1-yl)piperidin-1-yl)phenyl)amino)pyrimidin-4-yl)amino)phenyl)methanesulfonamide;
Compound No. 102: *N*-(4,5-dichloro-2-((5-chloro-2-((2-(4-(4-methylpiperazin-1 -yl)piperidin-1 -yl)quinolin-6-yl)amino)pyrimidin-4-yl)amino)phenyl)methanesulfonamide;
Compound No. 103: *N*-(4,5-dichloro-2-((5-chloro-2-((5-chloro-2-methoxy-4-(4-(4-methylpiperazin-1-yl)piperidin-1-yl)phenyl)amino)pyrimidin-4-yl)aminophenyl)methanesulfonamide;
Compound No. 104: *N*-(4,5-dichloro-2-((5-chloro-2-((4-(4-((2-(dimethylamino)ethyl)(methyl)amino)piperidin-1-yl)-2-methoxy-5-methylphenyl)amino)pyrimidin-4-yl)amino)phenyl)methanesulfonamide;
Compound No. 105: *N*-(4,5-dichloro-2-((5-chloro-2-((2-methoxy-5-methyl-4-(methyl(3-(4-methylpiperazin-1-yl)propyl)amino)phenyl)amino)pyrimidin-4-yl)amino)phenyl)methanesulfonamide;
Compound No. 106: *N*-(2-((5-bromo-2-((2-methoxy-5-methyl-4-(4-(4-methylpiperazin-1-yl)piperidin-1-yl)phenyl)amino)pyrimidin-4-yl)amino)-4-methylphenyl)methanesulfonamide;
Compound No. 107: *N*-(2-((5-bromo-2-((5-chloro-2-methoxy-4-(4-(4-methylpiperazin-1-yl)piperidin-1-yl)phenyl)amino)pyrimidin-4-yl)amino)-4-methylphenyl)methanesulfonamide;
Compound No. 108: *N*-(2-((5-bromo-2-((5-fluoro-2-methoxy-4-(4-(4-methoxy-1-yl)piperidin-1-yl)phenyl)amino)pyrimidin-4-yl)amino)-4-methylphenyl)methanesulfonamide;
Compound No. 109: *N*-(2-((5-bromo-2-((2-chloro-5-methyl-4-(4-(4-methylpiperazin-1-yl)piperidin-1-yl)phenyl)amino)pyrimidin-4-yl)amino)-4-methylphenyl)methanesulfonamide;
Compound No. 110: *N*-(5-chloro-2-((2-methoxy-5-methyl-4-(4-(4-methylpiperazin-1-yl)piperidin-1-yl)phenyl)amino)pyrimidin-4-yl)-*N*-(4-(methylsulfonamido)benzo[d]thiazol-5-yl)methanesulfonamide;
Compound No. 111: *N*-(5-chloro-2-((2-methoxy-6-(4-(4-methylpiperazin-1-yl)piperidin-1-yl)pyridin-3-yl)amino)pyrimidin-4-yl)-*N*-(4-(methylsulfonamido)benzo[d]thiazol-5-yl)methanesulfonamide;
Compound No. 112: *N*-(5-chloro-2-((3-fluoro-4-(4-(4-methylpiperazin-1-yl)piperidin-1-yl)phenyl)amino)pyrimidin-4-yl)-*N*-(4-(methylsulfonamido)benzo[d]thiazol-5-yl)methanesulfonamide;
Compound No. 113: *N*-(5-chloro-2-((5-fluoro-6-(4-(4-methylpiperazin-1-yl)piperidin-1-yl)pyridin-3-yl)amino)pyrimidin-4-yl)-*N*-(4-(methylsulfonamido)benzo[d]thiazol-5-yl)methanesulfonamide;
Compound No. 114: *N*-(5-chloro-2-((5-chloro-2-methoxy-4-(4-(4-methylpiperazin-1-yl)piperidin-1-yl)phenyl)amino)pyrimidin-4-yl)-*N*-(4-(methylsulfonamido)benzo[d]thiazol-5-yl)methanesulfonamide;
Compound No. 115: *N*-(6-((5-chloro-2-((2-methoxy-5-methyl-4-(4-(4-methylpiperazin-1-yl)piperidin-1-yl)phenyl)amino)pyrimidin-4-yl)amino)quinolin-5-yl)methanesulfonamide;
Compound No. 116: *N*-(6-((5-chloro-2-((2-methoxy-5-methyl-6-(4-(4-methylpiperazin-1-yl)piperidin-1-yl)pyridin-3-yl)amino)pyrimidin-4-yl)amino)quinolin-5-yl)methanesulfonamide;
Compound No. 117: *N*-(6-((5-chloro-2-((2-methoxy-6-(4-morpholinopiperidin-1-yl)pyridin-3-yl)amino)pyrimidin-4-yl)amino)quinolin-5-yl)methanesulfonamide;
Compound No. 118: *N*-(6-((5-chloro-2-((5-chloro-2-methoxy-4-(4-(4-methoxypiperazin-1-yl)piperidin-1-yl)phenyl)amino)pyrimidin-4-yl)amino)quinolin-5-yl)methanesulfonamide;
Compound No. 119: *N*-(6-((5-chloro-2-((2-methoxy-4-(4-methylpiperazin-1-yl)phenyl)amino)pyrimidin-4-yl)amino)quinolin-5-yl)methanesulfonamide;
Compound No. 120: *N*-(2-((5-chloro-2-((2-methoxy-5-methyl-4-(4-(4-methylpiperazin-1-yl)piperidin-1-yl)phenyl)amino)pyrimidin-4-yl)(methyl)amino)phenyl)methanesulfonamide;
Compound No. 121: *N*-(2-((5-chloro-2-((2-methoxy-5-methyl-6-(4-(4-methylpiperazin-1-yl)piperidin-1-yl)pyridin-3-yl)amino)pyrimidin-4-yl)(methyl)amino)phenyl)methanesulfonamide;
Compound No. 122: (6-((5-chloro-2-((2-methoxy-5-methyl-6-(4-(4-methylpiperazin-1-yl)piperidin-1-yl)pyridin-3-yl)amino)pyrimidin-4-yl)amino)quinoxalin-5-yl)dimethylphosphine oxide;
Compound No. 123: (6-((5-chloro-2-((2-methoxy-6-(4-(4-methylpiperazin-1-yl)piperidin-1-yl)pyridin-3-yl)amino)pyrimidin-4-yl)amino)quinoxalin-5-yl)dimethylphosphine oxide;
Compound No. 124: (6-((5-chloro-2-((5-fluoro-6-(4-(4-methylpiperazin-1-yl)piperidin-1-yl)pyridin-3-yl)amino)pyrimidin-4-yl)amino)quinoxalin-5-yl)dimethylphosphine oxide;
Compound No. 125: (6-((5-chloro-2-((3-fluoro-4-(4-(4-methylpiperazin-1-yl)piperidin-1-yl)phenyl)amino)pyrimidin-4-yl)amino)quinoxalin-5-yl)dimethylphosphine oxide;
Compound No. 126: (6-((5-chloro-2-((2-methoxy-6-((3S,5R)-3,4,5-trimethylpiperazin-1-yl)pyridin-3-yl)amino)pyrimidin-4-yl)amino)quinoxalin-5-yl)dimethylphosphine oxide;
Compound No. 127: (6-((5-chloro-2-((6-(3-(dimethylamino)pyrrolidin-1-yl)-2-methoxypyridin-3-yl)amino)pyrimidin-4-yl)amino)quinoxalin-5-yl)dimethylphosphine oxide;
Compound No. 128: (6-((5-chloro-2-((2-methoxy-6-(piperazin-1-yl)pyridin-3-yl)amino)pyrimidin-4-yl)amino)quinoxalin-5-yl)dimethylphosphine oxide;
Compound No. 129: 4-(5-((5-chloro-4-((5-(dimethylphosphoryl)quinoxalin-6-yl)amino)pyrimidin-2-yl)amino)-6-methoxypyridin-2-yl)thiomorpholine 1,1-dioxide;
Compound No. 130: (6-((5-bromo-2-((2-methoxy-5-methyl-4-(4-(4-methylpiperazin-1-yl)piperidin-1-yl)phenyl)amino)pyrimidin-4-yl)amino)quinoxalin-5-yl)dimethylphosphine oxide;
Compound No. 131: *N*-(6-((5-chloro-2-((2-methoxy-5-methyl-4-(4-(4-methylpiperazin-1-yl)piperidin-1-yl)phenyl)amino)pyrimidin-4-yl)amino)quinoxalin-5-yl)methanesulfonamide;
Compound No. 132: *N*-(6-((5-chloro-2-((2-methoxy-6-(4-(4-methylpiperazin-1-yl)piperidin-1-yl)pyridin-3-yl)amino)pyrimidin-4-yl)amino)quinoxalin-5-yl)methanesulfonamide;
Compound No. 133: *N*-(6-((5-chloro-2-((2-methoxy-4-(4-(4-methylpiperazin-1-yl)piperidin-1-yl)phenyl)amino)pyrimidin-4-yl)amino)quinoxalin-5-yl)methanesulfonamide;
Compound No. 134: *N*-(6-((5-chloro-2-((2-methoxy-5-methyl-6-(4-(4-methylpiperazin-1-yl)piperidin-1-yl)pyridin-3-yl)amino)pyrimidin-4-yl)amino)quinoxalin-5-yl)methanesulfonamide;
Compound No. 135: *N*-(6-((5-chloro-2-((2-methoxy-6-((3S,SR)-3,4,5-trimethylpiperazin-1-yl)pyridin-3-yl)amino)pyrimidin-4-yl)amino)quinoxalin-5-yl)methanesulfonamide;
Compound No. 136: *N*-(6-((5-chloro-2-((5-chloro-2-methoxy-4-(4-(4-methylpiperazin-1-yl)piperidin-1-yl)phenyl)amino)pyrimidin-4-yl)amino)quinoxalin-5-yl)methanesulfonamide;
Compound No. 137: *N*-(2-((5-chloro-2-((5-chloro-2-methoxy-4-(4-(4-methylpiperazin-1-yl)piperidin-1-yl)phenyl)amino)pyrimidin-4-yl)amino)-4-(trifluoromethyl)phenyl)methanesulfonamide;
Compound No. 138: *N*-(2-((5-chloro-2-((2-methoxy-5-methyl-4-(4-(4-methylpiperazin-1-yl)piperidin-1-yl)phenyl)amino)pyrimidin-4-yl)amino)-4-(trifluoromethyl)phenyl)methanesulfonamide;
Compound No. 139: *N*-(2-((5-chloro-2-((4-(4-(4-cyclopropylpiperazin-1-yl)piperidin-1-yl)-2-methoxy-5-methylphenyl)amino)pyrimidin-4-yl)amino)-4-(trifluoromethyl)phenyl)methanesulfonamide;
Compound No. 140: *N*-(6-((5-chloro-2-((2-methoxy-5-methyl-4-(4-(4-methylpiperazin-1-yl)piperidin-1-yl)phenyl)amino)pyrimidin-4-yl)amino)-2,3-dihydrobenzofuran-5-yl)methanesulfonamide;
Compound No. 141: *N*-(2-((6-amino-5-chloro-2-((2-methoxy-5-methyl-4-(4-(4-methylpiperazin-1-yl)piperidin-1-yl)phenyl)amino)pyrimidin-4-yl)amino)-5-fluorophenyl)methanesulfonamide;
Compound No. 142: ((2-((5-bromo-2-((2-methoxy-5-methyl-4-(4-(4-methylpiperazin-1-yl)piperidin-1-yl)phenyl)amino)pyrimidin-4-yl)amino)phenyl)imino)dimethyl-λ⁶-sulfanone;
Compound No. 143: ((2-((5-bromo-2-((5-chloro-2-methoxy-4-(4-(4-methylpiperazin-1-yl)piperidin-1-yl)phenyl)amino)pyrimidin-4-yl)amino)phenyl)imino)dimethyl-*λ*⁶-sulfanone;
Compound No. 144: ((2-((5-bromo-2-((2-chloro-5-methyl-4-(4-(4-methylpiperazin-1-yl)piperidin-1-yl)phenyl)amino)pyrimidin-4-yl)amino)phenyl)imino)dimethyl-*λ*⁶-sulfanone;
Compound No. 145: *N*-(2-((5-bromo-2-((2-methoxy-6-(1-methylazepan-4-yl)pyridin-3-yl)amino)pyrimidin-4-yl)amino)-5-fluorophenyl)methanesulfonamide;
Compound No. 146: *N*-(2-((5-bromo-2-((5-fluoro-2-methoxy-4-(4-(4-methylpiperazin-1-yl)piperidin-1-yl)phenyl)amino)pyrimidin-4-yl)amino)-5-fluorophenyl)methanesulfonamide;
Compound No. 147: *N*-(2-((5-bromo-2-((2-methoxy-6-(4-(4-methylpiperazin-1-yl)piperidin-1-yl)pyridin-3-yl)amino)pyrimidin-4-yl)amino)-5-fluorophenyl)methanesulfonamide;
Compound No. 148: *N*-(2-((5-bromo-2-((2-methoxy-5-methyl-4-(4-(trifluoromethyl)-[1,4'-bipiperidin]-1'-yl)phenyl)amino)pyrimidin-4-yl)amino)-5-fluorophenyl)methanesulfonamide;
Compound No. 149: *N*-(2-((5-bromo-2-((5-chloro-4-(3-(dimethylamino)-[1,4'-bipiperidin]-1'-yl)-2-methoxyphenyl)amino)pyrimidin-4-yl)amino)-5-fluorophenyl)methanesulfonamide;
Compound No. 150: *N*-(2-((5-bromo-2-((2-methoxy-5-methyl-4-(4-(trifluoromethyl)-[1,4'-bipiperidin]-1'-yl)phenyl)amino)pyrimidin-4-yl)amino)-4-chlorophenyl)methanesulfonamide;
Compound No. 151: *N*-(4-chloro-2-((5-chloro-2-((5-chloro-2-methoxy-4-(4-(4-methylpiperazin-1-yl)piperidin-1-yl)phenyl)amino)pyrimidin-4-yl)amino)phenyl)methanesulfonamide;
Compound No. 152: *N*-(4-chloro-2-((6-chloro-3-((-2-methoxy-5-methyl-4-(4-(4-methylpiperazin-1-yl)piperidin-1-yl)phenyl)amino)-1,2,4-triazin-5-yl)amino)phenyl)methanesulfonamide;
Compound No. 153: (4-chloro-2-((5-chloro-2-((2-methoxy-5-methyl-4-(4-(4-methylpiperazin-1-yl)piperidin-1-yl)phenyl)amino)pyrimidin-4-yl)amino)phenyl)dimethylphosphine oxide;
Compound No. 154: (4-chloro-2-((5-chloro-2-((2-chloro-5-methyl-4-(4-(4-methylpiperazin-1-yl)piperidin-1-yl)phenyl)amino)pyrimidin-4-yl)amino)phenyl)dimethylphosphine oxide;
Compound No. 155: *N*-(2-((5-bromo-2-((2-methoxy-5-methyl-4-(4-(4-methylpiperazin-1-yl)piperidin-1-yl)phenyl)amino)pyrimidin-4-yl)amino)-4-chlorophenyl)-*N*-methylmethanesulfonamide;
Compound No. 156: *N*-(2-((5-bromo-2-((5-chloro-2-methoxy-4-(4-(4-methylpiperazin-1-yl)piperidin-1-yl)phenyl)amino)pyrimidin-4-yl)amino)-4-chlorophenyl)-*N*-methylmethanesulfonamide;
Compound No. 157: *N*-(4-chloro-2-((5-chloro-2-((2-chloro-4-(4-(3-(dimethylamino)pyrrolidin-1-yl)piperidin-1-yl)-5-methylphenyl)amino)pyrimidin-4-yl)amino)phenyl)methanesulfonamide;
Compound No. 158: *N*-(4-chloro-2-((5-chloro-2-((2-chloro-5-methyl-4-(4-(4-methylpiperazin-1-yl)piperidin-1-yl)phenyl)amino)pyrimidin-4-yl)amino)phenyl)methanesulfonamide;
Compound No. 159: *N*-(4-chloro-2-((5-chloro-2-((5-chloro-2-methoxy-4-(4-(4-methylpiperazin-1-yl)piperidin-1-yl)phenyl)amino)pyrimidin-4-yl)amino)phenyl)methanesulfonamide;
Compound No. 160: *N*-(2-((5-chloro-2-((2-methoxy-5-methyl-4-(4-(4-methylpiperazin-1-yl)piperidin-1-yl)phenyl)amino)pyrimidin-4-yl)amino)-4-isopropylphenyl)methanesulfonamide;
Compound No. 161: *N*-(2-((5-chloro-2-((5-chloro-2-methoxy-4-(4-(4-methylpiperazin-1-yl)piperidin-1-yl)phenyl)amino)pyrimidin-4-yl)amino)-4-isopropylphenyl)methanesulfonamide;
Compound No. 162: *N*-(4-chloro-2-((5-chloro-2-((2-chloro-5-methyl-4-(4-(4-methyl-1,4-diazepan-1 -yl)piperidin-1 -yl)phenyl)amino)pyrimidin-4-yl)amino)phenyl)methanesulfonamide;
Compound No. 163: *N*-(4-chloro-2-((5-chloro-2-((5-chloro-2-methoxy-4-(4-(4-methyl-1,4-diazepan-1-yl)piperidin-1-yl)phenyl)amino)pyrimidin-4-yl)amino)phenyl)methanesulfonamide;
Compound No. 164: *N*-(2-((6-chloro-3-((2-methoxy-5-methyl-4-(4-(4-methylpiperazin-1-yl)piperidin-1-yl)phenyl)amino)-1,2,4-triazin-5-yl)amino)-5-fluorophenyl)methanesulfonamide;
Compound No. 165: *N*-(6-((5-chloro-2-((2-methoxy-5-methyl-4-(4-(4-methyl-1,4-diazepan-1-yl)piperidin-1-yl)phenyl)amino)pyrimidin-4-yl)amino)-2,3-dihydrobenzofuran-5-yl)methanesulfonamide;
Compound No. 166: 5-chloro-*N*⁴-(5-chloro-2-(1*H*-1,2,3-triazol-4-yl)phenyl)-*N*²-(2-methoxy-5-methyl-4-(4-(4-methylpiperazin-1-yl)piperidin-1-yl)phenyl)pyrimidine-2,4-diamine;
Compound No. 167: *N*-(4-chloro-2-((5-chloro-2-((1-(3-(dimethylamino)propyl)-1*H*-pyrazol-4-yl)amino)pyrimidin-4-yl)amino)phenyl)methanesulfonamide;
Compound No. 168: *N*-(6-((5-chloro-2-((5-chloro-2-methoxy-4-(4-(4-methylpiperazin-1-yl)piperidin-1-yl)phenyl)amino)pyrimidin-4-yl)amino)-2,3-dihydrobenzofuran-5-yl)methanesulfonamide;
Compound No. 169: *N*-(6-((5-bromo-2-((2-methoxy-5-methyl-4-(4-(4-methylpiperazin-1-yl)piperidin-1-yl)phenyl)amino)pyrimidin-4-yl)amino)-2,3-dihydrobenzofuran-5-yl)methanesulfonamide;
Compound No. 170: *N*-(4-chloro-2-((5-chloro-2-((5-chloro-2-methoxy-4-(4-(4-methylpiperazin-1-yl)piperidin-1-yl)phenyl)amino)pyrimidin-4-yl)amino)phenyl)-*N-*methylmethanesulfonamide;
Compound No. 171: *N*-(4-chloro-2-((5-chloro-2-((2-chloro-5-methyl-4-(4-(4-methylpiperazin-1-yl)piperidin-1-yl)phenyl)amino)pyrimidin-4-yl)amino)phenyl)-*N-*methylmethanesulfonamide;
Compound No. 172: *N*-(6-((5-chloro-2-((2-chloro-5-methyl-4-(4-(4-methyl-1,4-diazepan-1-yl)piperidin-1-yl)phenyl)amino)pyrimidin-4-yl)amino)-2,3 - dihydrobenzofuran-5-yl)methanesulfonamide;
Compound No. 173: *N*-(6-((5-chloro-2-((2-chloro-5-methyl-4-(4-(4-methylpiperazin-1-yl)piperidin-1-yl)phenyl)amino)pyrimidin-4-yl)amino)-2,3-dihydrobenzofuran-5-yl)methanesulfonamide;
Compound No. 174: *N*-(6-((5-chloro-2-((5-chloro-2-methoxy-4-(4-(4-methyl-1,4-diazepan-1-yl)piperidin-1-yl)phenyl)amino)pyrimidin-4-yl)amino)-2,3-dihydrobenzofuran-5-yl)methanesulfonamide;
Compound No. 175: *N*-(6-((5-bromo-2-((2-chloro-5-methyl-4-(4-(4-methylpiperazin-1-yl)piperidin-1-yl)phenyl)amino)pyrimidin-4-yl)amino)-2,3-dihydrobenzofuran-5-yl)methanesulfonamide;
Compound No. 176: *N*-(4-chloro-2-((5-chloro-2-((2-methoxy-5-methyl-4-(4-(4-methylpiperazin-1-yl)piperidin-1-yl)phenyl)amino)pyrimidin-4-yl)amino)-5-fluorophenyl)methanesulfonamide;
Compound No. 177: *N*-(2-((5-bromo-2-((2-chloro-5-methyl-4-(4-(4-methylpiperazin-1-yl)piperidin-1-yl)phenyl)amino)pyrimidin-4-yl)amino)-4-chloro-5-fluorophenyl)methanesulfonamide;
Compound No. 178: *N*-(2-((5-bromo-2-((5-chloro-2-methoxy-4-(4-(4-methylpiperazin-1-yl)piperidin-1-yl)phenyl)amino)pyrimidin-4-yl)amino)-4-chloro-5-fluorophenyl)methanesulfonamide;
Compound No. 179: *N*-(4-chloro-2-((5-chloro-2-((2-chloro-4-(4-(3-(dimethylamino)pyrrolidin-1-yl)piperidin-1-yl)-5-methylphenyl)amino)pyrimidin-4-yl)amino)phenyl)-*N*-methylmethanesulfonamide;
Compound No. 180: *N*-(4-chloro-2-((5-chloro-2-((2-chloro-5-methyl-4-(4-(4-methyl-1,4-diazepan-1-yl)piperidin-1-yl)phenyl)amino)pyrimidin-4-yl)amino)phenyl)-*N*-methylmethanesulfonamide;
Compound No. 181: *N*-(4-chloro-2-((5-chloro-2-((5-chloro-2-methoxy-4-(4-(4-methylpiperazin-1-yl)piperidin-1-yl)phenyl)amino)pyrimidin-4-yl)amino)-5-fluorophenyl)methanesulfonamide;
Compound No. 182: *N*-(4-chloro-2-((5-chloro-2-((2-chloro-5-methyl-4-(4-(4-methylpiperazin-1-yl)piperidin-1-yl)phenyl)amino)pyrimidin-4-yl)amino)-5-fluorophenyl)methanesulfonamide;
Compound No. 183: *N*-(6-((5-bromo-2-((5-chloro-2-methoxy-4-(4-(4-methylpiperazin-1-yl)piperidin-1-yl)phenyl)amino)pyrimidin-4-yl)amino)-2,3-dihydrobenzofuran-5-yl)methanesulfonamide;
Compound No. 184: *N*-(6-((5-bromo-2-((5-ethyl-2-methoxy-4-(4-(4-methylpiperazin-1-yl)piperidin-1-yl)phenyl)amino)pyrimidin-4-yl)amino)-2,3-dihydrobenzofuran-5-yl)methanesulfonamide;
Compound No. 185: 5-chloro-*N*⁴-(5-chloro-2-(1*H*-1,2,3-triazol-5-yl)phenyl)-*N*²-(2-chloro-5-methyl-4-(4-(4-methylpiperazin-1-yl)piperidin-1-yl)phenyl)pyrimidine-2,4-diamine;
Compound No. 186: 5-chloro-*N*⁴-(5-chloro-2-(1*H*-1,2,3-triazol-5-yl)phenyl)-*N*²-(5-chloro-2-methoxy-4-(4-(4-methylpiperazin-1-yl)piperidin-1-yl)phenyl)pyrimidine-2,4-diamine;
Compound No. 187: 5-bromo-*N*⁴-(5-chloro-2-(1*H*-1,2,3-triazol-5-yl)phenyl)-*N*²-(2-methoxy-5-methyl-4-(4-(4-methylpiperazin-1-yl)piperidin-1-yl)phenyl)pyrimidine-2,4-diamine;
Compound No. 188: *N*-(6-((5-bromo-2-((2-methoxy-5-methyl-4-(4-(4-methyl-1,4-diazepan-1-yl)piperidin-1-yl)phenyl)amino)pyrimidin-4-yl)amino)-2,3-dihydrobenzofuran-5-yl)methanesulfonamide;
Compound No. 189: *N*-(6-((5-bromo-2-((5-chloro-2-methoxy-4-(4-(4-methyl-1,4-diazepan-1-yl)piperidin-1-yl)phenyl)amino)pyrimidin-4-yl)amino)-2,3-dihydrobenzofuran-5-yl)methanesulfonamide;
Compound No. 190: (6-((5-chloro-2-((5-ethyl-2-methoxy-4-(4-(4-methylpiperazin-1-yl)piperidin-1-yl)phenyl)amino)pyrimidin-4-yl)amino)quinoxalin-5-yl)dimethylphosphine oxide;
Compound No. 191: *N*-(2-((5-bromo-2-((5-ethyl-2-methoxy-4-(4-(4-methylpiperazin-1-yl)piperidin-1-yl)phenyl)amino)pyrimidin-4-yl)amino)-4-chlorophenyl)methanesulfonamide;
Compound No. 192: (6-((5-bromo-2-((5-ethyl-2-methoxy-4-(4-(4-methylpiperazin-1-yl)piperidin-1-yl)phenyl)amino)pyrimidin-4-yl)amino)quinoxalin-5-yl)dimethylphosphine oxide;
Compound No. 193: (6-((5-bromo-2-((2-chloro-5-methyl-4-(4-(4-methylpiperazin-1-yl)piperidin-1-yl)phenyl)amino)pyrimidin-4-yl)amino)quinoxalin-5-yl)dimethylphosphine oxide;
Compound No. 194: (6-((5-bromo-2-((5-chloro-2-methoxy-4-(4-(4-methylpiperazin-1-yl)piperidin-1-yl)phenyl)amino)pyrimidin-4-yl)amino)quinoxalin-5-yl)dimethylphosphine oxide;
Compound No. 195: 5-bromo-*N*⁴-(5-chloro-2-(1*H*-1,2,3-triazol-5-yl)phenyl)-*N*²-(5-chloro-2-methoxy-4-(4-(4-methylpiperazin-1-yl)piperidin-1-yl)phenyl)pyrimidine-2,4-diamine;
Compound No. 196: *N-*(6-((5-bromo-2-((4-(3-(dimethylamino)-[1,4'-bipiperidin]-1'-yl)-2-methoxy-5-methylphenyl)amino)pyrimidin-4-yl)amino)-2,3-dihydrobenzofuran-5-yl)methanesulfonamide;
Compound No. 197: *N*-(6-((5-chloro-2-((2-chloro-4-(3-(dimethylamino)-[1,4'-bipiperidin]-1'-yl)-5-methylphenyl)amino)pyrimidin-4-yl)amino)-2,3-dihydrobenzofuran-5-yl)methanesulfonamide;
Compound No. 198: *N-*(4-chloro-2-((5-chloro-2-((2,5-dichloro-4-(4-(4-methylpiperazin-1-yl)piperidin-1-yl)phenyl)amino)pyrimidin-4-yl)amino)phenyl)methanesulfonamide;
Compound No. 199: *N*-(2-((5-chloro-2-((2-methoxy-5-methyl-4-(4-(4-methylpiperazin-1-yl)piperidin-1-yl)phenyl)amino)pyrimidin-4-yl)amino)-4-methoxyphenyl)methanesulfonamide;
Compound No. 200: *N-*(2-((5-chloro-2-((2-chloro-5-methyl-4-(4-(4-methylpiperazin-1-yl)piperidin-1-yl)phenyl)amino)pyrimidin-4-yl)amino)-4-methoxyphenyl)methanesulfonamide;
Compound No. 201: *N-*(2-((5-chloro-2-((5-chloro-2-methoxy-4-(4-(4-methylpiperazin-1-yl)piperidin-1-yl)phenyl)amino)pyrimidin-4-yl)amino)-4-methoxyphenyl)methanesulfonamide;
Compound No. 202: *N-*(2-((5-chloro-2-((2,5-dichloro-4-(4-(4-methylpiperazin-1-yl)piperidin-1-yl)phenyl)amino)pyrimidin-4-yl)amino)-4-methoxyphenyl)methanesulfonamide;
Compound No. 203: *N-*(2-((5-chloro-2-((5-ethyl-2-methoxy-4-(4-(4-methylpiperazin-1-yl)piperidin-1-yl)phenyl)amino)pyrimidin-4-yl)amino)-4-methoxyphenyl)methanesulfonamide;
Compound No. 204: *N-*(2-((5-chloro-2-((2-methoxy-5-methyl-4-(4-(4-methyl-1,4-diazepan-1-yl)piperidin-1-yl)phenyl)amino)pyrimidin-4-yl)amino)-4-methoxyphenyl)methanesulfonamide;
Compound No. 205: *N-*(6-((5-chloro-2-((2,5-dichloro-4-(4-(4-methylpiperazin-1-yl)piperidin-1-yl)phenyl)amino)pyrimidin-4-yl)amino)-2,3-dihydrobenzofuran-5-yl)methanesulfonamide;
Compound No. 206: *N-*(6-((5-chloro-2-((5-ethyl-2-methoxy-4-(4-(4-methylpiperazin-1-yl)piperidin-1-yl)phenyl)amino)pyrimidin-4-yl)amino)-2,3-dihydrobenzofuran-5-yl)methanesulfonamide;
Compound No. 207: *N-*(6-((5-chloro-2-((2-methoxy-5-methyl-4-(4-(piperazin-1-yl)piperidin-1-yl)phenyl)amino)pyrimidin-4-yl)amino)-2,3-dihydrobenzofuran-5-yl)methanesulfonamide;
Compound No. 208: *N-*(6-((5-chloro-2-((4-(4-(4-cyclopropylpiperazin-1-yl)piperidin-1-yl)-2-methoxy-5-methylphenyl)amino)pyrimidin-4-yl)amino)-2,3-dihydrobenzofuran-5-yl)methanesulfonamide;
Compound No. 209: *N-*(6-((5-chloro-2-((4-(4-(3-(dimethylamino)pyrrolidin-1-yl)piperidin-1-yl)-2-methoxy-5-methylphenyl)amino)pyrimidin-4-yl)amino)-2,3-dihydrobenzofuran-5-yl)methanesulfonamide;
Compound No. 210: *N-*(6-((5-chloro-2-((2-chloro-4-(4-(3-(dimethylamino)pyrrolidin-1 -yl)piperidin-1 -yl)-5-methylphenyl)amino)pyrimidin-4-yl)amino)-2,3-dihydrobenzofuran-5-yl)methanesulfonamide;
Compound No. 211: *N-*(6-((5-bromo-2-((4-(4-(3-(dimethylamino)pyrrolidin-1-yl)piperidin-1-yl)-2-methoxy-5-methylphenyl)amino)pyrimidin-4-yl)amino)-2,3-dihydrobenzofuran-5-yl)methanesulfonamide;
Compound No. 212: *N-*(6-((5-bromo-2-((4-(4-(4-cyclopropylpiperazin-1-yl)piperidin-1-yl)-2-methoxy-5-methylphenyl)amino)pyrimidin-4-yl)amino)-2,3-dihydrobenzofuran-5-yl)methanesulfonamide;
Compound No. 213: *N-*(6-((5-bromo-2-((2-chloro-4-(4-(3-(dimethylamino)pyrrolidin-1 -yl)piperidin-1 -yl)-5-methylphenyl)amino)pyrimidin-4-yl)amino)-2,3-dihydrobenzofuran-5-yl)methanesulfonamide;
Compound No. 214: *N-*(6-((5-bromo-2-((2-chloro-4-(3-(dimethylamino)-[1,4'-bipiperidin]-1'-yl)-5-methylphenyl)amino)pyrimidin-4-yl)amino)-2,3-dihydrobenzofuran-5-yl)methanesulfonamide;
Compound No. 215: *N-*(6-((5-bromo-2-((2-methoxy-5-methyl-4-(4-(piperazin-1-yl)piperidin-1-yl)phenyl)amino)pyrimidin-4-yl)amino)-2,3-dihydrobenzofuran-5-yl)methanesulfonamide;
Compound No. 216: *N-*(6-((5-bromo-2-((5-chloro-4-(3-(dimethylamino)-[1,4'-bipiperidin]-1'-yl)-2-methoxyphenyl)amino)pyrimidin-4-yl)amino)-2,3-dihydrobenzofuran-5-yl)methanesulfonamide;
Compound No. 217: *N-*(6-((5-bromo-2-((2,5-dichloro-4-(4-(4-methylpiperazin-1-yl)piperidin-1-yl)phenyl)amino)pyrimidin-4-yl)amino)-2,3-dihydrobenzofuran-5-yl)methanesulfonamide;
Compound No. 218: *N-*(6-((5-chloro-2-((4-(3-(dimethylamino)-[1,4'-bipiperidin]-1'-yl)-2-methoxy-5-methylphenyl)amino)pyrimidin-4-yl)amino)-2,3-dihydrobenzofuran-5-yl)methanesulfonamide;
Compound No. 219: *N-*(6-((5-chloro-2-((5-chloro-4-(3-(dimethylamino)-[1,4'-bipiperidin]-1'-yl)-2-methoxyphenyl)amino)pyrimidin-4-yl)amino)-2,3-dihydrobenzofuran-5-yl)methanesulfonamide;
Compound No. 220: *N-*(6-((5-chloro-2-((2-methoxy-4-(4-(4-methylpiperazin-1-yl)piperidin-1-yl)phenyl)amino)pyrimidin-4-yl)amino)-2,3-dihydrobenzofuran-5-yl)methanesulfonamide;
Compound No. 221: *N-*(6-((5-chloro-2-((2-chloro-4-(4-(4-methylpiperazin-1-yl)piperidin-1-yl)phenyl)amino)pyrimidin-4-yl)amino)-2,3-dihydrobenzofuran-5-yl)methanesulfonamide;
Compound No. 222: *N-*(6-((5-chloro-2-((5-fluoro-2-methoxy-4-(4-(4-methylpiperazin-1-yl)piperidin-1-yl)phenyl)amino)pyrimidin-4-yl)amino)-2,3-dihydrobenzofuran-5-yl)methanesulfonamide;
Compound No. 223: *N-*(6-((5-chloro-2-((2-(4-(4-methylpiperazin-1-yl)piperidin-1-yl)quinolin-6-yl)amino)pyrimidin-4-yl)amino)-2,3-dihydrobenzofuran-5-yl)methanesulfonamide;
Compound No. 224: *N-*(6-((5-chloro-2-((2-methoxy-5-methyl-4-(4-((1-methyl-1*H*-pyrazol-5-yl)amino)piperidin-1-yl)phenyl)amino)pyrimidin-4-yl)amino)-2,3-dihydrobenzofuran-5-yl)methanesulfonamide;
Compound No. 225: *N-*(6-((5-chloro-2-((2-methoxy-5-methyl-4-(4-(4-(trifluoromethyl)piperazin-1-yl)piperidin-1-yl)phenyl)amino)pyrimidin-4-yl)amino)-2,3-dihydrobenzofuran-5-yl)methanesulfonamide;
Compound No. 226: *N-*(4-chloro-2-((5-chloro-2-((2,5-dichloro-4-(4-(4-methylpiperazin-1-yl)piperidin-1-yl)phenyl)amino)pyrimidin-4-yl)amino)phenyl)-*N-*methylmethanesulfonamide;
Compound No. 227: *N-*(2-((5-bromo-2-((2-chloro-4-(4-(3-(dimethylamino)pyrrolidin-1-yl)piperidin-1-yl)-5-methylphenyl)amino)pyrimidin-4-yl)amino)-4-chlorophenyl)-*N-*methylmethanesulfonamide;
Compound No. 228: *N-*(2-((5-bromo-2-((2,5-dichloro-4-(4-(4-methylpiperazin-1-yl)piperidin-1-yl)phenyl)amino)pyrimidin-4-yl)amino)-4-chlorophenyl)-*N-*methylmethanesulfonamide;
Compound No. 229: *N-*(2-((5-chloro-2-((5-chloro-2-methoxy-4-(4-(4-methyl-1,4-diazepan-1-yl)piperidin-1-yl)phenyl)amino)pyrimidin-4-yl)amino)-4-methoxyphenyl)methanesulfonamide;
Compound No. 230: *N-*(2-((5-chloro-2-((2-chloro-4-(4-(3-(dimethylamino)pyrrolidin-1 -yl)piperidin-1 -yl)-5-methylphenyl)amino)pyrimidin-4-yl)amino)-4-methoxyphenyl)methanesulfonamide;
Compound No. 231: *N-*(2-((5-chloro-2-((2-methoxy-4-(4-(4-methylpiperazin-1-yl)piperidin-1-yl)phenyl)amino)pyrimidin-4-yl)amino)-4-methoxyphenyl)methanesulfonamide;
Compound No. 232: *N-*(2-((5-chloro-2-((4-(3-(dimethylamino)-[1,4'-bipiperidin]-1'-yl)-2-methoxy-5-methylphenyl)amino)pyrimidin-4-yl)amino)-4-methoxyphenyl)methanesulfonamide;
Compound No. 233: *N-*(2-((5-chloro-2-((4-(4-(4-cyclopropylpiperazin-1-yl)piperidin-1-yl)-2-methoxy-5-methylphenyl)amino)pyrimidin-4-yl)amino)-4-methoxyphenyl)methanesulfonamide;
Compound No. 234: *N-*(2-((5-chloro-2-((2-methoxy-5-methyl-4-(4-(trifluoromethyl)-[1,4'-bipiperidin]-1'-yl)phenyl)amino)pyrimidin-4-yl)amino)-4-methoxyphenyl)methanesulfonamide;
Compound No. 235: *N-*(2-((5-chloro-2-((4-(4-(3-(dimethylamino)pyrrolidin-1-yl)piperidin-1-yl)-2-methoxy-5-methylphenyl)amino)pyrimidin-4-yl)amino)-4-methoxyphenyl)methanesulfonamide;
Compound No. 236: *N-*(2-((5-chloro-2-((2-chloro-4-(3-(dimethylamino)-[1,4'-bipiperidin]-1'-yl)-5-methylphenyl)amino)pyrimidin-4-yl)amino)-4-methoxyphenyl)methanesulfonamide;
Compound No. 237: *N-*(2-((5-chloro-2-((5-chloro-4-(3-(dimethylamino)-[1,4'-bipiperidin]-1'-yl)-2-methoxyphenyl)amino)pyrimidin-4-yl)amino)-4-methoxyphenyl)methanesulfonamide;
Compound No. 238: *N-*(6-((5-bromo-2-((2-methoxy-6-(4-methyl-1,4-diazepan-1-yl)pyridin-3-yl)amino)pyrimidin-4-yl)amino)-2,3-dihydrobenzofuran-5-yl)methanesulfonamide;
Compound No. 239: *N-*(6-((5-bromo-2-((5-fluoro-6-(4-(4-methylpiperazin-1-yl)piperidin-1-yl)pyridin-3-yl)amino)pyrimidin-4-yl)amino)-2,3-dihydrobenzofuran-5-yl)methanesulfonamide;
Compound No. 240: *N-*(6-((5-bromo-2-((2-methoxy-5-methyl-6-(4-(4-methylpiperazin-1-yl)piperidin-1-yl)pyridin-3-yl)amino)pyrimidin-4-yl)amino)-2,3-dihydrobenzofuran-5-yl)methanesulfonamide;
Compound No. 241: *N-*(6-((5-chloro-2-((2-methoxy-5-methyl-6-(4-(4-methylpiperazin-1-yl)piperidin-1-yl)pyridin-3-yl)amino)pyrimidin-4-yl)amino)-2,3-dihydrobenzofuran-5-yl)methanesulfonamide;
Compound No. 242: *N-*(6-((5-chloro-2-((5-fluoro-6-(4-(4-methylpiperazin-1-yl)piperidin-1-yl)pyridin-3-yl)amino)pyrimidin-4-yl)amino)-2,3-dihydrobenzofuran-5-yl)methanesulfonamide;
Compound No. 243: *N-*(6-((5-bromo-2-((2-methoxy-4-(4-(4-methylpiperazin-1-yl)piperidin-1-yl)phenyl)amino)pyrimidin-4-yl)amino)-2,3-dihydrobenzofuran-5-yl)methanesulfonamide;
Compound No. 244: *N-*(6-((5-bromo-2-((3-fluoro-4-(4-(4-methylpiperazin-1-yl)piperidin-1-yl)phenyl)amino)pyrimidin-4-yl)amino)-2,3-dihydrobenzofuran-5-yl)methanesulfonamide;
Compound No. 245: *N-*(6-((5-bromo-2-((2-methoxy-5-methyl-4-(4-(trifluoromethyl)-[1,4'-bipiperidin]-1'-yl)phenyl)amino)pyrimidin-4-yl)amino)-2,3-dihydrobenzofuran-5-yl)methanesulfonamide;
Compound No. 246: *N-*(6-((5-bromo-2-((2-chloro-4-(4-(4-methylpiperazin-1-yl)piperidin-1-yl)phenyl)amino)pyrimidin-4-yl)amino)-2,3-dihydrobenzofuran-5-yl)methanesulfonylamide;
Compound No. 247: *N-*(6-((5-bromo-2-((2-(4-(4-methylpiperazin-1-yl)piperidin-1-yl)quinolin-6-yl)amino)pyrimidin-4-yl)amino)-2,3-dihydrobenzofuran-5-yl)methanesulfonamide;
Compound No. 248: *N-*(6-((5-chloro-2-((2-methoxy-5-methyl-4-(4-(4-methylpiperazin-1-yl)piperidin-1-yl)phenyl)amino)pyrimidin-4-yl)amino)-2,3-dihydrobenzofuran-5-yl)-*N-*methylmethanesulfonamide;
Compound No. 249: *N-*(6-((5-chloro-2-((5-chloro-2-methoxy-4-(4-(4-methylpiperazin-1-yl)piperidin-1-yl)phenyl)amino)pyrimidin-4-yl)amino)-2,3-dihydrobenzofuran-5-yl)-*N-*methylmethanesulfonamide;
Compound No. 250: *N-*(6-((5-bromo-2-((2-methoxy-5-methyl-4-(4-(4-methylpiperazin-1-yl)piperidin-1-yl)phenyl)amino)pyrimidin-4-yl)amino)-2,3-dihydrobenzofuran-5-yl)-*N-*methylmethanesulfonamide;
Compound No. 251: *N-*(6-((5-bromo-2-((2-chloro-5-methyl-4-(4-(4-methylpiperazin-1-yl)piperidin-1-yl)phenyl)amino)pyrimidin-4-yl)amino)-2,3-dihydrobenzofuran-5-yl)-*N-*methylmethanesulfonamide;
Compound No. 252: *N-*(6-((5-bromo-2-((5-chloro-2-methoxy-4-(4-(4-methylpiperazin-1-yl)piperidin-1-yl)phenyl)amino)pyrimidin-4-yl)amino)-2,3-dihydrobenzofuran-5-yl)-*N-*methylmethanesulfonamide;
Compound No. 253: *N-*(6-((5-bromo-2-((2,5-dichloro-4-(4-(4-methylpiperazin-1-yl)piperidin-1-yl)phenyl)amino)pyrimidin-4-yl)amino)-2,3-dihydrobenzofuran-5-yl)-*N-*methylmethanesulfonamide;
Compound No. 254: *N-*(6-((5-bromo-2-((5-ethyl-2-methoxy-4-(4-(4-methylpiperazin-1-yl)piperidin-1-yl)phenyl)amino)pyrimidin-4-yl)amino)-2,3-dihydrobenzofuran-5-yl)-*N-*methylmethanesulfonamide;
Compound No. 255: *N-*(6-((5-bromo-2-((2-methoxy-5-methyl-4-(4-(4-(trifluoromethyl)piperazin-1-yl)piperidin-1-yl)phenyl)amino)pyrimidin-4-yl)amino)-2,3-dihydrobenzofuran-5-yl)-*N-*methylmethanesulfonamide;
Compound No. 256: *N-*(6-((5-bromo-2-((5-fluoro-2-methoxy-4-(4-(4-methylpiperazin-1-yl)piperidin-1-yl)phenyl)amino)pyrimidin-4-yl)amino)-2,3-dihydrobenzofuran-5-yl)-*N-*methylmethanesulfonamide;
Compound No. 257: *N-*(6-((5-bromo-2-((5-fluoro-2-methoxy-4-(4-(4-methyl-1,4-diazepan-1-yl)piperidin-1-yl)phenyl)amino)pyrimidin-4-yl)amino)-2,3-dihydrobenzofuran-5-yl)-*N-*methylmethanesulfonamide;
Compound No. 258: *N-*(6-((5-bromo-2-((4-(3-(dimethylamino)-[1,4'-bipiperidin]-1'-yl)-2-methoxy-5-methoxyphenyl)amino)pyrimidin-4-yl)amino)-2,3-dihydrobenzofuran-5-yl)-*N-*methylmethanesulfonamide;
Compound No. 259: *N-*(6-((5-chloro-2-((2,5-dichloro-4-(4-(4-methylpiperazin-1-yl)piperidin-1-yl)phenyl)amino)pyrimidin-4-yl)amino)-2,3-dihydrobenzofuran-5-yl)-*N-*methylmethanesulfonamide;
Compound No. 260: *N-*(2-((5-chloro-2-((2-methoxy-5-methyl-4-(4-(4-methylpiperazin-1-yl)piperidin-1-yl)phenyl)amino)pyrimidin-4-yl)amino)-4-methoxyphenyl)-*N-*methylmethanesulfonamide;
Compound No. 261: *N-*(6-((5-bromo-2-((4-(4-(4-cyclopropylpiperazin-1-yl)piperidin-1-yl)-2-methoxy-5-methylphenyl)amino)pyrimidin-4-yl)amino)-2,3-dihydrobenzofuran-5-yl)-*N-*methylmethanesulfonamide;
Compound No. 262: *N-*(6-((5-bromo-2-((4-(4-(3-(dimethylamino)pyrrolidin-1-yl)piperidin-1-yl)-2-methoxy-5-methylphenyl)amino)pyrimidin-4-yl)amino)-2,3-dihydrobenzofuran-5-yl)-*N-*methylmethanesulfonamide;
Compound No. 263: *N-*(6-((5-chloro-2-((2-chloro-5-methyl-4-(4-(4-methylpiperazin-1-yl)piperidin-1-yl)phenyl)amino)pyrimidin-4-yl)amino)-2,3-dihydrobenzofuran-5-yl)-*N-*methylmethanesulfonamide;
Compound No. 264: *N-*(6-((5-chloro-2-((5-chloro-2-methoxy-4-(4-(4-methyl-1,4-diazepan-1-yl)piperidin-1-yl)phenyl)amino)pyrimidin-4-yl)amino)-2,3-dihydrobenzofuran-5-yl)-*N-*methylmethanesulfonamide;
Compound No. 265: *N-*(6-((5-chloro-2-((2-methoxy-5-methyl-4-(4-(4-methyl-1,4-diazepan-1-yl)piperidin-1-yl)phenyl)amino)pyrimidin-4-yl)amino)-2,3-dihydrobenzofuran-5-yl)-*N-*methylmethanesulfonamide;
Compound No. 266: *N-*(6-((5-chloro-2-((4-(4-(4-cyclopropylpiperazin-1-yl)piperidin-1-yl)-2-methoxy-5-methylphenyl)amino)pyrimidin-4-yl)amino)-2,3-dihydrobenzofuran-5-yl)-*N-*methylmethanesulfonamide;
Compound No. 267: *N-*(6-((5-chloro-2-((4-(3-(dimethylamino)-[1,4'-bipiperidin]-1'-yl)-2-methoxy-5-methylphenyl)amino)pyrimidin-4-yl)amino)-2,3-dihydrobenzofuran-5-yl)-*N-*methylmethanesulfonamide;
Compound No. 268: *N-*(6-((5-chloro-2-((2,5-difluoro-4-(4-(4-methylpiperazin-1-yl)piperidin-1-yl)phenyl)amino)pyrimidin-4-yl)amino)-2,3-dihydrobenzofuran-5-yl)methanesulfonamide;
Compound No. 269: *N-*(5-((5-bromo-2-((2,5-difluoro-4-(4-(4-methylpiperazin-1-yl)piperidin-1-yl)phenyl)amino)pyrimidin-4-yl)amino)-2,3-dihydrobenzofuran-4-yl)methanesulfonamide;
Compound No. 270: *N-*(6-((2-((5-bromo-2-methoxy-4-(4-(4-methylpiperazin-1-yl)piperidin-1-yl)phenyl)amino)-5-chloropyrimidin-4-yl)amino)-2,3-dihydrobenzofuran-5-yl)methanesulfonamide;
Compound No. 271: *N-*(6-((2-((5-bromo-2-methoxy-4-(4-(4-methylpiperazin-1-yl)piperidin-1-yl)phenyl)amino)-5-bromopyrimidin-4-yl)amino)-2,3-dihydrobenzofuran-5-yl)methanesulfonamide;
Compound No. 272: 5-chloro-*N*²-(2-methoxy-5-methyl-4-(4-(4-methylpiperazin-1-yl)piperidin-1-yl)phenyl)-*N*⁴-(1-methylindolin-6-yl)pyrimidine-2,4-diamine;
Compound No. 273: 5-chloro-*N*²-(5-chloro-2-methoxy-4-(4-(4-methylpiperazin-1-yl)piperidin-1-yl)phenyl)-*N*⁴-(1-methylindolin-6-yl)pyrimidine-2,4-diamine;
Compound No. 274: 5-chloro-*N*²-(2-chloro-5-methyl-4-(4-(4-methylpiperazin-1-yl)piperidin-1-yl)phenyl)-*N*⁴-(1-methylindolin-6-yl)pyrimidine-2,4-diamine;
Compound No. 275: 5-chloro-*N*²-(2-methoxy-5-methyl-4-(4-(4-methyl-1,4-diazepan-1-yl)piperidin-1-yl)phenyl)-*N*⁴-(1-methylindolin-6-yl)pyrimidine-2,4-diamine;
Compound No. 276: *N-*(6-((5-chloro-2-((2-methoxy-5-methyl-4-(4-(4-methylpiperazin-1-yl)piperidin-1-yl)phenyl)amino)pyrimidin-4-yl)amino)-1-methylindolin-5-yl)methanesulfonamide;
Compound No. 277: 5-chloro-*N*²-(2-chloro-5-methyl-4-(4-(4-methylpiperazin-1-yl)piperidin-1-yl)phenyl)-*N*⁴-(indolin-6-yl)pyrimidine-2,4-diamine;
Compound No. 278: 5-chloro-*N*²-(5-chloro-2-methoxy-4-(4-(4-methylpiperazin-1-yl)piperidin-1-yl)phenyl)-*N*⁴-(indolin-6-yl)pyrimidine-2,4-diamine;
Compound No. 279: 5-chloro-*N*⁴-(indolin-6-yl)-*N*²-(2-methoxy-4-(4-(4-methylpiperazin-1-yl)piperidin-1-yl)phenyl)pyrimidine-2,4-diamine;
Compound No. 280: *N-*(6-((2-((5-bromo-4-(3-(dimethylamino)-[1,4'-bipiperidin]-1'-yl)-2-methoxyphenyl)amino)-5-chloropyrimidin-4-yl)amino)-2,3-dihydrobenzofuran-5-yl)methanesulfonamide;
Compound No. 281: *N-*(6-((5-bromo-2-((5-bromo-4-(3-(dimethylamino)-[1,4'-bipiperidin]-1'-yl)-2-methoxyphenyl)amino)pyrimidin-4-yl)amino)-2,3-dihydrobenzofuran-5-yl)methanesulfonamide;
Compound No. 282: *N-*(6-((2-((5-bromo-2-methoxy-4-(4-(4-methyl-1,4-diazepan-1-yl)piperidin-1-yl)phenyl)amino)-5-chloropyrimidin-4-yl)amino)-2,3-dihydrobenzofuran-5-yl)methanesulfonamide; and
Compound No. 283: *N-*(6-((2-((5-bromo-4-(4-(3-(dimethylamino)pyrrolidin-1-yl)piperidin-1-yl)-2-methoxyphenyl)amino)-5-chloropyrimidin-4-yl)amino)-2,3-dihydrobenzofuran-5-yl)methanesulfonamide.

10. The compound according to claim 1, wherein the pharmaceutically acceptable salt is a salt of an inorganic or inorganic acid selected from the group consisting of hydrochloric acid, hydrobromic acid, sulfuric acid, phosphoric acid, nitric acid, acetic acid, glycolic acid, lactic acid, pyruvic acid, malonic acid, succinic acid, glutaric acid, fumaric acid, malic acid, mandelic acid, tartaric acid, citric acid, ascorbic acid, palmitic acid, maleic acid, hydroxymaleic acid, benzoic acid, hydroxybenzoic acid, phenylacetic acid, cinnamic acid, salicylic acid, methanesulfonic acid, benzenesulfonic acid, and toluenesulfonic acid.

11. A pharmaceutical composition for preventing, ameliorating or treating cancer comprising the compound according to any one of claims 1 to 10 as an active ingredient.

12. The pharmaceutical composition according to claim 11, wherein the cancer is caused by an EGFR mutation.

13. The pharmaceutical composition according to claim 11, wherein the pharmaceutical composition is applied to a patient with an EGFR mutation.

14. The pharmaceutical composition according to claim 11, wherein the cancer is selected from the group consisting of glioblastoma, triple-negative breast cancer, colorectal cancer, lung cancer, anaplastic large cell lymphoma, gastrointestinal stromal tumor, multiple myeloma, acute myeloid leukemia, liver cancer, stomach cancer, thyroid cancer, head and neck cancer, and combinations thereof.

15. The pharmaceutical composition according to claim 14, wherein the cancer is lung cancer.

16. The pharmaceutical composition according to claim 11, wherein the pharmaceutical composition is administered to a patient with exon 19 deletion/T790M/C797S triple mutation or L858R/T790M/C797S triple mutation as an EGFR-related mutation.

17. The pharmaceutical composition according to claim 11, wherein the pharmaceutical composition is administered to a patient with exon 19 deletion/T790M double mutation or L858R/T790M double mutation as an EGFR-related mutation.

18. The pharmaceutical composition according to claim 11, wherein the pharmaceutical composition is administered to a patient with an exon 19 deletion mutation or L858R mutation as an EGFR-related mutation.
